(19) Europäisches Patentamt / European Patent Office / Office européen des brevets

(11) EP 1 689 789 B1

(12) **FASCICULE DE BREVET EUROPEEN**

(45) Date de publication et mention de la délivrance du brevet:
**11.11.2009 Bulletin 2009/46**

(21) Numéro de dépôt: **04805527.1**

(22) Date de dépôt: **24.11.2004**

(51) Int Cl.:
***C08B 37/16*** *(2006.01)* ***A61K 47/40*** *(2006.01)*

(86) Numéro de dépôt international:
**PCT/FR2004/002998**

(87) Numéro de publication internationale:
**WO 2005/054303 (16.06.2005 Gazette 2005/24)**

# (54) DIMERES DE CYCLODEXTRINES ET LEURS DERIVES, LEURS PROCEDES DE PREPARATION ET LEUR UTILISATION NOTAMMENT POUR LA SOLUBILISATION DE SUBSTANCES PHARMACOLOGIQUEMENT ACTIVES

NEUE CYCLODEXTRINDIMERE UND DERIVATE DAVON, HERSTELLUNGSVERFAHREN DAFÜR UND VERWENDUNG DAVON, INSBESONDERE ZUR SOLUBILISIERUNG VON PHARMAKOLOGISCHEN WIRKSTOFFEN

NOVEL CYCLODEXTRIN DIMERS AND DERIVATIVES THEREOF, METHODS FOR PREPARING THEM AND THEIR USE, IN PARTICULAR, FOR SOLUBILIZING PHARMACOLOGICALLY ACTIVE SUBSTANCES

(84) Etats contractants désignés:
**AT BE BG CH CY CZ DE DK EE ES FI FR GB GR HU IE IS IT LI LU MC NL PL PT RO SE SI SK TR**

(30) Priorité: **26.11.2003 FR 0313873**

(43) Date de publication de la demande:
**16.08.2006 Bulletin 2006/33**

(73) Titulaires:
- **CENTRE NATIONAL DE LA RECHERCHE SCIENTIFIQUE (CNRS)**
  **75794 Paris Cedex 16 (FR)**
- **UNIVERSITE JOSEPH FOURIER**
  **F-34000 Saint-Martin D'Heres (FR)**
- **CONSEJO SUPERIOR INVESTIGACIONES CIENTIFICAS (CSIC)**
  **28006 Madrid (ES)**
- **UNIVERSIDAD DE SEVILLA**
  **41013 Sevilla (ES)**

(72) Inventeurs:
- **DEFAYE, Jacques**
  **F-38330 Saint Ismier (FR)**
- **ORTIZ-MELLET, Carmen**
  **E-41011 Seville (ES)**
- **GARCIA-FERNANDEZ, José Manuel**
  **E-41011 Seville (ES)**
- **BENITO, Juan, M.**
  **E-41110 Bollulos M. (ES)**
- **GOMEZ-GARCIA, Marta**
  **E-41009 Seville (ES)**
- **YU, Jian-Xin**
  **Dallas, TX 75205-2292 (US)**

(74) Mandataire: **Grosset-Fournier, Chantal Catherine**
**Grosset-Fournier & Demachy**
**54, Rue Saint-Lazare**
**75009 Paris (FR)**

(56) Documents cités:
**EP-A- 0 639 380** **WO-A-01/51524**
**WO-A-90/02141** **WO-A-95/21870**
**WO-A-97/33919** **WO-A-03/052060**
**US-A1- 2002 094 950**

- **BAUSSANNE I. ET AL.: "Synthesis and comparative lectin-binding affinity of mannosyl-coated beta-cyclodextrin-dendrimer constructs" CHEM. COMMUN., 2000, pages 1489-1490, XP008032689 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 689 789 B1

## Description

La présente invention concerne de nouveaux dérivés dimères de cyclodextrines, ainsi que leurs procédés de préparation. La présente invention concerne également l'utilisation de ces nouveaux dérivés pour la solubilisation de substances pharmacologiquement actives dans un milieu aqueux.

Les cyclodextrines, ou cyclomaltooligosaccharides, sont des oligosaccharides cycliques qui sont connus pour leur aptitude à inclure dans leur cavité des molécules diverses, de taille adaptée à celle de la structure hôte. Le caractère généralement apolaire de ces associations conduit à inclure préférentiellement des structures moléculaires de type hydrophobe, permettant notamment la solubilisation dans l'eau et les milieux biologiques de composés peu ou pas solubles dans ces milieux et éventuellement d'améliorer leur stabilisation. Ces propriétés sont actuellement utilisées en particulier pour le transport de médicaments.

La solubilité relativement faible dans l'eau des cyclodextrines, et notamment de la plus accessible d'entre elles sur le plan économique, la β-cyclodextrine (18 g/l, soit 15 mmol/l, à 25°C) limite cependant leur utilisation dans ce but. D'un autre côté, les cyclodextrines ne possédant pas de capacité de reconnaissance vis-à-vis de récepteurs biologiques dans l'organisme, ces entités ne peuvent pas être utilisées pour le ciblage et la vectorisation de principes actifs.

Pour remédier à cet état de fait, les cyclodextrines ont été modifiées chimiquement pour améliorer leur solubilité dans l'eau d'une part et, d'autre part, pour incorporer dans leur structure des signaux de reconnaissance cellulaire. Ainsi, les demandes internationales WO 95/19994, WO 95/21870 et WO 97/33919 et la demande de brevet européen EP 0 403 366 décrivent des dérivés de cyclodextrines dont une ou plusieurs fonctions alcool primaire sont substituées par des groupes monosaccharidiques ou oligosaccharidiques via un atome d'oxygène ou de soufre ou bien via un groupement thiourée, ainsi que leur utilisation. Ces cyclodextrines ramifiées sont en particulier susceptibles de servir d'hôte pour le taxol et ses dérivés, en particulier le Taxotère®, qui sont des agents antitumoraux et antiparasitaires, comme il est décrit par P. Potier dans Chem. Soc. Rev., 21, 1992, pp. 113-119. On obtient ainsi des complexes d'inclusion, ce qui permet de solubiliser dans l'eau ces agents antitumoraux. A titre d'exemple, la solubilité dans l'eau du Taxotère® qui est 0,004 g/L, peut être augmentée jusqu'à 6,5 g/L par addition de $6^{I}$-*S*-α-maltosyl-$6^{I}$-thiocyclomaltoheptaose à sa suspension aqueuse, comme cela est décrit dans le document WO 95/19994.

Le document EP-A-0 605 753 décrit des complexes d'inclusion du taxol utilisant des cyclodextrines ramifiées telles que les maltosyl-cyclodextrines, pour augmenter la solubilité de ce diterpène dans l'eau.

Des dérivés de cyclodextrines comportant un ou plusieurs substituants glycosyle ou maltosyle liés à la cyclodextrine par un atome de soufre sont également décrits par V. Lainé et al. dans J. Chem. Soc., Perkin Trans., 2, 1995, pp. 1479-1487. Ces dérivés ont été utilisés pour solubiliser un agent antiinflammatoire tel que la prédnisolone.

Le document WO 97/33919 décrit les procédés de préparation des thiouréido-cyclodextrines par couplage des $6^{I}$-amino-$6^{I}$-désoxycyclodextrines ou encore des dérivés peraminés correspondants avec des isothiocyanates d'alkyle ou des mono- ou oligosaccharides.

L'incorporation de substituants glucidiques sur les cyclodextrines conduit à des dérivés dotés d'une solubilité dans l'eau beaucoup plus élevée si on la compare à la cyclodextrine de départ. En même temps, cette approche permet de conférer à la cyclodextrine une affinité particulière pour certains sites biologiques, car les substituants glucidiques sont bien connus comme marqueurs de reconnaissance cellulaire. Ainsi, ce type de modification de la cyclodextrine peut permettre le ciblage et la vectorisation d'une substance active incluse dans la cavité de la cyclodextrine.

L'affinité d'un marqueur glucidique pour un récepteur spécifique de membrane cellulaire (lectine) est en règle générale faible. Pour atteindre des affinités utiles pour le ciblage et la vectorisation, il faut envisager une présentation multiple et simultanée du ligand. Dans le cas de dérivés de cyclodextrines monosubstitués en position alcool primaire (c'est-à-dire des cyclodextrines dans lesquelles seul l'un des groupes OH de la couronne des alcools primaires est substitué), ce problème peut être partiellement résolu par l'incorporation de structures glycodendritiques, comme décrit par I. Baussanne et al. dans Chem. Commun, 2000, pp. 1489-1490 et par J. M. Benito et al dans Chem. Eur. J., 2003, sous presse. Cependant, le procédé de préparation de tels composés est compliqué.

Par ailleurs, les résultats récents décrits par I. Baussanne et al. dans ChemBioChem 2001, pp. 777-783 montrent que les dérivés de la β-cyclodextrine comportant des substituants de type glycosylthiouréido, obtenus à partir de la per-(C-6)-amine correspondante, ne montrent pas une affinité suffisante vis-à-vis des lectines complémentaires. Néanmoins, la capacité de solubilisation de ces dérivés vis a vis d'agents antitumoraux de la famille du paclitaxel, tel que le docetaxel (Taxotère®) reste plus faible que celle observée dans le cas des dérivés des cyclodextrines monosubstitués en position alcool primaire.

Dans tous les exemples commentés de dérivés de cyclodextrines mono- ou polysubstitués, on observe que la proportion molaire de cyclodextrine : agent antitumoral solubilisé dans l'eau est significativement inférieure à 1:1. En fait, la présence de deux cycles aromatiques dans les dérivés du Taxol et du Taxotère®, susceptibles d'inclusion dans la cavité hydrophobe de la cyclodextrine, permet de supposer un caractère ditopique pour ces molécules et, en conséquence, une tendance à la formation de complexes de type sandwich avec les dérivés de cyclodextrines.

Le document D1 (WO 90/02141 A) est considéré comme représentant l'état de la technique le plus proche des

revendications 1-20 et décrit des cyclodextrines liées entre elles par l'atome de carbone en position 6 d'une de leurs unités glucose (figures 7B, 8A-D). Ces cyclodextrines liées sont utiles pour former des complexes avec des molécules de principe actif possédant deux sites apolaires ou hydrophobes. Le complexe ainsi constitué est plus soluble et plus stable que celui formé à partir d'une cyclodextrine non liée (pages 18-21, paragraphe C. "Linked Cyclodextrins"). Les deux cyclodextrines peuvent être liées par n'importe quelle type de liaison covalente; elle sont de préférence liées entre elles par un groupe formant deux liaisons de type amine, amide, ether et/ou ester. La longueur du groupe de liaison peut être variable afin de s'adapter à la taille des molécules de principe actif à complexer. Un des procédés de préparation des ces cyclodextrines liées consiste à faire réagir des 6A-amino-6A-désoxy-béta-cyclodextrines avec un diester m-nitrophényle d'acide succinique. Un deuxième procédé consiste à faire réagir des dérivés tosylés de cyclodextrines (6A-O-toluènesulphonyle cyclodextrines) avec un sel d'acide dicarboxylique (acide succinique, glutarique, téréphtalique).

A ce jour, il n'existe aucun dérivé de cyclodextrines obtenu par un procédé simple, permettant d'augmenter la solubilisation de substances ditopiques pharmacologiquement actives et présentant également une affinité suffisante vis-à-vis des récepteurs biologiques complémentaires.

Un des buts de la présente invention est de fournir de nouveaux dérivés de cyclodextrines dimères comportant deux sous-unités de cyclodextrine liées en position alcool primaire, présentant non seulement un intérêt pour la solubilisation des substances actives, en particulier des antitumoraux de la famille du taxol comme le Taxotère®, mais également une forte affinité vis-à-vis de récepteurs membranaires spécifiques, ce qui permet d'envisager par leur intermédiaire un transport efficace et sélectif de la substance ditopique active vers des organes cibles.

Un autre objectif de l'invention est de fournir un procédé de préparation simple à mettre en oeuvre, et permettant d'obtenir de nouveaux dérivés de cyclodextrines dimères avec un bon rendement, de l'ordre d'au moins 50%, et de préférence 70%, sans avoir à effectuer de purifications longues et compliquées.

La présente invention concerne un composé répondant à la formule générale suivante :

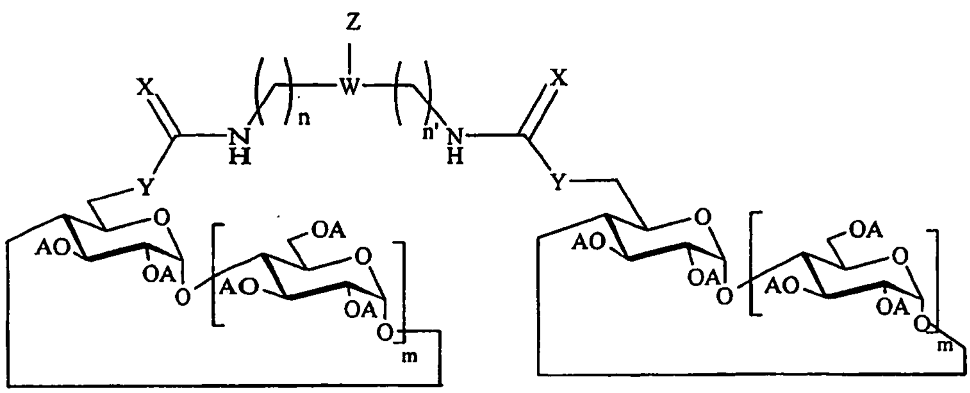

(I)

dans laquelle :

- m représente un nombre entier égal à 5, 6 ou 7;
- n et n' représentent un nombre entier compris de 1 à 5, n et n' pouvant être identiques ou différents ;
- les groupes A, identiques ou différents, représentent un atome d'hydrogène, un groupe acyle, alkyle, hydroxyalkyle ou sulfoalkyle de 1 à 16 atomes de carbone,
- X représente O ou S,
- Y représente un substituant avec une fonction aminée qui fait partie d'une fonction urée (cas où X = O) ou d'une fonction thiourée (cas où X = S), telle que:

    * un groupement $-NR_1-$, $R_1$ représentant un atome d'hydrogène ou un groupe alkyle comprenant de 1 à 6 atomes de carbone, ou
    * un groupement amide de formule $-NH-CO-(CH_2)_q-NR_1-$, q représentant un nombre entier compris de 1 à 5 et $R_1$ étant tel que défini ci-dessus, ou
    * un groupement cystéaminyle de formule $-S-(CH_2)_r-NR_1-$, r représentant un nombre entier compris de 2 à 5 et $R_1$ étant tel que défini ci-dessus,

- W représente CH ou N ;
- Z représente :

* un atome d'hydrogène ou

* un substituant carbamate de formule

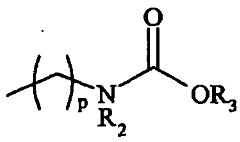

ou

* un substituant aminé de formule

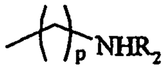

ou

* un groupe ammonium quaternaire de formule

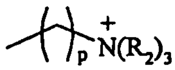

ou

* un substituant urée ou thiourée de formule

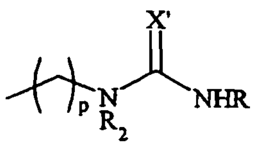

ou

* un groupe de formule

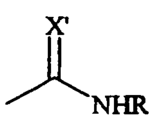

ou

* un groupe de la forme $C(=O)OR_3$, un groupe de la forme $C(=O)R_2$ ou un groupe portant les fonctionnalités amine, ammonium quaternaire urée ou thiourée, de formules respectives

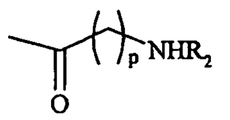 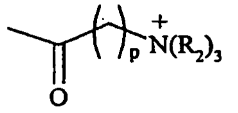 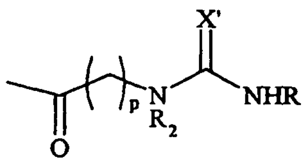

p représentant un nombre entier compris de 0 à 5, lorsque W représente CH, et de 2 à 5, lorsque W représente N,
X' représentant O ou S,
$R_2$ représentant un atome d'hydrogène ou un groupe alkyle comprenant de 1 à 6 atomes de carbone, et étant notamment un groupe méthyle, éthyle, propyle ou butyle,
$R_3$ représentant un substituant permettant l'hydrolyse du groupement carbamate afin de libérer la fonction amine, tel que les groupements *tert*-butyle, 9-fluorénylméthyle, benzyle, allyle ou 2,2,2-trichloroéthyle, et
R représentant un atome d'hydrogène, un groupe alkyle, linéaire ou ramifié, de 1 à 12 atomes de carbone, ou un groupe aromatique tel que le groupe phényle, benzyle ou naphtyle, ou des dérivés de ces groupements portant des substituants sur le cycle aromatique tels que les substituants méthyle, éthyle, chlore, brome, iode, nitro, hydroxyle, méthoxyle ou acétamido, ou
R représentant un élément de reconnaissance biologique tel qu'un dérivé d'acide aminé, un peptide, un monosaccharide, un oligosaccharide, un élément de multiplication à plusieurs ramifications, lesquelles ramifications comportent des groupements glucidiques qui peuvent être identiques ou différents, ou encore une sonde de visualisation ou de détection fluorescente ou radioactive.

L'expression "élément de reconnaissance biologique" désigne une structure moléculaire complémentaire d'un récepteur biologique, susceptible d'être reconnue par ce dernier et de conduire à une réponse spécifique : induction et régulation de la biosynthèse d'une enzyme, inhibition de l'activité d'une enzyme par fixation sur son site actif, induction d'une réponse immunitaire suite à une affection bactérienne, inhibition d'un processus inflammatoire par blocage du site actif d'une sélectine, etc.

L'expression "élément de multiplication à plusieurs ramifications" désigne notamment une chaîne carbonée ramifiée comprenant soit un carbone quaternaire tétrasubstitué comme les dérivés du tris(2-aminométhyl)méthylamine (TRIS) et du pentaérythritol, soit un atome d'azote trisubstitué comme le tris(2-aminoéthyl)amine (TREN). Ces éléments de multiplication peuvent aussi être incorporés en combinaison avec un deuxième élément de ramification comprenant, notamment, un atome d'azote tertiaire comme les dérivés du tris(2-aminoéthyl)amine (TREN).

L'expression "sonde de visualisation ou de détection fluorescente ou radioactive" désigne une structure moléculaire permettant la détection d'un système par une technique physicochimique, telle que la fluorescence ou la radioactivité. Parmi les sondes fluorescentes, on peut notamment citer les dérivés de la fluorescéine, du dansyle (5-(diméthylamino)-1-naphtalènesulfonyle) ou de la coumarine. Parmi les sondes radioactives, on peut citer les produits marqués par un isotope radioactif.

La formule (I) susmentionnée concerne à la fois les dérivés de cyclodextrines dimères aussi bien symétriques que non symétriques. Les composés symétriques correspondent à la formule (I) dans laquelle n et n' sont égaux et les composés non symétriques correspondent à la formule (I) dans laquelle n et n' sont différents.

Dans ces nouveaux dérivés, on a constaté que le caractère dimère est intéressant, notamment pour augmenter l'efficacité de complexation et de solubilisation dans l'eau de molécules ditopiques, en particulier les dérivés du paclitaxel, du docétaxel et des stéroïdes.

L'expression "élément de dimérisation" désigne un dérivé possédant deux groupement susceptibles de subir une réaction de couplage avec des fonctionnalités de type amine situées sur le précurseur de cyclodextrine, notamment deux groupements isocyanate ou isothiocyanate. Un aspect intéressant de la présente invention est que l'élément de dimérisation peut être, en même temps, un élément de multiplication tel que défini ci-dessus comprenant, notamment, un atome de carbone ou d'azote trisubstitué. Deux des substituants sur cet atome trisubstitué portent les groupements réactifs isocyanate ou isothiocyanate, le troisième substituant pouvant être de nature variable et très diverse. A titre d'exemple d'élément de dimérisation avec un atome de carbone trisubstitué, on peut mentionner les dérivés du 1,2,3-propanetriamine et, plus généralement, des alkanetriamines, dont deux des groupes amine sont transformés en isocyanate ou isothiocyanate. A titre d'exemple d'élément de dimérisation avec un atome d'azote trisubstitué, on peut mentionner les dérivés du tris(2-aminoethyl)amine (TREN), du bis(5-aminopentyl)amine et, plus généralement, des tris et bis(ω-aminoalkane)amines, dont deux des groupes amines sont transformés en isocyanate ou isothiocyanate. La réaction de couplage de l'élément de dimérisation avec l'un des précurseurs de cyclodextrine susmentionnés conduit à un dérivé de cyclodextrine dimère répondant à la formule générale (I) dans laquelle W représente CH, lorsque l'atome trisubstitué dans l'élément de dimérisation est un atome de carbone, et dans laquelle W représente N lorsqu'il s'agit d'un atome d'azote.

Un aspect intéressant de la présente invention est que l'élément de dimérisation peut porter un troisième substituant pouvant générer un groupement réactif dans une étape ultérieure à la réaction de dimérisation, notamment un groupement carbamate qui peut être hydrolysé pour générer un groupement amine libre. Eventuellement, le groupement amine peut porter un substituant alkyle tel qu'un groupe méthyle, éthyle, propyle ou butyle. Ce groupement amine permet d'associer la cyclodextrine dimère à un motif hydrophile et de reconnaissance cellulaire tel qu'un dérivé glucidique, ou encore un acide aminé ou un peptide, par des liaisons de type amide, urée et thiourée qui sont très stables et donnent lieu à des structures bien définies. La liaison urée ou thiourée est créée dans une dernière étape et permet de coupler la cyclodextrine à de nombreux substituants, en particulier des substituants comportant un élément de multiplication à plusieurs ramifications, lesdites ramifications portant divers motifs glucidiques ou encore une sonde de visualisation ou de détection fluorescente ou radioactive.

Les uréido-, thiouréido- uréidocystéaminyl- et thiouréidocystéaminyl-cyclodextrines dimères répondant à la formule (I) donnée ci-dessus dans laquelle W représente CH ou N et Z représente un substituant portant une fonctionnalité amine du type $NHR_1$ ($R_1$ = H ou substituant alkyle), et dans tous les cas où Z représente N, peuvent être isolées sous forme de sel d'ammonium ou de base libre. Elles sont toujours isolées sous forme de sel d'ammonium lorsque Z comprend un groupement ammonium quaternaire, chargé positivement, du type $^+N(R_2)_3$ ($R_2$ = substituant alkyle). Dans le cas du sel, le contre-ion est un anion monovalent, en particulier un halogénure tel que le chlorure, le bromure ou l'iodure. Les composés de formule (I), dans laquelle W représente CH ou N, et Z représente un substituant portant un groupement amine du type $NHR_1$, ou bien W représente N et Z représente H, peuvent être utilisés comme précurseurs pour l'incorporation de nouveaux substituants sur la cyclodextrine dimère, notamment par formation d'une nouvelle liaison urée ou thiourée. Lorsque Z dans la formule (I) porte un groupement $NH_2$, les urées et thiourées obtenues sont *N,N'*-disubstituées, alors que, lorsque Z porte un groupement $NHR_2$, $R_2$ représentant un substituant alkyle, tel que méthyle, éthyle, propyle ou butyle, ou bien lorsque W = N et Z = H, les urées et thiourées obtenues sont *N,N',N''*-trisubstituées.

Dans le cas des uréido-, thiouréido-, uréidocystéaminyl- et thiouréidocystéaminyl-cyclodextrines dimères de formule (I), dans laquelle Z représente un substituant comportant un groupe thiourée du type $NR_2C(=X')NHR$, les substituants R peuvent être de divers types. Ainsi, R peut représenter un atome d'hydrogène, un substituant alkyle de 1 à 12 atomes de carbone linéaire ou ramifié, ou un groupe aromatique tel que phényle, benzyle, naphtyle ou des dérivés de ces groupements portant des substituants sur le cycle aromatique, lesdits substituants étant tels que définis précédemment. R peut représenter aussi, en particulier, des groupes dérivés d'acides aminés, de peptides, de monosaccharides ou d'oligosaccharides éventuellement substitués. A titre d'exemple de groupes dérivés de monosaccharides, on peut citer ceux dérivés du glucose, du mannose et du galactose, en configuration anomérique $\alpha$ ou $\beta$**.** Dans le cas où le groupe dérivé du monosaccharide est substitué, un ou plusieurs des groupes hydroxyle du monosaccharide peuvent être remplacés par des groupes alcoxy de 1 à 16 atomes de carbone, des groupes acyloxy comme le groupe acétoxy, des groupes amine et amide. Les groupes dérivés d'oligosaccharides peuvent être les groupes maltosyle, maltotriosyle, lactosyle, ou encore des tri- ou tétrasaccharides marqueurs d'affinité cellulaire du type Lewis X ou sialyl Lewis X, ou encore des oligosaccharides dérivés de l'héparine. Ils peuvent également être substitués par des groupes alcoxy, acyloxy, des groupements aminés, sulfatés ou phosphatés.

Selon l'invention, R peut également représenter un groupe comportant un élément de multiplication ramifié, par exemple un groupe dérivé du tris(2-hydroxyméthyl)méthylamine (TRIS), du pentaérythritol ou du tris(2-aminoéthyl) amine (TREN), comportant dans les ramifications des groupes dérivés de mono- ou d'oligosaccharides qui peuvent être identiques ou différents. A titre d'exemples, on peut citer les groupes dérivés de mono- ou oligosaccharides déjà cités précédemment, qui peuvent également comporter des substituants oxygénés ou aminés. Ces groupes glucidiques peuvent être liés à l'élément de multiplication par une liaison oxygénée, soufrée ou aminée. Dans le cas où R comprend un élément de ramification, une des ramifications peut aussi porter une sonde de type fluorescent, en particulier un dérivé de la fluorescéine ou encore une sonde radioactive. Ces éléments de multiplication peuvent aussi être incorporés en combinaison avec un deuxième élément de ramification comprenant, notamment, un atome d'azote tertiaire comme les dérivés du tris(2-aminoéthyl)amine (TREN).

Un composé avantageux selon la présente invention est un composé tel que défini ci-dessus, **caractérisé en ce que** n et n' sont égaux.

Un tel composé est un composé symétrique et est donc plus commodément accessible et plus facilement caractérisable par les méthodes physico-chimiques classiques qu'un composé non symétrique.

Un tel composé répond à la formule (A) suivante :

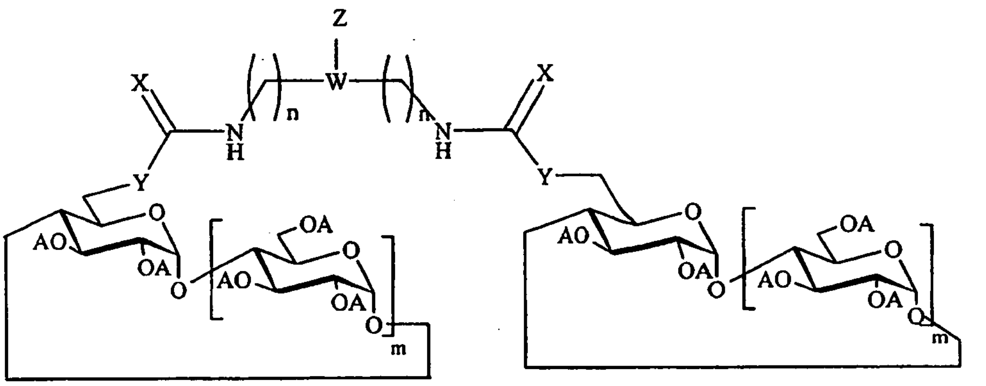

(A)

dans laquelle m, n, A, X, Y, W et Z sont tels que définis ci-dessus.

Un composé avantageux selon la présente invention est un composé tel que défini ci-dessus, **caractérisé en ce que** tous les groupes A représentent un atome d'hydrogène.

Un tel composé répond à la formule (B) suivante :

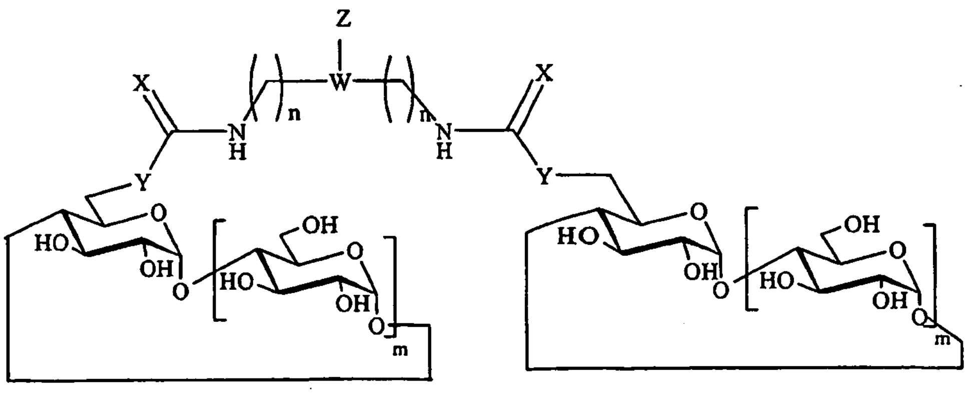

(B)

dans laquelle m, n, X, Y, W et Z sont tels que définis ci-dessus.

La présente invention concerne également un composé tel que défini ci-dessus, **caractérisé en ce que** Y représente soit un groupe $NR_1$, soit un groupe $-NH-CO-(CH_2)_q-NR_1-$, soit un groupe $-S-(CH_2)_r-NR_1-$, et répondant respectivement à l'une des formules suivantes :

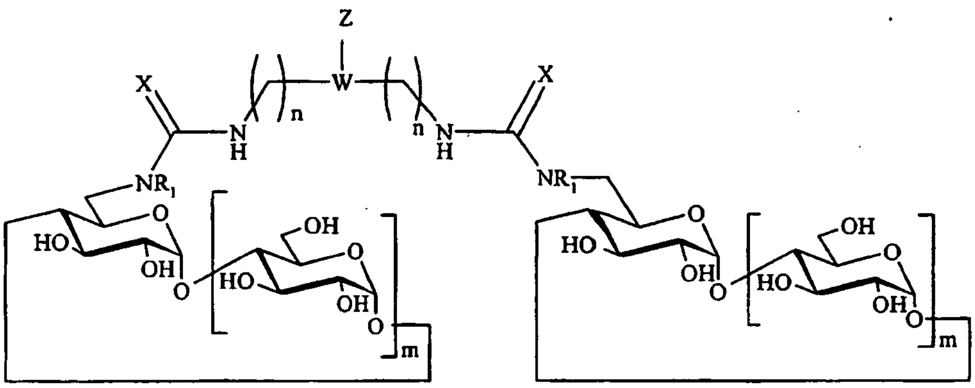

(I-a)

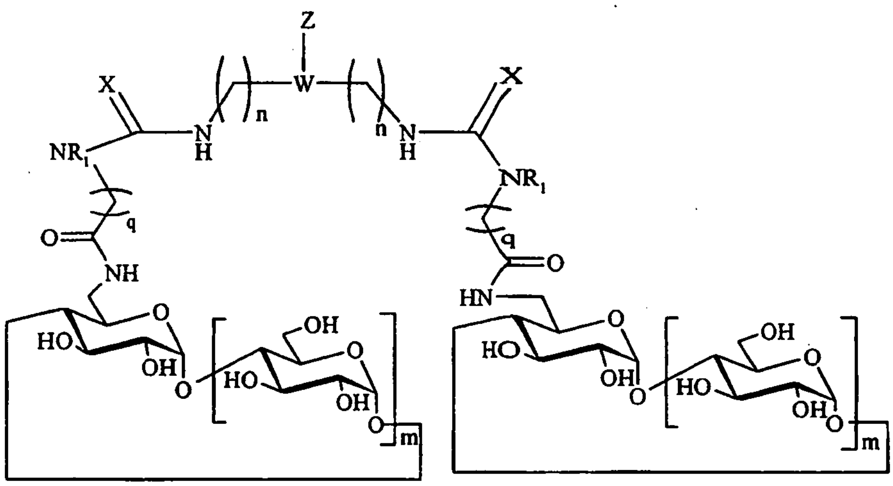

(I-b)

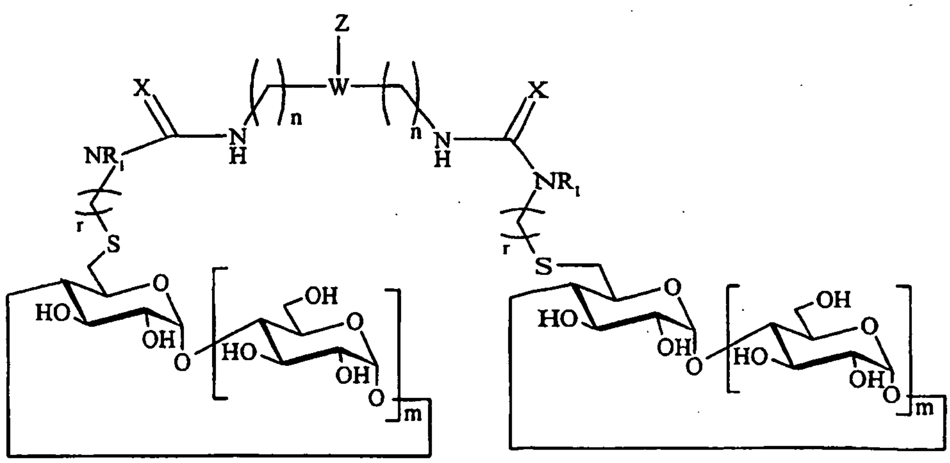

(I-c)

dans lesquelles n, m, q, r, X, W, Z et $R_1$ sont tels que définis ci-dessus.

Les composés susmentionnés sont des composés monosubstitués sur chaque cycle de la cyclodextrine. Dans ce type de composés, l'accès à la cavité de la cyclodextrine est moins encombré que dans le cas de dérivés polysubstitués. En plus, le caractère dimère peut apporter de meilleures capacités de complexation pour certaines molécules invitées ditopiques.

Les composés de formule (I-a) et (I-b) sont des composés dimères dérivés de la cyclodextrine, nommés uréido- et thiouréido-cyclodextrines dimères. Les composés de formule (I-c) sont des composés dimères dérivés de la cyclodextrine, nommés uréidocystéaminyl- et thiouréidocyctéaminyl-cyclodextrines dimères.

Un composé avantageux selon la présente invention est **caractérisé en ce que** Z représente soit un groupe $-(CH_2)_p-NHR_2$, soit un groupe $-(CH_2)_p-N^+(R_2)_3$, soit un groupe de formule

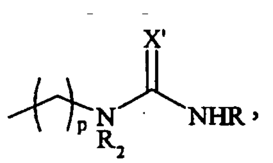

dans laquelle X' représente un atome de soufre, et répondant respectivement à l'une des formules suivantes :

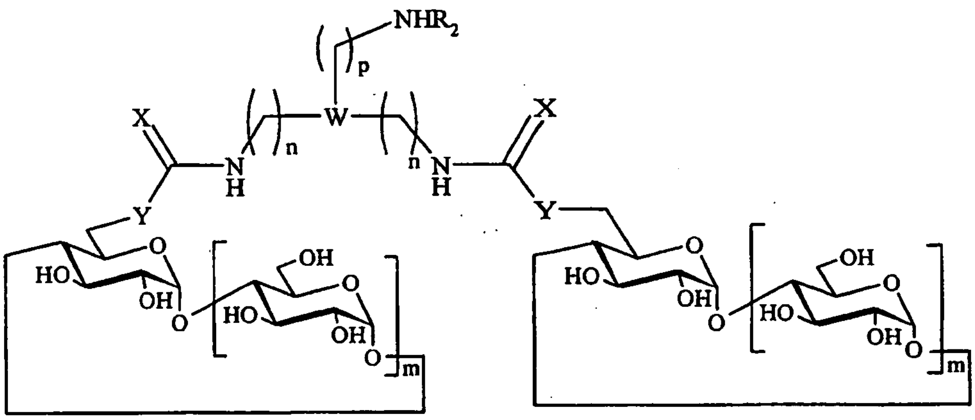

(I-d)

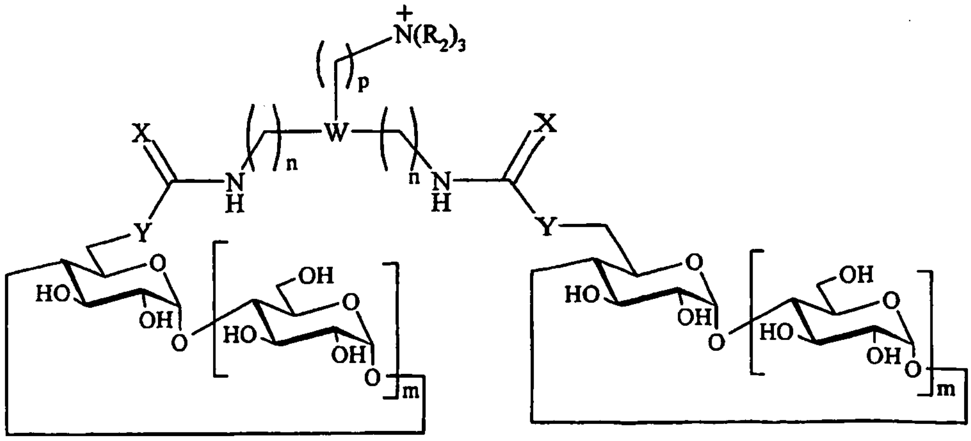

(I-e)

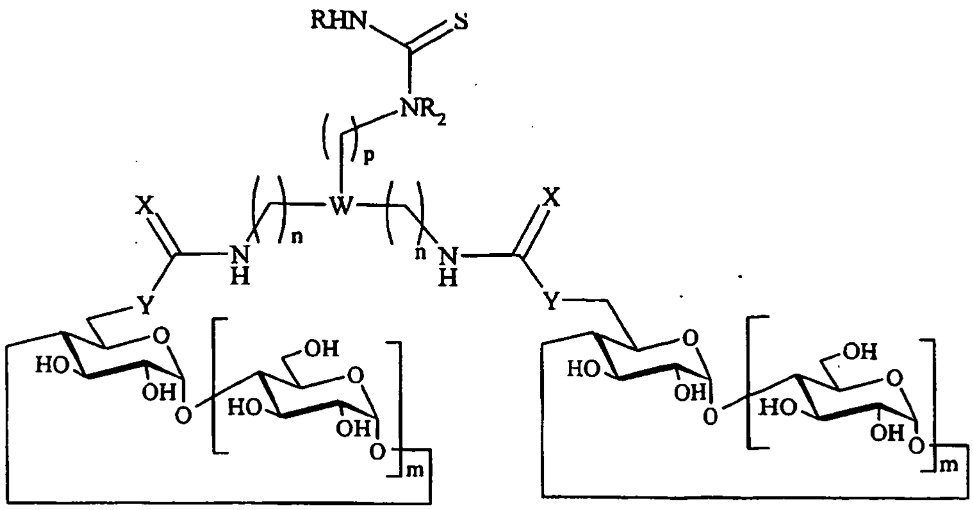

(I-f)

dans lesquelles n, m, p, X, W, Y, R et $R_2$ sont tels que définis ci-dessus.

Ces composés sont particulièrement intéressants dans la mesure où ils donnent accès à une gamme de produits avec des propriétés physico-chimiques modulables en ce qui concerne notamment la solubilité, le caractère neutre ou chargé, intéressant pour les interactions membranaires, ou encore les propriétés de reconnaissance vis-à-vis de récepteurs biologiques.

Un composé avantageux selon la présente invention est un composé tel que défini ci-dessus, **caractérisé en ce que** W représente un atome d'azote et en ce que Z représente soit un groupe de formule $-CO-(CH_2)_p-NHR_2$, soit un

groupe de formule + -CO-$(CH_2)$p-$N(R_2)_3$, soit un groupe de formule

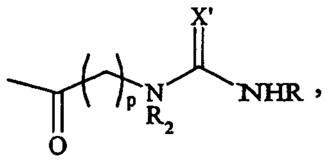

,

dans laquelle X' représente un atome de soufre, soit un groupe de formule

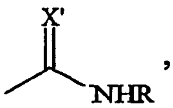

,

dans laquelle X' représente un atome de soufre et répondant respectivement à l'une des formules suivantes :

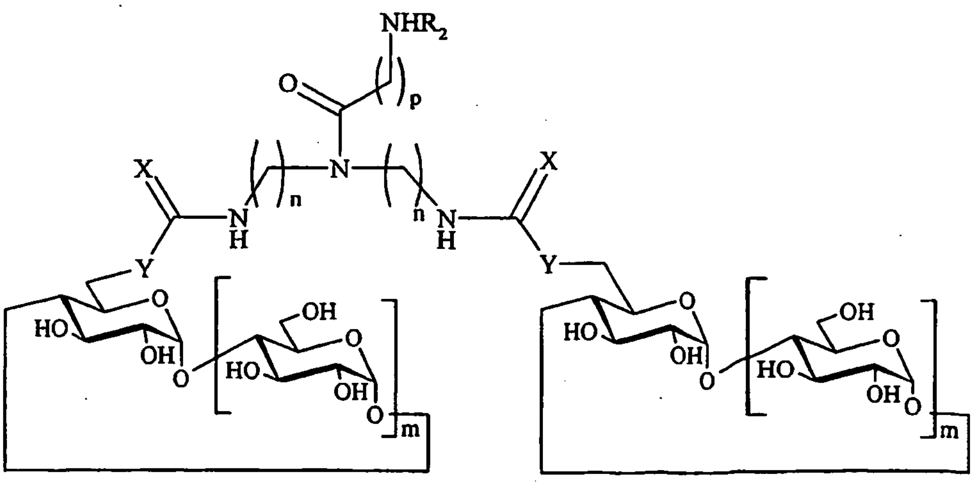

(I-g)

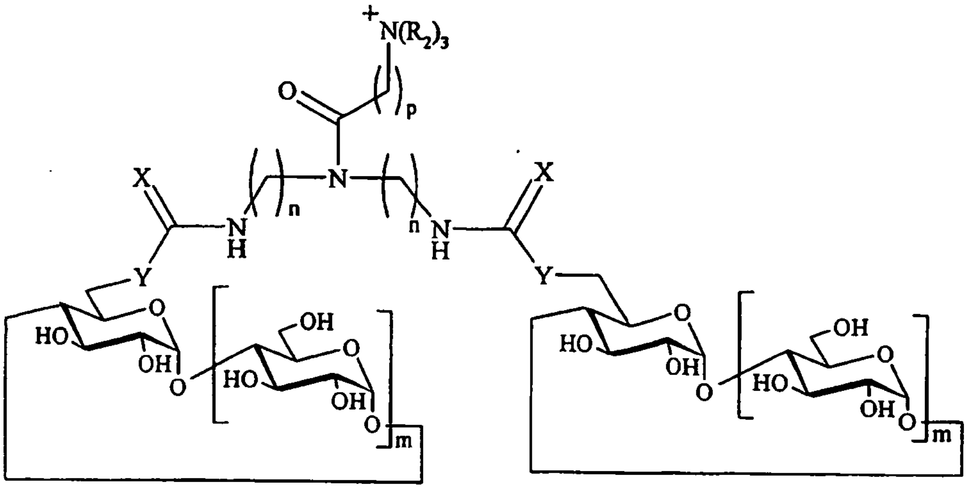

(I-h)

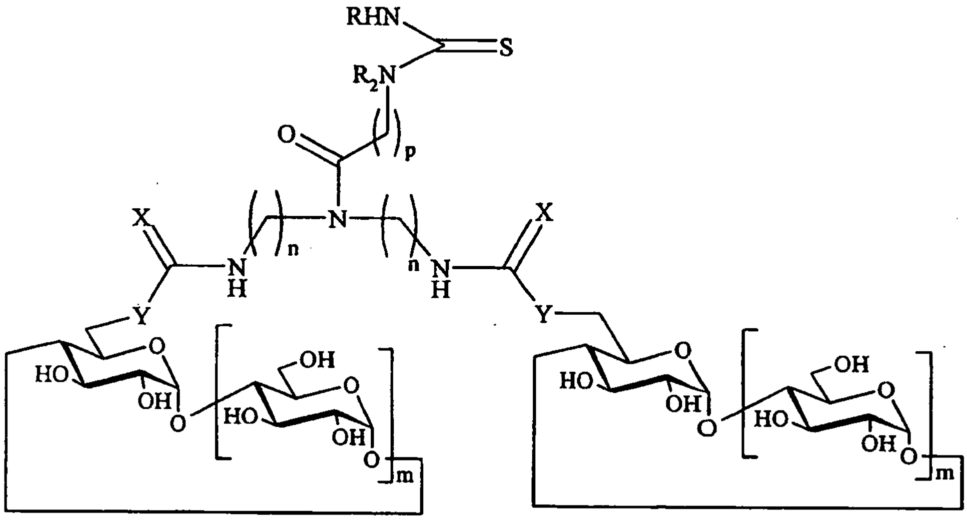

(I-i)

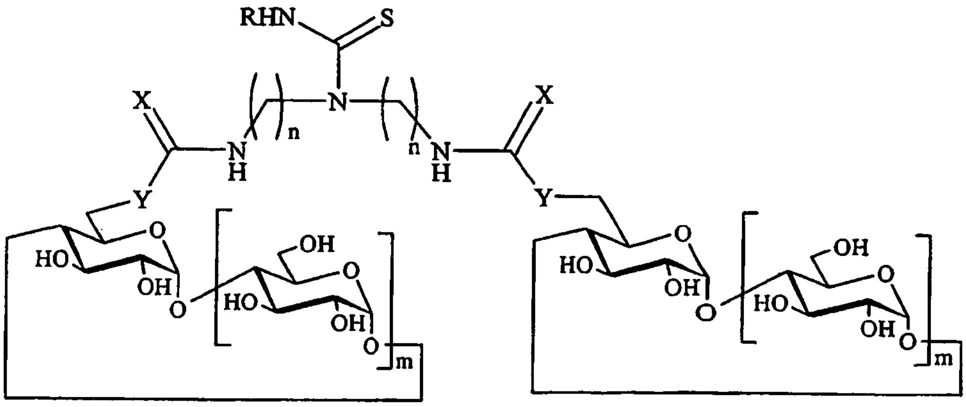

(I-j)

dans lesquelles n, m, p, X, Y, R et $R_2$ sont tels que définis ci-dessus.

Un composé préféré selon la présente invention est un composé tel que défini ci-dessus, de formule (I), **caractérisé en ce que** n est égal à 2, X représente un atome de soufre, W représente un atome d'azote et Y représente un groupe NH.

Un tel composé répond à la formule (C) suivante :

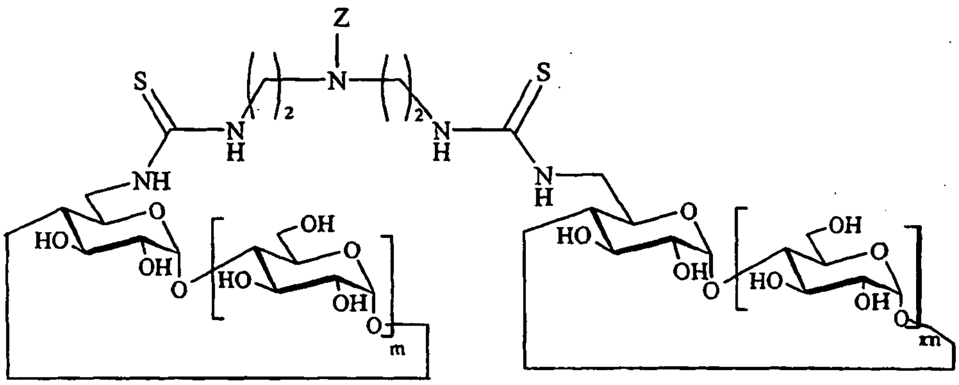

(C)

dans laquelle m et Z sont tels que définis ci-dessus.

Un tel composé présente un caractère non chargé et est commodément accessible en peu d'étapes à partir

d'une grande variété d'unités de base commerciales.

Un composé préféré selon la présente invention est un composé tel que défini ci-dessus, de formule (C), **caractérisé en ce que** Z représente l'un des groupements suivants : $-(CH_2)_2-NH_2$, $-(CH_2)_2-NH-CS-NHR$ ou $-(CH_2)_2-NH-Boc$ (Boc = *tert*-butoxycarbonyl), R étant tel que défini ci-dessus, et répondant à l'une des formules suivantes :

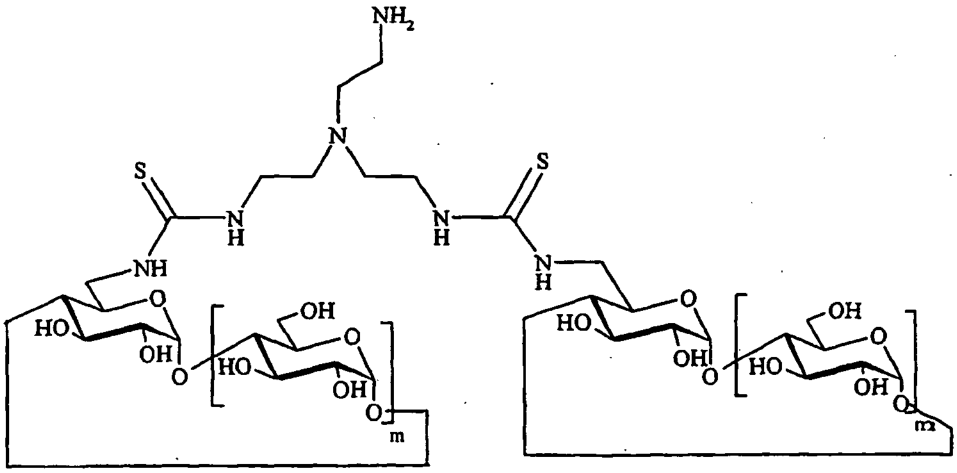

(D)

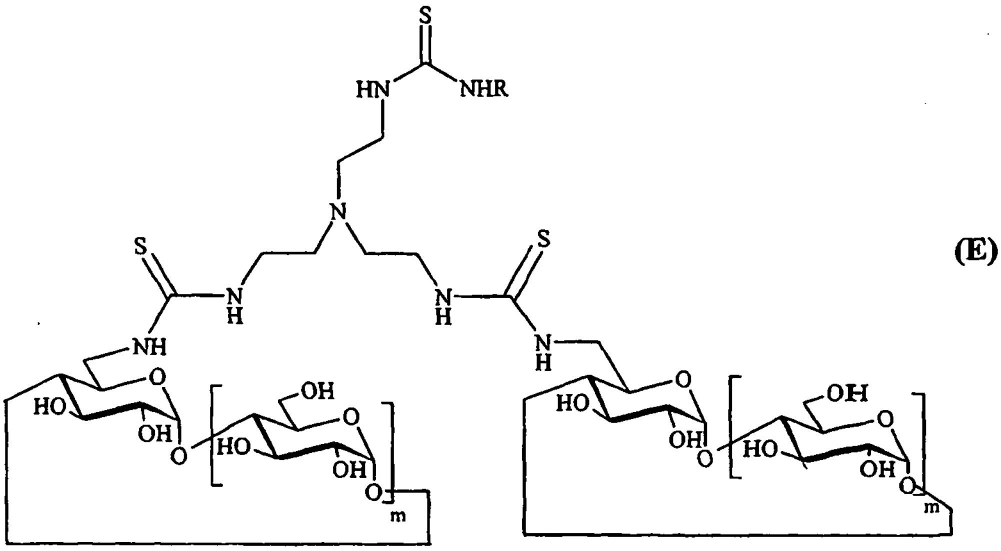

(E)

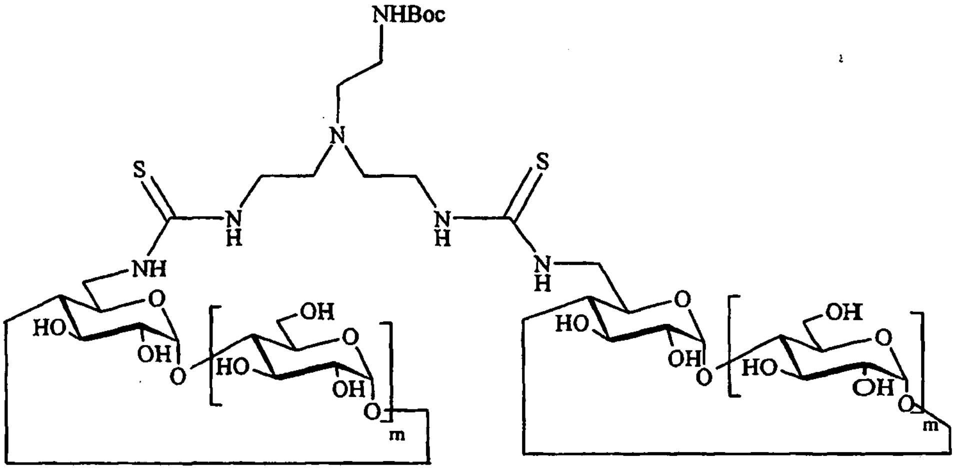

(F)

m étant tel que défini ci-dessus.

Les composés susmentionnés de formules (D), (E) et (F) présentent un caractère nettement basique et peuvent éventuellement être protonés sous forme de sels. Ils peuvent présenter un intérêt pour le ciblage vers des entités à caractère acide.

Selon un mode de réalisation avantageux, les composés de l'invention sont **caractérisés en ce que** R est choisi parmi les groupes suivants :

- un groupe alkyle de 1 à 12 atomes de carbone, linéaire ou ramifié, et étant de préférence le groupe méthyle ;
- un groupe aromatique tel que phényle, benzyle, naphtyle ou des dérivés de ces groupements portant des substituants sur le cycle aromatique, et étant de préférence le groupe phényle ;
- le groupe α-D-mannopyranosyle, de formule suivante (III) :

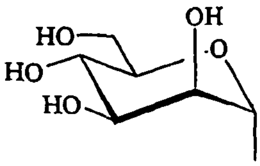

(III)

- le groupe β-lactosyle, de formule suivante (III-a) :

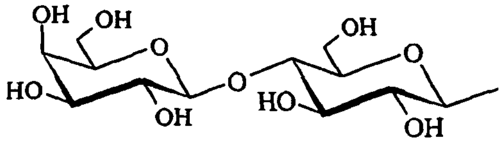

(III-a)

- le groupe dérivé du trisaccharide Lewis X ou du tétrasaccharide sialyl Lewis X, respectivement de formule suivante (III-b) et (III-c) :

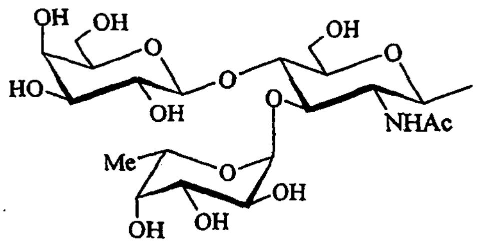

(III-b)

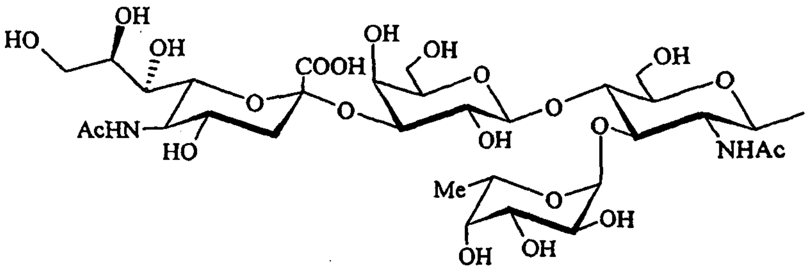

(III-c)

- un oligosaccharide dérivé de l'héparine, de formule suivante (III-d) :

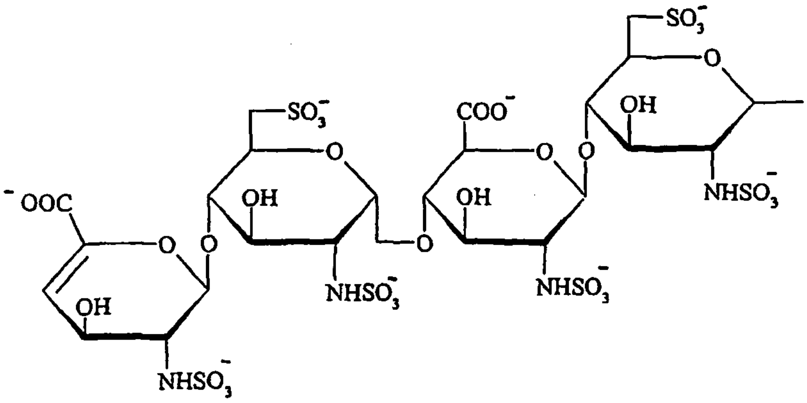

(III-d)

Ainsi, la présente invention concerne notamment les composés répondant à l'une des formules suivantes :

- composé de formule (E) dans laquelle R représente le groupe méthyle :

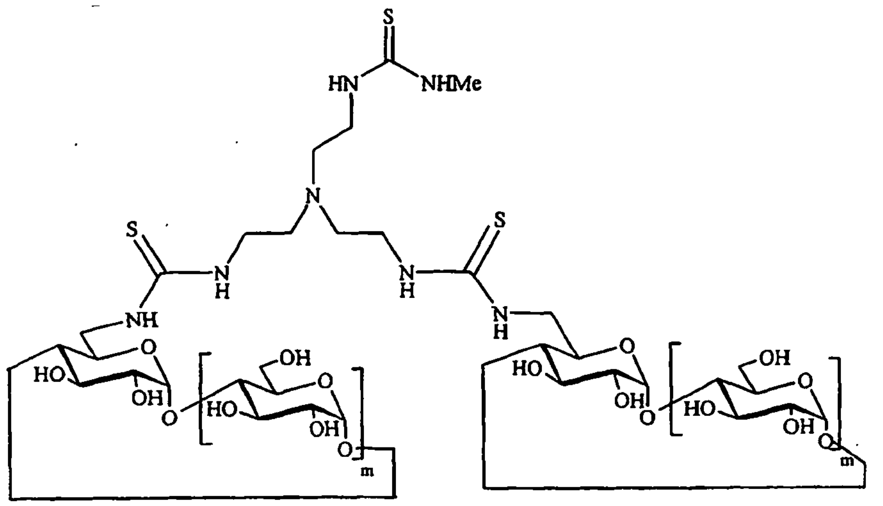

(E-a)

- composé de formule (E) dans laquelle R représente le groupe phényle :

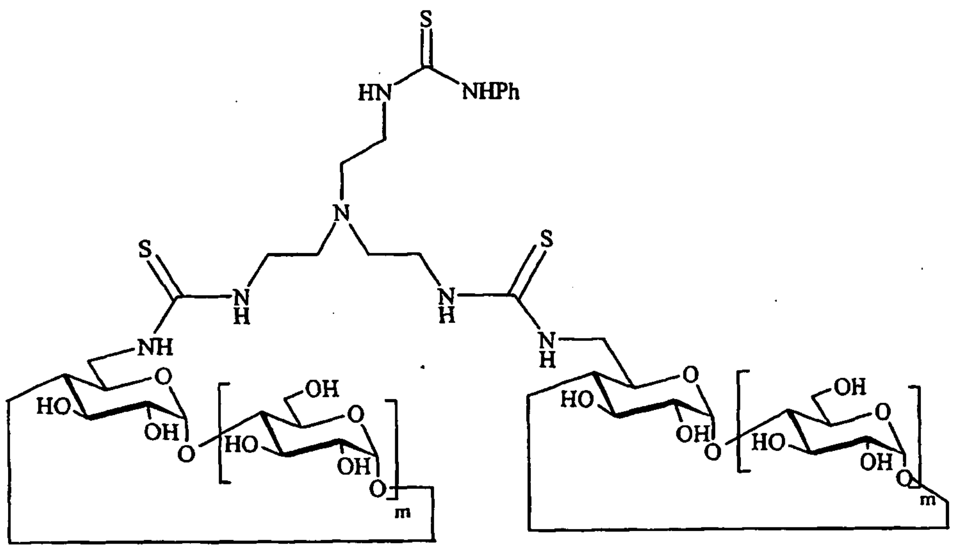

(E-b)

- composé de formule (E) dans laquelle R représente le groupe α-D-mannopyranosyle de formule (III) susmentionnée :

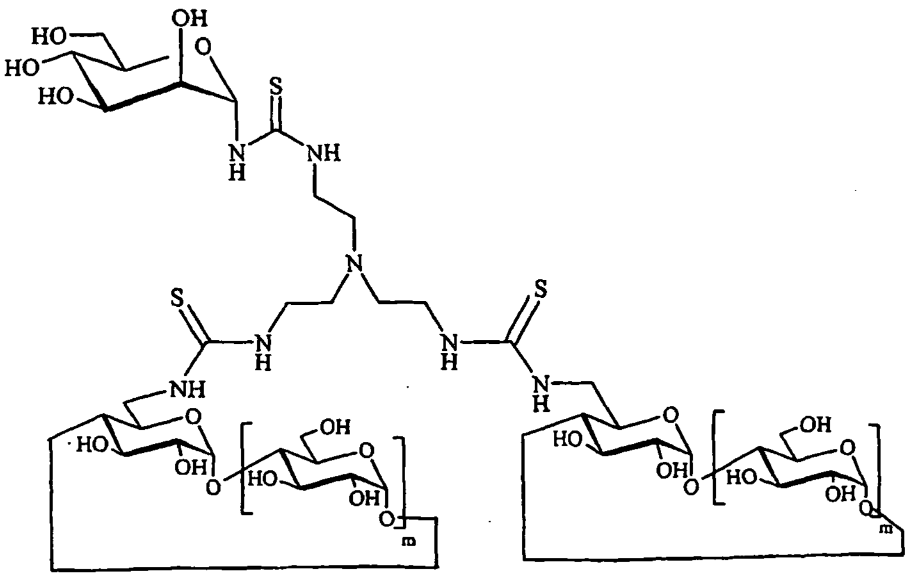

(E-c)

- composé de formule (E) dans laquelle R représente le groupe β-lactosyle de formule (III-a) susmentionnée :

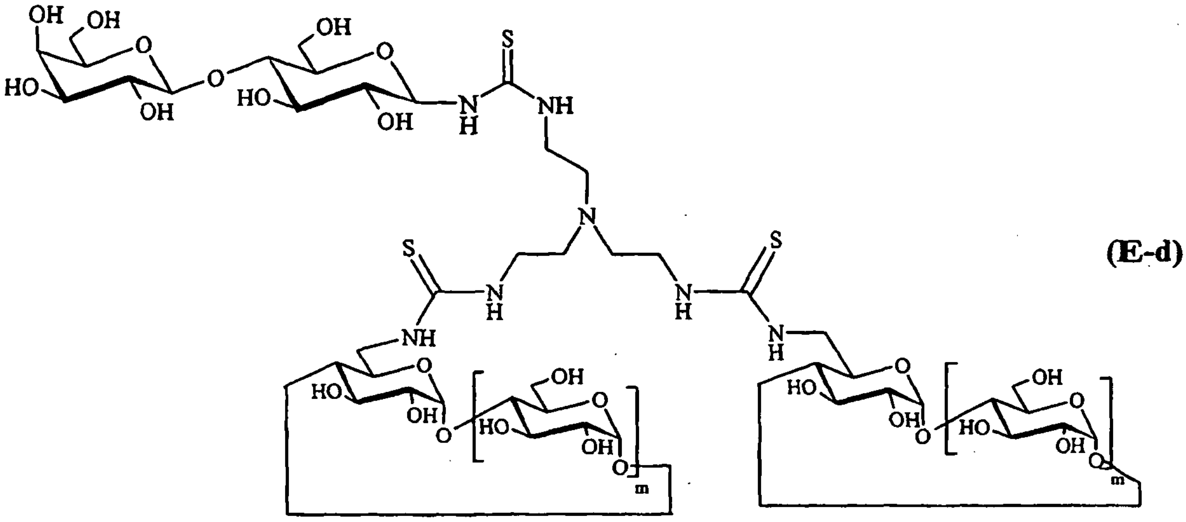

(E-d)

- composé de formule (E) dans laquelle R représente le groupe dérivé du trisaccharide Lewis X de formule (III-b) susmentionnée :

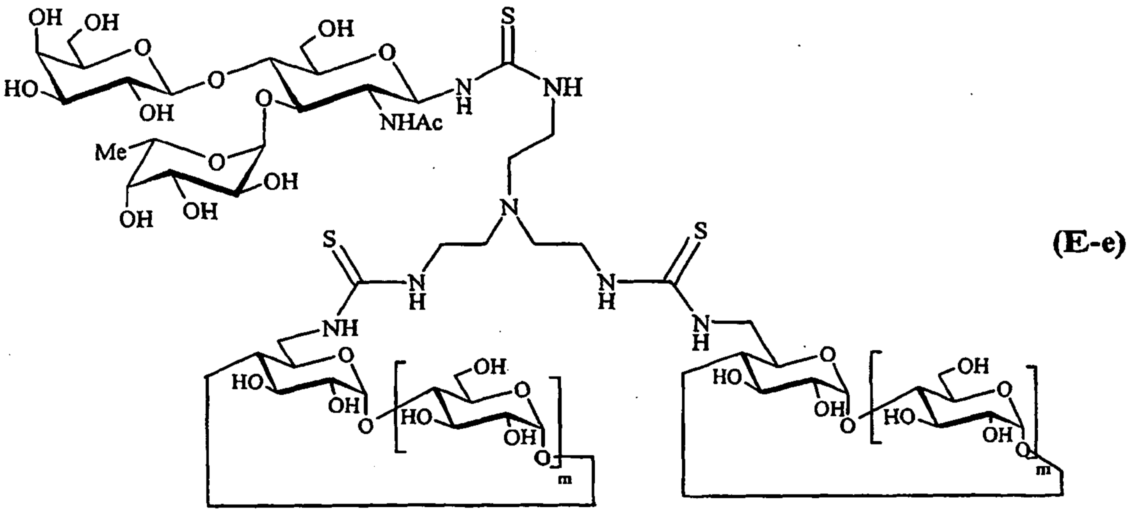

(E-e)

- composé de formule (E) dans laquelle R représente le groupe dérivé du tétrasaccharide sialyl Lewis X de formule (III-c) susmentionnée :

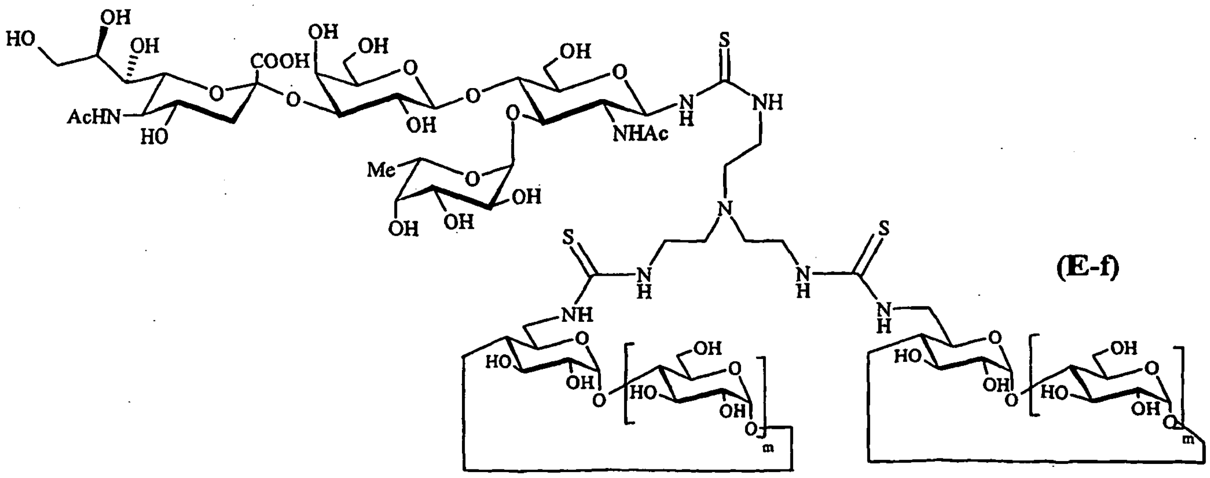

(E-f)

- composé de formule (E) dans laquelle R représente un oligosaccharide dérivé de l'héparine, tel que l'oligosaccharide de formule (III-d) susmentionnée :

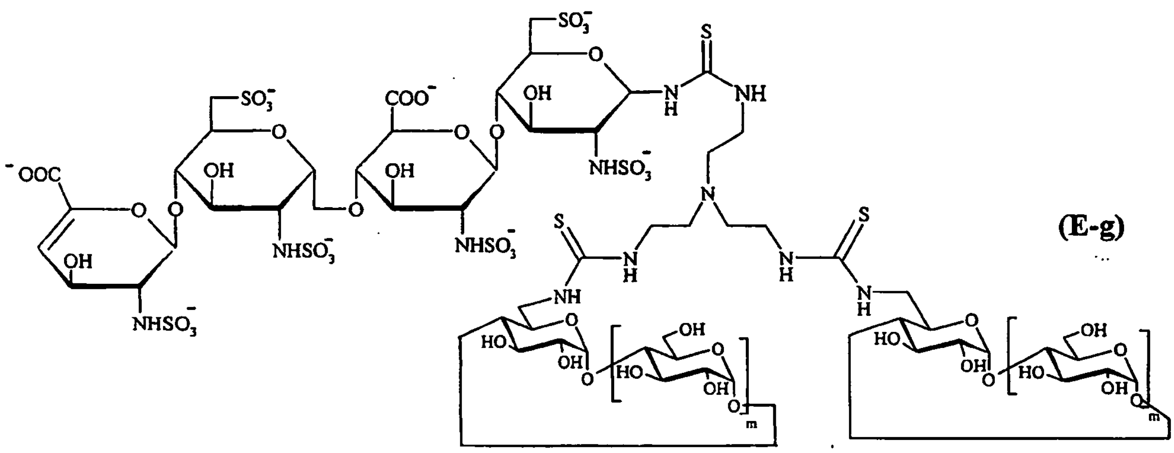

(E-g)

La présente invention concerne également un composé tel que défini ci-dessus, notamment de formule (I),

**caractérisé en ce que** R comporte un élément de ramification dérivé du tris(2-hydroxyméthyl)méthylamine, et représente l'un des groupes suivants :

- le groupe tris(α-D-mannopyranosyloxyméthyl)méthyle, de formule suivante (IV):

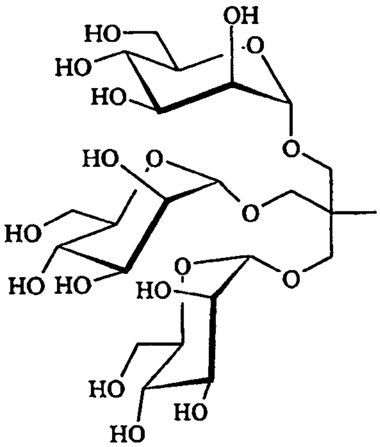

**(IV)**

- le groupe tris(β-lactosyloxyméthyl)méthyle, de formule suivante (IV-a) :

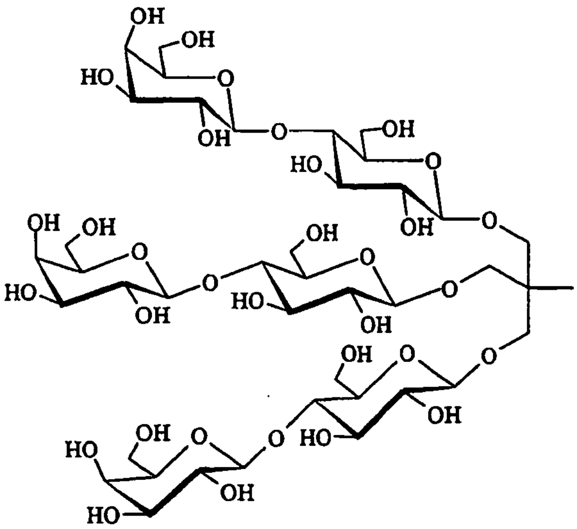

**(IV-a)**

Ainsi, la présente invention concerne notamment les composés répondant à l'une des formules suivantes :

- composé de formule (E) dans laquelle R représente le groupe tris(α-D-mannopyranosyloxyméthyl)méthyle, de formule (IV) susmentionnée :

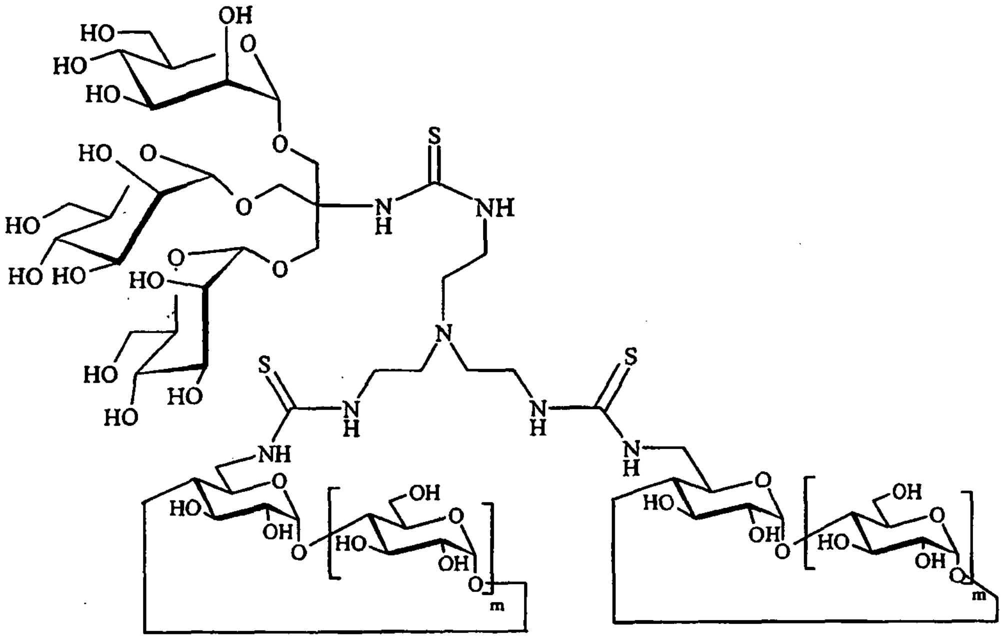

(E-h)

- composé de formule (E) dans laquelle R représente le groupe tris(β-lactosyloxyméthyl)méthyle, de formule (IV-a) susmentionnée :

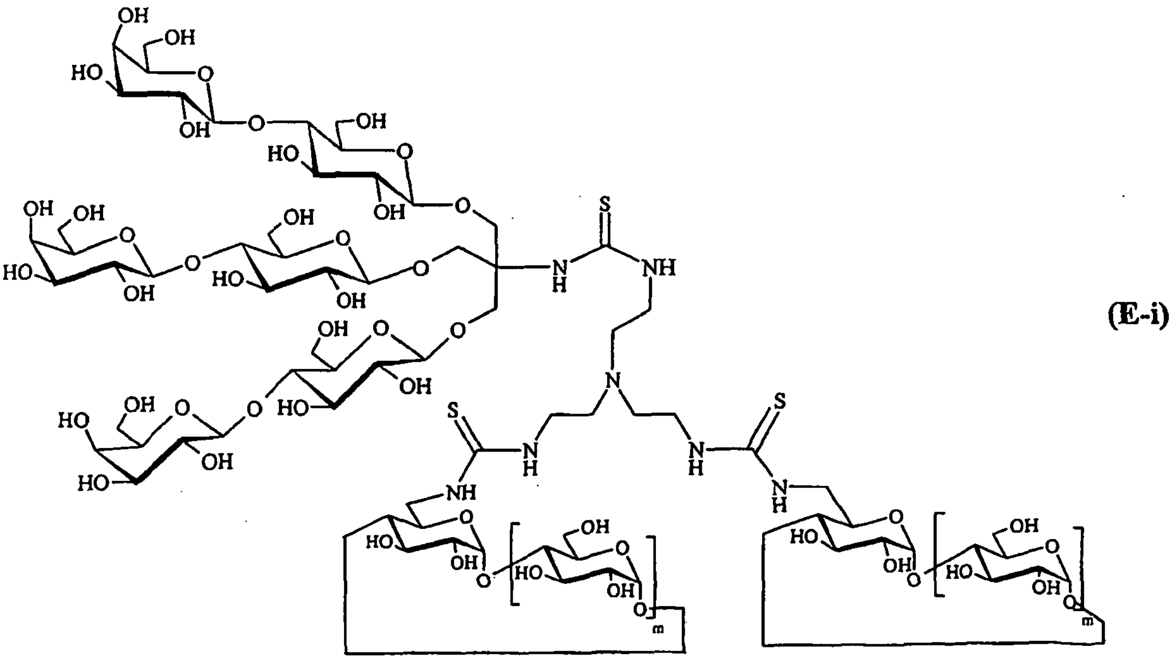

(E-i)

La présente invention concerne également un composé tel que défini ci-dessus, de formule (I), **caractérisé en ce que** R comporte un élément de ramification dérivé du pentaérythritol, ledit composé répondant à l'une des formules suivantes :

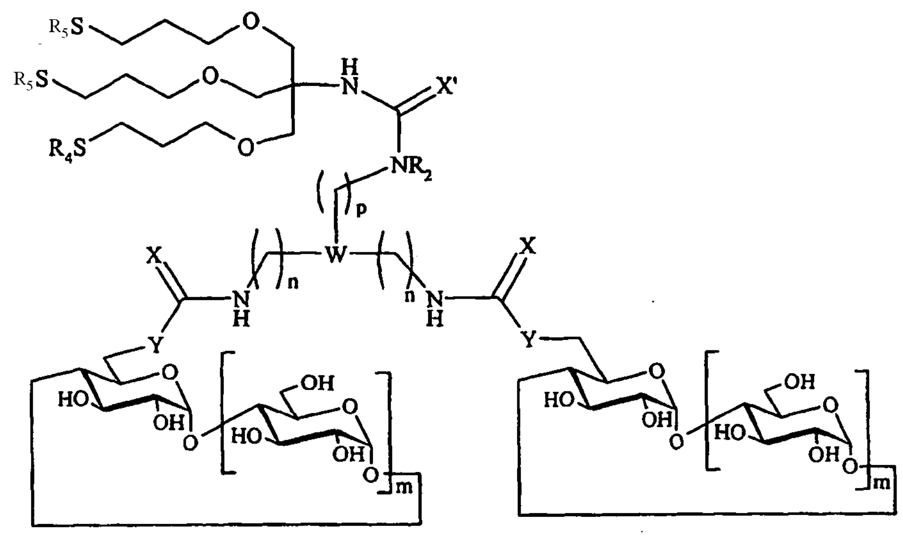

(II-a)

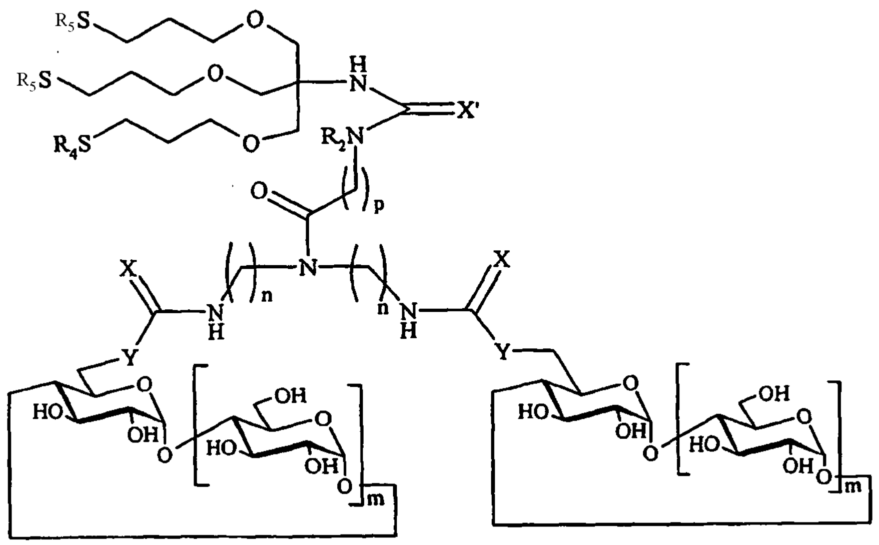

(II-b)

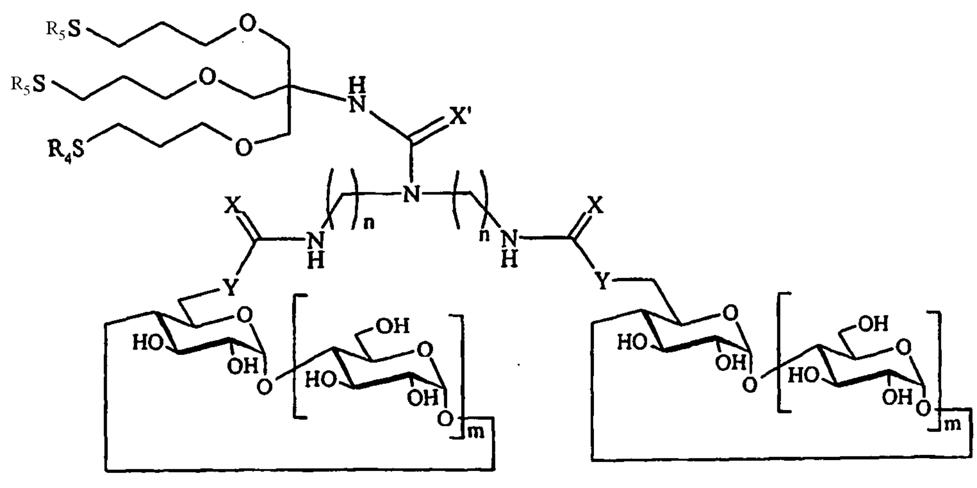

(II-c)

dans lesquelles m, n, p, X, X', Y sont tels que définis ci-dessus, et
$R_5$ et $R_4$ représentent des dérivés glucidiques qui peuvent être différents ou identiques ou encore une sonde

fluorescente ou radioactive.

La présente invention concerne également un composé tel que défini ci-dessus, répondant aux formules susmentionnées (I-f), (I-i) ou (I-j), **caractérisé en ce que** R comporte un élément de ramification dérivé du pentaérythritol, ledit composé répondant à l'une des formules (II-a-bis), (II-b-bis) ou (II-c-bis) suivantes :

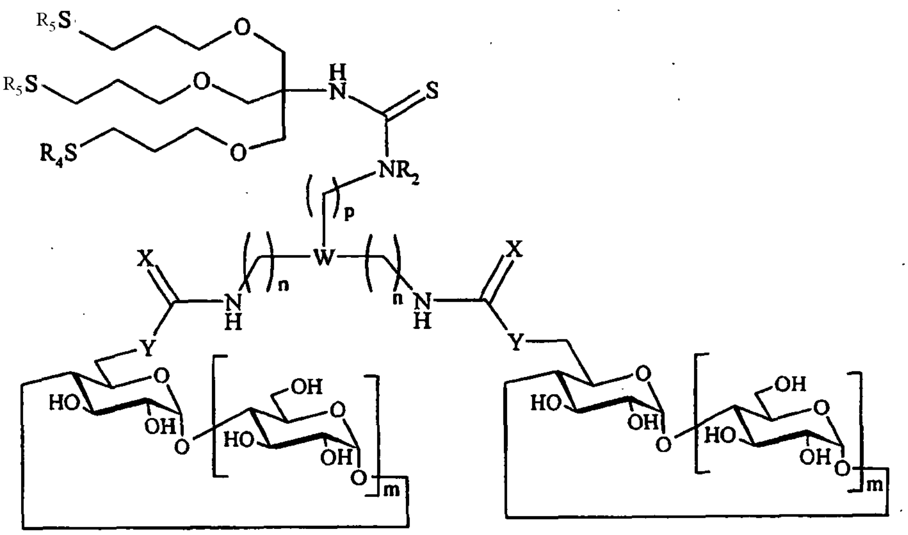

**(II-a-bis)**

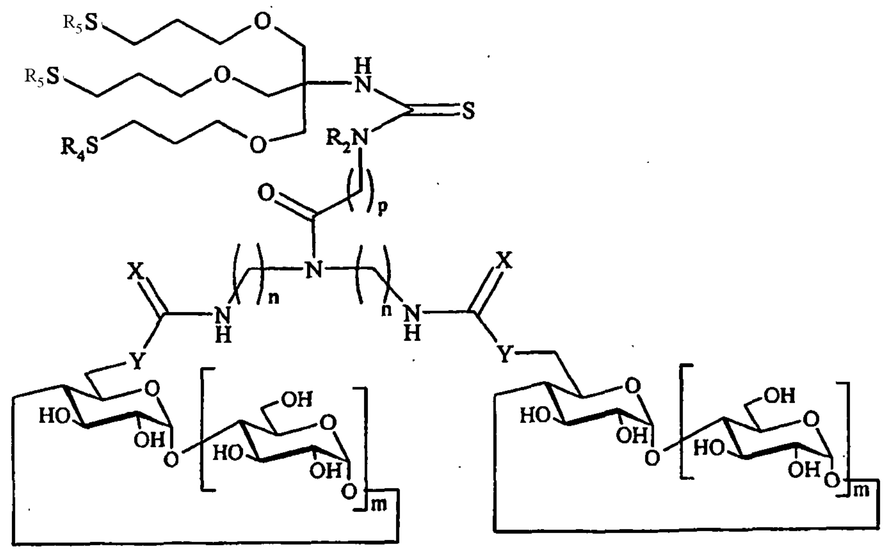

**(II-b-bis)**

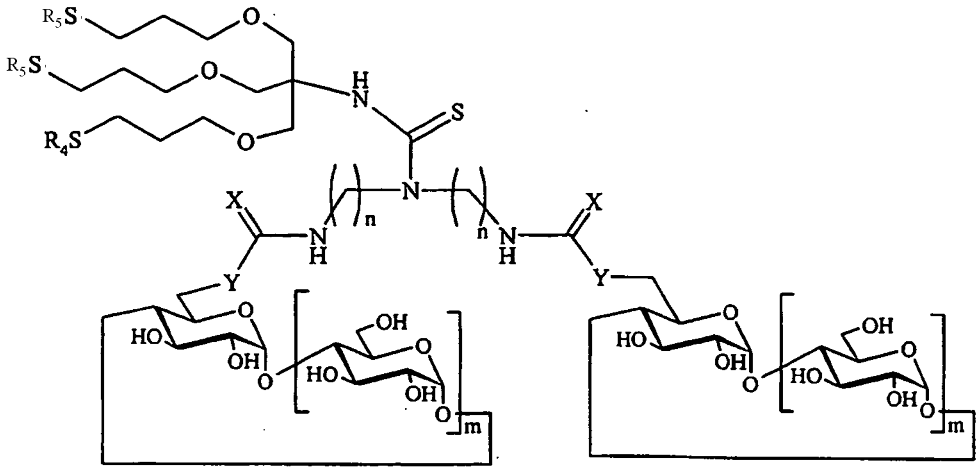

(II-c-bis)

dans lesquelles m, n, p, X, Y, $R_5$ et $R_4$ sont tels que définis ci-dessus.

Un composé avantageux selon la présente invention est un composé répondant à la formule susmentionnée (II-a), dans laquelle n et p sont égaux à 2, X et X' représentent un atome de soufre, Y représente un groupe NH et $R_2$ représente un atome d'hydrogène. Un tel composé répond à la formule suivante (G) :

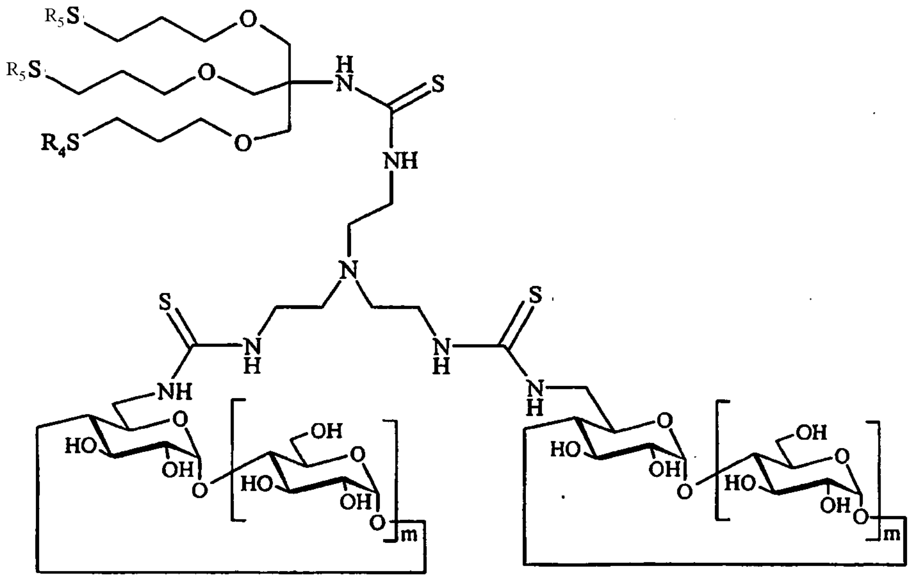

(G)

La présente invention concerne également un composé tel que défini ci-dessus, répondant à l'une des formules (II-a), (II-b) ou (II-c), **caractérisé en ce que** $R_5$ et $R_4$ représentent l'un des groupes suivantes :

- le groupe α-D-mannopyranosyle, de formule (III) tel que défini ci-dessus, ou
- le groupe β-lactosyle, de formule (III-a) tel que défini ci-dessus, ou
- le groupe β-D-glucopyranosyle, de formule (VI) suivante :

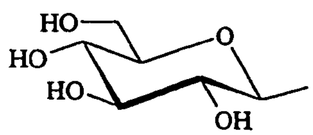

(VI)

$R^5$ et $R^4$ pouvant être identiques ou différents.

Les composés préférés de l'invention sont notamment des composés de formule (G) dans lesquels les substituants $R_5$ et $R_4$ sont tels que définis ci-dessus. De tels composés répondent aux formules suivantes :

- composé de formule (G) dans laquelle $R_5$ et $R_4$ représentent un groupe α-D-mannopyranosyle, de formule (III) susmentionnée :

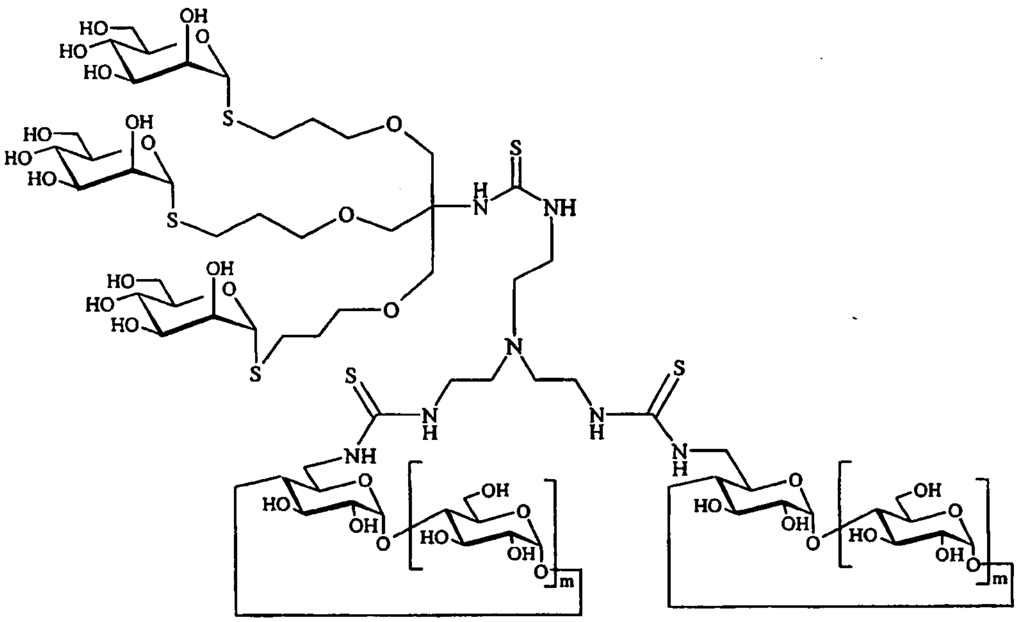

**(G-a)**

- composé de formule (G) dans laquelle $R_5$ et $R_4$ représentent un groupe β-D-glucopyranosyle, de formule (VI) susmentionnée :

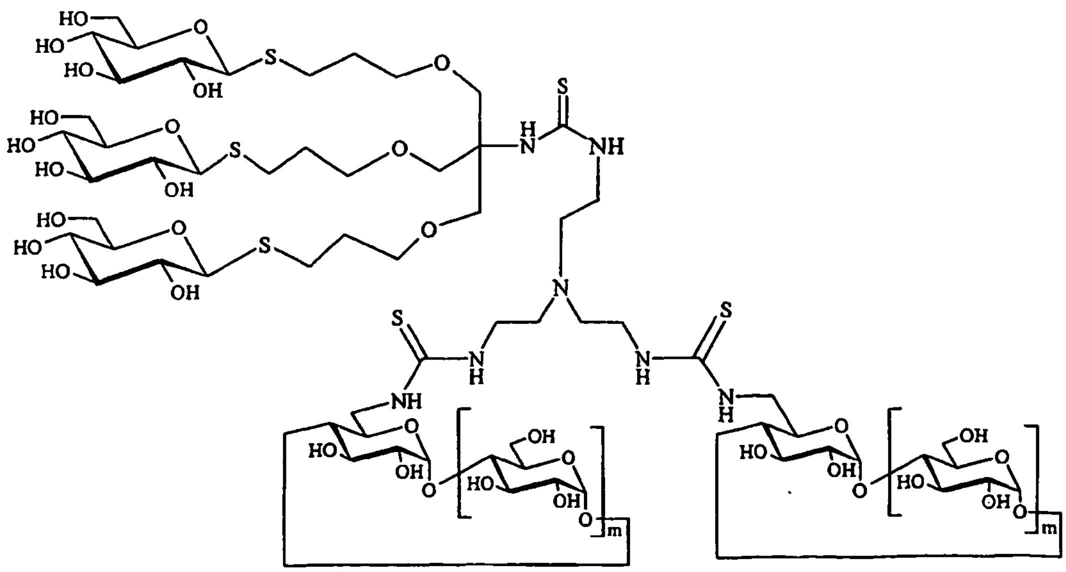

**(G-b)**

- composé de formule (G) dans laquelle $R_5$ et $R_4$ représentent un groupe β-lactosyle, de formule (III-a) susmentionnée :

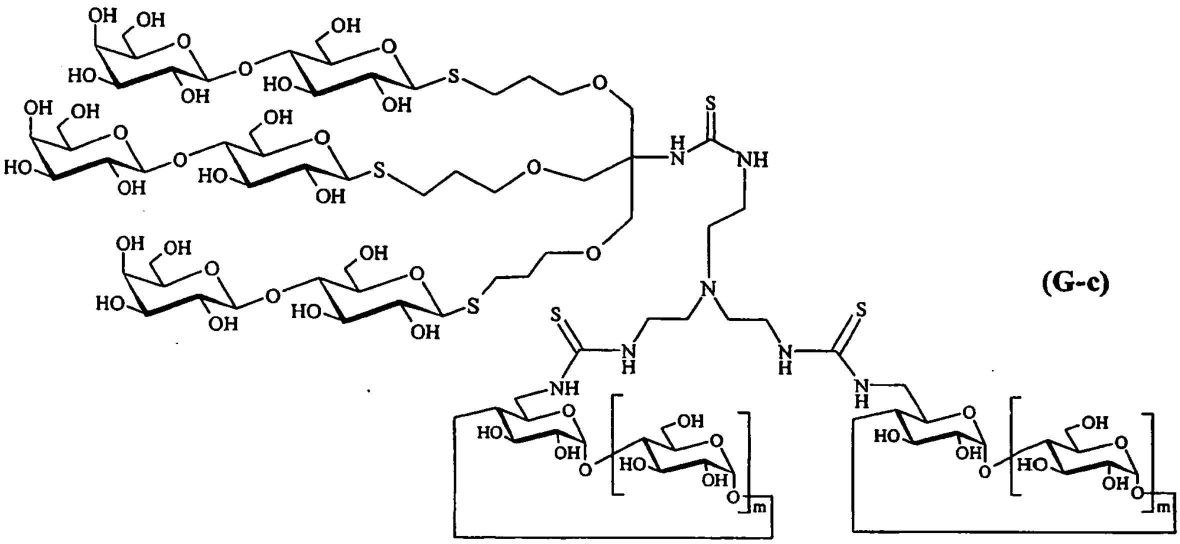

(G-c)

- composé de formule (G) dans laquelle $R_5$ représente un groupe α-D-mannopyranosyle de formule (III) susmentionnée, et $R_4$ représente un groupe β-D-glucopyranosyle, de formule (VI) susmentionnée :

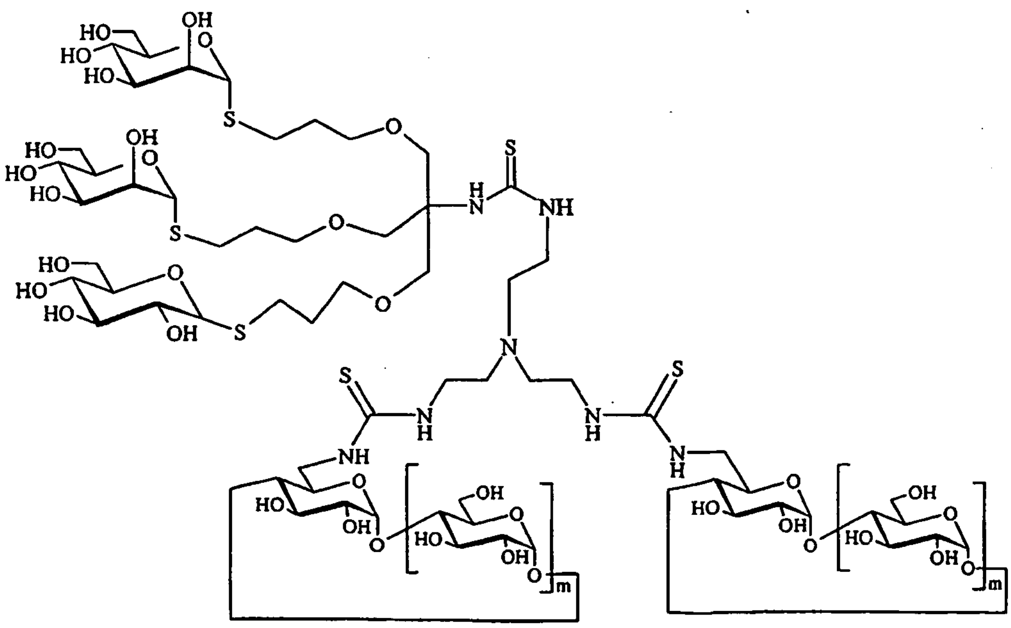

(G-d)

- composé de formule (G) dans laquelle $R_5$ représente un groupe α-D-mannopyranosyle de formule (III) susmentionnée, et $R_4$ représente un groupe β-lactosyle, de formule (III-a) susmentionnée :

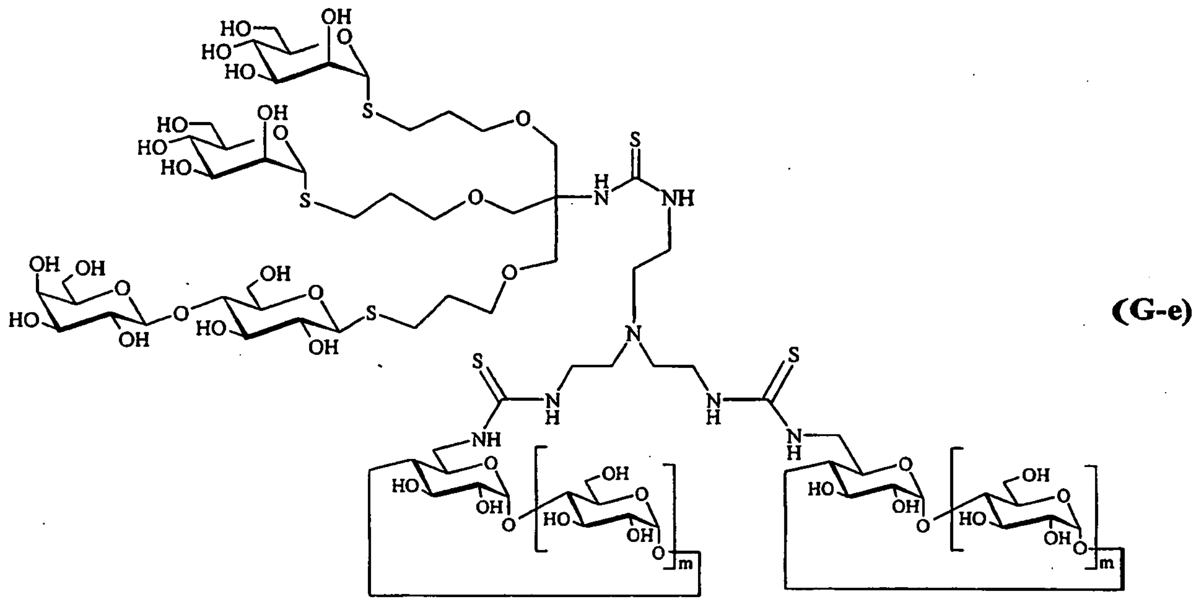

**(G-e)**

- composé de formule (G) dans laquelle $R_5$ représente un groupe β-D-glucopyranosyle, de formule (VI) susmentionnée, et $R_4$ représente un groupe α-D-mannopyranosyle, de formule (III) susmentionnée :

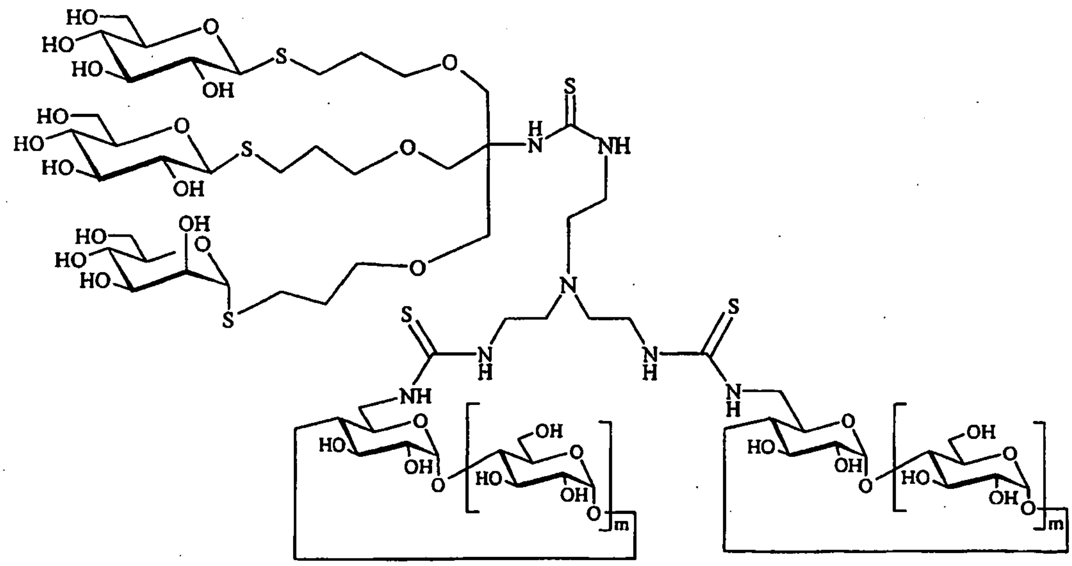

**(G-f)**

- composé de formule (G) dans laquelle $R_5$ représente un groupe β-D-glucopyranosyle, de formule (VI) susmentionnée, et $R_4$ représente un groupe β-lactosyle, de formule (III-a) susmentionnée :

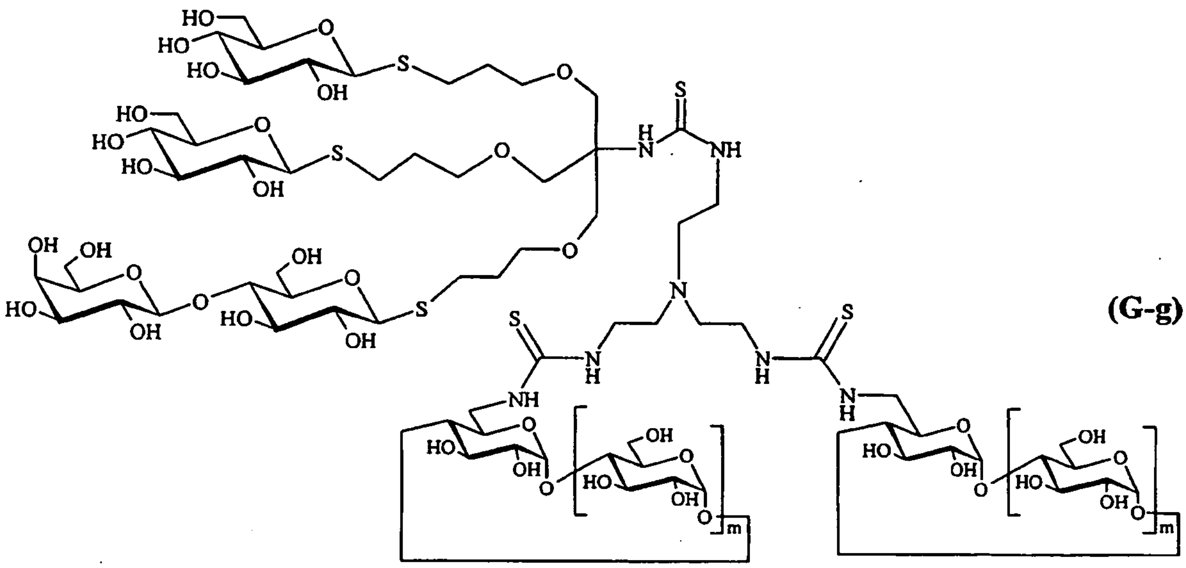

(G-g)

- composé de formule (G) dans laquelle $R_5$ représente un groupe β-lactosyle, de formule (III-a) susmentionnée, et $R_4$ représente un groupe α-D-mannopyranosyle, de formule (III) susmentionnée :

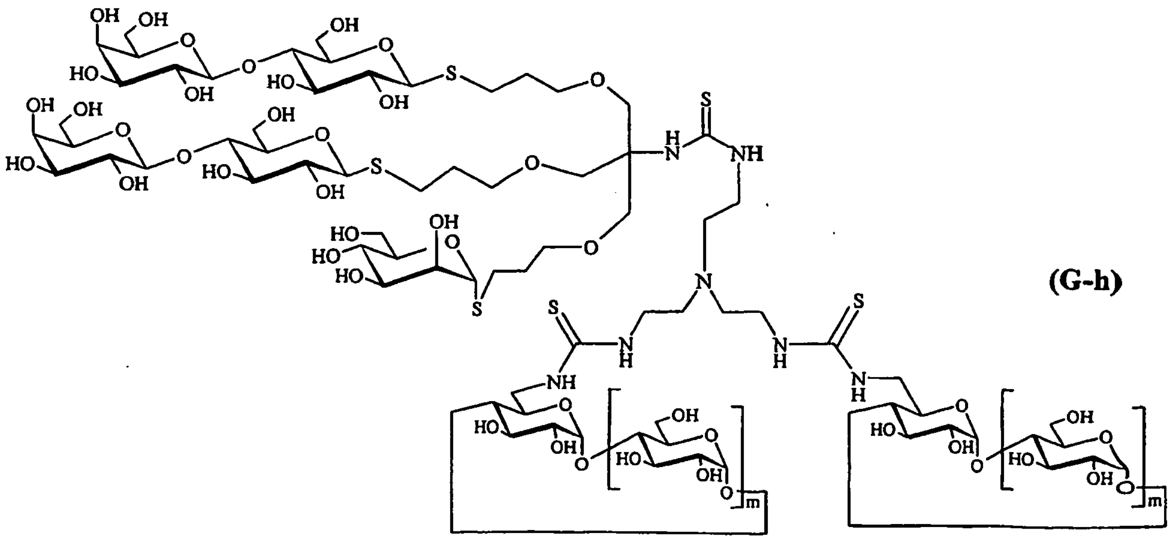

(G-h)

- composé de formule (G) dans laquelle $R_5$ représente un groupe β-lactosyle, de formule (III-a) susmentionnée, et $R_4$ représente un groupe α-D-glucopyranosyle, de formule (VI) susmentionnée :

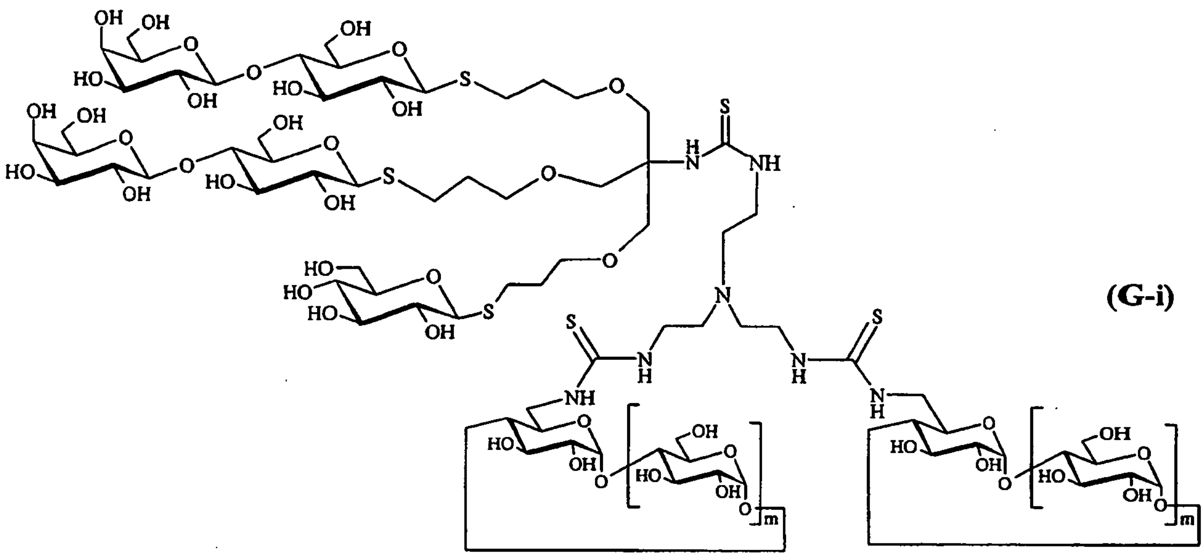

**(G-i)**

La présente invention concerne également un composé tel que défini ci-dessus, de formule (I), **caractérisé en ce que** R comporte un élément de ramification dérivé du tris(2-aminoéthyl)amine (TREN), ledit composé répondant à l'une des formules suivantes :

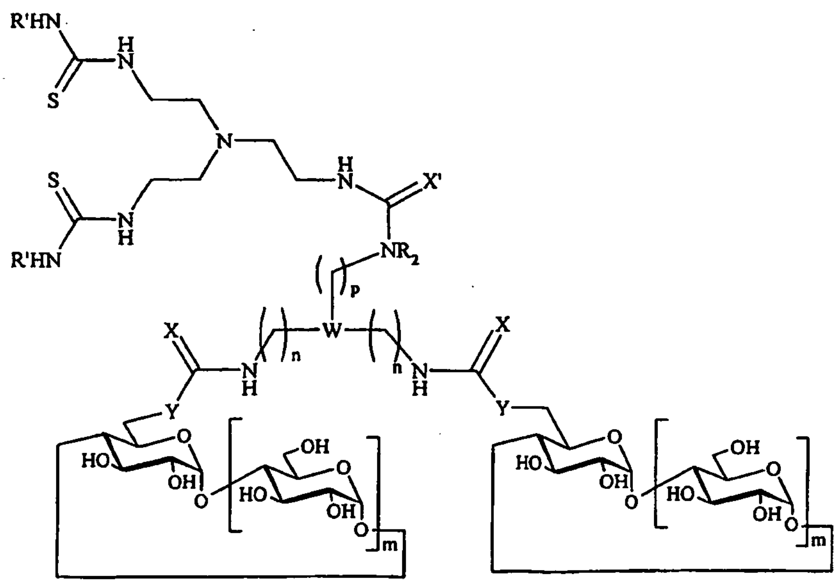

**(V-a)**

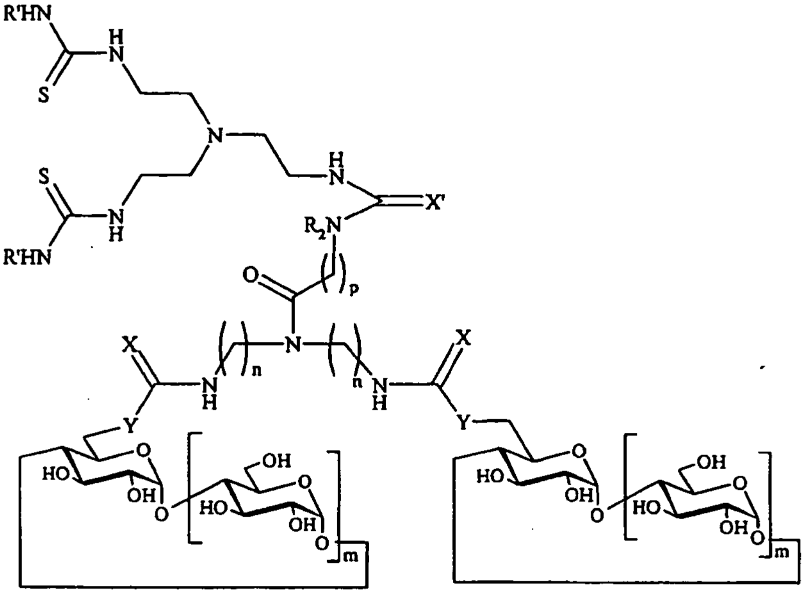

(V-b)

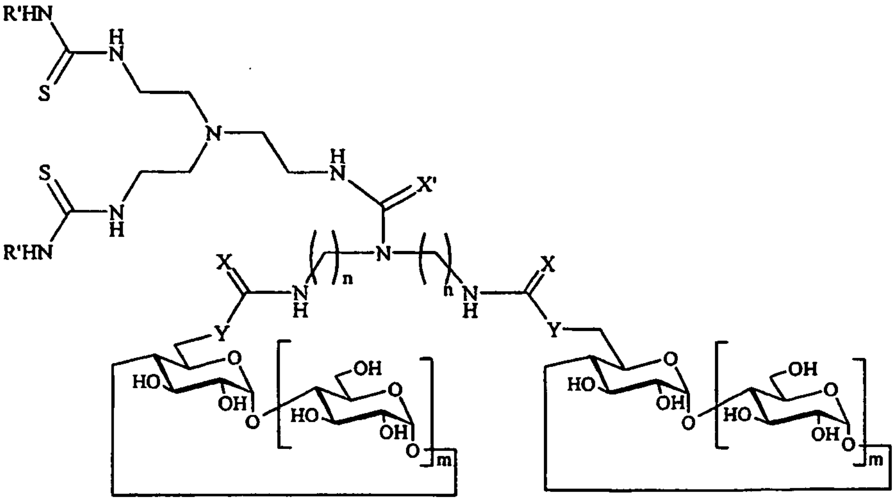

(V-c)

m, n, p, X, X', Y étant tels que définis ci-dessus, et R' ayant la définition donnée précédemment pour R.

La présente invention concerne également un composé tel que défini ci-dessus, répondant notamment à la formule (I-f), (I-i) ou (I-j), **caractérisé en ce que** R comporte un élément de ramification dérivé du tris(2-aminoéthyl) amine (TREN), ledit composé répondant à l'une des formules (V-a-bis), (V-b-bis) ou (V-c-bis) suivantes :

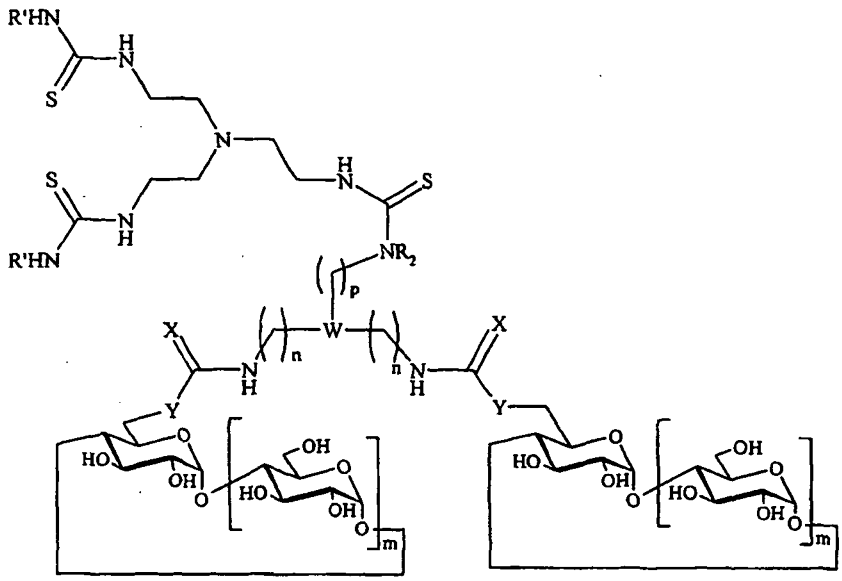

(V-a-bis)

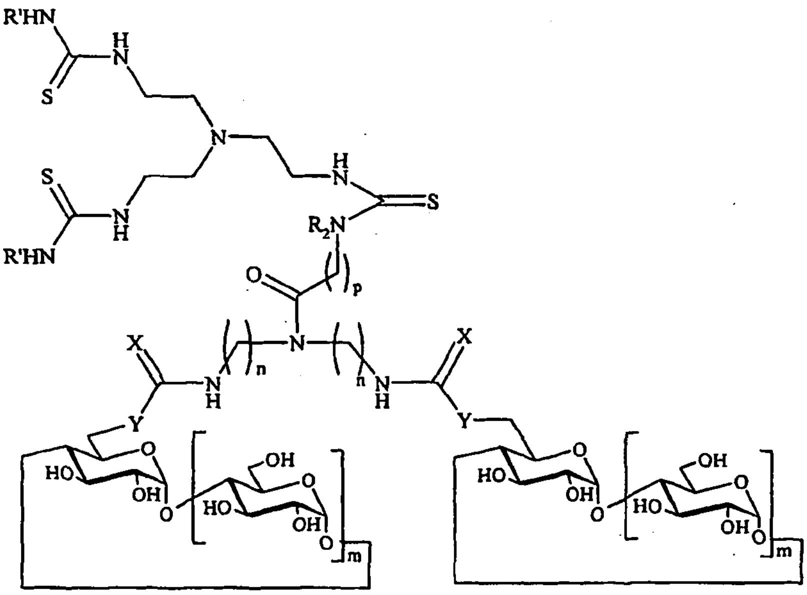

(V-b-bis)

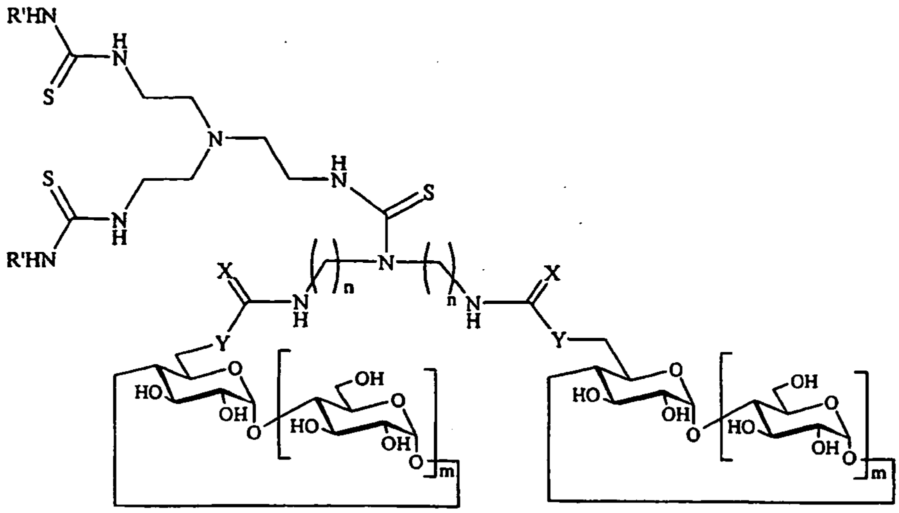

**(V-c-bis)**

m, n, p, X, Y et R' étant tels que définis ci-dessus.

Un composé avantageux selon la présente invention est un composé tel que défini ci-dessus, de formule (V-a), **caractérisé en ce que** Y représente NH, X et X' représentent un atome de soufre, W représente un atome d'azote, $R^2$ représente un atome d'hydrogène et en ce que n est égal à 2. Un tel composé répond à la formule suivante :

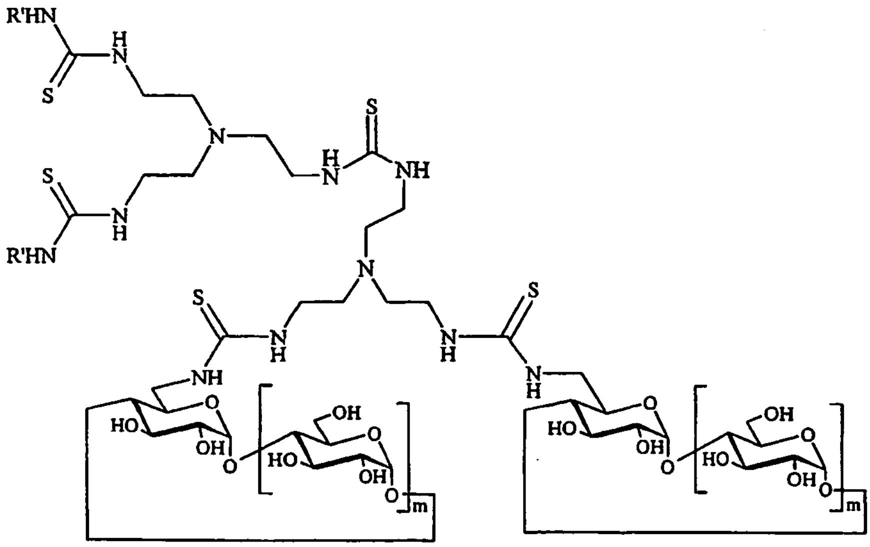

**(H)**

m et R' étant tel que défini ci-dessus.

La présence de deux substituants R' dans les composés de formule (H) est particulièrement intéressante lorsque R' représente un élément de reconnaissance biologique, et notamment lorsque cet élément de reconnaissance est un dérivé glucidique, puisque dans ce cas l'affinité pour les récepteurs biologiques complémentaires est améliorée du fait de la présentation dendritique multivalente du ligand.

La présente invention concerne également un composé tel que défini ci-dessus, de formule (V-a), (V-b) ou (V-c), **caractérisé en ce que** R' représente :

- le groupe α-D-mannopyranosyle, de formule (III), ou
- le groupe β-lactosyle de formule (III-a), ou
- le groupe tris(α-D-mannopyranosyloxyméthyl)méthyle, de formule (IV), ou
- le groupe tris(β-lactyloxyméthyl)méthyle, de formule (IV-a).

Ainsi, la présente invention concerne notamment les composés répondant à l'une des formules suivantes :

- composé de formule (H) dans laquelle R' représente le groupe α-D-mannopyranosyle de formule (III) :

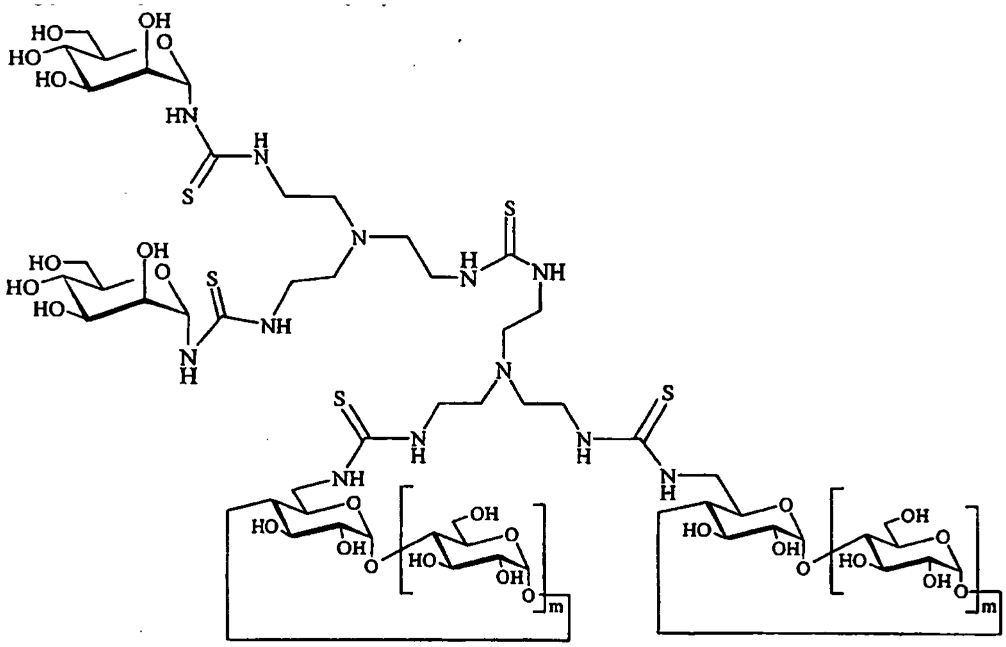

(H-a)

- composé de formule (H) dans laquelle R' représente le groupe β-lactosyle de formule (III-a) :

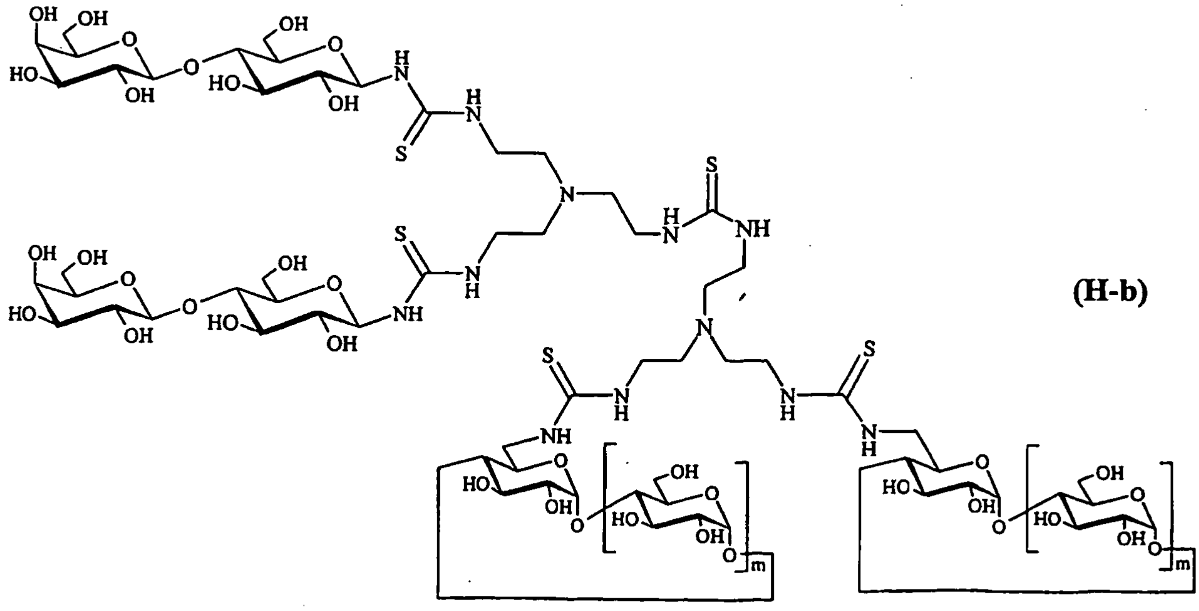

(H-b)

- composé de formule (H) dans laquelle R' représente le groupe tris(α-D-mannopyranosyloxyméthyl)méthyle de formule (IV) :

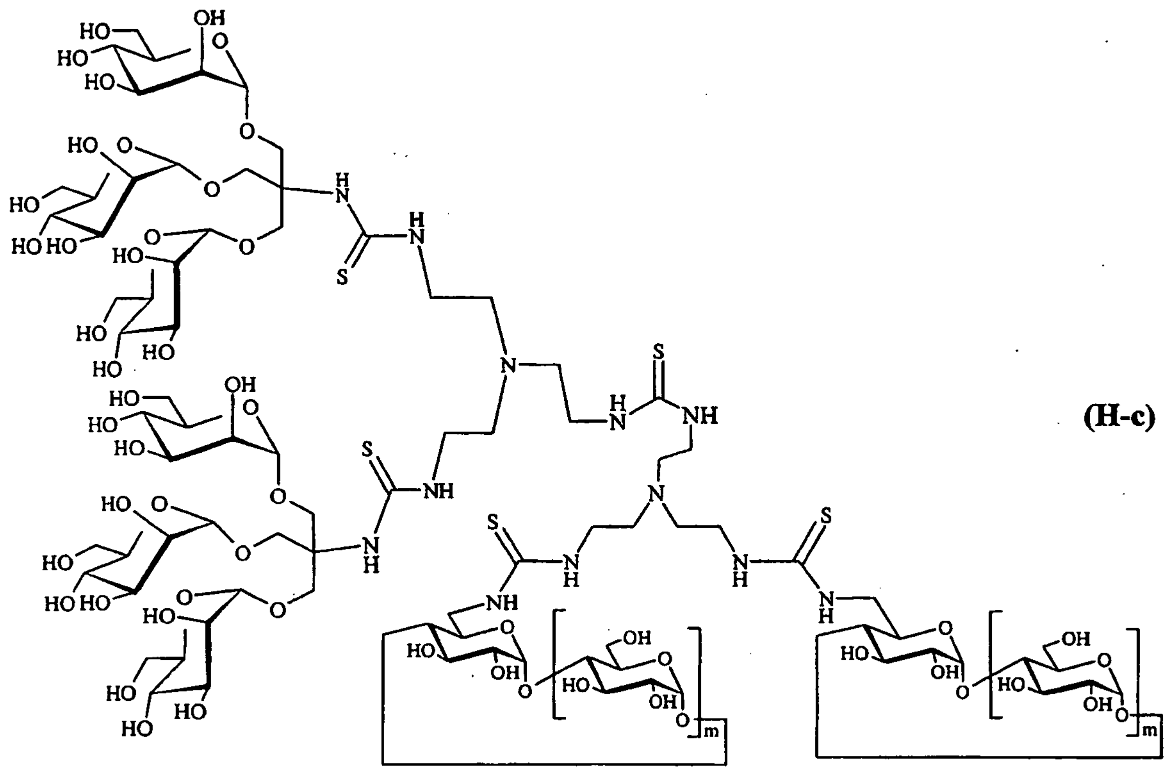

(H-c)

- composé de formule (H) dans laquelle R' représente le groupe tris(β-lactosyloxyméthyl)méthyle, de formule (IV-a) :

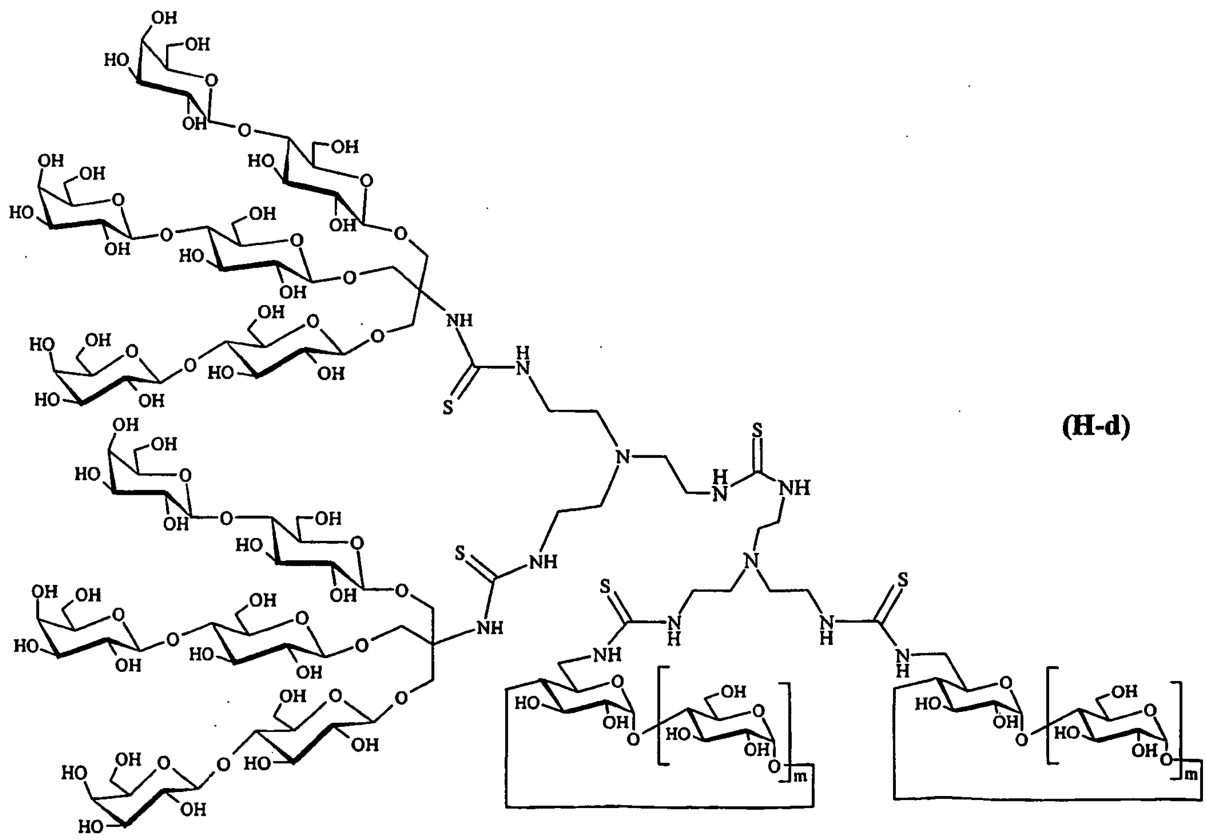

(H-d)

La présente invention concerne également un composé tel que défini ci-dessus, **caractérisé en ce que** m est égal à 6.

L'utilisation de dérivés de cyclodextrines monosubstitués en position alcool primaire avec un groupement portant une fonctionnalité amine, comme précurseurs pour la préparation des cyclodextrines dimères de l'invention, présente des avantages en comparaison avec d'autres méthodes préalablement décrites. Notamment, la formation de deux liaisons de type urée ou thiourée par réaction avec un élément de dimérisation de type diisocyanate ou diisothiocyanate présente des avantages du point de vue de la simplicité opératoire, des rendements et de la purification du produit final qui ne nécessite le plus souvent pas de séparation chromatographique. Un autre aspect intéressant de la présente invention en comparaison avec d'autres méthodes préalablement décrites est l'utilisation d'éléments de dimérisation possédant une fonctionnalité amine protégée, qui peut être utilisée pour l'incorporation de nouveaux substituants sur le dérivé de cyclodextrine dimère, tel qu'un élément de bioreconnaissance, notamment par formation d'une nouvelle liaison urée ou thiourée.

La présente invention concerne également un procédé de préparation d'un composé tel que défini ci-dessus, de formule (I), **caractérisé en ce qu'**il comprend les étapes suivantes :

- la réaction d'un composé sélectivement fonctionnalisé en position alcool primaire avec un groupement aminé, de formule (VII) suivante :

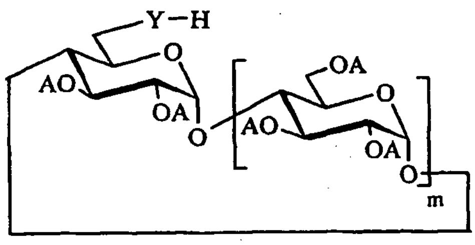

**(VII)**

m, A et Y étant tels que définis ci-dessus, et A étant de préférence un atome d'hydrogène,
avec un élément de dimérisation de type diisocyanate ou diisothiocyanate, notamment portant une fonctionnalité amine protégée sous forme d'un groupe carbamate ou portant une fonctionnalité sel d'ammonium quaternaire chargé positivement, de formule (VIII) suivante :

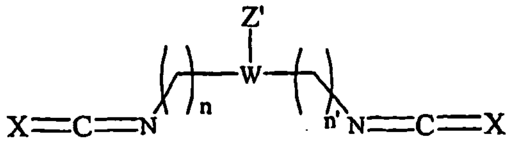

**(VIII)**

* n et n' étant tels que définis ci-dessus, et étant de préférence égaux,
* W et X étant tels que définis ci-dessus,
* Z' représentant un groupe répondant à l'une des formules suivantes :

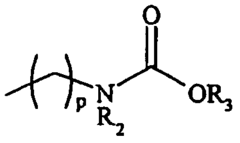 ou 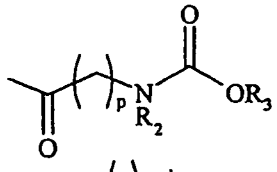 ou 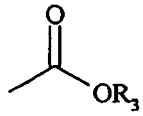 ou

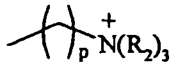 ou 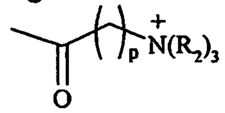 ou 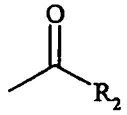

p, $R_2$ et $R_3$ étant tels que définis ci-dessus,
afin d'obtenir un composé tel que défini ci-dessus de formule (I), et répondant à la formule (IX) suivante :

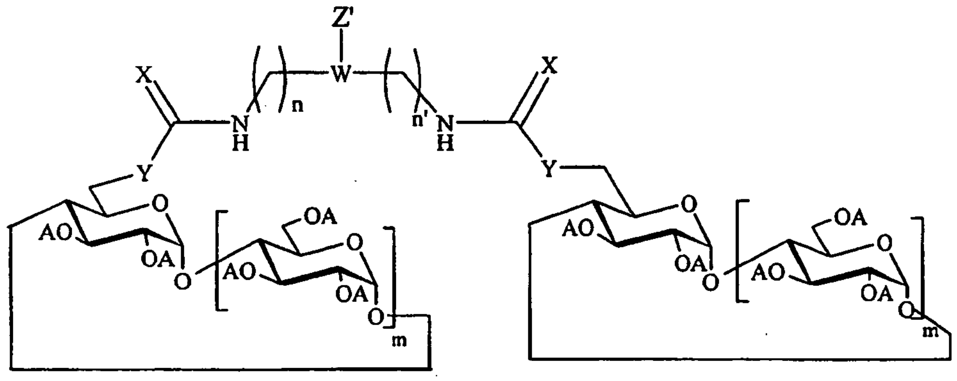

(IX)

- et éventuellement la réaction d'hydrolyse du groupement

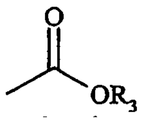

tel que défini ci-dessus, présent dans les composés de formule (IX) susmentionnée, dans laquelle Z' contient un tel groupement, afin d'obtenir un composé portant une fonctionnalité amine libre et répondant à la formule (X) suivante :

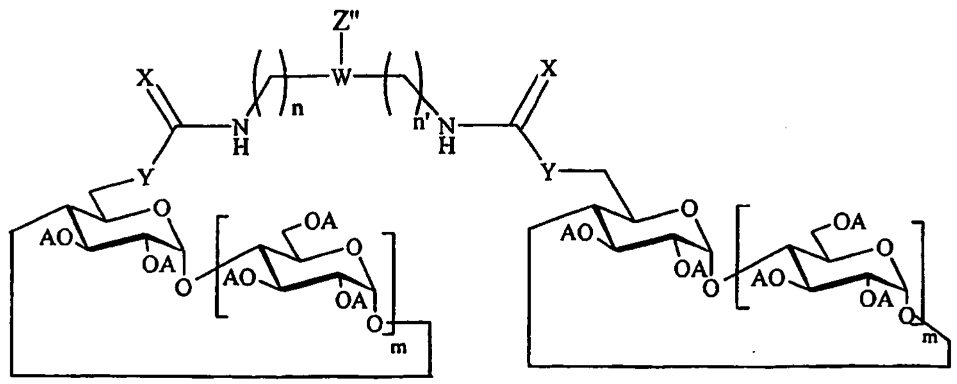

(X)

* n, n,' A, X, Y, W et m étant tels que définis précédemment, et
* Z" correspondant à l'hydrolysat du groupe Z' contenant une fonction $-COOR_3$, et représentant un atome d'hydrogène ou répondant à l'une des formules suivantes :

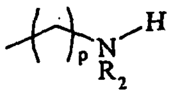

ou

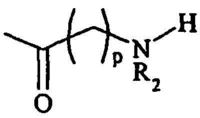

p et $R_2$ étant tels que définis ci-dessus,

- et éventuellement la réaction d'un composé de formule (X) tel qu'obtenu à l'étape précédente, avec un isocyanate ou un isothiocyanate de formule (XI) suivante :

R-N=C=X'

R et X' étant tels que définis ci-dessus,
afin d'obtenir un composé tel que défini ci-dessus de formule (I), et répondant à la formule (XII) suivante :

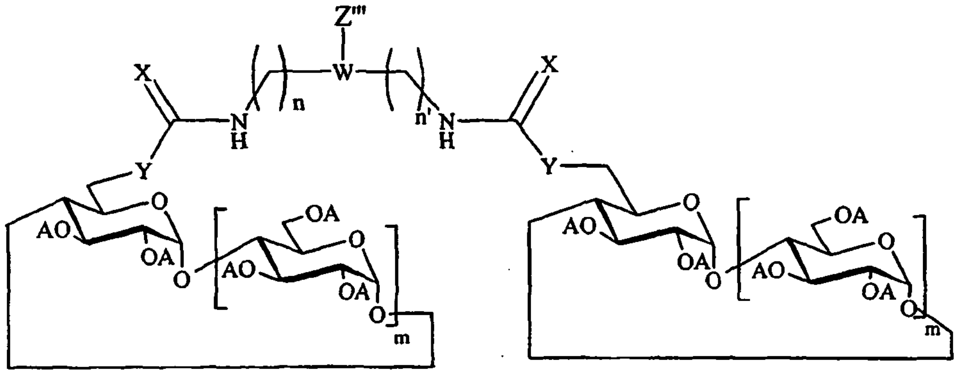

**(XII)**

* n, n,' A, X, Y, W et m étant tels que définis précédemment, et
* Z''' répondant à l'une des formules suivantes :

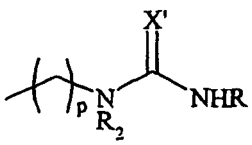

ou

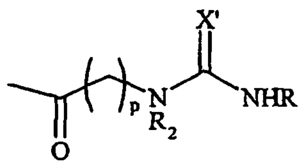

ou

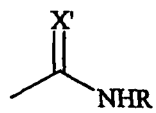

p, $R_2$, X' et R étant tels que définis ci-dessus.

La présente invention concerne également un procédé de préparation d'un composé tel que défini ci-dessus, de formule (I-a), **caractérisé en ce qu**'il comprend les étapes suivantes :

- la réaction d'un composé sélectivement fonctionnalisé en position alcool primaire avec un groupement aminé, de formule (VII-1) suivante :

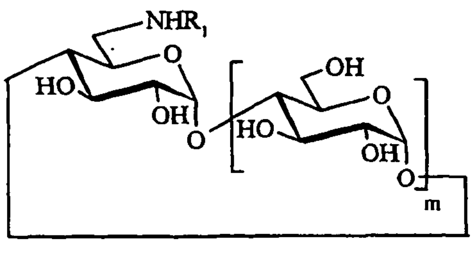

**(VII-1)**

m étant tel que défini ci-dessus,
avec un élément de dimérisation de type diisocyanate ou diisothiocyanate, notamment portant une fonctionnalité amine protégée sous forme d'un groupe carbamate, de formules (VIII-a), (VIII-b) ou (VIII-c) suivantes :

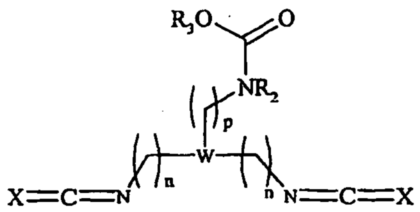

(VIII-a)

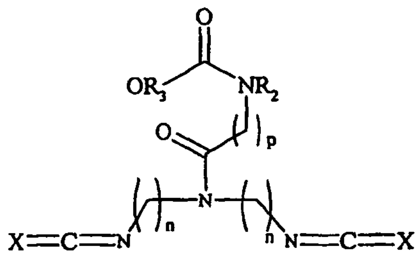

(VIII-b)

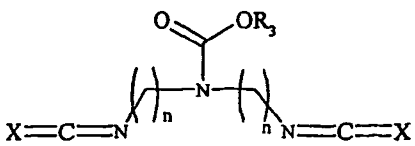

(VIII-c)

n, p, $R_2$, $R_3$, W et X étant tels que définis ci-dessus,
afin d'obtenir un composé tel que défini ci-dessus répondant respectivement aux formules (IX-1-a), (IX-1-b) ou (IX-1-c) :

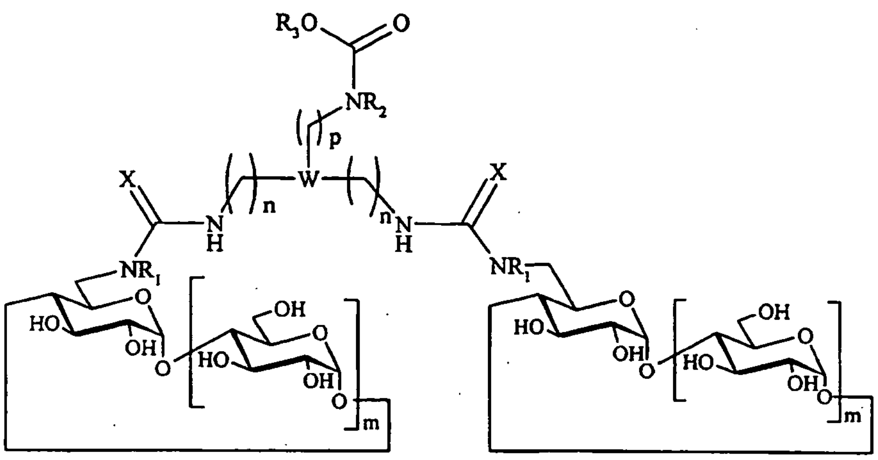

(IX-1-a)

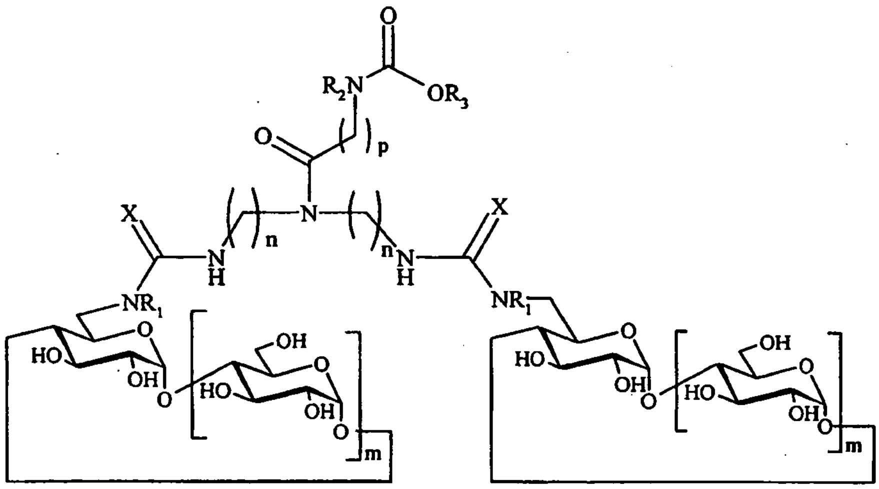

(IX-1-b)

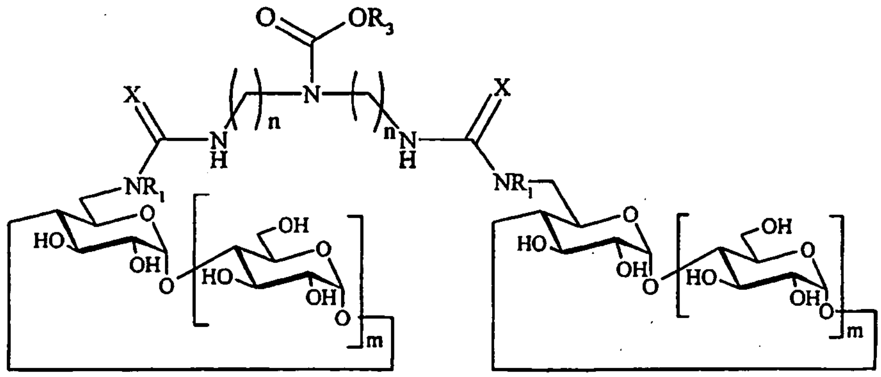

(IX-1-c)

- et éventuellement la réaction d'hydrolyse du groupement

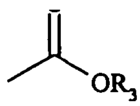

tel que défini ci-dessus, présent dans les composés de formules (IX-1-a), (IX-1-b) ou (IX-1-c) susmentionnées, afin d'obtenir un composé portant une fonctionnalité amine libre et répondant respectivement aux formules (X-1-a), (X-1-b) ou (X-1-c) suivantes :

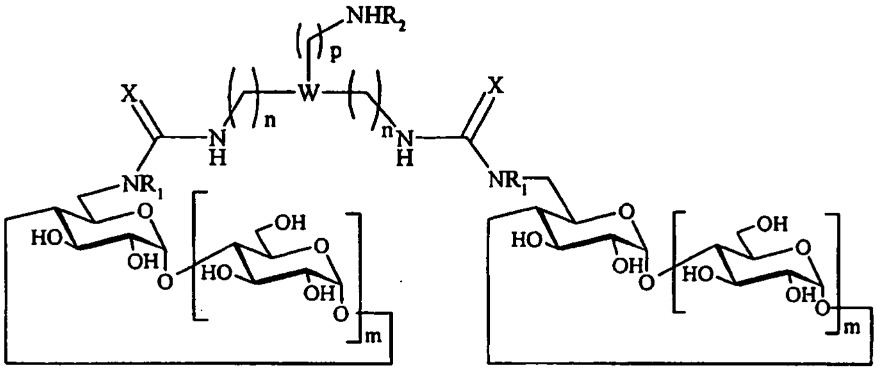

**(X-1-a)**

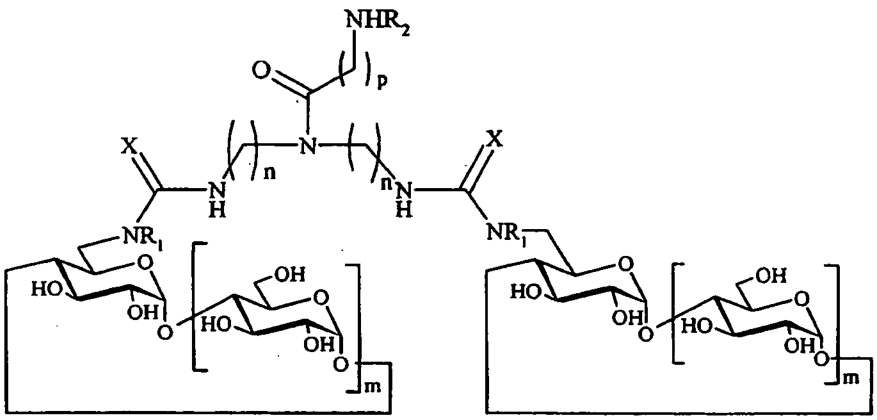

**(X-1-b)**

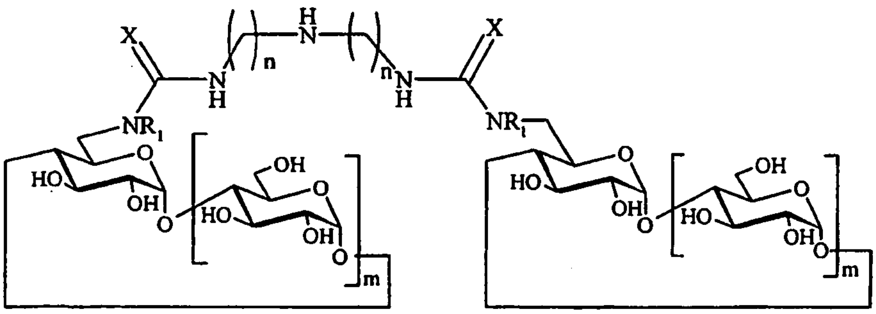

**(X-1-c)**

- et éventuellement la réaction d'un composé de formule (X-1-a), (X-1-b) ou (X-1-c) tel qu'obtenu à l'étape précédente, avec un isocyanate ou un isothiocyanate de formule (XI) suivante :

R-N=C=X'

R et X' étant tels que définis ci-dessus,
afin d'obtenir un composé tel que défini ci-dessus répondant respectivement aux formules (XII-1-a), (XII-1-b) ou (XII-1-c) :

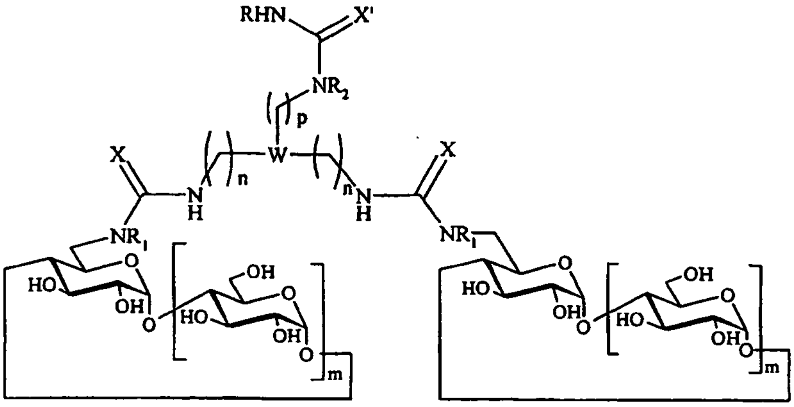

(XII-1-a)

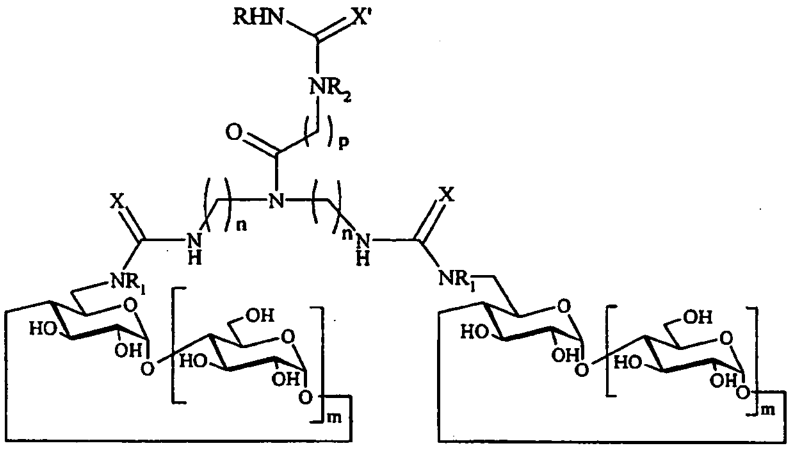

(XII-1-b)

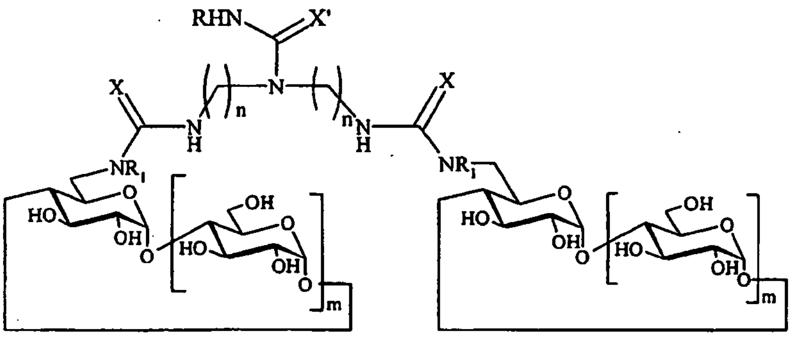

(XII-1-c)

La présente invention concerne également un procédé de préparation d'un composé tel que défini ci-dessus, de formule (I-b), **caractérisé en ce qu**'il comprend les étapes suivantes :

- la réaction d'un composé sélectivement fonctionnalisé en position alcool primaire avec un groupement aminé, de formule (VII-2) suivante :

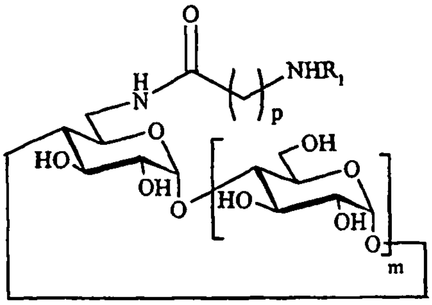

(VII-2)

m, p et $R_1$ étant tels que définis ci-dessus,

avec un élément de dimérisation de type diisocyanate ou diisothiocyanate, notamment portant une fonctionnalité amine protégée sous forme d'un groupe carbamate, de formules (VIII-a), (VIII-b) ou (VIII-c) susmentionnées,

afin d'obtenir un composé tel que défini ci-dessus répondant respectivement aux formules (IX-2-a), (IX-2-b) ou (IX-2-c) :

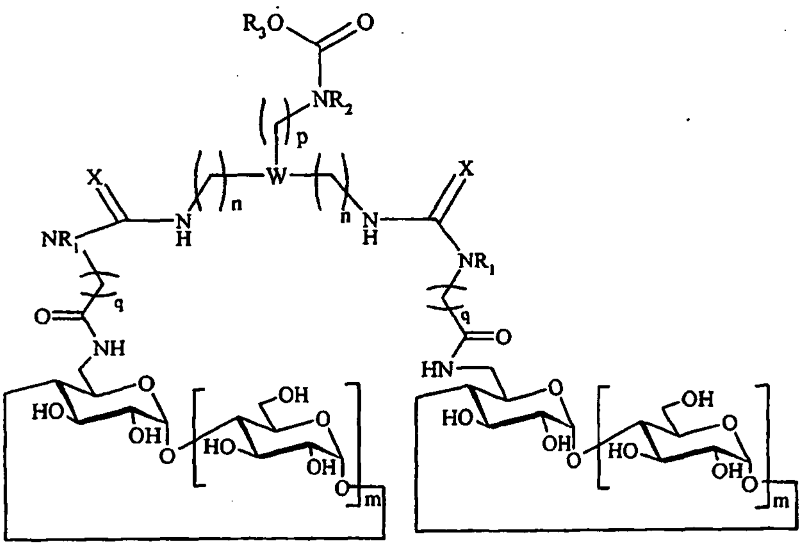

(IX-2-a)

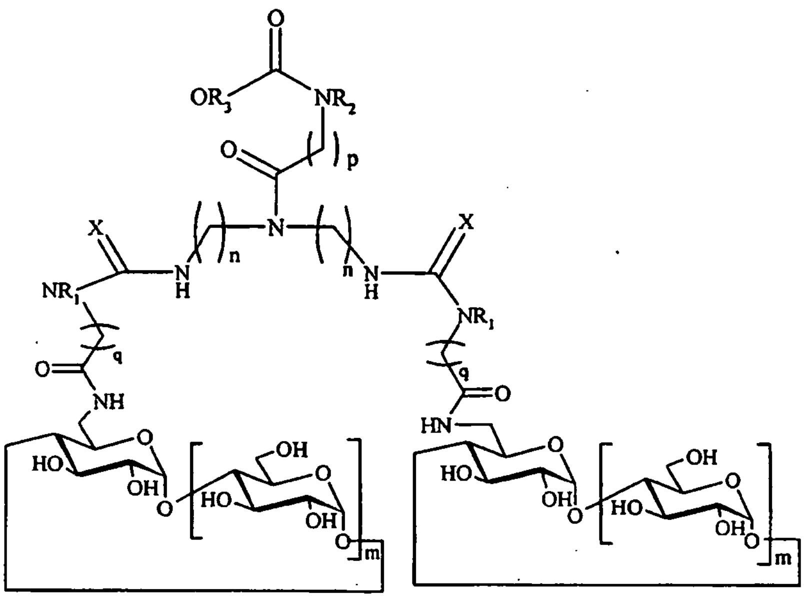

(IX-2-b)

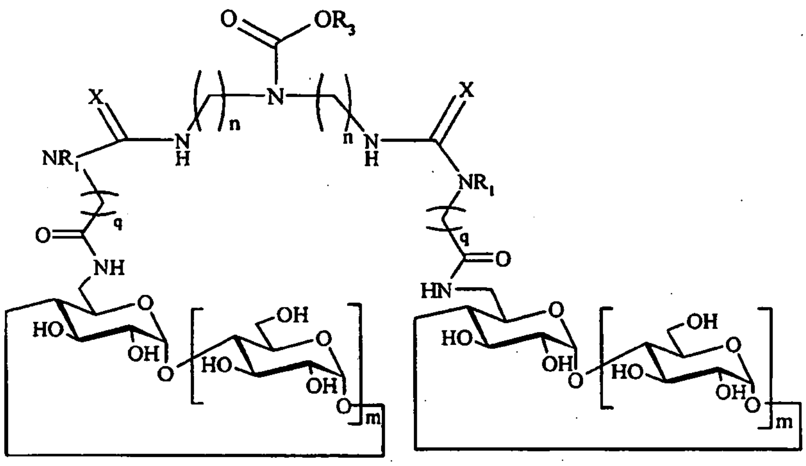

(IX-2-c)

- et éventuellement la réaction d'hydrolyse du groupement

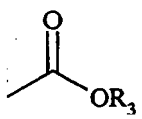

tel que défini ci-dessus, présent dans les composés de formules (IX-2-a), (IX-2-b) ou (IX-2-c) susmentionnées, afin d'obtenir un composé portant une fonctionnalité amine libre et répondant respectivement aux formules (X-2-a), (X-2-b) ou (X-2-c) suivantes :

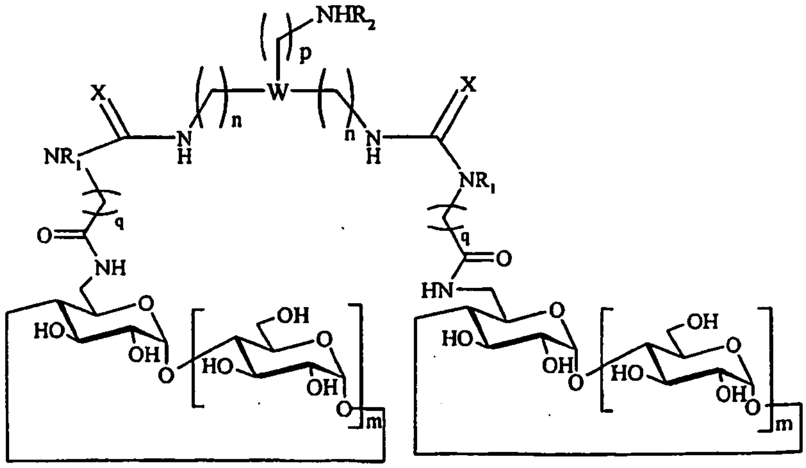

**(X-2-a)**

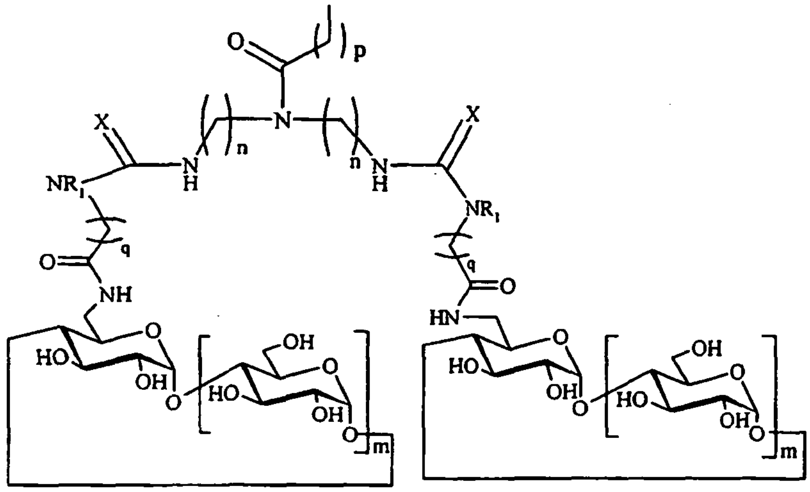

**(X-2-b)**

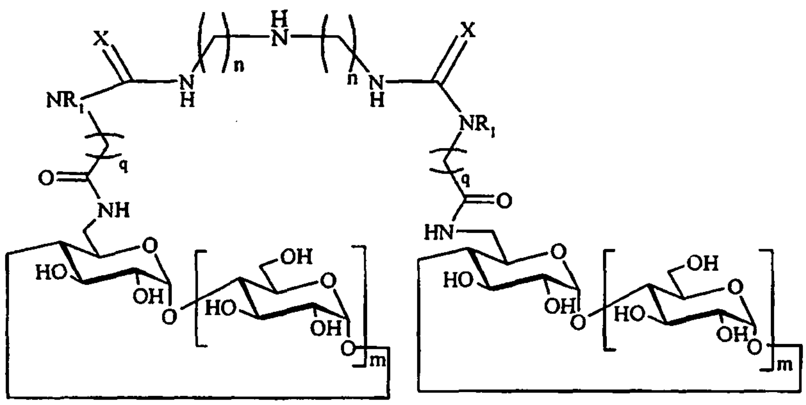

**(X-2-c)**

- et éventuellement la réaction d'un composé de formule (X-2-a), (X-2-b) ou (X-2-c) tel qu'obtenu à l'étape précédente, avec un isocyanate ou un isothiocyanate de formule (XI) suivante :

R-N=C=X'

R et X' étant tels que définis ci-dessus,

afin d'obtenir un composé tel que défini ci-dessus répondant respectivement aux formules (XII-2-a), (XII-2-b) ou (XII-2-c) :

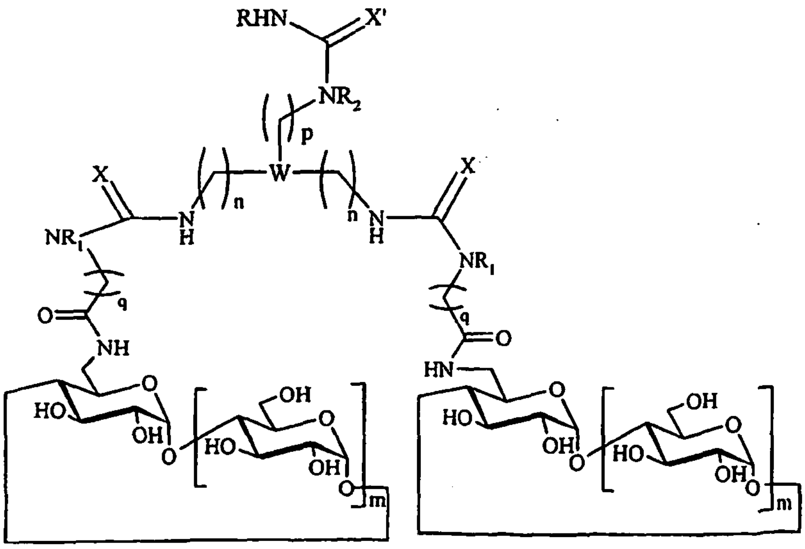

(XII-2-a)

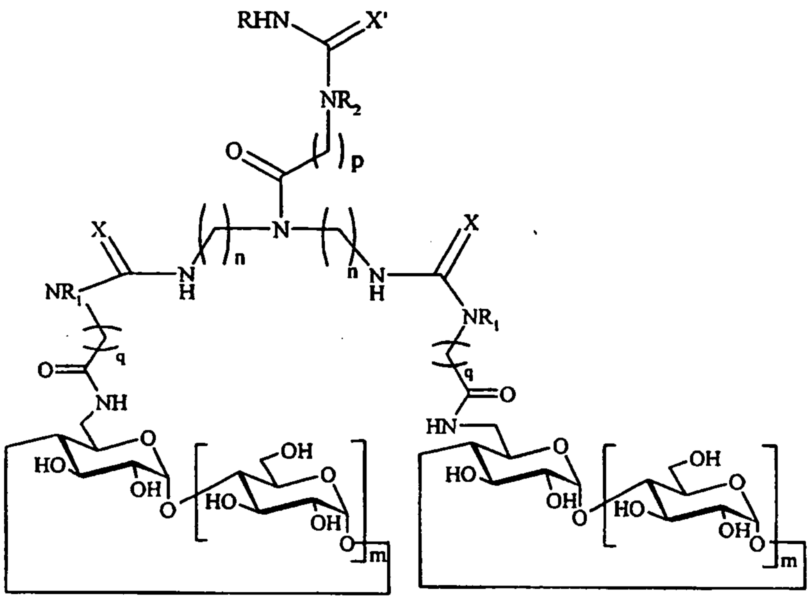

(XII-2-b)

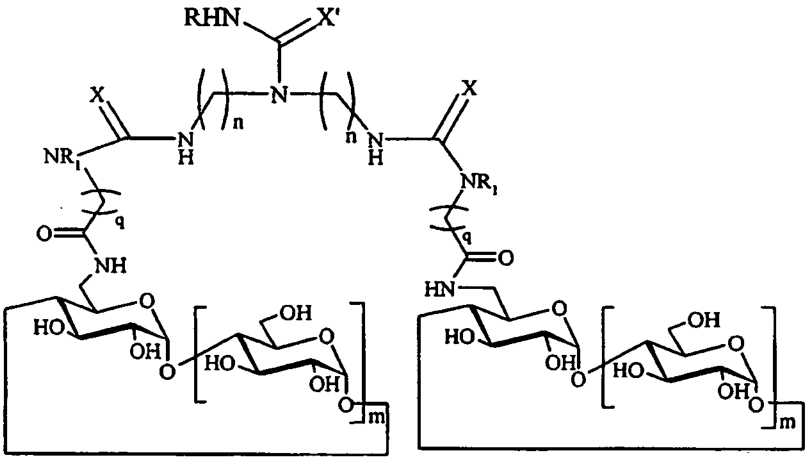

(XII-2-c)

La présente invention concerne également un procédé de préparation d'un composé tel que défini ci-dessus, de formule (I-c), **caractérisé en ce qu**'il comprend les étapes suivantes :

- la réaction d'un composé sélectivement fonctionnalisé en position alcool primaire avec un groupement aminé, de formule (VII-3) suivante :

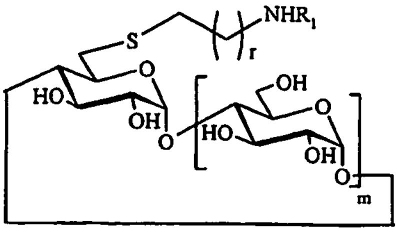

(VII-3)

m, r et $R_1$ étant tels que définis ci-dessus,
avec un élément de dimérisation de type diisocyanate ou diisothiocyanate, notamment portant une fonctionnalité amine protégée sous forme d'un groupe carbamate, de formules (VIII-a), (VIII-b) ou (Vin-c) susmentionnées,
afin d'obtenir un composé tel que défini ci-dessus répondant respectivement aux formules (IX-3-a), (IX-3-b) ou (IX-3-c) :

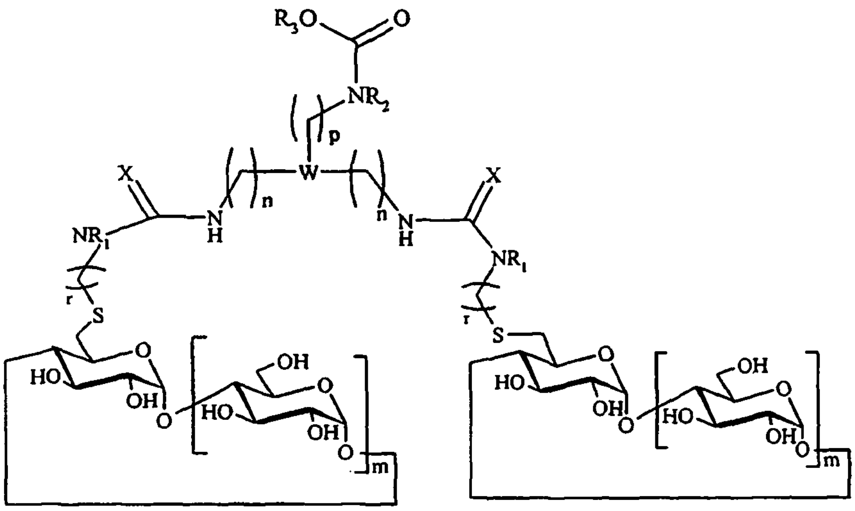

(IX-3-a)

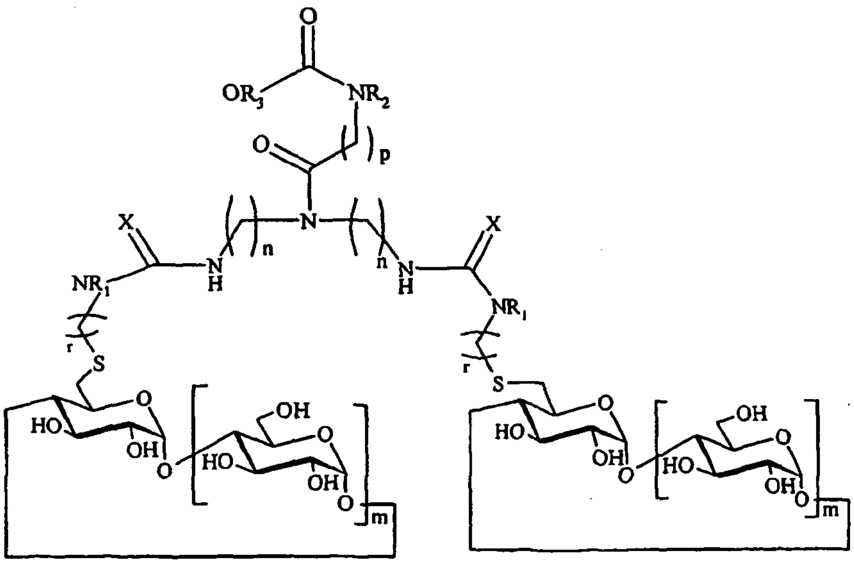

(IX-3-b)

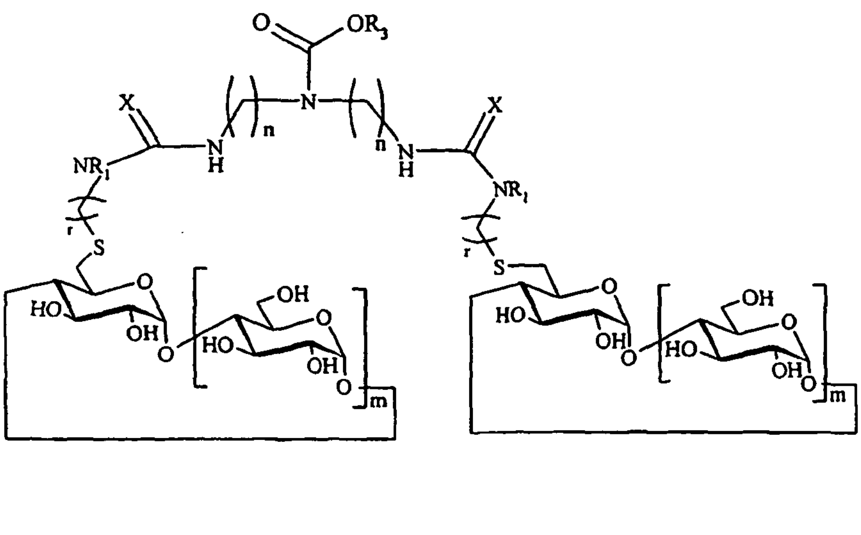

(IX-3-c)

- et éventuellement la réaction d'hydrolyse du groupement

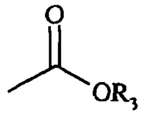

tel que défini ci-dessus, présent dans les composés de formules (IX-3-a), (IX-3-b) ou (IX-3-c) susmentionnées, afin d'obtenir un composé portant une fonctionnalité amine libre et répondant respectivement aux formules (X-3-a), (X-3-b) ou (X-3-c) suivantes :

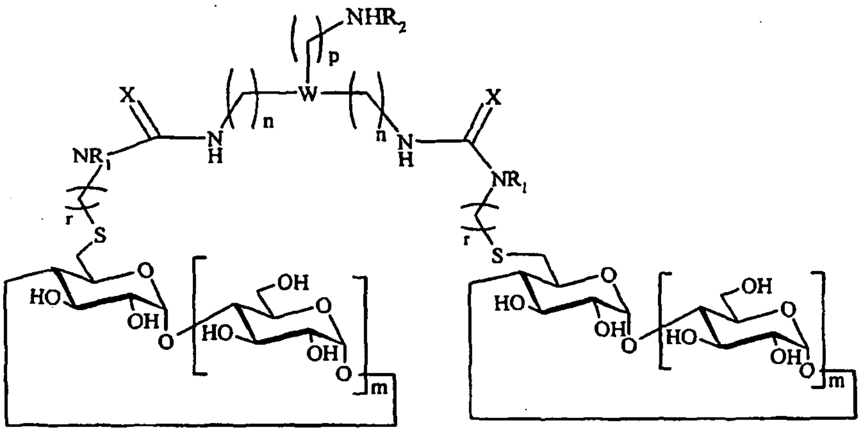

(X-3-a)

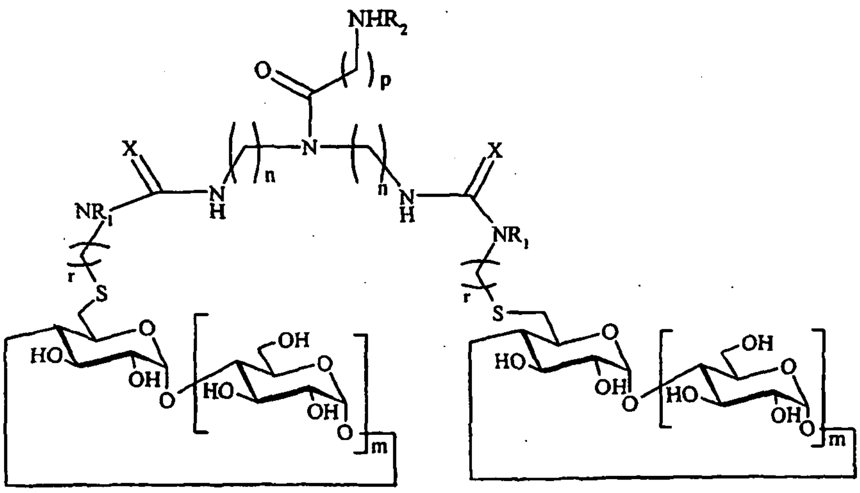

(X-3-b)

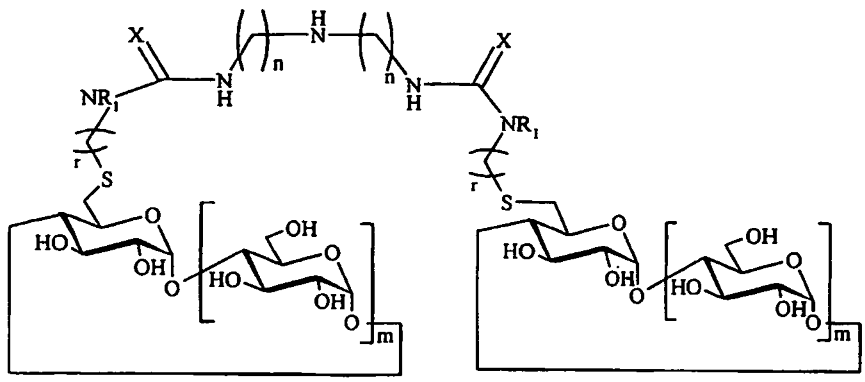

(X-3-c)

- et éventuellement la réaction d'un composé de formule (X-3-a), (X-3-b) ou (X-3-c) tel qu'obtenu à l'étape précédente, avec un isocyanate ou un isothiocyanate de formule (XI) suivante :

R-N=C=X'

R et X' étant tels que définis ci-dessus, afin d'obtenir un composé tel que défini ci-dessus répondant respectivement aux formules (XII-3-a), (XII-3-b) ou (XII-3-c) :

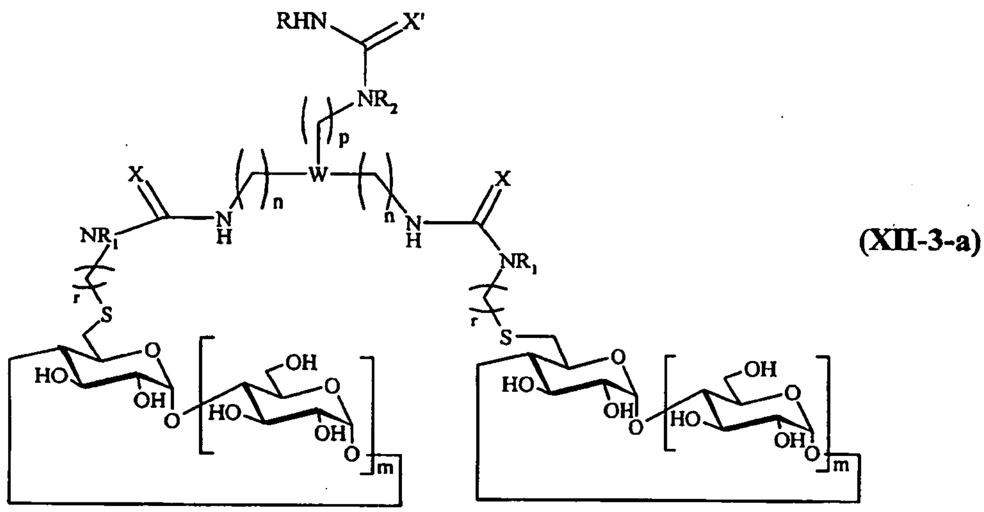

(XII-3-a)

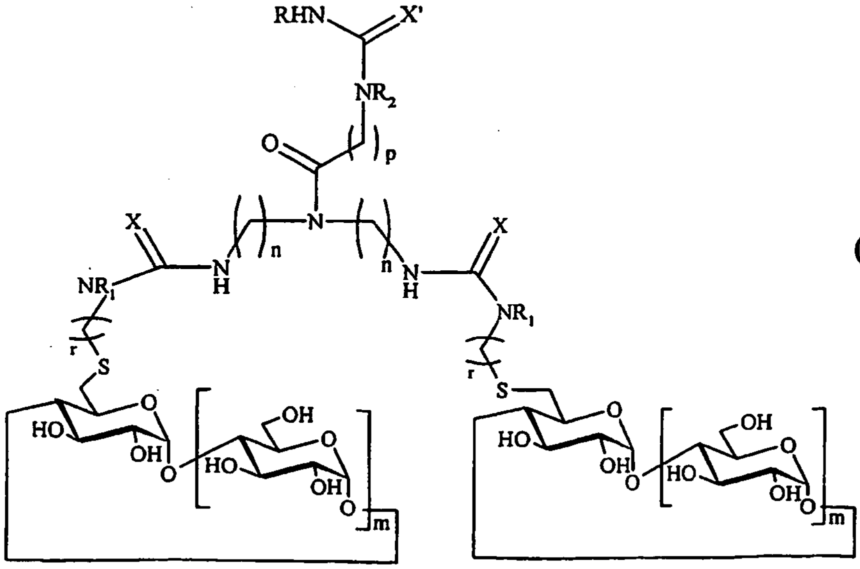

(XII-3-b)

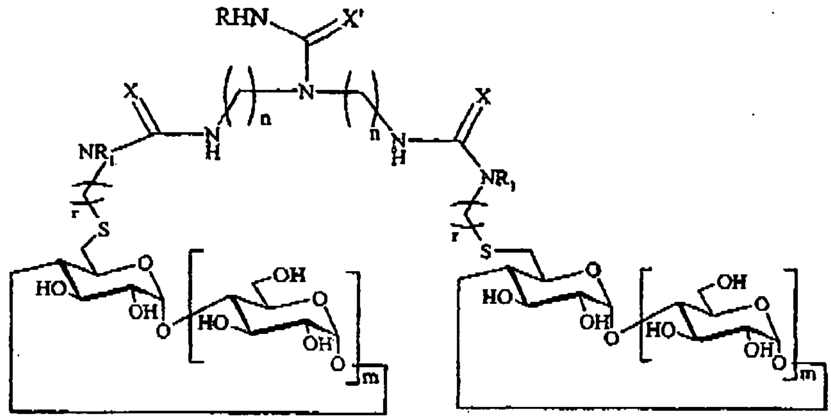

(XII-3-c)

Il est à noter par ailleurs que la méthode de synthèse développée est très flexible en ce qui concerne la nature du bras espaceur entre les positions alcool primaire des deux sous-unités de cyclodextrine, en particulier sa longueur, ce qui permet d'optimiser la géométrie du récepteur de cyclodextrine dimère pour la complexation efficace d'une molécule invitée déterminée. Ce bras espaceur peut être construit notamment à partir du dérivé $6^I$-amino-$6^I$-désoxy de la cyclodextrine correspondante, d'un dérivé incorporant un substituant amide de type $6^I$-(ω-aminoalkanamido)-$6^I$-désoxy, ou encore d'un dérivé incorporant un substituant cystéaminyle ou, plus généralement, d'un substituant dérivé d'un ω-aminoalkanethiol. Ces trois familles de précurseurs sont accessibles d'une façon simple à partir des dérivés des cyclodextrines monofonctionnalisés en position alcool primaire de type $6^I$-O-p- toluènesulfonyle, qui peuvent être préparés en une étape à partir des cyclodextrines commerciales. On peut suivre à ce propos le procédé décrit par J. Defaye et col. dans la demande de brevet internationale WO 99/61483 ou les procédés décrits dans l'article de revue de L. Jicsinszky et col. dans Comprehensive Supramolecular Chemistry, Vol. 3 (Editeurs J. Szejtli et T. Osa), Pergamon, Oxford, 1996, pp. 57-198. La substitution nucléophile du groupement *p*-toluènesulfonate soit par l'anion azure ($N_3^-$), suivie d'une réaction de réduction, ou encore par une amine primaire conduit aux dérivés de cyclodextrines de type $6^I$-amino-$6^I$-désoxy. A titre d'exemple, on peut utiliser pour ces transformations le procédé décrit par I. Baussanne et al. dans le document Chem. Commun, 2000, pp. 1489-1490 ou les procédés décrits dans l'article de revue de L. Jicsinszky et col. dans Comprehensive Supramolecular Chemistry, Vol. 3 (Editeurs J. Szejtli et T. Osa), Pergamon, Oxford, 1996, pp. 57-198, ou encore pour les dérivés alkylés des cyclodextrines le procédé décrit par Jicsinszinsky et al. dans Carbohydr. Polym. 2041, 45, 139-145. L'acylation sélective du groupement amine dans les $6^I$-amino-$6^I$-désoxycyclodextrines par des dérivés d'acides ω-aminoalkanecarboxyliques, le groupement amine terminal étant protégé sous forme d'un carbamate, et la libération du groupe amine en bout de chaîne dans le produit résultant conduit aux dérivés de type $6^I$-(ω-aminoalkanamido)-$6^I$-désoxycyclodextrines. Finalement, les dérivés du type cystéaminyle peuvent être obtenus par

réaction de la $6^I$-désoxy-$6^I$-*p*-toluenesulfonylcyclodextrine correspondante ou bien d'un dérivé de type $6^I$-désoxy-$6^I$-halogènocyclodextrine avec la cystéamine ou, de façon générale, avec un ω-aminoalkanethiol. Eventuellement, le groupement amine dans l'un quelconque des précurseurs susmentionnés peut porter un substituant alkyle tel qu'un groupe méthyle, éthyle, propyle ou butyle.

Le groupement amine dans les dérivés de cyclodextrines utilisés comme précurseurs des cyclodextrines dimères de l'invention, permet d'associer deux sous-unités de cyclodextrine à un élément de dimérisation possédant deux groupements complémentaires, notamment deux groupements isocyanate ou isothiocyanate. La réaction de couplage conduit à un dérivé de cyclodextrine dimère avec deux liaisons urée ou thiourée, qui sont très stables et qui donnent lieu à des structures bien définies. Lorsque le groupement amine dans le dérivé de cyclodextrine précurseur est une amine primaire $NH_2$, les urées ou thiourées obtenues sont *N,N'*-disubstituées, alors que lorsqu'il s'agit d'un groupement amine secondaire de type $NHR_1$, $R_1$ représentant un substituant alkyle, tel que méthyle, éthyle, propyle ou butyle, les urées ou thiourées obtenues sont *N,N',N"*-trisubstituées.

La première et la dernière étape du procédé susmentionné consistent en des réactions de couplage entre un dérivé aminé et un dérivé d'un hétérocummulène de type isocyanate ou isothiocyanate, effectués dans un mélange eau-acétone (2:1 à 1:2) à pH 8-9 (hydrogénocarbonate de sodium à température ambiante pendant 2-24 h). Le rendement est compris entre environ 60 et 90% pour la première étape et entre 50 et 85% pour la dernière étape.

La deuxième étape correspond à la libération d'un groupement amine protégé sous forme de carbamate. De préférence, ce groupement carbamate est un dérivé de type *tert*-butyloxycarbonyle, et sa déprotection est effectuée par hydrolyse acide avec un mélange acide trifluoroacétique-eau 1:1 à température ambiante ou à 40°C pendant 2 heures, le rendement étant quantitatif.

Les dérivés de type uréido- et thiouréido-cyclodextrine dimères de l'invention répondant à la formule (I) dans laquelle Y représente le groupe $NR_1$ (composés de formule (I-a)), peuvent être préparés par un procédé qui consiste à faire réagir un dérivé de $6^I$-amino-$6^I$-désoxycyclodextrine avec un diisocyanate ou un diisothiocyanate de formule (VIII-a), (VIII-b) ou (VIII-c) susmentionnée. Cette réaction peut se faire dans un solvant organique tel que la pyridine ou encore dans un mélange d'eau avec un solvant organique miscible tel que l'acétone. Pour la préparation du dérivé de cyclodextrine sélectivement monofonctionnalisé en position alcool primaire précurseur, on peut utiliser le procédé décrit par I. Baussanne et al. dans le document Chem. Commun, 2000, pp. 1489-1490 ou les procédés décrits dans l'article de revue par L. Jicsinszky et col. dans Comprehensive Supramolecular Chemistry, Vol. 3 (Editeurs J. Szejtli et T. Osa), Pergamon, Oxford, 1996, pp. 57-198.

Les dérivés de type uréido- et thiouréido-cyclodextrine dimères de l'invention répondant à la formule (I) dans laquelle Y répresente le groupe $-NH-CO-(CH_2)_q-NR_1$ (composés de formule (I-b)), peuvent être préparés par un procédé qui consiste à faire réagir un dérivé de $6^I$-(ω-aminoalkyl-$6^I$-désoxycyclodextrine avec un diisocyanate ou un diisothiocyanate de formule (VIII-a), (VIII-b) ou (VIII-c) susmentionnée. Cette réaction peut se faire dans un solvant organique tel que la pyridine ou encore dans un mélange d'eau avec un solvant organique tel que l'acétone. Le dérivé de cyclodextrine sélectivement monofonctionnalisé en position alcool primaire précurseur peut être préparé à partir du dérivé $6^I$-amino-$6^I$-désoxycyclodextrine correspondant par N-acylation sélective avec un dérivé d'acide ω-(*N*-*tert*-butyloxycarbonyl-amino)alkylcarboxylique, tel que le N-*tert*-butyloxycarbonylglycine (Boc-glycine), suivi d'hydrolyse acide du groupement N-Boc par l'acide trifluoroacétique-eau 1:1.

Les dérivés de type uréidocystéaminyl- et thiouréidocystéaminyl-cyclodextrine dimère de l'invention répondant à la formule (I) dans laquelle Y représente le groupe $-S-(CH_2)_r-NR_1$ (composés de formule (I-c)), peuvent être préparés par un procédé qui consiste à faire réagir un dérivé de $6^I$-*S*-(ω-aminoalkyl)-$6^I$-désoxycyclodextrine avec un diisocyanate ou un diisothiocyanate de formule (VIII-a), (VM-b) ou (VIII-c) susmentionnée. Cette réaction peut se faire dans un solvant organique tel que la pyridine ou encore dans un mélange d'eau avec un solvant organique tel que l'acétone.

Le diisocyanate ou diisothiocyanate de formule (VIII-a), (VIII-b) ou (VIII-c) susmentionnée peut être obtenu à partir d'un dérivé de triamine précurseur dans lequel un des groupements amines est sélectivement protégé sous forme de groupement carbamate, de préférence le groupement Boc, par isocyanation ou isothiocyanation des deux groupements amines libres restants.

L'invention concerne aussi des dérivés de type uréido-, thiouréido-, uréidocystéaminyl- et thiouréidocystéaminyl-cyclodextrines dimères répondant à la formule (I) dans laquelle Z représente un substituant comportant un groupement de type uréido ou thiouréido. Le procédé de l'invention consiste à faire réagir un dérivé de cyclodextrine dimère portant un groupement amine répondant à l'une des formules suivantes (X-1-a), (X-1-b) ou (X-1c), (X-2-a), (X-2-b) ou (X-2-c), ou (X-3-a), (X-3-b) ou (X-3-c) susmentionnées, obtenu suivant le procédé indiqué ci-dessus, avec un isocyanate ou isothiocyanate de formule R-NCX' dans laquelle R et X' ont la signification donnée ci-dessus. Cette réaction peut être faite dans un solvant organique tel que la pyridine ou encore dans un mélange d'eau avec un solvant organique tel que l'acétone.

Lorsque R est un groupe dérivé d'un monosaccharide ou d'un oligosaccharide, l'isocyanate de formule R-NCO peut être préparé par réaction d'un dérivé du phosgène, tel que le triphosgène, sur un aminodésoxyglycose ou une glycosylamine ou bien par réaction d'un glycosylisocyanate avec un agent d'oxydation. On peut suivre pour cela les

procédés décrits dans l'article de revue de R. Richter et H. Ulrich dans « The Chemistry of Cyanates and Their Thio Derivatives », Part 2, S. Patai (Ed.), Wiley, Chichester, 1977, pp. 619-818 ou le procédé décrit par Y. Ichikawa et col. dans Synlett, 2000, 1253-1256. Lorsque R est un groupe dérivé d'un monosaccharide ou d'un oligosaccharide, l'isothiocyanate de formule R-NCS peut être préparé par réaction du thiophosgène sur un aminodésoxyglycose ou une glycosylamine. On peut suivre pour cette réaction les procédés décrits par J. M. García Fernández et C. Ortiz Mellet dans Adv. Carbohydr. Chem. Biochem. 1999, 55, pp. 35-135.

Lorsque R comporte un élément de multiplication ramifié dérivé du tris(2-hydroxymethyl)méthylamine (TRIS), on peut préparer l'isocyanate ou l'isothiocyanate correspondant par réaction d'un dérivé du phosgène ou du thiophosgène sur le dérivé aminé portant des substituants glucidiques sur les positions alcool primaire, comme décrit dans le document Chem. Commun., 2000, pp. 1489-1490. Le glycodendron trivalent aminé précurseur peut être obtenu par réaction de glycosidation d'un dérivé du TRIS avec la fonction amine convenablement protégée sous forme de dérivé carbobenzoxy, comme décrit par P. R. Ashton et al. dans J. Org. Chem. 1998, 63, pp. 3429-3437.

Lorsque R comporte un élément de multiplication ramifié dérivé du pentaérythritol, les glycodendrons convenablement fonctionnalisés avec un groupement isothiocyanate peuvent être préparés à partir du pentaérythritol commercial par une séquence de réactions qui implique :

(i) une triallylation sélective par traitement avec le bromure d'allyle, ce qui laisse un seul groupe hydroxyle libre ;
(ii) l'addition radicalaire d'un 1-thiosucre à la double liaison des groupements allyle ; cette réaction peut se faire, soit par activation par la lumière ultraviolette, soit en présence d'un initiateur de radicaux libres tel que l'azobis (isobutyronitrile) ou l'acide *p*-nitroperbenzoïque. A titre d'exemple, on peut adapter les conditions réactionnelles décrites par D. A. Fulton et J. F. Stoddart dans Org. Lett. 2000, 2, pp. 1113-1116 ou par X.-B. Meng et al. dans Carbohydr. Res. 2002, 337, pp. 977-981. Cette réaction permet, notamment, l'addition séquentielle des différentes ramifications glucidiques. On peut ainsi accéder à des glycodendrons homogènes aussi bien qu'hétérogènes dans lesquels les substituants glucidiques répondent aux structures mentionnées ci-dessus. Cette approche permet également l'incorporation à la structure d'un substituant autre qu'un dérivé glucidique, en particulier une sonde de type fluorescent telle qu'un dérivé de la fluorescéine. Les 1-thiosucres précurseurs peuvent être préparés, soit à partir des halogénures de glycosyle correspondants par réaction avec la thiourée suivie d'hydrolyse du sel d'isothiouronium résultant, soit à partir de glycals par addition radicalaire de l'acide thioacétique à la double liaison. On peut suivre pour ces réactions les procédés décrits dans les articles de revue publiés par J. Defaye et J. Gelas dans Studies in Natural Products Chemistry, Vol. 8 (Editeur Atta.ur Rahman), Elsevier, Amsterdam, 1991, pp. 315-357 et par H. Driguez dans Top. Curr. Chem., 1997, 187, pp. 85-116.
(iii) la transformation du groupement alcool primaire restant en groupement isothiocyanate. Cette transformation peut se faire par exemple par transformation du groupe hydroxyle en un bon groupe partant tel que le *p*-toluènesulfonate ou le trifluorométhanesulfonate, suivie de déplacement nucléophile par l'anion azidure et isocyanation ou isothiocyanation de l'azide résultante par réaction avec la triphénylphosphine et le dioxide ou le disulfure de carbone. Pour cette transformation, on peut suivre le procédé décrit dans le document Chem. Commun., 2000, pp. 1489-1490.

Lorsque R comporte un élément de multiplication ramifié dérivé du tris(2-aminoethyl)amine (TREN), les glycodendrons convenablement fonctionnalisés avec un groupement isothiocyanate peuvent être préparés à partir du bis (2-aminoethyl)amine commercial par une séquence de réactions qui implique :

(i) la protection séléctive des deux groupements amine primaire sous forme de dérivés *tert*-butyloxycarbonyl, par réaction avec le 2-(*tert*-butyloxycarbonyloximino)-2-phenylacétonitrile (Boc-ON) dans le tetrahydrofurane (THF) ; cette réaction peut être faite suivant le procédé décrit dans Synthesis, 2002, 2195-2202,
(ii) la réaction de déplacement nucléophile du bis(2-*tert*-butyloxycarbonylaminoéthyl)amine, résultant de l'étape précédente, sur le *p*-toluènesulfonate de 2-azidoéthyle, ce dernier composé étant obtenu à partir du 2-bromoéthanol commercial par réaction avec l'azide de sodium dans la *N,N*-diméthylformamide (DMF), suivie de la réaction du 2-azidoéthanol résultant avec le chlorure de *p*-toluenesulfonyle,
(iii) l'hydrolyse des groupements Boc par l'acide trifluoroacétique-eau 1:1,
(iv) le couplage de la diamine résultant de l'étape précédente avec un isocyanate ou isothiocyanate de formule R-NCX', X' et R ayant la signification indiqué ci-dessus, et
(v) la transformation du groupe azide sur le dérivé résultant de l'étape précédente en groupe isothiocyanate par réaction avec la triphénylphosphine et le dioxide ou le disulfure de carbone, suivant les procédés indiqués ci-dessus.

Il est intéressant de noter que l'utilisation du dérivé du TREN comme élément de multiplication est compatible avec l'incorporation d'autres éléments de multiplication tel que ceux dérivés du TRIS ou du pentaérythritol, ce qui permet, notamment, de construire une architecture de type dendritique. Cette approche permet de multiplier le nombre de substituants et, en conséquence, d'augmenter la multivalence dans le cas des substituants de reconnaissance biologique.

Dans le cas des substituants glucidiques, cela permet notamment d'augmenter l'affinité vis-à-vis d'un récepteur biologique complémentaire (lectine).

Les procédés décrits ci-dessus pour l'obtention des uréido-, thiouréido-, uréidocystéaminyl- et thiouréidocystéaminyl-cyclodextrines de l'invention sont très intéressants car ils permettent d'obtenir les dérivés souhaités en un nombre réduit d'étapes et avec des rendements élevés. En plus, ces procédés permettent d'introduire une très grande diversité moléculaire dans la structure, avec la possibilité d'incorporer différents substituants aussi bien homogènes qu'hétérogènes, ceci étant compatible avec la préparation de librairies de composés non accessibles commodément par d'autres voies. Les dérivés qui incorporent des substituants oligosaccharidiques multivalents sont reconnus de façon très efficace par des lectines membranaires spécifiques, en fonction des substituants glucidiques incorporés.

Les composés de formule (F) susmentionnée peuvent être obtenus à partir d'un précurseur de cyclodextrine de type $6^I$-amino-$6^I$-désoxy et un élément de multiplication dérivé du TREN. D'après des études en modélisation moléculaire, la distance entre les deux cycles de cyclodextrines est particulièrement bien adaptée à la formation de complexes de type sandwich avec les dérives des taxanes, et notamment avec le docétaxel (Taxotère). Cette hypothèse a été confirmée expérimentalement (voir plus loin). Par ailleurs, le fait d'avoir un atome d'azote trisubstitué dans l'élément de dimérisation (c'est-à-dire, W = N dans la formule (I)) au lieu d'un groupement méthyne (W = CH) facilite la caractérisation de la molécule par RMN.

Les composés répondant à la formule (D) susmentionnée peuvent être obtenus aisément par hydrolyse acide du groupement protecteur Boc dans les dérivés de formule (F).

Les composés de formule (E) susmentionnée peuvent être préparés à partir des composés de formule (F) ci-dessus par réaction avec un dérivé d'isothiocyanate, comme décrit plus loin.

La présente invention concerne également un complexe d'inclusion d'un composé tel que défini ci-dessus, de formule (I), avec une molécule pharmacologiquement active, le rapport molaire entre le composé tel que défini ci-dessus et la molécule pharmacologiquement active étant d'environ 10:1 à environ 1:2.

L'expression "complexe d'inclusion" désigne l'entité supramoléculaire formée par le composé selon l'invention (molécule hôte) et la molécule pharmacologiquement active (molécule invitée), dans laquelle la molécule invitée est maintenue dans la cavité hydrophobe de la molécule hôte par l'intermédiaire d'interactions non covalentes.

L'expression "molécule pharmacologiquement active" désigne un principe actif doté d'activité thérapeutique, tel qu'un agent anticoagulant, antidiabétique, antiinflammatoire, antibiotique, antitumoral, etc...

La présente invention concerne également un complexe tel que défini ci-dessus, caractérisé en ce que la molécule pharmacologiquement active est une molécule ditopique, capable d'interagir simultanément, avec deux sous-unités de cyclodextrine, telle qu'une molécule ayant deux cycles aromatiques, comme par exemple un dérivé du Taxol, ou une taille suffisamment grande, comme par exemple un stéroïde.

La présente invention concerne également un complexe tel que défini ci-dessus, caractérisé en ce que la molécule pharmacologiquement active est un agent antitumoral, appartenant notamment à la famille du Taxol.

On peut notamment citer comme agents antitumoraux, ceux de synthèse non biologiques approuvés par la FDA (Food and Drug Administration) tels que :

- des agents alkylants : nitrosourées telles que : lomustine, carmustine et streptozocine ; huiles de moutarde telles que : mechloréthamine, melphalan, uracile moutarde à l'azote, chlorambucile, cyclophosphamide et iphosphamide ; d'autres agents d'alkylation tels que : cisplatine, carboplatine, mitomycine, thiotepane, decarbazine, procarbazine, hexaméthyl mélamine, triéthylène mélamine, bisulfane, pipobromane, et mitotane ;
- des antimétabolites : méthotrexate, trimétrexate, pentostatine, cytarabine, ara-CMP, fludarabine phosphate, hydroxyurée, fluorouracile, floxuridine, chlorodésoxyadénosine, gemcitabine, thioguanine et 6-mercaptopurine ;
- des agents de coupure de l'ADN : bléomycine ; poisons de la topoisomérase I tels que : topotecan, irinotecan, sel de sodium de la camptothécine et des analogues de topotecan et irinotecan ; des poisons de la topoisomérase II tels que les : daunorubicine, doxorubicine, idarubicine, mitoxantrone, téniposide et étoposide ;
- des agents d'intercalation de l'ADN : dactinomycine et mithramycine ;
- des inhibiteurs de la mitose : vinblastine, vincristine, navelbine, paclitaxel et docétaxel.

Un agent antitumoral préféré, appartenant à la famille du Taxol, est le docétaxel (Taxotère®).

On observe, de façon générale, un augmentation importante de la capacité de solubilisation des antitumoraux par les uréido-, thiouréido-, uréidocystéaminyl- et thiouréidocystéaminyl-cyclodextrines dimères de l'invention en comparaison avec les dérivés monomères de l'art antérieur.

On a évalué l'affinité des uréido-, thiouréido-, uréidocystéaminyl- et thiouréidocystéaminyl-cyclodextrines dimères de l'invention qui incorporent des substituants glucidiques pour des lectines membranaires spécifiques *in vitro* suivant le protocole ELLA (de l'anglais "Enzyme-Linked Lectin Assay"). On peut trouver de nombreux exemples d'application de ce protocole dans l'article de revue de J. J. Lundquist et E. J. Toon dans Chem. Rev. 2002, 102, pp. 555-578. Cette technique mesure la capacité d'un ligand mono- ou oligosaccharidique soluble à inhiber l'association entre une

lectine complémentaire et un autre ligand de référence fixé sur une microplaque de support plastique. Ainsi, les uréido-, thiouréido-, uréidocystéaminyl- et thiouréidocystéaminyl-cyclodextrines dimères portant des substituants α-D-mannopyranosyle sont reconnus par la concanavaline A ou par la lectine spécifique du mannose des macrophages, les dérivés avec des substituants β-lactosyle sont reconnus par la lectine spécifique du lactose d'*Arachys hypogaea* ou des hépatocytes, et les dérivés comportant le substituant tétrasaccharidique sialyl-Lewis X sont reconnus par les sélectines de l'endothélium impliquées dans le processus de l'inflammation.

On observe, de façon générale, une augmentation importante de l'affinité des dérivés de type cyclodextrine dimère qui incorporent des substituants glucidiques avec une présentation multivalente vis-à-vis des lectines spécifiques, en comparaison avec les conjugués de type monovalent ou avec les dérivés persubstitués en position alcool primaire de type thiouréido-cyclodextrine décrits dans le document ChemBioChem 2001.

Les uréido-, thiouréido-, uréidocystéaminyl- et thiouréidocystéaminyl-cyclodextrines dimères de l'invention comportant des éléments de reconnaissance cellulaire avec une présentation multivalente sont utilisables en particulier pour le transport vectorisé de substances pharmacologiquement actives vers les récepteurs de membrane complémentaire et aussi pour masquer ces récepteurs de membranes complémentaires. Ainsi, les uréido-, thiouréido-, uréidocystéaminyl- et thiouréidocystéaminyl-cyclodextrines dimères polymannosylés permettent de cibler ou de bloquer le récepteur spécifique du mannose des macrophages, les dérivés polylactosylés le récepteur spécifique du lactose des hépatocytes et les dérivés incorporant des substituants dérivés du tétrasaccharide sialyl Lewis X les sélectines de l'endothélium. Dans ce contexte, ces dérivés sont utilisables en tant que molécules actives pour la prévention et le traitement des processus d'infection et de cancérisation impliquant des phénomènes d'adhésion et, également, pour la prévention et le traitement des maladies liées au dérèglement du processus de l'inflammation.

Ces composés d'inclusion peuvent être préparés par des procédés classiques, par exemple par dispersion de la molécule active en solution ou à l'état pur dans une solution du dérivé de cyclodextrine, en présence ou non d'un cosolvant, comme décrit dans le document WO 97/33919.

Ces complexes d'inclusion peuvent être préparés par exemple en ajoutant à une solution ou à une suspension du composé de l'invention de formule (I) la molécule pharmacologiquement active en solution ou à l'état pur. Le complexe d'inclusion ainsi formé peut être isolé par lyophilisation.

Dans le cas où on ajoute la molécule pharmacologiquement active en solution, par exemple un agent antitumoral de la famille du Taxol, on utilise une solution concentrée de la molécule dans un solvant organique miscible à l'eau, par exemple l'acétone, et on soumet le mélange obtenu à une agitation et à un barbotage de gaz inerte tel que l'azote, pour éliminer le solvant organique.

Dans le cas des composés de la famille du Taxol, tels que le Taxotère®, il est aussi possible de disperser ce produit à l'état pur dans une solution stérile d'un composé selon l'invention.

La présente invention concerne également une composition pharmaceutique comprenant un composé tel que défini ci-dessus, ou un complexe d'inclusion tel que défini ci-dessus, avec un véhicule pharmacologiquement acceptable.

La présente invention concerne une composition pharmaceutique telle que définie ci-dessus, sous forme de solution aqueuse.

La présente invention concerne une composition pharmaceutique telle que définie ci-dessus, caractérisée en ce qu'elle contient par dose unitaire d'environ 50 mg à environ 500 mg de l'un des composés tels que définis ci-dessus, ou en ce qu'elle contient par dose unitaire d'environ 100 mg à environ 750 mg de l'un des complexes tels que définis ci-dessus.

Ces compositions pharmaceutiques, qui peuvent être administrées par voie orale ou parentérale, sont par exemple des solutions, des poudres, des suspensions, etc..., en particulier des solutions injectables.

D'autres caractéristiques et avantages de l'invention apparaîtront mieux à la lecture des exemples suivants donnés bien entendu à titre illustratif et non limitatif.

## PARTIE EXPÉRIMENTALE

### Exemple 1 : Préparation de 2-*tert*-butoxycarbonylaminoéthyl bis[2-[*N*'-(6$^I$-désoxycyclomaltoheptaos-6$^I$-yl) thiouréido]éthyl]amine (composé n° 1).

Ce composé répond à la formule (I) donnée ci-dessus avec A = H, n = n' = 2, m = 6, dans laquelle X représente un atome de soufre, Y représente NH, W représente un atome d'azote et Z représente le groupe $(CH_2)_2$NHBoc (voir aussi la formule (F) donnée ci-dessus avec m = 6). Le composé n° 1 répond donc à la formule suivante :

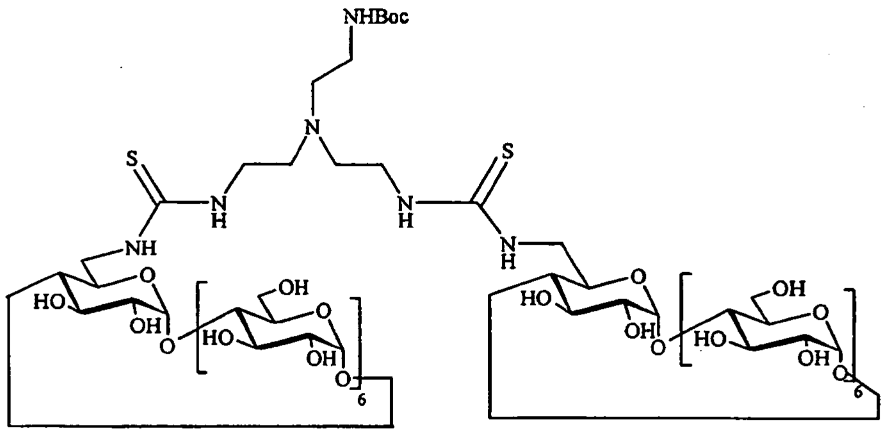

Ce composé est préparé en effectuant les étapes suivantes:

**a) Préparation du bis(2-aminoéthyl) 2-*tert*-butoxycarbonylaminoéthyl amine.**

A une solution agitée de tris(2-aminoéthyl)amine (TREN : $N(CH_2-CH_2-NH_2)_3$) (5 g, 35 mmol) dans le dioxane (30 mL) on ajoute goutte à goutte une solution de carbonate de di-*tert*-butyle (1,52 g ; 7 mmoles) dans le dioxane (30 ml). Le mélange réactionnel est agité à température ambiante pendant 16 h, puis évaporé sous pression réduite. Le résidu et repris dans l'eau (10 mL) et extrait par le dichlorométhane (6 $\times$ 15 mL). L'évaporation du solvant organique conduit au composé bis(2-aminoéthyl) 2-*tert*-butoxycarbonylaminoéthyl amine sous forme d'une huile (1,19 g ; 90%) ayant les caractéristiques suivantes :

- spectre de masse ($FAB^+$) : *m/z* 247 $[100, M + H]^+$ ;
- données de $^1H$ RMN (500 MHz, $CD_3OD$) : $\delta$ 2,69 (t ; 4 H ; $^3J_{H,H}$ 6,3 Hz ; $CH_2NH_2$) ; 2,67 (t ; 2 H ; $^3J_{H,H}$ 6,3 Hz ; $CH_2NHBoc$) ; 2,50 (t ; 6 H ;$CH_2N$) ; 1,43 (s ; 9 H ; $CMe_3$) ;
- données de $^{13}C$ RMN (125,7 MHz ; MeOD) : $\delta$ 158,5 (CO) ; 79,8 ($CMe_3$) ; 58,2 ($NCH_2CH_2NH_2$) ; 55,3 ($NCH_2CH_2NHBoc$) ; 40,2 ($CH_2NH_2$) ; 39,7 ($CH_2NHBoc$) ; 28,9 ($CMe_3$).

**b) Préparation du bis(2-isothiocyanatoéthyl) 2-*tert*-butoxycarbonylamino-éthylamine.**

A un mélange hétérogène de bis(2-aminoéthyl) 2-*tert*-butoxycarbonylaminoéthyl amine (1,19 g ; 5 mmol)(composé tel qu'obtenu à l'étape précédente) et de carbonate de calcium (1,5 g ; 15 mmol ; 3 équiv) dans l'eau-dichlorométhane (1:1, 16 mL), on ajoute le thiophosgène (0,58 mL ; 7,5 mmol ; 1,5 équiv). Le mélange réactionnel est agité a température ambiante pendant 1 h, puis concentré. La phase organique est séparée, séchée par addition de sulfate de magnésium anhydre, filtrée et concentrée. Le résidu résultant est purifié par chromatographie sur colonne de gel de silice avec l'acétate d'éthyle-éther de pétrole 1:3. On obtient ainsi 0,99 g (60 %) du composé bis(2-isothiocyanatoéthyl) 2-*tert*-butoxycarbonylamino-éthylamine sous forme d'une huile incolore ayant les caractéristiques suivantes :

- spectre de masse ($FAB^+$) : *m/z* 353 (100%, $[M + Na]^+$), 331 (80%, $[M + H]^+$)
- donnés de $^1H$ RMN (400 MHz, $CDCl_3$) : $\delta$ 5,06 (bs, 1 H, NHBoc) ; 3,52 (t, 4 H, $^3J_{H,H}$ 6,5 Hz, $CH_2NCS$) ; 3,14 (q, 2 H, $^3J_{H,H}$ 6,5 Hz, $CH_2NHBoc$); 2,83 (t, 4 H, $CH_2CH_2NCS$) ; 2,65 (t, 2 H, $CH_2CH_2NHBoc$); 1,40 (s, 9 H, $CMe_3$)
- donnés de $^{13}C$ RMN (100.6 MHz, $CDCl_3$) : $\delta$ 156,2 (CO) ; 132,8 (NCS) ; 79,4 ($CMe_3$) ; 54,3 ($NCH_2CH_2NCS$) ; 53,8 ($NCH_2CH_2NHBoc$) ; 43,9 ($CH_2NCS$) ; 38,7 ($CH_2NHBoc$) ; 28,4 ($CMe_3$).

**c) Préparation du composé n° 1**

Ce composé est obtenu par réaction de couplage entre le bis(2-isothiocyanatoéthyl) 2-*tert*-butoxycarbonylaminoéthylamine (0,146 g ; 0.44 mmol) et le $6^I$-amino-$6^I$-deoxycyclomaltoheptaose (1 g ; 0,88 mmol) dans la pyridine (8 mL). Le mélange réactionnel est agité à 40 °C pendant 24 heures, puis concentré et le résidu purifié par chromatographie sur colonne de gel de silice avec l'acétonitrile-eau-hydroxyde d'ammonium à 28% 6:3:1. La réaction peut aussi s'effectuer dans l'eau-acétone 1:1 à pH 8-9 (hydrogénocarbonate de sodium solide) à température ambiante pendant 12 heures. On obtient ainsi le composé no. 1 (0.86 g, 75%) sous forme d'un solide blanc ayant les caractéristiques suivantes :

- $[\alpha]_D$ +97,1° (*c* 0,7 ; $H_2O$)
- spectre de masse (MALDITOF$^+$) : *m/z* 2629 (100%, $[M + Na]^+$)
- $^1H$ RMN (500 MHz, $D_2O$, 323 K) : δ 5,26 (d, 2 H, $J_{1,2}$ = 3,5 Hz, H-$1^{II}$) ; 5,24-5,21 (m, 12 H, H-$1^{I, III-VII}$) ; 4,51 (m, 2 H, H-$6a^I$) ; 4,28 (m, 2 H, H-$6a^{II}$) ; 4,18 (m, 2 H, H-$6b^{II}$) ; 4,16 (bt, 2 H, $J_{4,5}$ = $J_{5,6a}$ = 9,5 Hz, H-$5^I$) ; 4,11-4,00 (m, 50 H, H-$3^{I-VII}$, H-$5^{II-VII}$, H-$6a^{II-VII}$, H-$6b^{II-VII}$) ; 3,84-3,72 (m, 30 H, C$H_2$NHCS, H-$2^{I-VII}$, H-$4^{II-VII}$) ; 3,58 (m, 2 H, H-$6b^I$) ; 3,55 (t, 2 H, $J_{3,4}$ = 9,5 Hz, H-$4^1$) ; 3,28, 3,25 (2 m, 2 H, C$H_2$NHBoc) ; 2,95 (m, 4 H, C$H_2$CH$_2$NHCS) ; 2,94, 2,89 (2 m, 2 H, C$H_2$CH$_2$NHBoc) ; 1,68 (s, 9 H, $CMe_3$) ;
- 1D-TOCSY, unités I et II (500 MHz, $D_2O$, 323 K) : δ 5,21 (d, 1 H, $J_{1,2}$ = 3,5 Hz, H-$1^I$) ; 4,08 (t, 2 H, $J_{2,3}$ = $J_{3,4}$ = 10,0 Hz, H-$3^{II}$) ; 4,05 (t, 2 H, $J_{2,3}$ = $J_{3,4}$ 9,5 Hz, H-$3^I$) ; 4,05 (m, 2 H, H-$5^{II}$) ; 3,82 (dd, 2 H, H-$2^I$) ; 3,81 (dd, 2 H, H-$2^{II}$) ; 3,80 (t, 2 H, H-$4^{II}$);
- $^{13}C$ RMN (125,7 MHz, $D_2O$, 323 K) : δ 184,3 (CS) ; 159,9 (CO) ; 105,2-104,6 (C-$1^{I-VII}$); 86,7 (C-$4^I$) ; 84,3-83,6 (C-$4^{II-VII}$) ; 82,9 ($CMe_3$) ; 76,1-75,9 (C-$3^{I-VII}$) ; 75,2-74,7 (C-$2^{I-VII}$, C-$5^{II-VII}$) ; 73,4 (C-$5^I$) ; 63,4-62,8 (C-$6^{II-VII}$) ; 55,8 (*C*H$_2$CH$_2$NHBoc) ; 55,4 (*C*H$_2$CH$_2$NHCS) ; 48,4 (*C*H$_2$NHCS); 45,8 (C-$6^I$) ; 41,6 ($CH_2$NHBoc) ; 31,7 (C*Me*$_3$).

Une méthode alternative pour la préparation du bis(2-isocyanatoéthyl) 2-*tert*-butoxycarbonylaminoéthylamine, précurseur pour la préparation du composé no. 1 et décrit au paragraphe b) consiste en les étapes suivantes :

**- 2-(*tert*-butoxycarbonylamino)éthylamine**

A une solution agitée magnétiquement d'éthylènediamine (2,4 g ; 40 mmol) dans le tétrahydrofurane (30 mL) à 0°C, on ajoute goutte à goutte sur une période d'1 heure une solution de carbonate de di-*tert*-butyle (2,18 g ; 10 mmol ; 0,25 équiv) dans le tétrahydrofurane (30 mL). On poursuit l'agitation du mélange réactionnel pendant 2 heures puis on le concentre sous pression réduite. Le résidu est dissous dans l'eau (10 mL) et extrait par le dichlorométhane (3 × 15 mL) conduisant à 1,45 g (90%) du composé du titre sous forme d'un sirop qui est utilisé directement dans l'étape suivante.
$R_f$ 0,40 (10:1:1 MeCN-eau-$NH_4OH$)
$^1H$ RMN (500 MHz ; MeOD) : δ 3,07 (t, 2 H, $^3J_{H,H}$ 6,3 Hz, C$H_2$NH$_2$) ; 2,65 (t, 2 H, C$H_2$NHBoc) ; 1,41 (s, 9 H, $CMe_3$)
$^{13}C$ RMN (125,7 MHz ; MeOD) : δ 157,2 (CO) ; 78,6 (*C*Me$_3$) ; 42,6 ($CH_2NH_2$); 41,0 (C$H_2$NHBoc) ; 27,3 (C*Me*$_3$).

**- 2-Azidoéthyl *p*-toluènesulfonate**

Une solution de 2-bromoéthanol (5,06 g ; 40,4 mmol) et d'azidure de sodium (3,12 g ; 48 mmol ; 1,2 équiv) dans l'eau (15 mL) est agitée à 60°C pendant 4 heures, extraite par le dichlorométhane (4 × 20 mL), séchée sur sulfate de sodium, filtrée et concentrée (on peut noter que le 2-azidoéthanol est trop volatil pour être séché sous le vide d'une pompe à palette). Le produit brut huileux est dissous dans la pyridine (20 mL) à 0°C et le chlorure de tosyle (7,2 g ; 48 mmol ; 1,2 équiv) est ajouté par petites portions. La solution est amenée à température ambiante et agitée toute la nuit. On ajoute ensuite de l'eau (10 mL) et le produit est extrait par le dichlorométhane (3 × 20 mL), séché sur sulfate de sodium et concentré sous pression réduite pour conduire au composé du titre (7 g ; 29 mmol ; 72%).
$^1H$ RMN (500 MHz, $CDCl_3$) : δ 7,79 ; 7,34 (2 d, 4 H, $^3J_{H,H}$ 8,0 Hz, CH-Ar) ; 4,13 (t, 2 H, $^3J_{H,H}$ 6 Hz, C$H_2$OTs) ; 3,46 (t, 2 H, $CH_2N_3$) ; 2,43 (s, 3 H, Me)
$^{13}C$ RMN (125,7 MHz ; MeOD) : δ 145,2 ; 132,6 ; 130,0 ; 128,0 (Ar) ; 68,0 ($CH_2$OTs) ; 49,6 ($CH_2N_3$) ; 21,6 (Me).

**- 2-(*tert*-Butoxycarbonylamino)éthyl bis(2-azidoéthyl)amine**

Un mélange de 2-(*tert*-butoxycarbonylamino)éthylamine (10 mg ; 0,6 mmol), de 2-azidoéthyl *p*-toluènesulfonate (370 mg ; 1,5 mmol ; 1,25 équiv) et de carbonate de potassium (2,4 mmol, 2 équiv) est porté à reflux dans le nitrométhane pendant 16 heures. La solution est alors concentrée et le produit résiduel est dissous dans le dichlorométhane (10 mL), lavé par l'eau (5 mL) et purifié par chromatographie sur colonne (EtOAc-hexane 1:3) conduisant au produit du titre (130 mg, 70%).
$^1H$ RMN (500 MHz ; $CDCl_3$) : δ 5.06 (bs, 1 H, NH) ; 3,32 (t, 4 H, $^3J_{H,H}$ 6,6 Hz, $CH_2N_3$); 3,18 (q, 2 H, $CH_2$NHBoc) ; 2,72 (t, 4 H, C$H_2$CH$_2$N$_3$) ; 2,64 (t, 2 H, C$H_2$CH$_2$NHBoc) ; 1,44 (s, 9 H, $CMe_3$)
$^{13}C$ RMN (125,7 MHz ; MeOD) : δ 157,2 (CO) ; 75,5 ($CMe_3$) ; 54,5 (*C*H$_2$CH$_2$NHBoc); 54,2 ($CH_2CH_2N_3$) ; 50,0 ($CH_2N_3$) ; 38,9 (*C*H$_2$NHBoc) ; 28,8 (C*Me*$_3$).

**- Bis (2-isothiocyanatoéthyl) 2-tert- butoxycarbonylaminoéthylamine**

A une solution de 2-(*tert*-butoxycarbonylamino)éthyl bis(2-azidoéthyl)amine (130 mg ; 0,44 mmol) dans le dioxane (5 mL), triphénylphosphine (0,48 mmol ; 1,1 équiv) et le sulfure de carbone (4,4 mmol ; 10 équiv) sont ajoutés. Le mélange est agité sous azote à température ambiante pendant 1 jour, la solution est concentrée et le résidu purifié

par chromatographie sur colonne de gel de silice (EtOAc-éther de pétrole 1:3) conduisant au produit du titre (102 mg, 70%) identique à celui décrit au paragraphe b) précédent.

**<u>Exemple 2</u> : Préparation de 2-aminoéthyl bis[2-[*N*'-(6[I]-désoxycyclomalto-heptaos-6[I]-yl)thiouréido]éthyl]amine (composé n° 2).**

Ce composé répond à la formule (I) donnée ci-dessus avec A = H, n = n' = 2, m = 6, dans laquelle X représente un atome de soufre, Y représente NH, W représente un atome d'azote et Z représente le groupe $(CH_2)_2NH_2$ (voir aussi la formule (D) donnée ci-dessus avec m = 6).

Le composé n° 2 répond donc à la formule suivante :

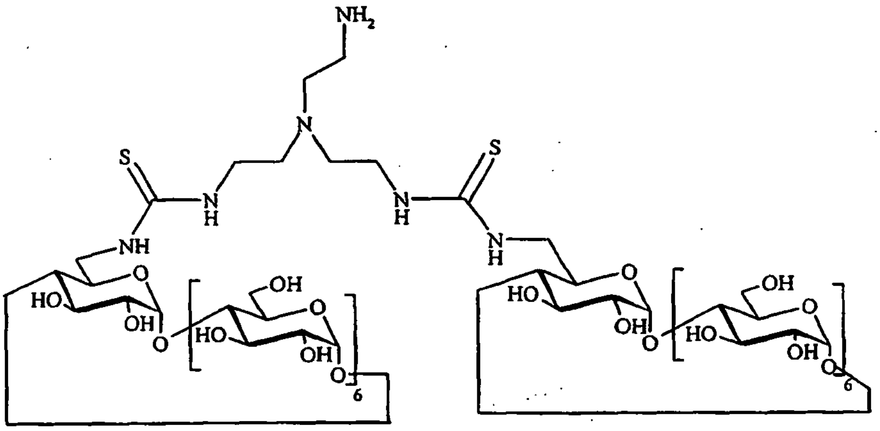

Ce composé est obtenu à partir du composé n° 1 (0,1 g ; 0,14 mmol) par traitement avec l'acide trifluoroacétique-eau (1:1) à 40°C pendant 2 heures. L'évaporation des solvants à pression réduite, la coévaporation des traces d'acide avec de l'eau et la lyophilisation du résidu résultant conduit au composé n° 2 sous forme d'un solide blanc hygroscopique (rendement quantitatif d'après la RMN) ayant les caractéristiques suivantes :

- $[\alpha]_D$ +126,1° (*c* 2,3; $H_2O$)
- spectre de masse (MALDITOF$^+$) : *m*/*z* 2498 (100%, $[M + H]^+$)
- $^1H$ RMN (500 MHz, $D_2O$, 333 K) : δ 5,33-5,31 (m, 14 H, H-$1^{I-VII}$) ; 4,42 (m, 2 H, H-$6a^I$) ; 4,37 (m, 6 H, $CH_2N$) ; 4,27 (bt, 2 H, $J_{4,5} = J_{5,6a}$ 10,0 Hz, H-$5^I$) ; 4,21-4,07 (m, 50 H, H-$3^{I-VII}$, H-$5^{II-VII}$, H-$6a^{II-VII}$, H-$6b^{II-VII}$) ; 3,92-3,89 (m, 18 H, $CH_2$NHCS, H-$2^{I-VII}$) ; 3,83-3,81 (m, 14 H, $CH_2NH_2$, H-$4^{II-VII}$) ; 3,72 (m, 2 H, H-$6b^I$) ; 3,70 (t, 2 H, $J_{3,4}$ = 10,0 Hz, H-$4^I$).
- 1D-TOCSY, unité I et ponts d'éthylène (500 MHz, $D_2O$, 323 K) : δ 5,32 (d, 1 H, $J_{1,2}$ = 3,5 Hz, H-$1^I$) ; 4,19 (t, 2 H, $J_{2,3} = J_{3,4}$ = 10,0 Hz, H-$3^I$) ; 3,92 (dd, 2 H, H-$2^I$) ; 3,94 (m, 4 H, $CH_2$NHCS) ; 3,85 (m, 2 H, $CH_2NH_2$).
- $^{13}C$ RMN (125,7 MHz ; $D_2O$ ; 333 K) : δ 183,1 (CS) ; 102,5-101,8 (C-$1^{I-VII}$) ; 83,6 (C-$4^I$) ; 81,6-81,3 (C-$4^{II-VII}$) ; 73,6-73,3 (C-$3^{I-VII}$) ; 42,6-71,7 (C-$2^{I-VII}$, C-$5^{I-VII}$); 70,4 (C-$5^I$) ; 60,9-60,7 (C-$6^{II-VII}$) ; 54,8 ($CH_2CH_2NH_2$) ; 50,9 ($CH_2CH_2$NHCS) ; 45,8 (C-$6^I$, $CH_2$NHCS) ; 39,6 ($CH_2NH_2$).

Le composé n° 2 est utilisé directement comme précurseur pour la préparation de dérivés de cyclodextrines dimères répondant à la formule (I) donnée ci-dessus avec A = H, n = n' = 2, m = 6, dans laquelle X représente un atome de soufre, Y représente NH, W représente un atome d'azote et Z représente le groupe $-(CH_2)_2$-NH-CX-NHR, R ayant la signification donnée ci-dessus, sans purification additionnelle.

**<u>Exemple 3</u>: Préparation de bis[2-[*N*'-(6[I]-désoxycyclomaltoheptaos-6[I]-yl)thiouréido]éthyl] 2-(*N*'-methylthiouréido)éthylamine (composé n° 3).**

Ce composé répond à la formule (I) donnée ci-dessus avec A = H, n = n' = 2, m = 6, dans laquelle X représente un atome de soufre, Y représente NH, W représente un atome d'azote et Z représente le groupe $-(CH_2)_2$-NH-CS-NHMe (voir aussi formule (E) avec m = 6, dans laquelle R représente le groupe méthyle). Ce composé répond à la formule suivante :

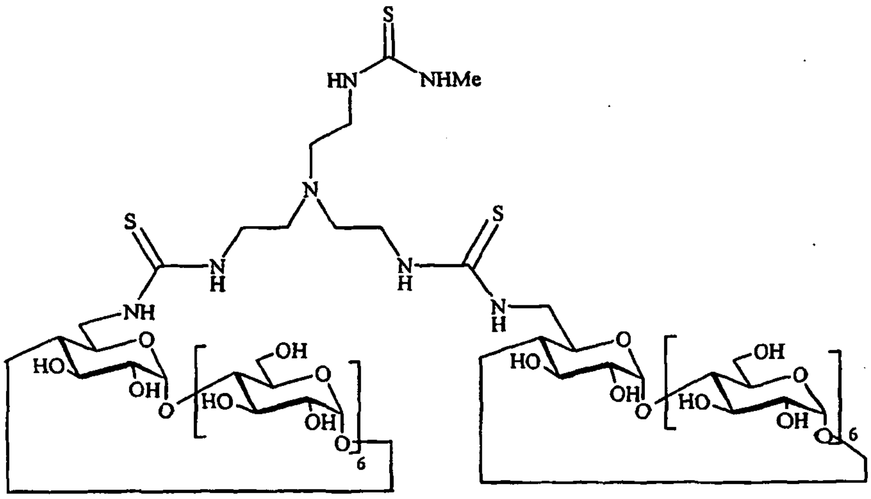

Ce composé est obtenu par la réaction de couplage entre le composé n° 2 (122 mg ; 0,05 mol) et le méthyl isothiocyanate commercial (4 mg ; 0,05 5 mmol) dans l'eau-acetone (1:1 ; 4 mL) à pH 8-9 (hydrogénocarbonate de sodium solide) à température ambiante pendant 12 heures. L'acétone est éliminé par évaporation à pression réduite, la solution aqueuse est alors extraite par le dichlorométhane (2 $\times$ 5 mL) et lyophilisée. Le résidu est repris dans l'eau (5 mL) et déminéralisé par traitement avec la résine échangeuse ionique mixte Duolite MB-6113 ($H^+$, $OH^-$). La résine est filtrée et lavée par l'eau et la solution aqueuse résultant est lyophilisée à nouveau. On obtient ainsi le composé n° 3 (129 mg, 97%) sous forme d'un solide blanc ayant les propriétés suivantes :

- $[\alpha]_D$ +85.4' (*c* 1,0 ; $H_2O$)
- spectre de masse (MALDITOF$^+$) : *m*/*z* 2594 (100%, $[M + Na]^+$)
- solubilité dans l'eau : 325 g·$L^{-1}$ (126 mmol·$L^{-1}$)
- $^1$H RMN (500 MHz, $D_2O$, 353 K): $\delta$ 5,55-5,52 (m, 14 H, H-$^{I-VII}$) ; 4,67 (m, 2 H, H-6a$^I$) ; 4,52 (m, 2 H, H-5$^I$) ; 4,44-4,35 (m, 50 H, H-3$^{I-VII}$, H-5$^{II-VII}$, H-6a$^{II-VII}$, H-6b$^{II-VII}$) ; 4,13-4,10 (m, 14 H, H-2$^{I-VII}$) ; 4,07-4,04 (m, 18 H, H-4$^{II-VII}$, C$H_2$NHCS) ; 3,89 (m, 4 H, H-4$^I$, H-6b$^I$) ; 3,52 ( s, 3 H, MeN) ; 3,32 (m, 6 H, $NCH_2$)
- $^{13}$C RMN (125,7 MHz ; $D_2O$ ; 353 K) : $\delta$ 182,2 (CS) ; 102,7-100,9 (C-1$^{I-VII}$) ; 83,9 (C-4$^I$) ; 81,8-81,4 (C-4$^{II-VII}$) ; 73,8-73,6 (C-3$^{I-VII}$) ; 72,8-72,4 (C-2$^{I-VII}$, C-5$^{II-VII}$) ; 70,6 (C-5$^I$); 61,4-61,0 (C-6$^{II-VII}$) ; 53,4 ($NCH_2$) ; 45,7 (C-6$^I$) ; 42,4 ($CH_2NH$); 31,9 (Me).

**<u>Exemple 4</u>: Préparation du bis[2-[*N'*-(6$^I$-désoxycyclomaltobeptaos-6$^I$-yl)thiouréido]éthyl] 2-(*N'*-phénylthiouréido)éthylamine (composé n° 4).**

Ce composé répond à la formule (I) donnée ci-dessus avec A = H, n = n' = 2, m = 6, dans laquelle X représente un atome de soufre, Y représente NH, W représente un atome d'azote et Z représente le groupe $(CH_2)_2NHC(=S)NHPh$ (voir aussi formule (E) avec m = 6, dans laquelle R représente le groupe phényle). Ce composé répond à la formule suivante :

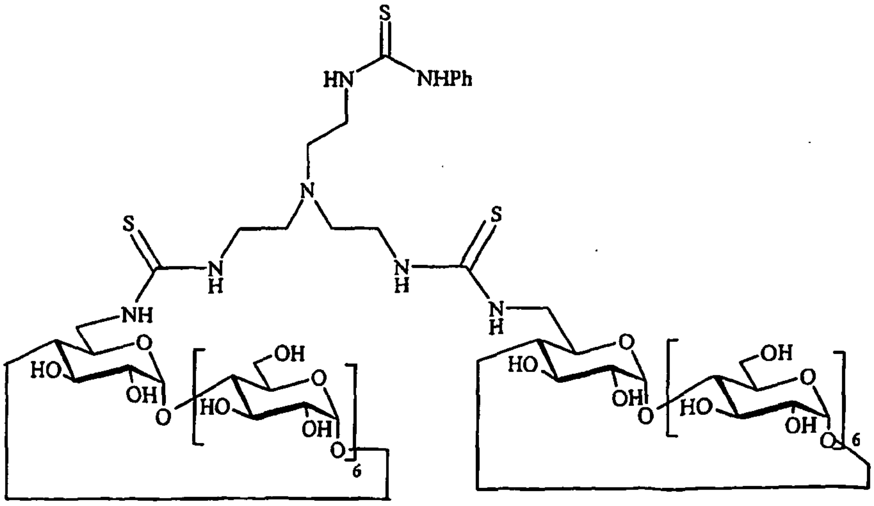

Ce composé est obtenu par réaction de couplage entre le composé n° 2 (122 mg ; 0,05 mmol) et le phényl isothiocyanate commercial (7,4 mg ; 0,055 mmol) dans l'eau-acétone (1:1, 4 mL) à pH 8-9 (hydrogénocarbonate de sodium solide) à température ambiante pendant 12 heures, comme décrit ci-dessus pour la préparation du composé n° 3. Après déminéralisation et lyophilisation, on obtient le composé n° 4 (101,4 mg ; 77%) sous forme d'un solide blanc ayant les propriétés suivantes :

- $[\alpha]_D$ +97,4° (*c* 1,0 ; $H_2O$)
- spectre de masse (MALDITOF$^+$) : *m/z* 2655 (100%, $[M + Na]^+$)
- solubilité dans l'eau : 115 g·L$^{-1}$ (62 mmol·L$^{-1}$)
- $^1$H RMN (500 MHz, $D_2O$, 353 K) : δ 7,40-7,30 (m, 5 H, Ph) ; 5,55-5,52 (m, 14 H, H-1$^{I-VII}$) ; 4,66 (m, 2 H, H-6a$^I$) ; 4,51 (m, 2 H, H-5$^I$) ; 4,44-4,35 (m, 50 H, H-3$^{I-VII}$), H-5$^{II-VII,}$ H-6a$^{II-VII}$, H-6b$^{II-VII}$) ; 4,13-4,10 (m, 14 H, H-2$^{I-VII}$) ; 4,07-4,04 (m, 18 H, H-4$^{II-VII}$, C$H_2$NHCS) ; 3,89 (m, 4 H, H-4$^I$, H-6b$^I$) ; 3,32 (m, 6 H, $NCH_2$) ;
- $^{13}$C RMN (125,7 MHz; $D_2O$; 353 K) : δ 182,2 (CS) ; 138,0 ; 128,9 ; 127,5 ; 127,1 (Ph) ; 102,7-100,9 (C-1$^{I-VII}$); 84,0 (C-4$^I$) ; 81,8-81,4 (C-4$^{II-VII}$) ; 73,8-73,6 (C-3$^{I-VII}$) ; 72,8-72,4 (C-2$^{I-VII}$, C-5$^{II-VII}$) ; 70,5 (C-5$^I$) ; 61,4-61,0 (C-6$^{II-VII}$) ; 53,3 ($NCH_2$); 45,6 (C-6$^I$) ; 42,3 ($CH_2NH$)

**<u>Exemple 5</u> : Préparation de bis[2-[*N'*-(6$^I$-désoxycydomaltoheptaos-6$^I$-yl)thiouréido]éthyl] 2-[*N'*-(α-D-mannopyranosyl)thiouréido]éthylamine (composé n° 5).**

Ce composé répond à la formule (I) donnée ci-dessus avec A = H, n = n' = 2, m = 6, dans laquelle X représente un atome de soufre, Y représente NH, W représente un atome d'azote et Z représente le groupe $(CH_2)_2NHC(=S)NHR$, R répondant à la formule (III) (voir aussi formule (E-c) avec m = 6). Ce composé répond donc à la formule suivante :

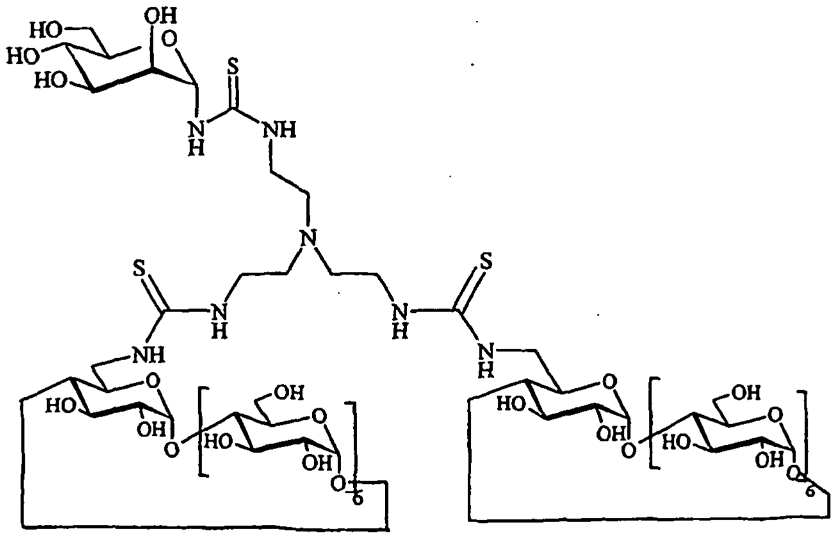

Ce composé est obtenu selon les étapes suivantes :

**a) Préparation du bis[2-[*N'*-(6$^{I}$-désoxycyclomaltoheptaos-6$^{I}$-yl)thiouréido] éthyl] 2-[*N'*-(2,3,4,6-tetra-*O*-acétyl-α-D-mannopyranosyl)thiouréido]éthylamine.**

Ce composé est obtenu par réaction de couplage entre le composé n° 2 (122 mg ; 0,05 mmol) et le 2,3,4,6-tétra-*O*-acétyl-α-D-mannopyranosyl isothiocyanate (19 mg ; 0,05 mmol) dans l'eau-acétone (1:1, 4 mL) à pH 8-9 (hydrogénocarbonate de sodium solide) à température ambiante pendant 4 heures. On élimine l'acétone sous pression réduite, la solution aqueuse est lyophilisée, et le résidu est repris dans l'eau (5 mL), déminéralisé et lyophilisé à nouveau comme décrit ci-dessus pour le composé n° 3. On obtient ainsi le composé du titre (137 mg, 95%) sous forme d'un solide blanc ayant les propriétés suivantes :

- $[\alpha]_D$ +99,6° (c 2,4 ; $H_2O$)
- $^{1}H$ RMN (500 MHz, $D_2O$, 343 K) : δ 6,28 (bs, 1 H, H-1') ; 5,81 (dd, 1 H, $J_{2,3}$ 3,5 Hz, $J_{3,4}$ 8,5 Hz, H-3') ; 5,74 (t, 1 H, $J_{1,2}$ 3,5 Hz, H-2') ; 5,60 (t, 1 H, $J_{4,5}$ 8,5 Hz, H-4') ; 5,44-5,43 (m, 14 H, H-1$^{I-VII}$) ; 4,76 (dd, 1 H, $J_{5,6a}$ 4,5 Hz, $J_{6a,6b}$ 13,0 Hz, H-6a') ; 4,66 (m, 2 H, H-6a$^{I}$) ; 4,55 (m, 2 H, H-6a$^{II}$) ; 4,54 (bd, 1 H, H-6b') ; 4,43 (m, 1 H, H-5') ; 4,42 (m, 4 H, H-5$^{I}$, H-6b$^{II}$) ; 4,38-4,13 (m, 50 H, H-3$^{I-VII}$, H-5$^{II-VII}$, H-6a$^{II-VII}$, H-6b$^{II-VII}$) ; 4,03-4,01 (m, 20 H, C$H_2$NHCS, H-2$^{I-VII}$) ; 4,00-3,93 (m, 12 H, H-4$^{II-VII}$) ; 3,84 (m, 2 H, H-6b$^{I}$) ; 3,77 (t, 2 H, $J_{3,4}$ = 9,5 Hz, H-4$^{I}$) ; 3,31 (m, 6 H, NC$H_2$) ; 2,62 ; 2,53 ; 2,52 ; 2,47 (4 s, 12 H, MeCO)
- $^{13}C$ RMN (125,7 MHz ; $D_2O$ ; 343 K) : δ 183,0 (CS) ; 173,8 ; 172,9 (CO) ; 102,5-101,9 (C-1$^{I-VII}$) ; 83,7 (C-4$^{I}$) ; 81,6-81,2 (C-4$^{II-VII}$, C-1') ; 73,6-73,4 (C-3$^{I-VII}$) ; 72,6-71,7 (C-2$^{I-VII}$, C-5$^{II-VII}$) ; 70,3 (C-5$^{I}$, C-5') ; 69,8-69,6 (C-2', C-3') ; 67,1 (C-4') ; 60,9-60,6 (C-6$^{II-VII}$) ; 60,3 (C-6') ; 53,2 ($NCH_2$) ; 46,0 (C-6$^{I}$) ; 42,0 ($CH_2$NHCS) ; 20,4 ; 20,2 ; 20,1 (MeCO).

**b) Préparation du composé n° 5**

Une solution de bis[2-[*N'*-(6$^{I}$-désoxycyclomaltoheptaos-6$^{I}$-yl)thiouréido]ethyl] 2-[*N'*-(2,3,4,6-tétra-*O*-acétyl-α-D-mannopyranosyl)thiouréido]éthylamine (50 mg, 17 μmol) dans le méthanol sec (5 mL) est additionnée d'une solution 1 N de méthylate de sodium dans le méthanol (70 mL ; 0,5 équiv) et la solution est agitée à 0°C pendant 10 minutes. On observe la précipitation d'un solide blanc qui est redissous par addition d'eau (0,5 mL). La solution aqueuse est agitée à 0°C pendant 10 minutes, neutralisée et déminéralisée par traitement, successivement, avec la résine échangeuse acide Amberlite IR-120 ($H^+$) et la résine mixte Duolite MB-6331 ($H^+$, $OH^-$), filtrée et lyophilisée. On obtient ainsi le composé n° 5 (46,2 mg ; 100%) sous forme d'un solide blanc ayant les caractéristiques suivantes :

- $[\alpha]_D$ +113,0° (*c* 1,0 ; $H_2O$)
- spectre de masse (MALDITOF$^+$) : *m/z* 2742 (100%, $[M + Na]^+$)
- solubilité dans l'eau: 375 $g{\cdot}L^{-1}$(138 $mmol{\cdot}L^{-1}$)
- $^{13}C$ RMN (125,7 MHz; $D_2O$; 333 K) : δ 183,8 ; 183,2 (CS) ; 102,6-101,9 (C-1$^{I-VII}$) ; 83,8 (C-4$^{I}$) ; 82,3-81,3 (C-4$^{II-VII}$) ; 77,9 (C-1') ; 74,0-73,3 (C-3$^{I-VII}$) ;73,0-72,3 (C-2$^{I-VII}$, C-5$^{II-VII}$) ; 70,9 (C-5$^{I}$, C-5') ; 70,7, 70,4 (C-3', C-2') ; 67,1 (C-4') ;

61,6 (C-6') ; 61,4-60,6 (C-$6^{II-VII}$) ; 55,0 ; 54,7 ($CH_2N$) ; 46,2 (C-$6^{I}$) ; 40,0 ($CH_2NHCS$).

**Exemple 6: Préparation du bis[2-[*N'*-($6^{I}$-désoxycyclomaltoheptaos-$6^{I}$-yl)thiouréido]éthyl] 2-[*N'*-(tris(α-D-mannopyranosyloxy-méthyl)méthyl]thiouréido]éthylamine (composé n° 6).**

Ce composé répond à la formule (I) donné ci-dessus avec A = H, n = n' = 2, m = 6, dans laquelle X représente un atome de soufre, Y représente NH, W représente un atome d'azote et Z représente le groupe $(CH_2)_2NHC(=S)NHR$, R répondant à la formule (IV) telle que définie précédemment (voir aussi formule (E-h) avec m = 6). Ce composé répond donc à la formule suivante :

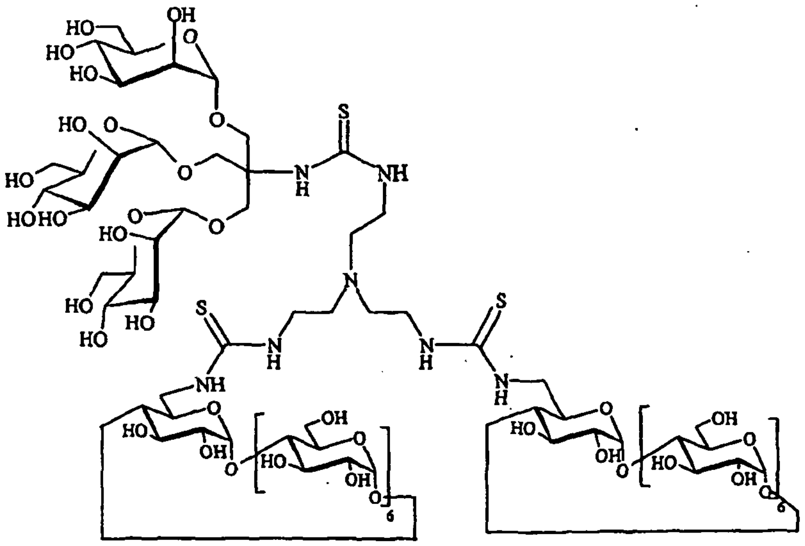

Ce composé est obtenu en effectuant les étapes suivantes :

**a) Préparation de bis[2-[*N'*-($6^{I}$-désoxycyclomaltoheptaos-$6^{I}$-yl)thiouréido] éthyl] 2-[*N'*-[tris(2,3,4,6-tetra-*O*-acétyl-α-D-mannopyranosyloxyméthyl)méthyl] thiouréido]éthylamine.**

Ce composé est obtenu par la réaction de couplage entre le composé n° 2 (122 mg ; 0,05 mmol) et le tris (2,3,4,6-tétra-*O*-acétyl-α-D-mannopyranosyloxyméthyl)méthyl isothiocyanate (57,7 mg; 0,05 mmol) dans l'eau-acétone (1:1, 4 mL) à pH 8-9 (hydrogénocarbonate de sodium solide) à température ambiante pendant 16 heures. On élimine l'acétone sous pression réduite, la solution aqueuse est lyophilisée, et le résidu est repris dans l'eau (5 mL), déminéralisé et lyophilisé à nouveau comme décrit ci-dessus pour le composé n° 3. On obtient ainsi le composé du titre (155 mg, 85%) sous forme d'un solide blanc ayant les propriétés suivantes :

- $[\alpha]_D$ +55.1° (*c* 1.0, $H_2O$)
- $^{1}H$ RMN (500 MHz, $D_2O$, 353 K) : δ 5,80-5,74 (m, 9 H, H-2', H-3', H-4') ; 5,55-5,52 (m, 14 H, H-$1^{I-VII}$) ; 5,50 (bs, 3 H, H-1') ; 4,85 (bd, 3 H, $J_{6a,6b}$ 12,0 Hz, H-6a') ; 4,71 (bd, 3 H, H-6b') ; 4,65 (m, 5 H, H-5') ; 4,67 (m, 2 H, H-$6a^{I}$); 4,52 (m, 2 H, H-$5^{I}$) ; 4,44-4,35 (m, 56 H, H-$3^{I-VII}$, H-$5^{II-VII}$. H-$6a^{II-VII}$, H-$6b^{II-VII}$, $OCH_2$) ; 4,13-4,10 (m, 14 H, H-$2^{I-VII}$) ; 4,07-4,04 (m, 18 H, H-$4^{II-VII}$, C$H_2$NHCS) ; 3,89 (m, 4 H, H-$4^{I}$, H-$6b^{I}$) ; 3,32 (m, 6 H, $NCH_2$) ; 2,65 ; 2,62 ; 2,57 ; 2,52 (4 s, 36 H, MeCO) ;
- $^{13}C$ RMN (125.7 MHz, $D_2O$, 353 K) : δ 182,2 (CS) ; 173,9 ; 173,0 (CO) ; 102,7-100,9 (C-$1^{I-VII}$) ; 98,4 (C-1') ; 83,9 (C-$4^{I}$) ; 81,8-81,4 (C-$4^{II-VII}$) ; 73,8-73,6 (C-$3^{I-VII}$) ; 72,8-72,4 (C-$2^{I-VII}$, C-$5^{II-VII}$) ; 70,6 (C-$5^{I}$) ; 70,3 (C-2') ; 70,0 (C-3') ; 69,4 (C-5') ; 66,8 (C-4', $OCH_2$) ; 62,9 (C-6') ; 61,4-61,0 (C-$6^{II-VII}$) ; 53,4 ($NCH_2$) ; 45,7 (C-$6^{I}$) ; 42,4 ($CH_2NH$) ; 20,7 (Me).

**b) Préparation du composé n° 6**

Une solution de bis[2-[*N'*-($6^{I}$-désoxycyclomaltoheptaos-$6^{I}$-yl)thiouréido]éthyl] 2-[*N'*-[tris(2,3,4,6-tétra-*O*-acétyl-α-D-mannoppanosyloxyméthyl)méthyl]thiouréido]éthyl amine (80 mg, 22 μmol) dans le méthanol sec (5 mL) est additionnée d'une solution 1 N de méthylate de sodium dans le méthanol (70 mL ; 0,5 équiv) et la solution est agitée à

température ambiante pendant 1 heure. On observe la précipitation d'un solide blanc qui est redissous dans l'eau (0,5 mL). La solution aqueuse est agitée à 0°C pendant 30 minutes, neutralisée et déminéralisée par traitement, successivement, avec la résine échangeuse acide Amberlite IR-120 ($H^+$) et la résine mixte Duolite MB-6331 ($H^+$, $OH^-$), comme indiqué ci-dessus pour la préparation du composé n° 5, filtrée et lyophilisée. On obtient ainsi le composé n° 6 (68,5 mg ; rendement 99%) en forme d'un solide blanc ayant les caractéristiques suivantes :

- $[\alpha]_D$ +95,2° (*c* 1,0 ; $H_2O$)
- solubilité dans l'eau : 400 $g{\cdot}L^{-1}$ (127 $mmol{\cdot}L^{-1}$)
- spectre de masse (MALDITOF$^+$) : *m/z* 3170 (100%, $[M + Na]^+$)
- $^1H$ RMN (500 MHz, $D_2O$, 353 K) : δ 5,54 (m, 14 H, H-$1^{I-VII}$) ; 5,35 (m, 9 H, H-2', H-3', H-4') ; 5,31 (bs, 3 H, H-1') ; 4,61 (d, 3 H, $^2J_{H,H}$ 10,0 Hz, $OCH_2$a) ; 4,50-4,32 (m, 63 H, H-$3^{I-VII}$, H-$5^{I-VII}$, H-$6a^{I-VII}$, H-$6b^{II-VII}$, $CH_2N$, $OCH_2$b) ; 4,14-4,10 (m, 14 H, H-$2^{I-VII}$) ; 4,10-4,03 (m, 18 H, H-$4^{II-VII}$, $CH_2NH$) ; 3,95 (m, 4 H, H-$4^I$, H-$6b^I$)
- $^{13}C$ RMN (125,7 MHz, $D_2O$, 353 K) : δ 183,8 (CS) ; 103,7-102,0 (C-$1^{I-VII}$); 101,2 (C-1') ; 83,3 (C-$4^I$) ; 81,9-81,6 (C-$4^{II-VII}$) ; 74,2-73,5 (C-$3^{I-II}$) ; 72,9-72,1 (C-$2^{I-VII}$, C-$5^{II-VII}$) ; 71,6 (C-$5^I$) ; 70,9-70,5 (C-3', C-2', C-5') ; 67,5 (C-4',$OCH_2$) ; 61,7 (C-6') ; 61,2-60,9 (C-$6I^{I-VII}$) ; 55,1 ($CH_2N$) ; 46,0 (C-$6^I$) ; 39,9 ($CH_2NHCS$).

**<u>Exemple 7</u>: Préparation de bis[2-[*N'*-($6^I$-désoxycyclomaltoheptaos-$6^I$-yl)thiouréido]éthyl] 2-[*N'*-[2-[bis[2-[*N'*-(α-D-mannopyranosyl)thiouréido]éthyl] amino]éthyl]thiouréido]éthyl amine (composé n° 7).**

Ce composé répond à la formule (I) donné ci-dessus avec A = H, n = n' = 2, m = 6, dans laquelle X représente un atome de soufre, Y représente NH, W représente un atome d'azote et Z représente le groupe $(CH_2)_2NHC(=S)NH(CH_2)_2N[(CH_2)_2\ NHC(=S)NHR']_2$, R' répondant à la formule (III) donnée ci-dessus (voir aussi formule (H-a) avec m = 6). Ce composé répond donc à la formule suivante :

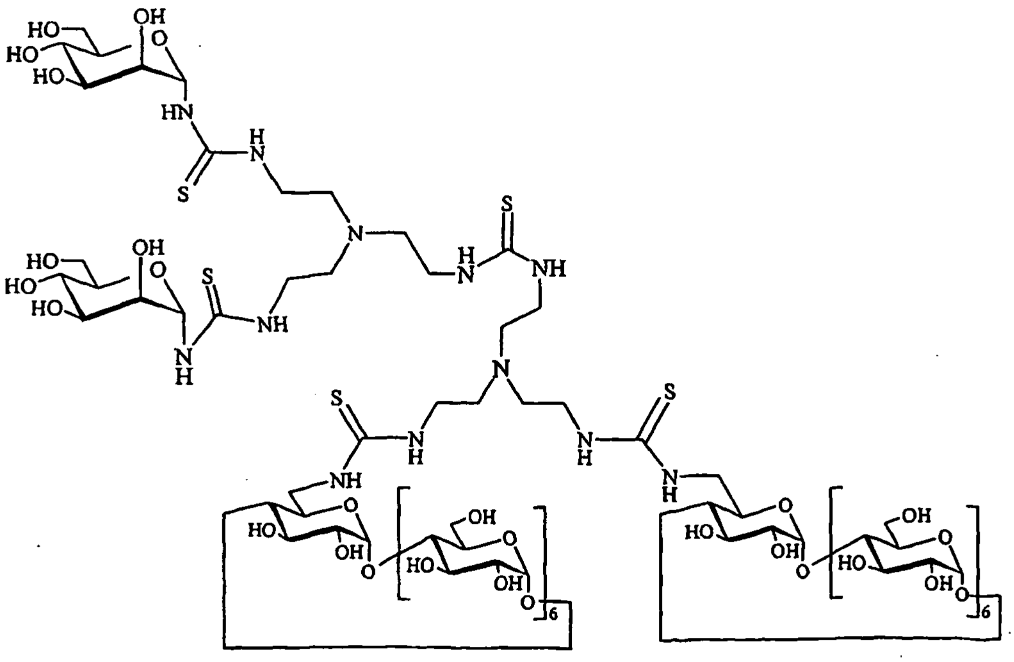

Ce composé est obtenu en effectuant les étapes suivantes :

**a) Préparation de bis(2-*tert*-butoxycarbonylaminoéthyl)amine**

Une solution de diéthylenetriamine (0,42 g ; 4,1 mmol) dans le tétrahydrofurane (THF ; 5 mL) est agité à 0°C sous azote pendant 20 minutes. Une solution de 2-(*tert*-butoxycarbonyloximino)-2-phénylacétonitrile (Boc-ON ; 2,04 g; 8.2 mmol) dans le THF (5mL) est alors ajoutée goutte à goutte et le mélange réactionnel est agité à 0°C pendant 1 heure. On élimine le solvant à pression réduite et le résidu résultant est purifié par chromatographie sur colonne de gel de silice avec le méthanol-hydroxyde d'ammonium 100:3. On obtient ainsi 1,2 g (rendement 96%) d'une huile ayant les caractéristiques suivantes :

- spectre de masse ($CI^+$) : *m*/*z* 304 $[M + H]^+$
- $^1H$ RMN (500 MHz, $CDCl_3$, 313 K) : δ 5,04 (bs, 2 H, NHBoc) ; 4,56 (bs, 1 H, NH) ; 3,19 (q, 4 H, $^3J_{H,H}$ 5.7 Hz, $CH_2NHBoc$) ; 2,71 (t, 4 H, $CH_2NH$) ; 1,41 (s, 18 H, $CMe_3$)
- $^{13}C$ RMN (125,7 MHz ; $CDCl_3$, 313 K) : δ 156,2 (CO) ; 79,2 ($CMe_3$) ; 48,7 ($CH_2NH$) ; 40,2 ($CH_2NHBoc$) ; 28,3 ($CMe_3$).

**b) Préparation du tosylate de 2-azidoéthyle.**

Un mélange de 2-bromoéthanol (3 g, 24 mmol) et d'azidure de sodium (3,12 g ; 48 mmol) dans l'acétonitrile (10 mL) est agité à reflux pendant 24 heures, puis filtré et le solvant est éliminé sous pression réduite. Le résidu est repris dans la pyridine (10 mL) et la solution résultante est additionnée de chlorure de tosyle (4,57 g ; 24 mmol) et agitée à température ambiante pendant 2 heures. On ajoute le dichlorométhane (40 mL) et la phase organique est lavée par une solution 2 N d'acide sulfurique (3 × 40 mL), séchée (sulfate de sodium anhydre) et concentrée. On obtient ainsi 4,63 g (rendement 80%) d'une huile ayant les caractéristiques suivantes :

- spectre de masse ($CI^+$) : *m*/*z* 242 $[M + H]^+$
- $^1H$ RMN (300 MHz, $CDCl_3$) : δ 7,81 ; 7,35 (2 d, 4 H, $^3J_{H,H}$ 8,3 Hz, Ph) ; 4,15 (t, 2 H, $^3J_{H,H}$ 5,0 Hz, $CH_2OTs$) ; 3,48 (t, 2 H, $CH_2N_3$)
- $^{13}C$ RMN (75,5 MHz ; $CDCl_3$) : δ 145.1 ; 132,4; 129,8; 127,8 (Ph) ; 67,9 ($CH_2OTs$) ; 49,4 ($CH_2N_3$) ; 21,5 (Me).

**c) Préparation de 2-azidoéthyl bis(2-*tert*-butoxycarbonylaminoéthyl)] amine.**

A une solution de bis(2-*tert*-butoxycarbonylaminoéthyl)amine (0,2 g ; 0,66 mmol) dans la *N*,*N*-diméthylformamide (2 mL) on ajoute le tosylate de 2-azidoéthyle (0,24 g ; 1,5 équiv ; 1 mmol). Le mélange réactionnel est agité à 60°C pendant une nuit, puis concentré à pression réduite, le résidu est repris dans le dichlorométhane (5 mL), la phase organique est lavée par l'eau (2 × 5 mL), séchée sur sulfate de sodium anhydre et concentrée. Le résidu est purifié par chromatographie sur colonne de gel de silice avec l'acétate d'éthyle-éther de pétrole. On obtient ainsi 0,125 g (rendement 70%) d'une huile ayant les caractéristiques suivantes :

- spectre de masse ($CI^+$) : *m*/*z* 373 $[M + H]^+$
- $^1H$ RMN (500 MHz, $CDCl_3$) : δ 5,05 (bs, 2 H, N*H*Boc) ; 3,29 (t, 2 H, $^3J_{H,H}$ 6,0 Hz, $CH_2N_3$) ; 3,15 (q, 4 H, $^3J_{H,H}$ 5,6 Hz, $CH_2NHBoc$) ; 2,67 (t, 2 H, $CH_2CH_2N_3$) ; 2,59 (t, 4 H, $CH_2CH_2NHBoc$) ; 1,43 (s, 18 H, $CMe_3$) ;
- $^{13}C$ RMN (125,7 MHz ; $CDCl_3$): δ 156,0 (CO); 79,1 ($CMe_3$) ; 53,9 ($CH_2CH_2NHBoc$); 53,5 ($CH_2CH_2N_3$); 49,6 ($CH_2N_3$); 38,5 ($CH_2NHBoc$); 28,3 ($CMe_3$).

**d) Préparation de bis(2-aminoéthyl)-2-azidoéthylamine.**

Ce composé est obtenu par hydrolyse des deux groupes carbamates dans le 2-azidoéthyl-bis(2-*tert*-butoxycarbonylaminoéthyl)amine (0,16 g; 0,43 mmol) par traitement avec l'acide trifluoroacétique-eau 1:1 (3 mL) à 40°C pendant 2 heures. L'évaporation des solvants sous pression réduite, la co-évaporation des traces d'acide avec de l'eau et la lyophilisation du résidu résultant conduit à un solide blanc hygroscopique (rendement quantitatif d'après la RMN) ayant les caractéristiques suivantes :

- spectre de masse ($CI^+$) : *m*/*z* 173 $[M + H]^+$
- $^1H$ RMN (500 MHz, $D_2O$) : δ 3,63 (t, 2 H, $^3J_{H,H}$ 6,0 Hz, $CH_2N_3$) ; 3,24 (t, 4 H, $3J_{H,H}$ 6,6 Hz, $CH_2NH_2$) ; 3,08 (t, 4 H, $CH_2CH_2NH_2$) ; 2,99 (t, 2 H, $CH_2CH_2N_3$)
- $^{13}C$ RMN (75,5 MHz ; $D_2O$): δ 52,7 ($CH_2N_3$); 50,3 ($CH_2NH_2$); 45,7 ($CH_2CH_2N_3$) ; 34,1 ($CH_2CH_2NH_2$).

Ce composé est utilisé directement dans l'étape suivante sans purification additionnelle.

**e) Préparation de 2-azidoéthyl bis[2-[*N'*-(2,3,4,6-tetra-*O*-acétyl-α-D-mannopyranosyl)thiouréido]éthyl]amine.**

Ce composé est obtenu par réaction de couplage entre le bis(2-aminoéthyl) 2-azidoéthylamine (132 mg ; 0,77 mmol) et le 2,3,4,6-tétra-*O*-acétyl-α-D-mannopyranosyl isothiocyanate (0,66 g ; 1,1 équiv ; 1,7 mmol) dan l'eau-acétone 4:7 (11 mL) à pH 8-9 (hydrogénocarbonate de sodium solide) à température ambiante pendant 16 heures. On élimine l'acétone à pression réduite et la suspension aqueuse est extraite par le dichlorométhane) (2 × 5 mL), la phase organique est séchée, concentrée et le résidu est purifié par chromatographie sur colonne de gel de silice avec l'acétate d'éthyle-éther de pétrole 2:1. On obtient ainsi 0,68 g (rendement 93%) d'un solide blanc ayant les caractéristiques suivantes :

- $[\alpha]_D$ +52,0° (*c* 1,0 ; dichlorométhane)
- spectre de masse (FAB$^+$) : *m*/*z* 973 [M + Na]
- $^1$H RMN (500 MHz, $CDCl_3$, 313 K) : δ 7,83 ; 7,26 (2 bs, 4 H, NH) ; 5,66 (bs, 2 H, H-1) ; 5,49 (dd, 2 H, $J_{2,3}$ 2,2 Hz, $J_{3,4}$ 9,4 Hz, H-3) ; 5,31 (bd, 2 H, H-2) ; 5,26 (t, 2 H, $J_{4,5}$ 9,4 Hz, H-4) ; 4,26 (dd, 2 H, $J_{5,6a}$ 4,8 Hz, $J_{6a,6b}$ 12,4 Hz, H-6a) ; 4,10 (dd, 2 H, $J_{5,6b}$ 2,4 Hz, H-6b) ; 3,95 (ddd, 2 H, H-5) ; 3,74 ; 3,67 (2 bs, 4 H, C$H_2$NH) ; 3,34 (t, 2 H, $^3J_{H,H}$ 5,6 Hz, $CH_2N_3$) ; 2,75 (m, 6 H, $CH_2N$) ; 2,12 ; 2,05 ; 2,01 ; 1,97 (4 s, 6 H, 8 *Me*CO) ;
- $^{13}$C RMN (125,7 MHz ; $CDCl_3$ ; 313 K) : δ 183,3 (CS) ; 170,5 ; 169,7 ; 169,6 (CO) ; 80,1 (C-1) ; 69,6 (C-5) ; 69,1 (C-3) ; 68,9 (C-2) ; 66,2 (C-4) ; 62,1 (C-6) ; 53,8 (*C*$H_2CH_2$NH) ; 52,9 (*C*$H_2CH_2N_3$) ; 49,5 ($CH_2N_3$) ; 43,4 ($CH_2$NH) ; 20,6 ; 20,4 (*Me*CO).

**f) Préparation du 2-isothiocyanatoéthyl bis[2-[*N'*-(2,3,4,6-tétra-*O*-acétyl-α-D- mannopyranosyl) thiouréido] éthyl] amine.**

A une solution de 2-azidoéthyl-bis[2-(*N'*-(2,3,4,6-tetra-*O*-acétyl-α-D-mannopyranosyl)thiouréido)éthyl]amine (0,68 g ; 0,71 mmol) dans le dioxane (10 mL) on ajoute la triphénylphosphine (0,22 g; 1.2 équiv; 0,85 mmol) et le disulfure de carbone (0,5 mL; 10 équiv; 7,1 mmol), et le mélange réactionnel est agité à température ambiante sous argon pendant 24 heures. Le solvant est éliminé à pression réduite et le résidu est purifié par chromatographie sur colonne de gel de silice avec l'acétate d'éthyle-éther de pétrole 3:1. On obtient ainsi 0,49 g (rendement 71%) d'un solide blanc ayant les caractéristiques suivantes :

- $[\alpha]_D$ +29.0° (*c* 1.0, dichlorométhane)
- spectre de masse (FAB$^+$): *m*/*z* 989 $[M + Na]^+$
- $^1$H RMN (300 MHz, $CDCl_3$, 313 K) : δ 7,81 ; 7,26 (2 bs, 4 H, NH) ; 5,68 (bs, 2 H, H-1) ; 5,51 (dd, 2 H, $J_{2,3}$ 3,3 Hz, $J_{3,4}$ 9,5 Hz, H-3) ; 5,36 (dd, 2 H, $J_{1,2}$ 2,1 Hz H-2) ; 5,27 (t, 2 H, $J_{4,5}$ 9,5 Hz, H-4) ; 4,27 (dd, 2 H, $J_{5,6a}$ 4,7 Hz, $J_{6a,6b}$ 12,3 Hz, H-6a) ; 4,11 (dd, 2 H, $J_{5,6b}$ 2,7 Hz, H-6b) ; 4,03 (ddd, 2 H, H-5) ; 3,76 (m, 4 H, C$H_2$NH) ; 3,61 (t, 2 H, $^3J_{H,H}$ 5,4 Hz, $CH_2$NCS) ; 2,73 (m, 6 H, $CH_2N$) ; 2,13 ; 2,07 ; 2,02 ; 1,99 (4 s, chaque 6 H, 8 *Me*CO) ;
- $^{13}$C RMN (125.7 MHz, $CDCl_3$, 313 K) : δ 183,4 (CS) ; 170,4 ; 170,1 ; 169,5 ; 169,4 (CO) ; 132,0 (NCS) ; 80,3 (C-1) ; 69,4 (C-5) ; 69,0 (C-3) ; 68,8 (C-2) ; 66,2 (C-4) ; 62,1 (C-6) ; 53,3 (*C*$H_2CH_2$NH) ; 52,6 ($CH_2CH_2$NCS) ; 42,9 ($CH_2$NH, *C*$H_2$NCS) ; 20,6 ; 20,3 (*Me*CO).

**g) Préparation du composé n° 7.**

Ce composé est obtenu par réaction de couplage entre le composé n° 2 (58 mg, 23 μmol) et le 2-isothiocyanatoéthyl bis[2-[*N'*-(2,3,4,6-tetra-*O*-acétyl-α-D-mannopyranosyl)thiouréido]éthyl]amine (25 mg, 26 μmol) dans l'eau-acétone 1:1 (2 mL) à température ambiante pendant 4 jours, suivi de désacétylation comme décrit ci-dessus pour le composé n° 5. Le résidu résultant est purifié par chromatographie de filtration sur gel (Sephadex G-25, eau). On obtient ainsi le composé n° 7 (36 mg, rendement 50%) sous forme d'un solide blanc ayant les caractéristiques suivantes :

- $[\alpha]_D$ +99,6° (*c* 2,4 ; $H_2O$)
- solubilité dans l'eau : 375 $g \cdot L^{-1}$ (120 $mmol \cdot L^{-1}$)
- spectre de masse (MALDITOF$^+$) : *m*/*z* 3151 (100%, $[M + Na]^+$)
- $^{13}$C RMN (125,7 MHz $D_2O$, 343 K) : δ 181,8 (CS) ; 102,3-101,0 (C-$1^{I-VII}$) ; 83,9 (C-$4^{I}$) ; 82,2-81,3 (C-$4^{II-VII}$) ; 78,1 (C-1') ; 74,0-73,6 (C-$3^{I-VII}$) ;72,8-72,1 (C-$2^{I-VII}$, C-$5^{II-VII}$) ; 71,2 ; 70,9 ; 70,5 (C-$5^{I}$, C-5', C-3', C-2') ; 67,4 (C-4') ; 61,7 (C-6') ; 61,2-61,0 (C-$6^{II-VII}$) ; 53,2, 52,6 ($CH_2N$) ; 45,7 (C-$6^{I}$) ; 42,3 ($CH_2$NHCS).

**Exemple 8: Préparation du bis[2-[*N'*-($6^I$-désoxycyclomaltoheptaos-$6^I$-yl)thiouréido]éthyl] 2-[*N'*-[2-[bis [2-[*N'*-[tris(α-D-mannopyranosyloxyméthyl) méthyl]thiouréido]éthyl]amino]éthyl]thiouréido]éthylamine (composé n° 8).**

Ce composé répond à la formule (I) donnée ci-dessus avec A = H, n = n' = 2, m = 6, dans laquelle X représente un atome de soufre, Y représente NH, W représente un atome d'azote et Z représente le groupe $(CH_2)_2$NHC(=S)NH $(CH_2)_2N[(CH_2)_2$ NHC(=S)NHR$]_2$, R répondant à la formule (IV) donné ci-dessus (voir aussi formule (H-c) avec m = 6). Ce composé répond donc à la formule suivante :

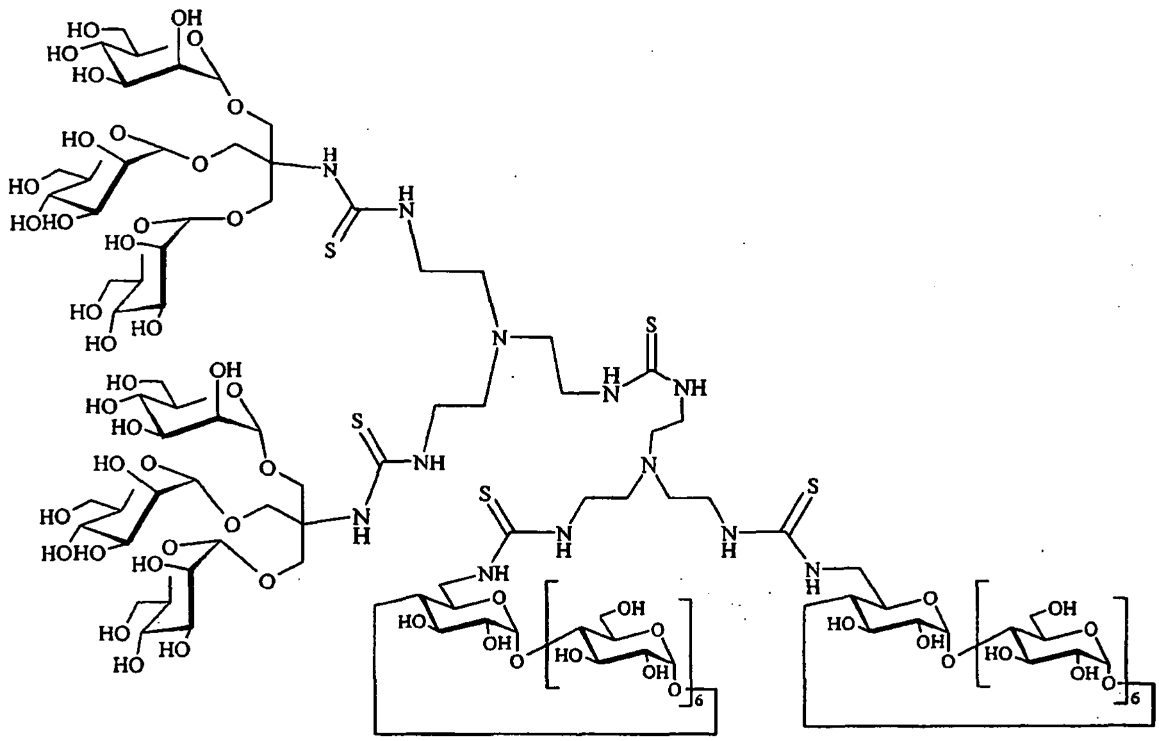

Ce composé est obtenu en effectuant les étapes suivantes :

**a) Préparation de 2-azidoéthyl bis[2-[*N'*-[tris(2,3,4,6-tétra-*O*-acétyl-α-D-mannopyranosyloxyméthyl)méthyl] thiouréido]éthyl]amine.**

Ce composé est obtenu par couplage entre le bis(2-aminoéthyl)-2-azidoéthylamine (45 mg ; 0,26 mmol), obtenu comme indiqué ci-dessus, et le tris(2,3,4,6-tétra-*O*-acétyl-α-D-mannopyranosyloxyméthyl)méthyl isothiocyanate (0,6 g ; 0,52 mmol) dans l'eau-acétone 1:5 (6 mL) à température ambiante pendant 24 heures. On élimine l'acétone à pression réduite et la suspension aqueuse est extraite par le dichlorométhane (2 × 5 mL), la phase organique est séchée, concentrée et le résidu est purifié par chromatographie sur colonne de gel de silice avec le dichlorométhane-méthanol 30:1. On obtient ainsi 0,51 g (rendement 79%) d'un solide blanc ayant les caractéristiques suivantes :

- $[\alpha]_D$ +47,2° (*c* 1,0 ; $CH_2Cl_2$) ;
- spectre de masse ($FAB^+$) : *m/z* 2503 $[M + Na]^+$
- $^1H$ RMN (500 MHz, $CDCl_3$, 313 K) : δ 6,87 ; 6,13 (2 bs, 4 H, NH) ; 5,27 (t, 6 H, $J_{3,4}$ = $J_{4,5}$ 9,6 Hz, H-4) ; 5,18 (dd, 6 H, $J_{2,3}$ 3,3 Hz, H-3) ; 5,18 (bs, 6 H, H-2) ; 4,88 (bs, 6 H, H-1) ; 4,31 (dd, 6 H, $J_{5,6a}$ 4,7 Hz, $J_{6a,6b}$ 12,4 Hz, H-6a) ; 4,28 (d, 6 H, $^2J_{H,H}$ 10,0 Hz, $OCH_2a$) ; 4,10 (dd, 6 H, $J_{5,6b}$ 2,3 Hz, H-6b) ; 4,03 (d, 6 H, $OCH_{2b}$) ; 4,00 (ddd, 6 H, H-5) ; 3,52 (m, 4 H, $CH_2NH$) ; 3,36 (m, 2 H, $CH_2N_3$); 2,77 (m, 6 H, $CH_2N$) ; 2,11 ; 2,08 ; 2,00 ; 1,94 (4 s, chaque 18 H, 24 *Me*CO) ;
- $^{13}C$ RMN (125,7 MHz ; $CDCl_3$ ; 313 K) : δ 182,1 (CS) ; 170,6 ; 169,8 ; 169,6 ; 169,4 (CO) ; 98,3 (C-1) ; 69,1 ; 68,9 (C-2, C-3, C-5) ; 66,5 ($OCH_2$) ; 65,8 (C-4) ; 62,1 (C-6) ; 60,8 ($C_q$) ; 52,2 ($CH_2N$) ; 49,2 ($CH_2N_3$) ; 41,6 ($CH_2NH$) ; 20,6 ; 20,5 ; 20,4 (*Me*CO).

**b) Préparation du 2-isothiocyanatoéthyl-bis[2-[*N'*-[tris(2,3,4,6-tétra-*O*-acétyl-α-D- mannopyranosyloxyméthyl) méthyl] thiouréido] éthyl] amine.**

A une solution de 2-azidoéthyl-bis[2-[*N'*-[tris(2,3,4,6-tetra-*O*-acétyl-α-D-mannopyranosyloxyméthyl)méthyl] thiouréido]éthyl]amine (0,51 g ; 0.20 mmol) dans le dioxane (5 mL), on ajoute la triphénylphosphine (59 mg ; 1,1 équiv ; 0,22 mmol) et le disulfure de carbone (0,13 mL ; 10 équiv ; 2,0 mmol), et le mélange réactionnel est agité à température ambiante sous argon pendant 24 heures. Le solvant est éliminé sous pression réduite et le résidu est purifié par chromatographie sur colonne de gel de silice avec l'éluant dichlorométhane-méthanol 50:1. On obtient ainsi 0,36 g (rendement 73%) d'un solide blanc ayant les caractéristiques suivantes :

- $[\alpha]_D$ +45,9° (c 1,0 ; $CH_2Cl_2$)
- spectre de masse (FAB$^+$) : *m/z* 2519 [M + Na]$^+$
- $^1$H RMN (500 MHz, $CDCl_3$, 313 K) : δ 6,88 ; 6,18 (2 bs, 4 H, NH) ; 5,27 (t, 6 H, $J_{3,4}$ = $J_{4,5}$ 9,8 Hz, H-4) ; 5,21 (dd, 6 H, $J_{2,3}$ 3,2 Hz, H-3) ; 5,19 (bs, 6 H, H-2) ; 4,89 (bs, 6 H, H-1) ; 4,31 (dd, 6 H, $J_{5,6a}$ 5,1 Hz, $J_{6a,6b}$ 12,4 Hz, H-6a) ; 4,30 (d, 6 H, $^2J_{H,H}$ 10,0 Hz, $OCH_2$a) ; 4,11 (dd, 6 H, $J_{5,6b}$ 2,0 Hz, H-6b) ; 4,09 (d, 6 H, $OCH_2$b) ; 4,01 (ddd, 6 H, H-5) ; 3,61 (m, 6 H, $CH_2NH$, $CH_2NCS$) ; 2,77 (m, 6 H, $CH_2N$) ; 2,11 ; 2,08 ; 2,01 ; 1,96 (4 s, chaque 18 H, 24 *Me*CO) ;
- $^{13}$C RMN (125,7 MHz ; $CDCl_3$ ; 313 K) : δ 182,1 (CS) ; 170,6 ; 169,8 ; 169,6 ; 169,4 (CO) ; 133,0 (NCS) ; 98,3 (C-1) ; 69,2 ; 69,1 ; 68,9 (C-2, C-3, C-5) ; 66,4 ($OCH_2$) ; 65,5 (C-4) ; 61,9 (C-6) ; 60,9 (Cq) ; 51,6 ($CH_2N$) ; 45,8 ($CH_2NCS$) ; 41,3 ($CH_2NH$) ; 20,7 ; 20,6 ; 20,5 (*Me*CO).

**c) Préparation du composé n° 8.**

Ce composé est obtenu par réaction de couplage entre le composé n° 2 (58 mg, 23 μmol) et le 2-isothiocyanatoéthyl-bis[2-[*N*'-[tris(2,3,4,6-tétra-*O*-acétyl-α-D-manno-pyranosyloxyméthyl)méthyl]thiouréido]éthyl]amine (65 mg, 26 μmol) dans l'eau-acétone 1:1 (2 mL) à température ambiante pendant 4 jours, suivi de désacétylation comme décrit ci-dessus pour le composé n° 6. Le résidu résultant est purifié par chromatographie de filtration sur gel (Sephadex G-25, eau). On obtient ainsi le composé n° 8 (49 mg, rendement 51%) sous forme d'un solide blanc ayant les caractéristiques suivantes :

- $[\alpha]_D$ +62,0° (*c* 1,0 ; $H_2O$)
- solubilité dans l'eau : 400 g·L$^{-1}$ (100 mmol·L$^{-1}$)
- spectre de masse (MALDITOF$^+$) : *m/z* 4008 (100%, [M + Na]$^+$)
- $^1$H RMN (500 MHz, $D_2O$, 333 K) : δ 5,34 (m, 14 H, H-1$^{I-VII}$) ; 5,15 (m, 18 H, H-2', H-3', H-4') ; 5,09 (bs, 6 H, H-1') ; 4,37 (d, 6 H, $^2J_{H,H}$ 10,5 Hz, $OCH_2$a) ; 4,31-4,10 (m, 72 H, H-3$^{I-VII}$ H-5$^{I-VII}$, H-6a$^{I-VII}$, H-6b$^{II-VII}$, $CH_2N$, $OCH_2$b) ; 4,09-4,04 (m, 14 H, H-2$^{I-VII}$) ; 4,03-3,88 (m, 24 H, H-4$^{II-VII}$, $CH_2NH$) ; 3,67 (m, 4 H, H-4$^I$, H-6b$^I$) ;
- $^{13}$C RMN (125,7 MHz ; $D_2O$; 333 K) : δ 181,5 (CS) ; 102,2 (C-1$^{I-VII}$) ; 100,8 (C-1') ; 81,4 (C-4$^{I-VII}$) ; 74,2-73,9-72,3 (C-2$^{I-VII}$, C-3$^{I-VII}$, C-5$^{I-VII}$); 71,3 ; 70,5 (C-3', C-2', C-5') ; 67,3 (C-4',$OCH_2$) ; 61,6 (C-6') ; 60,8 (C-6$^{II-VII}$) ; 52,8 ($CH_2N$) ; 42,6 (C-6$^I$) ; 37,6 ($CH_2NHCS$).

**<u>Exemple 9</u> : Evaluation de l'affinité des thiouréidocystéaminyl-cyclodextrines composés n° 5 à 8 pour la lectine spécifique de mannose concanavaline A (ConA)**

On a évalué l'affinité des composés n° 5 à 8 pour la lectine spécifique du mannose concanavaline A (ConA) suivant le protocole de test ELLA. On obtient ainsi la concentration des composés n° 5 à 8 nécessaire pour inhiber à 50% l'association de la ConA à un ligand de référence (le mannane de levure dans notre cas) fixé sur la cellule d'une plaque de microtitration ($IC_{50}$). Les valeurs d'$IC_{50}$ sont inversement proportionnelles aux affinités respectives.

Les cellules d'une plaque de microtitration (Nunc-Immuno™ plates MaxiSorp™) sont chargées avec 100 μL d'une solution mère de mannane de levure (Sigma, 10 μg/L$^{-1}$) dans une solution tampon phosphate saline (PBS ; pH 7,3 contenant $Ca^{2+}$ 0,1 mM et $Mn^{2+}$ 0,1 mM) pendant une nuit à température ambiante. Les cellules sont lavées (3 × 300 μL) par une solution tampon contenant 0,05% (v/v) de Tween 20 (PBTS). Ce protocole de lavage est répété après chaque incubation pendant l'essai. Les cellules sont alors additionnées d'une solution d'albumine du sérum bovin (BSA, 1%) dans le PBS (150 μg/cellule) suivi d'incubation pendant 1 h à 37°C, puis lavées.

Pour déterminer la concentration de lectine optimale pour les études d'inhibition, on ajoute dans les cellules chargées avec le mannane et traitées comme indiqué ci-dessus 100 μL d'une série de solutions de concanavaline A marquée avec la peroxydase de radis de $10^{-1}$ à $10^{-5}$ mg.mL$^{-1}$ dans le PBS. Après incubation à 37°C pendant 1 heure, les plaques sont lavées (PBST) et additionnées d'une solution (50 μL) du sel de diammonium de l'acide 2,2'-azinobis (3-éthylbenzothiazoline-6-sulfonique) (ABTS, 1 mg dans 4 mL) dans le tampon citrate (0,2 M ; pH 4,0 avec 0,015% d'eau oxygénée). La réaction est arrêtée après 20 minutes par addition de 50 μL/cellule d'acide sulfurique 1 M et les absorbances sont mesurées à 415 nm utilisant un lecteur ELISA. Les cellules témoin contenaient le tampon citrate-phosphate. La concentration de lectine marquée avec la peroxydase donnant une valeur d'absorbance entre 0,8 et 1,0 (typiquement entre $10^{-2}$ et $10^{-3}$ mg/mL$^{-1}$) à été utilisée dans les essais d'inhibition.

Pour les essais d'inhibition, on a utilisé des solutions mères des composés n° 5 à 8 à une concentration de 5 à 7 mg/mL$^{-1}$ dans le PBS. Dans une série d'expériences, les solutions de chaque composé (60 μL/cellule) dans le PBS, diluées séquentiellement au double, sont additionnées de ConA marquée à la peroxydase de radis de concentration appropriée comme indiqué ci-dessus (60 μL/cellule) dans une plaque de microtitration Nunclon™ (Delta) qui est incubée à 37°C pendant 1 heure. Les solutions (100 μL/cellule) sont alors transférées sur une plaque de microtitration chargée avec le mannane et traitée comme indiqué ci-dessus, laquelle est ensuite incubée à 37°C pendant 1 heure. Les cellules sont lavées (PBST) et additionnées de la solution de l'ABTS (50 μL/cellule). Après 20 minutes, la réaction est arrêtée

(acide sulfurique) et les absorbances sont mesurées.

Le pourcentage d'inhibition est calculé par la formule :

$$\% \text{ d'inhibition} = \frac{A_{\text{en absence d'inhibiteur}}}{A_{\text{en présence d'inhibiteur}}} \times 100$$

Dans le tableau 1 qui suit, on donne les valeurs d'ICso pour les composés n° 5 à 8 (moyenne de trois expériences indépendantes) en comparaison avec la valeur correspondante pour le méthyl-α-D-glucopyranoside, utilisé comme ligand monovalent de référence. On observe une augmentation importante de l'affinité pour la lectine dans le cas du dérivé trivalent et du dérivé hexavalent comportant des substituants mannopyranosyle. Ainsi, le composé n° 8, avec une présentation dendritique hexavalente du ligand mannopyranosyle, est reconnu jusqu'à 25 fois plus efficacement par la lectine spécifique de mannose ConA en comparaison avec le dérivé de cyclodextrine dimère monovalent, composé n° 5.

**Tableau 1**. Données ELLA pour l'inhibition de l'association entre le mannane de levure et la lectine ConA marquée par la peroxydase de radis par les composés n° 5 à 8.

| | Composé | | | | |
|---|---|---|---|---|---|
| | Me-α-D-Glc*p* | n° 5 | n° 6 | n° 7 | n° 8 |
| $IC_{50}$ (μM) | 865 | 1288 | 1130 | 265 | 49 |
| Affinité relative | 1 | 0,67 | 0,76 | 3,3 | 17,7 |
| Affinité molaire relative | 1 | 0,67 | 0,38 | 1,1 | 2,9 |

**<u>Exemple 10</u> : Inclusion du Taxotère® dans le bis[2-[*N'*-(6<sup>I</sup>-désoxycyclomaltoheptaos-6<sup>I</sup>-yl)thiouréido]éthyl] 2-(*N'*-methylthiouréido)éthyl amine (composé n° 3).**

On part du Taxotère à l'état pur et on disperse 8,3 mg (9,7 μmol) de ce produit dans 1 mL d'une solution contenant 10 mmol.$L^{-1}$ du composé n° 3 dans l'eau stérile, puis on agite la suspension obtenue à 70°C jusqu'à obtention d'une solution claire qui indique la complexation du Taxotère. Une fois formé, le complexe reste en solution à température ambiante. On obtient ainsi une augmentation de la solubilité du Taxotère (8,3 g·$L^{-1}$) de l'ordre de 2075 fois par rapport à celle du Taxotère en l'absence de cyclodextrine (0,004 g·$L^{-1}$).

**<u>Exemple 11</u> : Inclusion du Taxotère® dans le bis[2-[*N'*-(6<sup>I</sup>-désoxycyclomaltoheptaos-6<sup>I</sup>-yl)thiouréido]éthyl] 2-[*N'*-[tris( α-D-mannopyranosyloxyméthyl)méthyl]thiouréido]éthylamine (composé n° 6).**

On part du Taxotère à l'état pur et on disperse 6,6 mg (7,7 μmol) de ce produit dans 1 mL d'une solution contenant 8 mmol/$L^{-1}$ du composé n° 6 dans l'eau stérile, puis on agite la suspension obtenue à 70°C jusqu'à obtention d'une solution claire qui indique la complexation du Taxotère. Une fois formé, le complexe reste en solution à température ambiante. On obtient ainsi une augmentation de la solubilité du Taxotère (6,6 g.$L^{-1}$) de l'ordre de 1650 fois par rapport à celle du Taxotère en l'absence de cyclodextrine (0,004 g·$L^{-1}$).

**<u>Exemple 12</u> : Bis[2-[*N'*-(6<sup>I</sup>-désoxycyclomaltoheptaos-6<sup>I</sup>-yl)thiouréido]éthyl] 2-[*N'*-[2-[bis[2-[*N'*-[tris(α-D-mannopyranosyloxyméthyl)méthyl]thiouréido]éthyl] amino]éthyl]thiouréido]éthyl amine (composé n° 8).**

On part du Taxotère à l'état pur et on disperse 5,8 mg (68 μmol) de ce produit dans 1 mL d'une solution contenant 7 mmol/$L^{-1}$ du composé n° 6 dans l'eau stérile, puis on agite la suspension obtenue à 70°C jusqu'à obtention d'une solution claire qui indique la complexation du Taxotère. Une fois formé, le complexe reste en solution à température ambiante. On obtient ainsi une augmentation de la solubilité du Taxotère (5,8 g.$L^{-1}$) de l'ordre de 1450 fois par rapport à celle du Taxotère en l'absence de cyclodextrine (0,004 g/$L^{-1}$).

## Revendications

1. Composé répondant à la formule générale suivante :

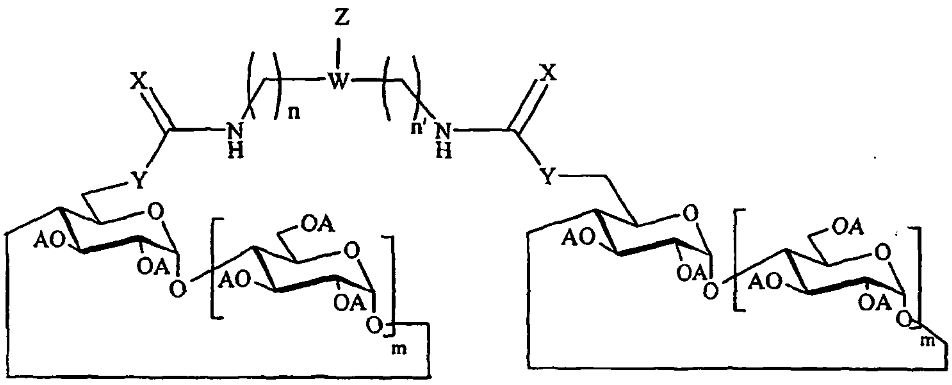

(I)

dans laquelle :

- m représente un nombre entier égal à 5, 6 ou 7 ;
- n et n' représentent un nombre entier compris de 1 à 5, n et n' pouvant être identiques ou différents ;
- les groupes A, identiques ou différents, représentent un atome d'hydrogène, un groupe acyle, alkyle, hydroxyalkyle ou sulfoalkyle de 1 à 16 atomes de carbone,
- X représente O ou S,
- Y représente :

  * un groupement $-NR_1-$, $R_1$ représentant un atome d'hydrogène ou un groupe alkyle comprenant de 1 à 6 atomes de carbone, ou
  * un groupement amide de formule $NH-CO-(CH_2)_q-NR_1-$, q représentant un nombre entier compris de 1 à 5 et $R_1$ étant tel que défini ci-dessus, ou
  * un groupement cystéaminyle de formule $-S-(CH_2)_r-NR_1-$, r représentant un nombre entier compris de 2 à 5 et $R_1$ étant tel que défini ci-dessus,

- W représente CH ou N ;
- Z représente :

  * un atome d'hydrogène ou
  * un substituant carbamate de formule

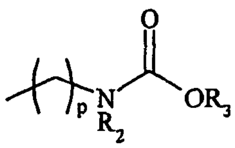

ou
  * un substituant aminé de formule

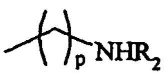

ou
  * un groupe ammonium quaternaire de formule

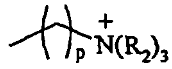

ou

* un substituant urée ou thiourée de formule

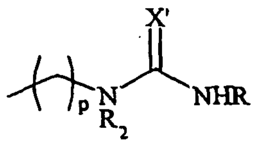

ou

* un groupe de formule

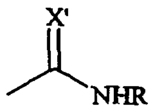

ou

* un groupe de la forme $C(=O)OR_3$, un groupe de la forme $C(=O)R_2$ ou un groupe portant les fonctionnalités amine, ammonium quaternaire urée ou thiourée, de formules respectives

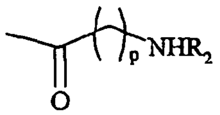

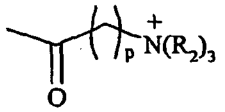

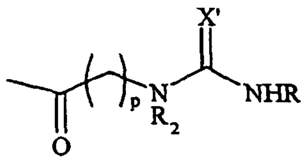

p représentant un nombre entier compris de 0 à 5, lorsque W représente CH,
et de 2 à 5, lorsque W représente N,
X' représentant O ou S,
$R_2$ représentant un atome d'hydrogène ou un groupe alkyle comprenant de 1 à 6 atomes de carbone, et étant notamment un groupe méthyle, éthyle, propyle ou butyle,
$R_3$ représentant un substituant permettant l'hydrolyse du groupement carbamate afin de libérer la fonction amine, tel que les groupements *tert*-butyle, 9-fluorénylméthyle, benzyle, allyle ou 2,2,2-trichloroéthyle, et :
R représentant un atome d'hydrogène, un groupe alkyle, linéaire ou ramifié, de 1 à 12 atomes de carbone, ou un groupe aromatique tel que le groupe phényle, benzyle ou naphtyle, ou des dérivés de ces groupements portant des substituants sur le cycle aromatique tels que les substituants méthyle, éthyle, chlore, brome, iode, nitro, hydroxyle, méthoxyle ou acétamido, ou
R représentant un élément de reconnaissance biologique tel qu'un dérivé d'acide aminé, un peptide, un monosaccharide, un oligosaccharide, un élément de multiplication à plusieurs ramifications, lesquelles ramifications comportent des groupements glucidiques qui peuvent être identiques ou différents, ou encore une sonde de visualisation ou de détection fluorescente ou radioactive.

**2.** Composé selon la revendication 1, **caractérisé en ce que** n et n' sont égaux.

**3.** Composé selon la revendication 1 ou 2, **caractérisé en ce que** tous les groupes A représentent un atome d'hydrogène.

**4.** Composé selon la revendication 3, **caractérisé en ce que** Y représente soit un groupe $NR_1$, soit un groupe -NH-CO-$(CH_2)_q$-$NR_1$-, soit un groupe -S-$(CH_2)_r$-$NR_1$-, et répondant respectivement à l'une des formules suivantes :

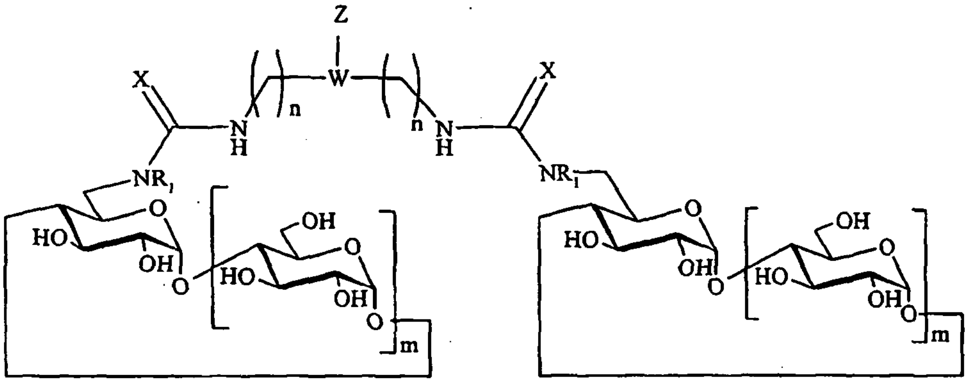

(I-a)

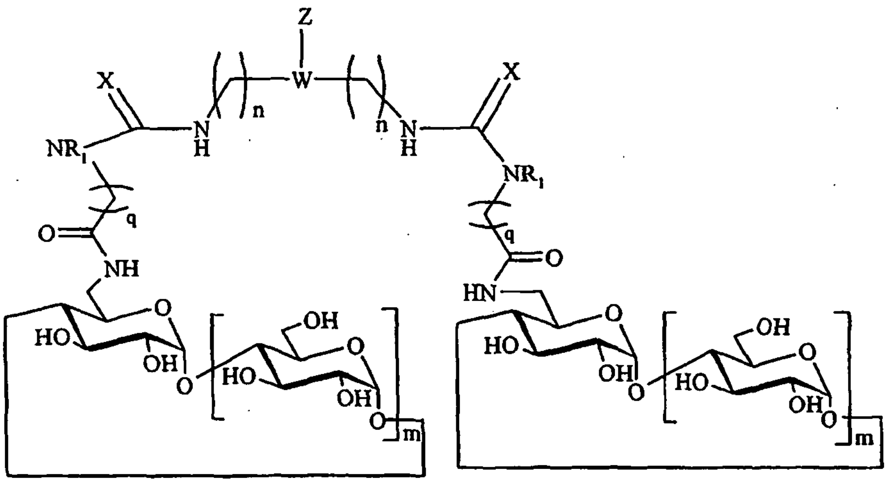

(I-b)

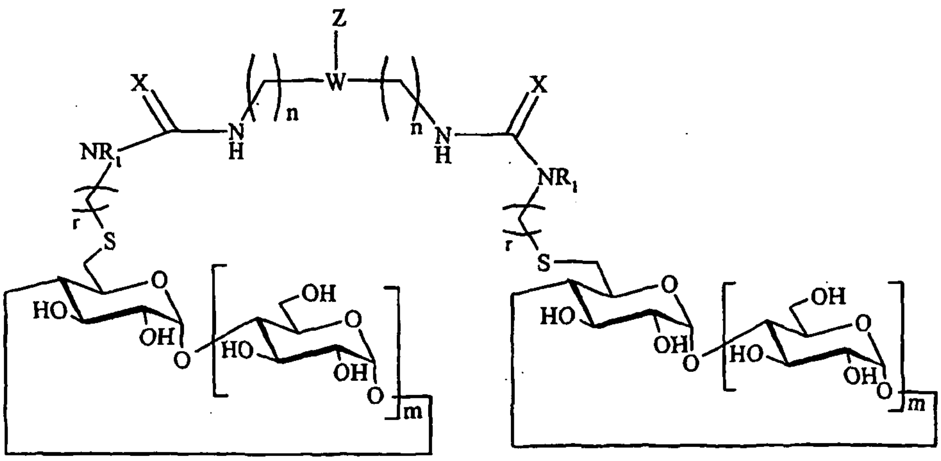

(I-c)

dans lesquelles n, m, q, r, X, W, Z et $R_1$ sont tels que définis dans la revendication 1.

**5.** Composé selon la revendication 3 ou 4, **caractérisé en ce que** Z représente soit un groupe $-(CH_2)_p-NHR_2$, soit un groupe $-(CH_2)_p-N^+(R_2)_3$, soit un groupe de formule

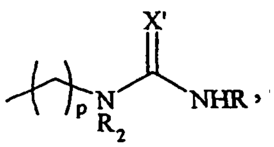

dans laquelle X' représente un atome de soufre,
et répondant respectivement à l'une des formules suivantes :

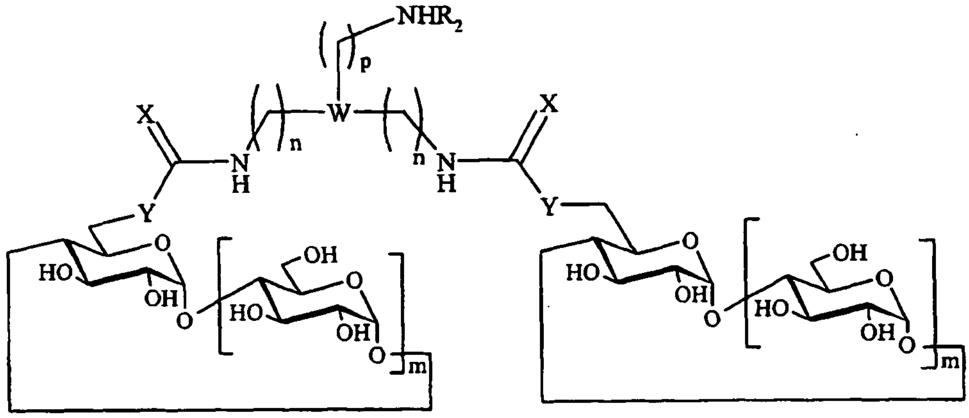

(I-d)

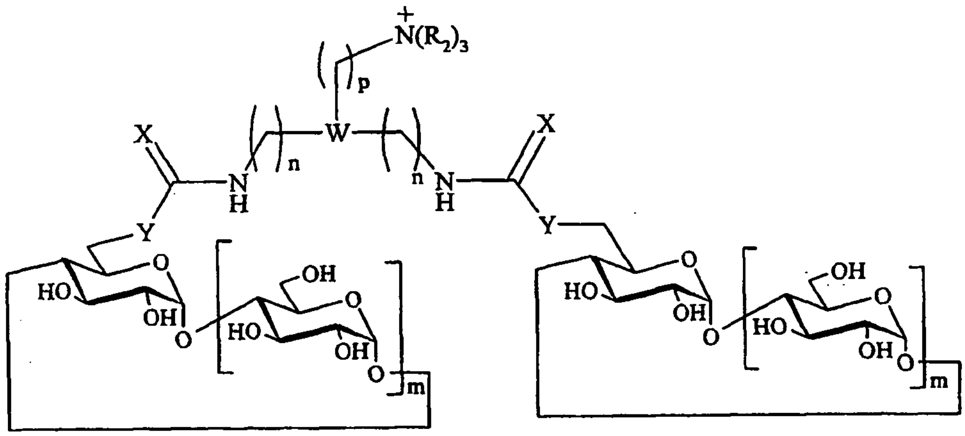

(I-e)

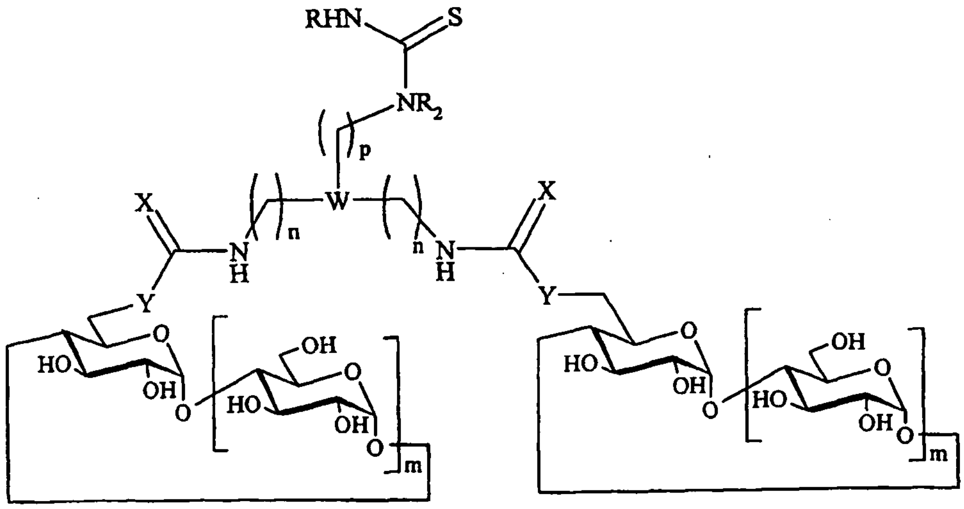

(I-f)

dans lesquelles n, m, p, X, W, Y, R et $R_2$ sont tels que définis dans la revendication 1.

**6.** Composé selon l'une quelconque des revendications 3 à 5, **caractérisé en ce que** W représente un atome d'azote et **en ce que** Z représente soit un groupe de formule $-CO-(CH_2)_p-NHR_2$, soit un groupe de formule $-CO-(CH_2)_p-N^+(R_2)_3$, soit un groupe de formule

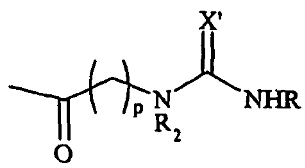

dans laquelle X' représente un atome de soufre, soit un groupe de formule

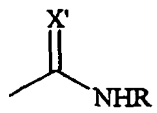

dans laquelle X' représente un atome de soufre et répondant respectivement à l'une des formules suivantes :

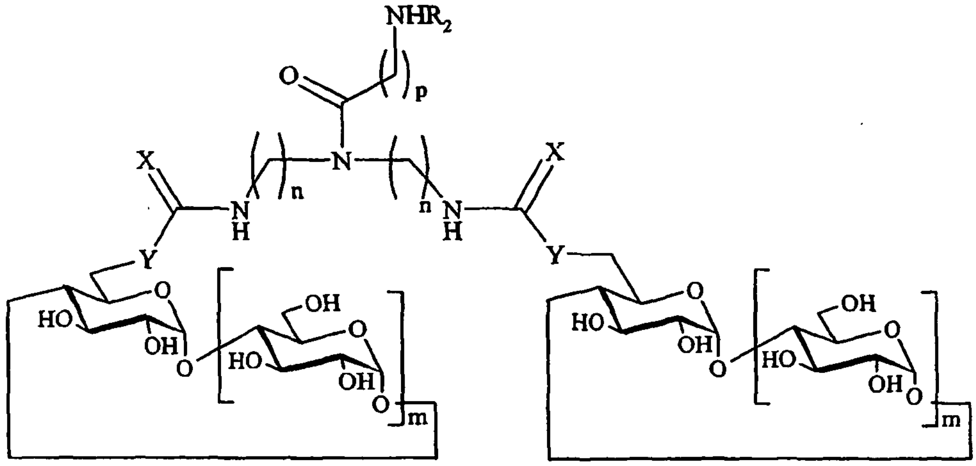

(I-g)

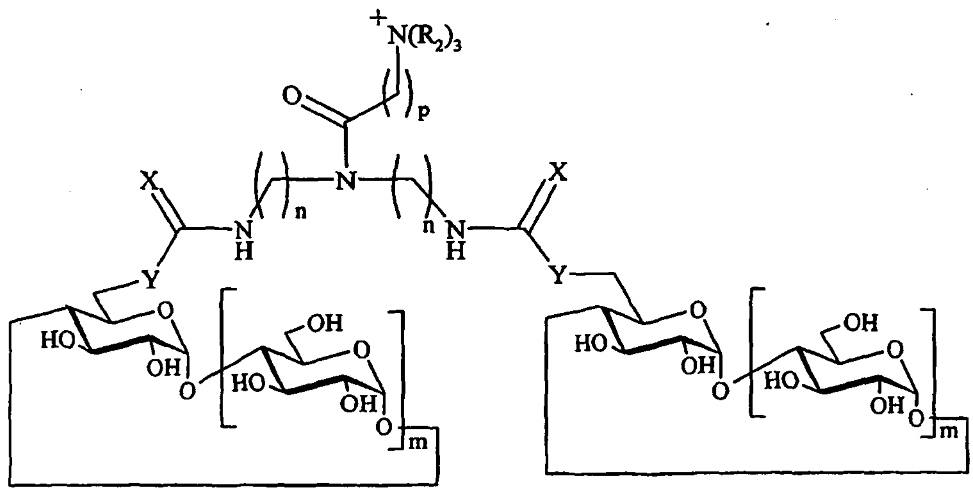

(I-h)

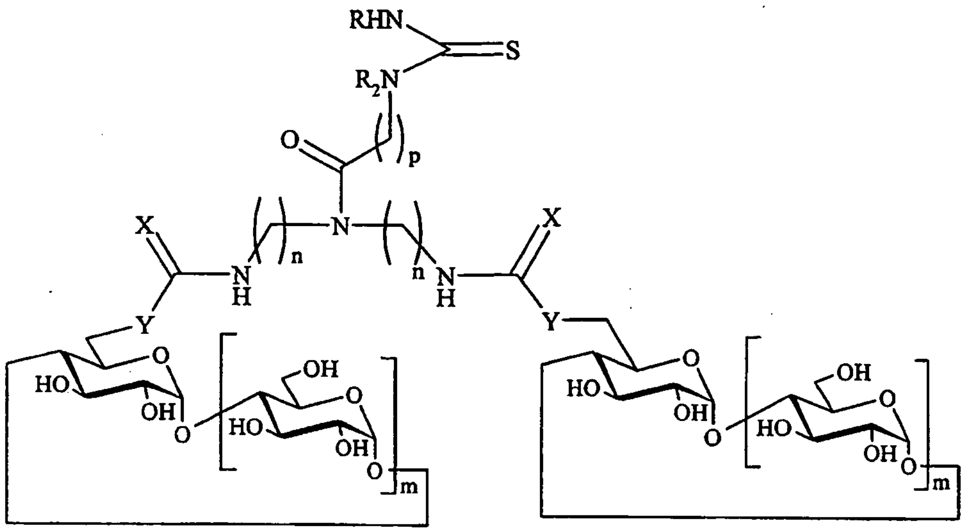

(I-i)

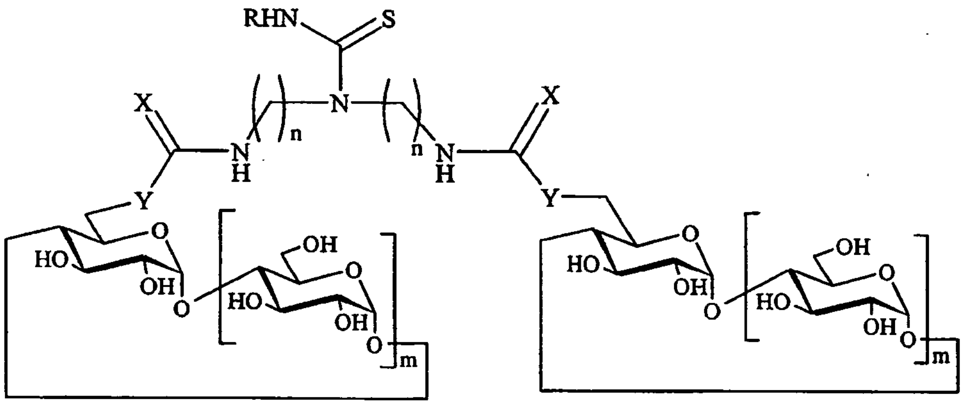

(I-j)

dans lesquelles n, m, p, X, Y, R et $R_2$ sont tels que définis dans la revendication 1.

**7.** Composé selon l'une quelconque des revendications 1 à 6, **caractérisé en ce que** R est choisi parmi les groupes suivants :

- un groupe alkyle de 1 à 12 atomes de carbone, linéaire ou ramifié, et étant de préférence le groupe méthyle ;
- un groupe aromatique tel que phényle, benzyle, naphtyle ou des dérivés de ces groupements portant des substituants sur le cycle aromatique, et étant de préférence le groupe phényle ;
- le groupe α-D-mannopyranosyle, de formule suivante (III) :

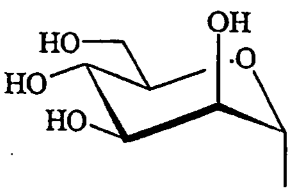

(III)

- le groupe β-lactosyle, de formule suivante (III-a) :

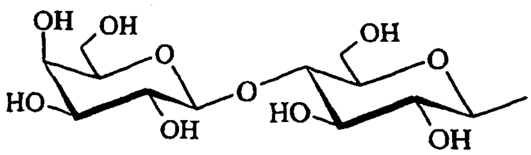

(III-a)

- le groupe dérivé du trisaccharide Lewis X ou du tétrasaccharide sialyl Lewis X, respectivement de formule suivante (III-b) et (III-c) :

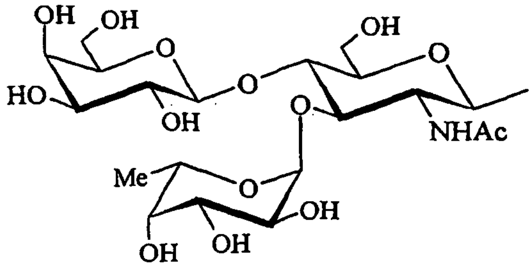

(III-b)

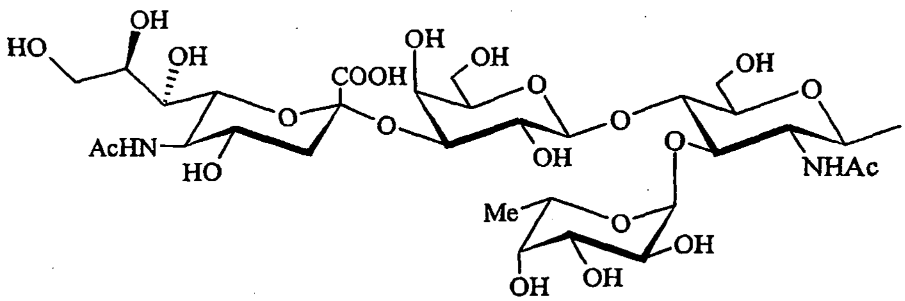

(III-c)

- un oligosaccharide dérivé de l'héparine, de formule suivante (III-d) :

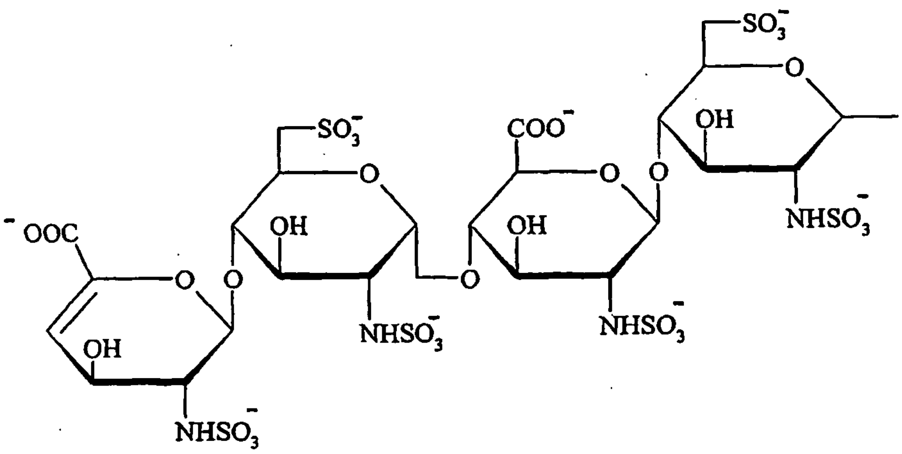

(III-d)

8. Composé selon l'une quelconque des revendications 1 à 6, **caractérisé en ce que** R comporte un élément de ramification dérivé du tris(2-hydroxyméthyl)méthylamine, et représente l'un des groupes suivants :

- le groupe tris(α-D-mannopyranosyloxyméthyl)méthyle, de formule suivante (IV) :

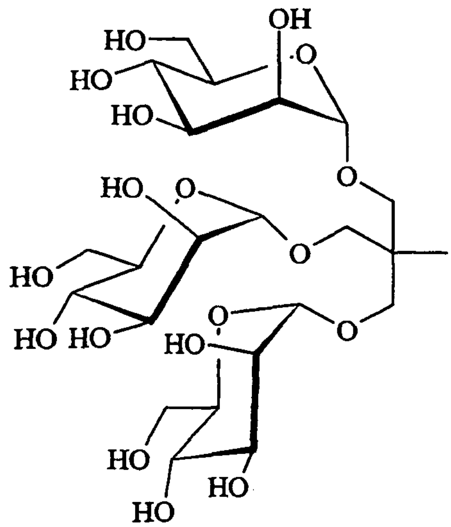

(IV)

- le groupe tris(β-lactosyloxyméthyl)méthyle, de formule suivante (IV-a) :

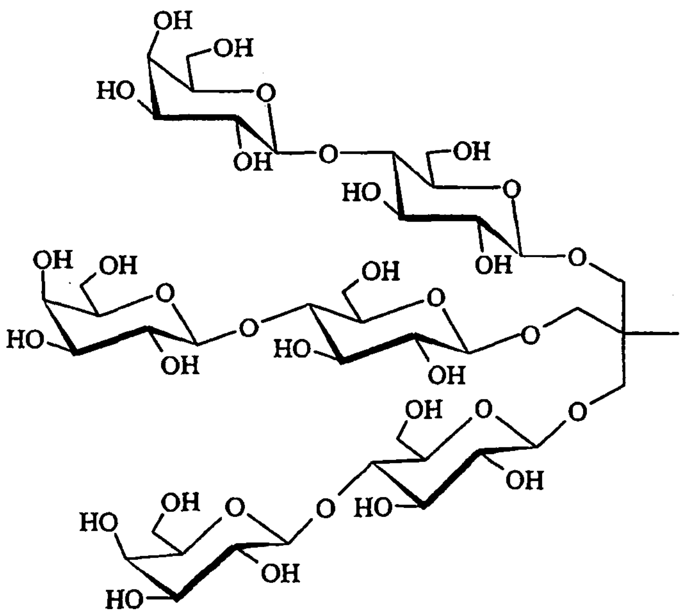

(IV-a)

9. Composé selon l'une quelconque des revendications 1 à 6, **caractérisé en ce que** R comporte un élément de ramification dérivé du pentaérythritol, ledit composé répondant à l'une des formules suivantes :

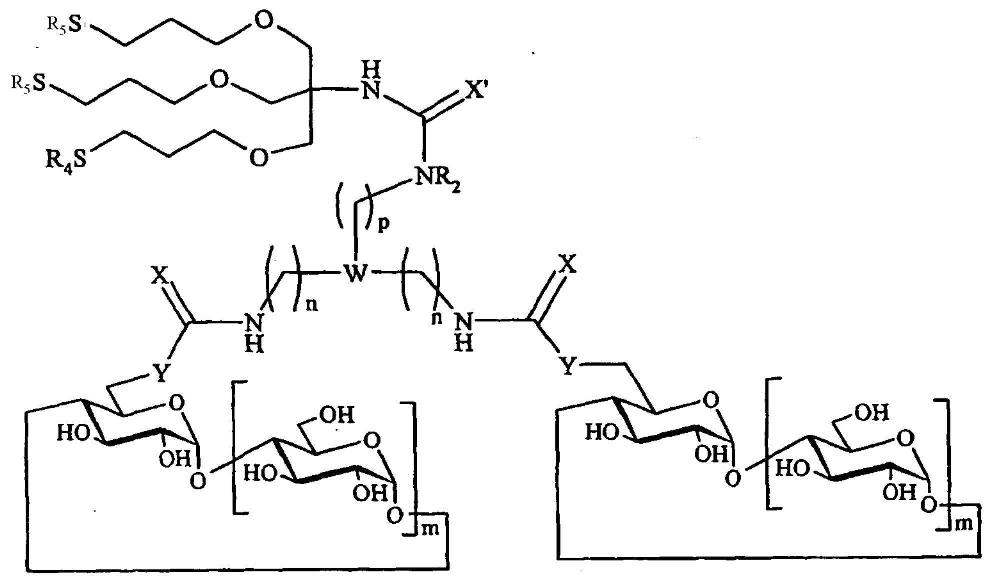

(II-a)

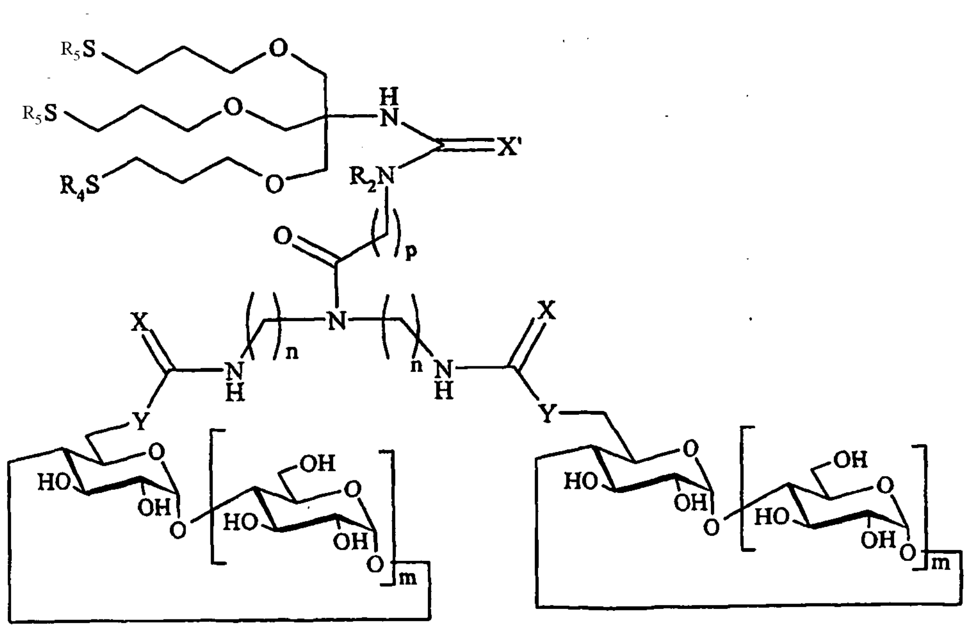

(II-b)

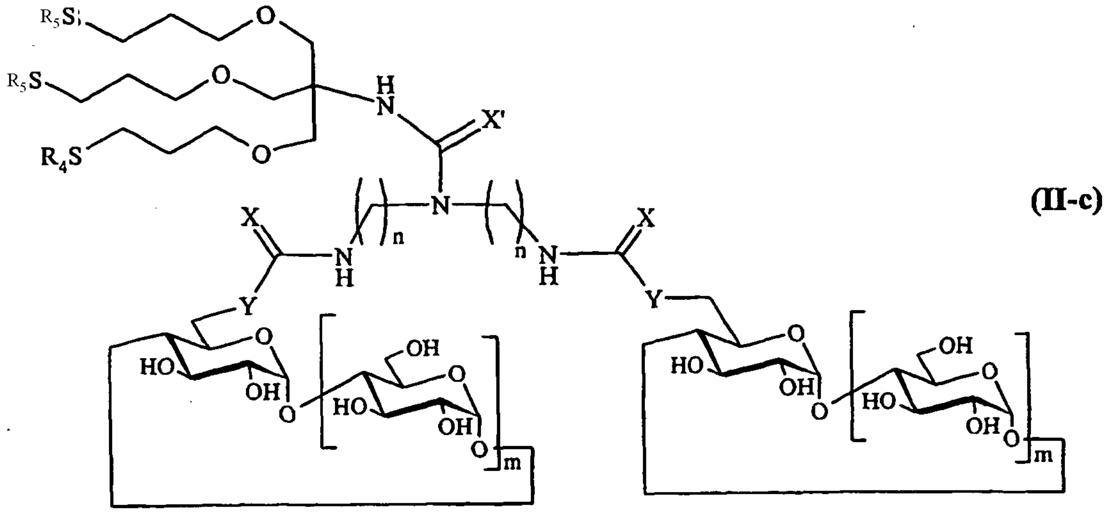

(II-c)

dans lesquelles m, n, p, X, X', Y sont tels que définis dans la revendication 1, et $R_5$ et $R_4$ représentent des dérivés glucidiques qui peuvent être différents ou identiques ou encore une sonde fluorescente ou radioactive.

**10.** Composé selon la revendication 9, **caractérisé en ce que** $R_5$ et $R_4$ représentent l'un des groupes suivantes :

- le groupe α-D-mannopyranosyle, de formule (III) tel que défini dans la revendication 7, ou
- le groupe β-lactosyle, de formule (III-a) tel que défini dans la revendication 7, ou
- le groupe β-D-glucopyranosyle, de formule (VI) suivante :

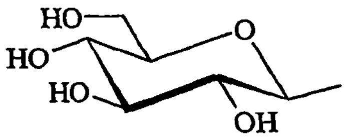

(VI)

$R^5$ et $R^4$ pouvant être identiques ou différents.

**11.** Composé selon l'une quelconque des revendications 1 à 6, **caractérisé en ce que** R comporte un élément de ramification dérivé du tris(2-aminoéthyl)amine (TREN), ledit composé répondant à l'une des formules suivantes :

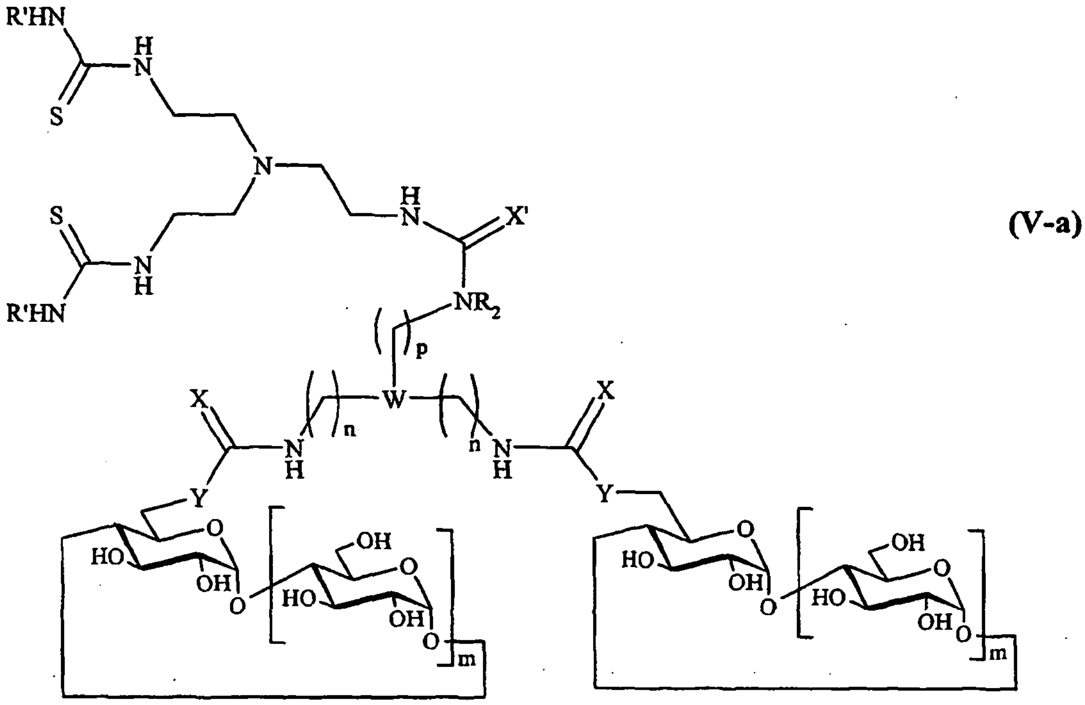

(V-a)

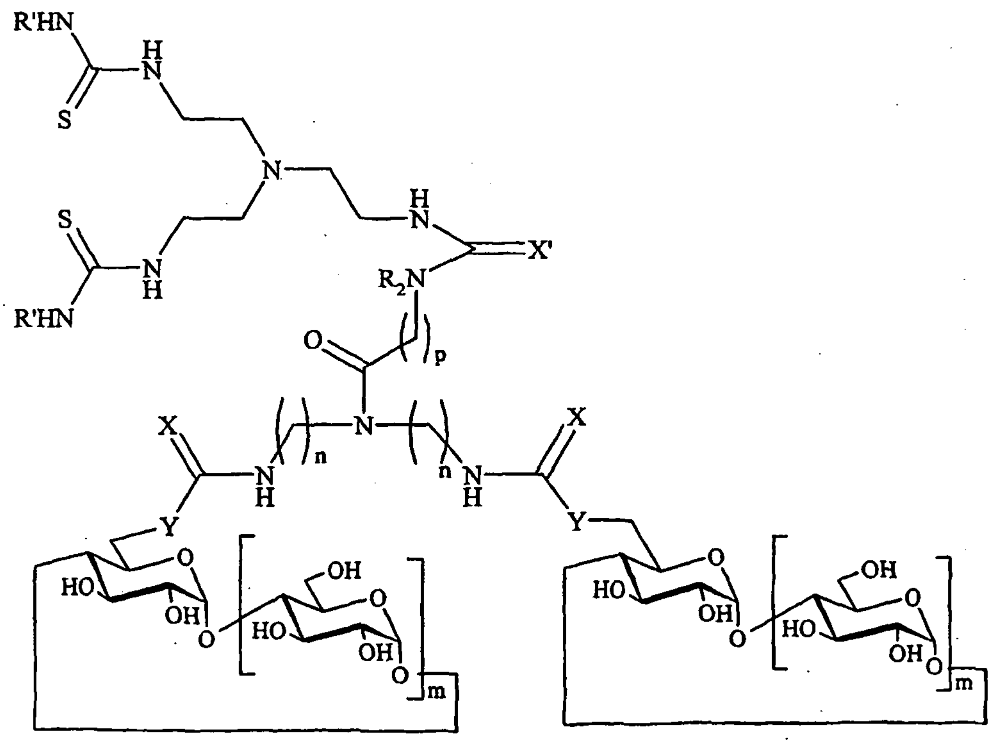

(V-b)

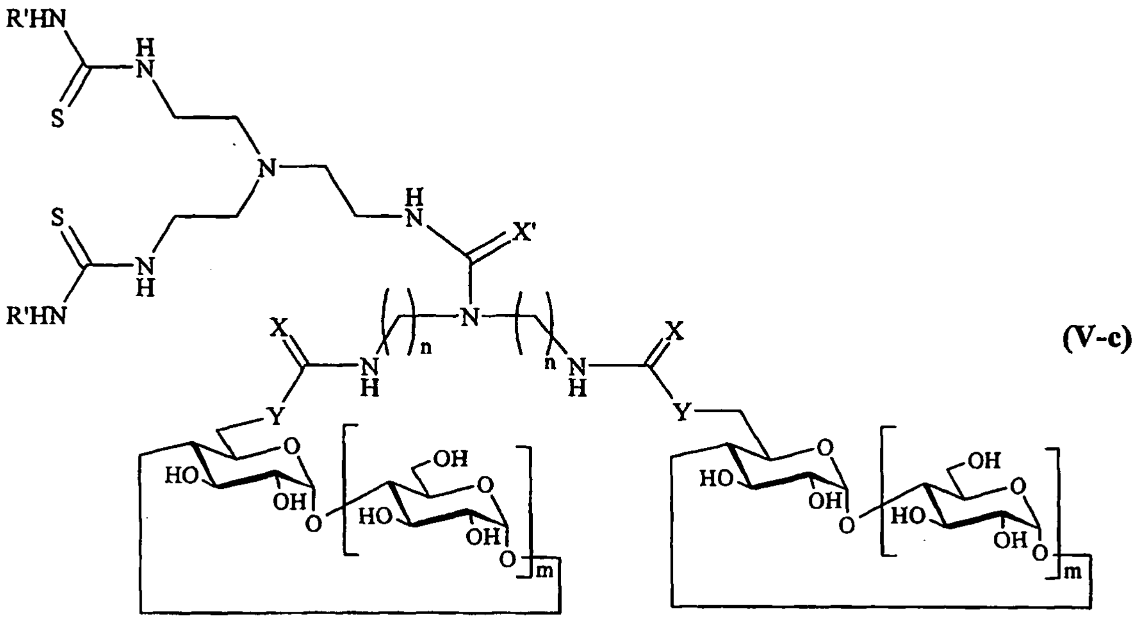

(V-c)

m, n, p, X, X', Y étant tels que définis dans la revendication 1, et
R' ayant la définition donnée précédemment pour R.

**12.** Composé selon la revendication 11, **caractérisé en ce que** R' représente

- le groupe α-D-mannopyranosyle, de formule (III), ou
- le groupe β-lactosyle de formule (III-a), ou
- le groupe tris(α-D-mannopyranosyloxyméthyl)méthyle, de formule (IV), ou
- le groupe tris(β-lactyloxyméthyl)méthyle, de formule (IV-a).

**13.** Composé selon l'une des revendications 1 à 12, **caractérisé en ce que** m est égal à 6.

**14.** Procédé de préparation d'un composé selon la revendication 1, **caractérisé en ce qu'**il comprend les étapes suivantes :

- la réaction d'un composé sélectivement fonctionnalisé en position alcool primaire avec un groupement aminé, de formule (VII) suivante :

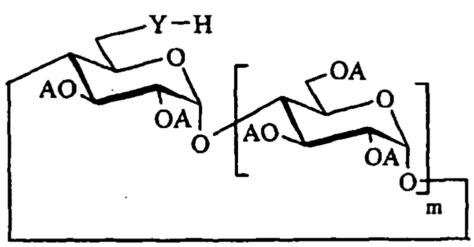

(VII)

m, A et Y étant tels que définis précédemment dans la revendication 1, et A étant de préférence un atome d'hydrogène,
avec un élément de dimérisation de type diisocyanate ou diisothiocyanate, notamment portant une fonctionnalité amine protégée sous forme d'un groupe carbamate ou portant une fonctionnalité sel d'ammonium quaternaire chargé positivement, de formule (VIII) suivante :

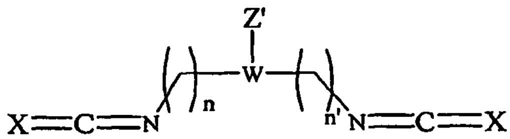

(VIII)

* n et n' étant tels que définis précédemment dans la revendication 1, et étant de préférence égaux,
* W et X étant tels que définis dans la revendication 1,
* Z' représentant un groupe répondant à l'une des formules suivantes :

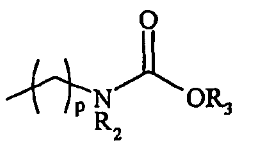 ou 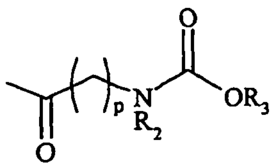 ou 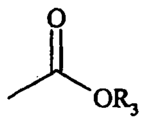 ou

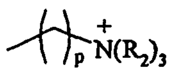 ou 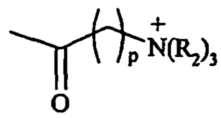 ou 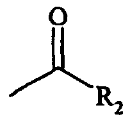

p, $R_2$ et $R_3$ étant tels que définis dans la revendication 1, afin d'obtenir un composé selon la revendication 1, répondant à la formule (IX) suivante :

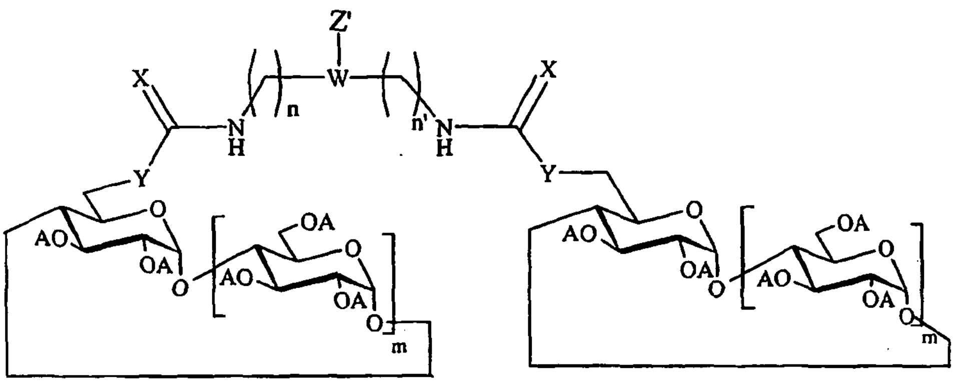

(IX)

- et éventuellement la réaction d'hydrolyse du groupement

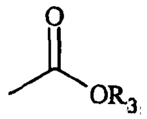

tel que défini ci-dessus, présent dans les composés de formule (IX) susmentionnée, dans laquelle Z' contient un tel groupement, afin d'obtenir un composé portant une fonctionnalité amine libre et répondant à la formule (X) suivante :

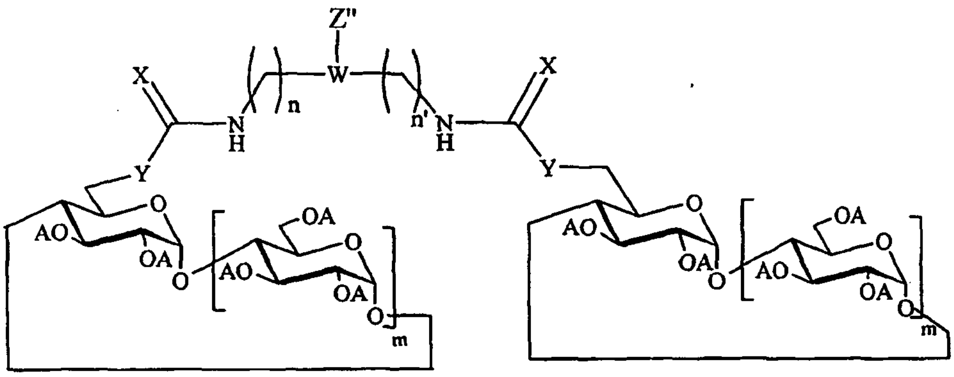

(X)

* n, n,' A, X, Y, W et m étant tels que définis précédemment, et
* Z" correspondant à l'hydrolysat du groupe Z' contenant une fonction $-COOR_3$, et représentant un atome d'hydrogène ou répondant à l'une des formules suivantes :

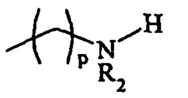

ou

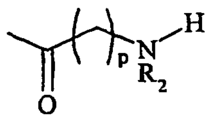

p et $R_2$ étant tels que définis dans la revendication 1,

- et éventuellement la réaction d'un composé de formule (X) tel qu'obtenu à l'étape précédente, avec un isocyanate ou un isothiocyanate de formule (XI) suivante :

R-N=C=X'

R et X' étant tels que définis dans la revendication 1,
afin d'obtenir un composé selon la revendication 1 répondant à la formule (XII) suivante :

ıte :

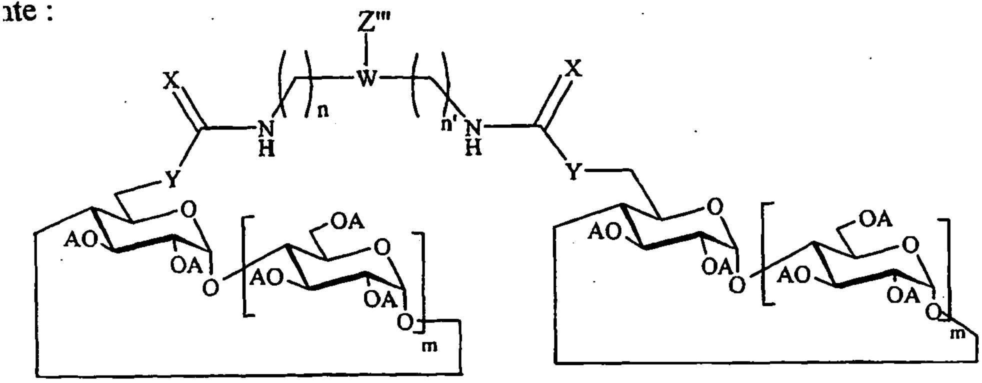

(XII)

* n, n,' A, X, Y, W et m étant tels que définis précédemment, et
* Z"' répondant à l'une des formules suivantes :

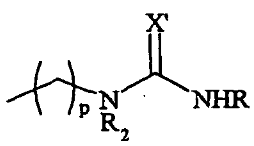

ou

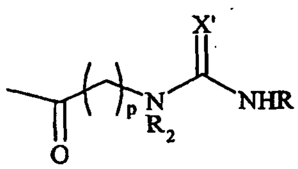

ou

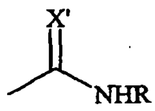

p, $R_2$, X' et R étant tels que définis dans la revendication 1.

**15.** Complexe d'inclusion d'un composé selon l'une quelconque des revendications 1 à 13, avec une molécule pharmacologiquement active, le rapport molaire entre le composé selon l'une des revendications 1 à 13 et la molécule pharmacologiquement active étant d'environ 10:1 à environ 1:2.

**16.** Complexe selon la revendication 15, **caractérisé en ce que** la molécule pharmacologiquement active est une molécule ditopique, capable d'interagir simultanément, avec deux sous-unités de cyclodextrine, telle qu'une molécule ayant deux cycles aromatiques, comme par exemple un dérivé du Taxol, ou une taille suffisamment grande, comme par exemple un stéroïde.

**17.** Complexe selon l'une quelconque des revendications 15 ou 16, **caractérisé en ce que** la molécule pharmacologiquement active est un agent antitumoral, appartenant notamment à la famille du Taxol.

**18.** Composition pharmaceutique comprenant un composé selon l'une quelconque des revendications 1 à 13, ou un complexe d'inclusion selon l'une des revendications 15 à 17, avec un véhicule pharmacologiquement acceptable.

**19.** Composition pharmaceutique selon la revendication 18, sous forme de solution aqueuse.

**20.** Composition pharmaceutique selon l'une quelconque des revendications 18 ou 19, **caractérisée en ce qu'**elle contient par dose unitaire d'environ 50 mg à environ 500 mg de l'un des composés selon l'une quelconque des revendications 1 à 13, ou **en ce qu'**elle contient par dose unitaire d'environ 100 mg à environ 750 mg de l'un des complexes selon l'une des revendications 15 à 17.

**Claims**

**1.** Compound corresponding to the following general formula:

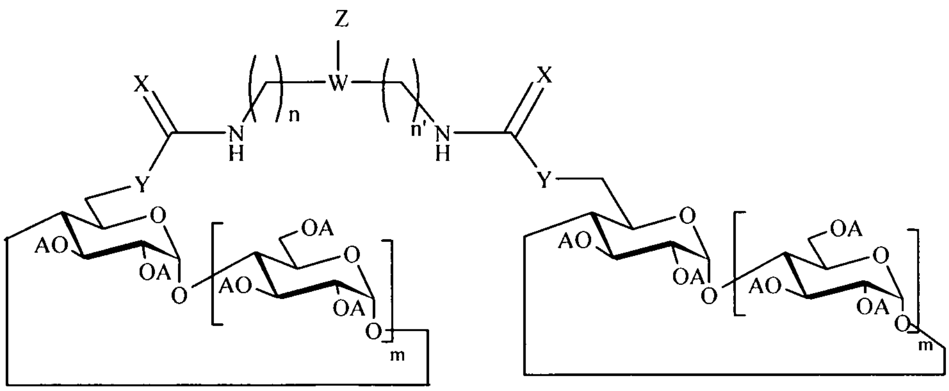

**(I)**

in which:

- m represents an integer equal to 5, 6 or 7;
- n and n' represent an integer from 1 to 5, n and n' being able to be identical or different;
- the A groups, identical or different, represent a hydrogen atom, an acyl, alkyl, hydroxyalkyl or sulphoalkyl group of 1 to 16 carbon atoms,
- X represents O or S,

- Y represents:

  * an $-NR_1-$ group, $R_1$ representing a hydrogen atom or an alkyl group comprising from 1 to 6 carbon atoms, or
  * an amide group of formula $-NH-CO-(CH_2)_q-NR_1-$, q representing an integer from 1 to 5 and $R_1$ being as defined above, or
  * a cysteaminyl group of formula $-S-(CH_2)_r-NR_1-$, r representing an integer from 2 to 5 and $R_1$ being as defined above,

- W represents CH or N;
- Z represents:

  * a hydrogen atom or
  * a carbamate substituent of formula

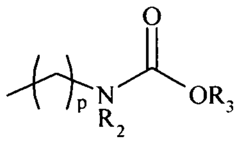

or
  * an amine substituent of formula

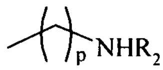

or
  * a quaternary ammonium group of formula

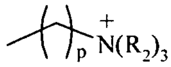

or
  * a urea or thiourea substituent of formula

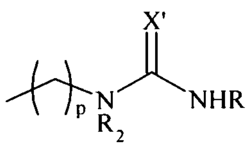

or
  * a group of formula

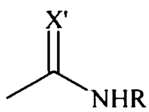

or
  * a group of the form $C(=O)OR_3$, a group of the form $C(=O)R_2$ or a group carrying the amine, ammonium quaternary urea or thiourea functionalities, of respective formulae

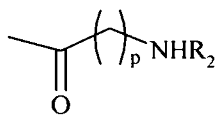

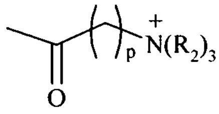

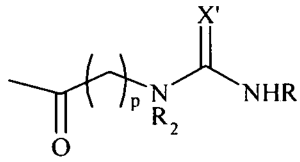

p representing an integer from 0 to 5, when W represents CH, and from 2 to 5, when W represents N,
X' representing O or S,
$R_2$ representing a hydrogen atom or an alkyl group comprising from 1 to 6 carbon atoms, and being in particular a methyl, ethyl, propyl or butyl group,
$R_3$ representing a substituent allowing the hydrolysis of the carbamate group in order to release the amine function, such as the *tert*-butyl, 9-fluorenylmethyl, benzyl, allyl or 2,2,2-trichloroethyl groups, and
R representing a hydrogen atom, a linear or branched alkyl group of 1 to 12 carbon atoms, or an aromatic group such as the phenyl, benzyl or naphthyl group, or derivatives of these groups carrying substituents on the aromatic ring such as the methyl, ethyl, chlorine, bromine, iodine, nitro, hydroxyl, methoxyl or acetamido substituents, or R representing a biological recognition element such as an amino acid derivative, a peptide, a monosaccharide, an oligosaccharide, a multiplication element with several branchings, which branchings comprise glucide groups which can be identical or different, or also a fluorescent or radioactive visualization or detection probe.

2. Compound according to claim 1, **characterized in that** n and n' are equal.

3. Compound according to claim 1 or 2, **characterized in that** all the A groups represent a hydrogen atom.

4. Compound according to claim 3, **characterized in that** Y represents either an $NR_1$ group, or an $-NH-CO-(CH_2)_q-NR_1-$ group, or an $-S-(CH_2)_r-NR_1-$ group, and corresponding to one of the following formulae respectively:

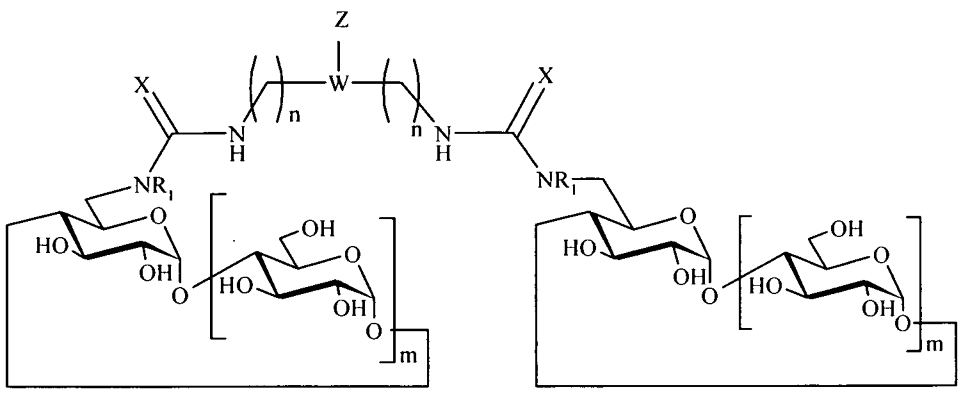

**(I-a)**

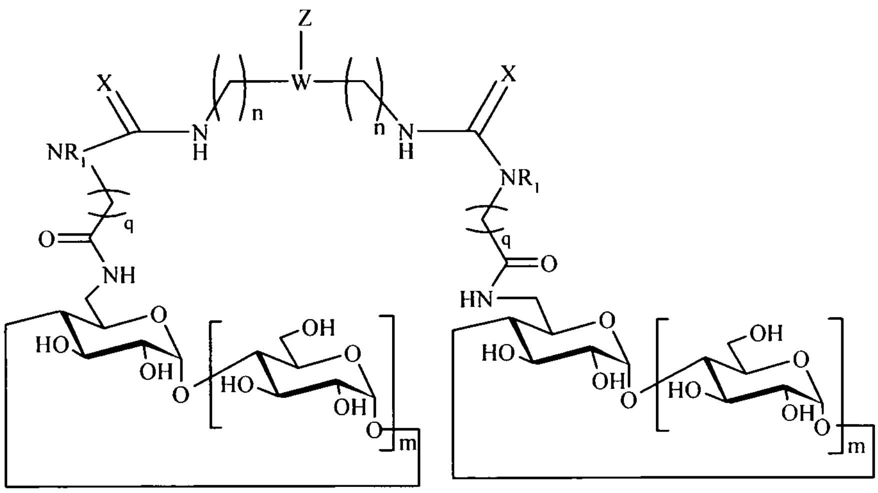

**(I-b)**

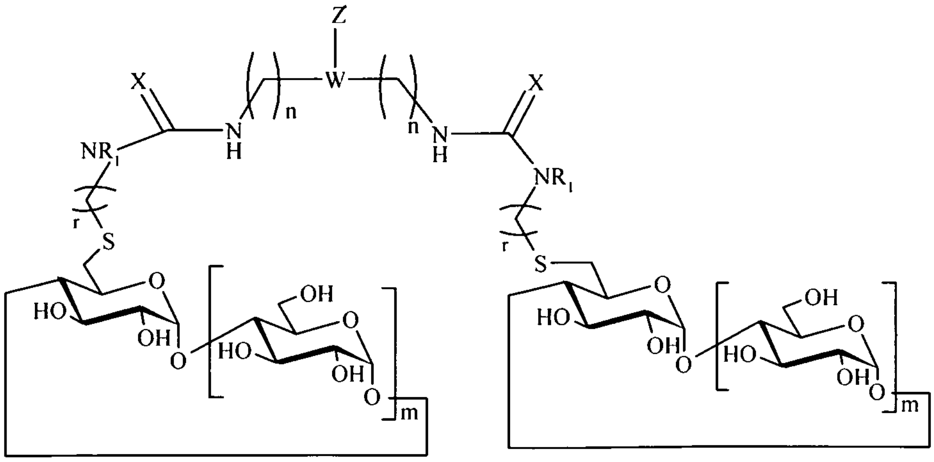

**(I-c)**

in which n, m, q, r, X, W, Z and $R_1$ are as defined in claim 1.

5. Compound according to claim 3 or 4, **characterized in that** Z represents either a $-(CH_2)_p-NHR_2$ group, or a $-(CH_2)_p-N^+(R_2)_3$ group, or a group of formula

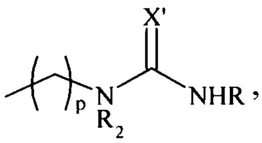

in which X' represents a sulphur atom,
and corresponding to one of the following formulae respectively:

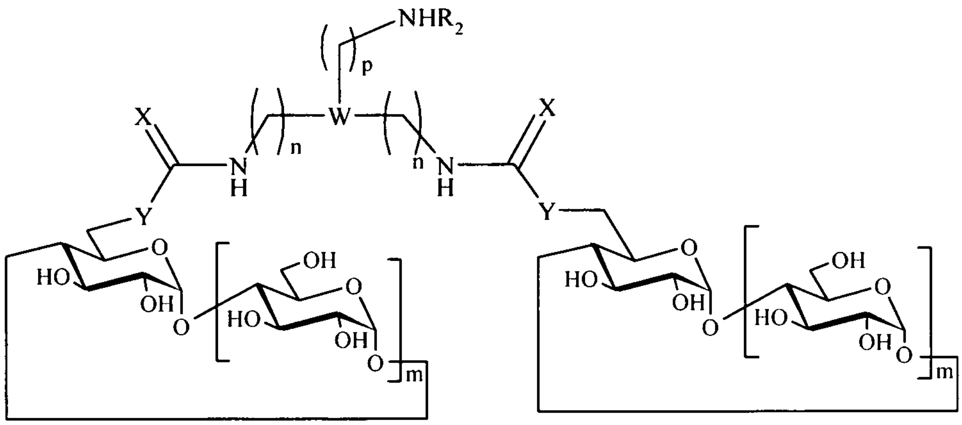

**(I-d)**

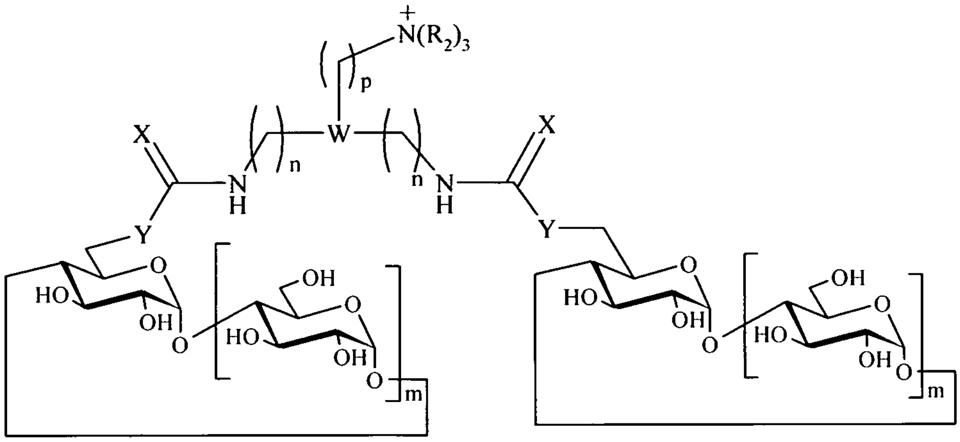

**(I-e)**

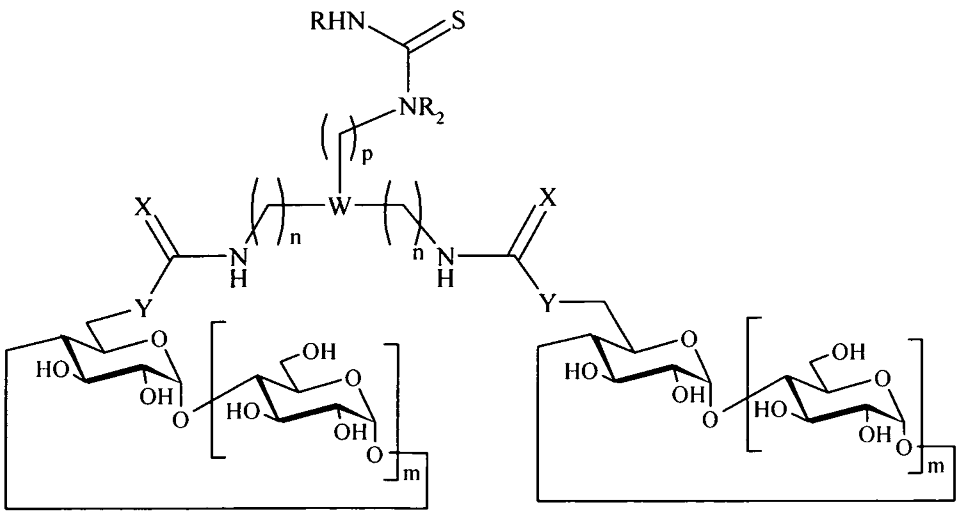

**(I-f)**

in which n, m, p, X, W, Y, R and $R_2$ are as defined in claim 1.

**6.** Compound according to any one of claims 3 to 5, **characterized in that** W represents a nitrogen atom and **in that** Z represents either a group of formula $-CO-(CH_2)_p-NHR_2$, or a group of formula $-CO-(CH_2)_p-N(R_2)_3$, or a group of formula

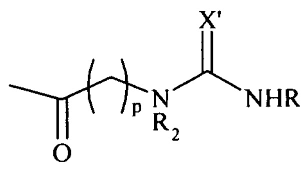

in which X' represents a sulphur atom, or a group of formula

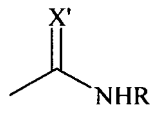

in which X' represents a sulphur atom and corresponding to one of the following formulae respectively:

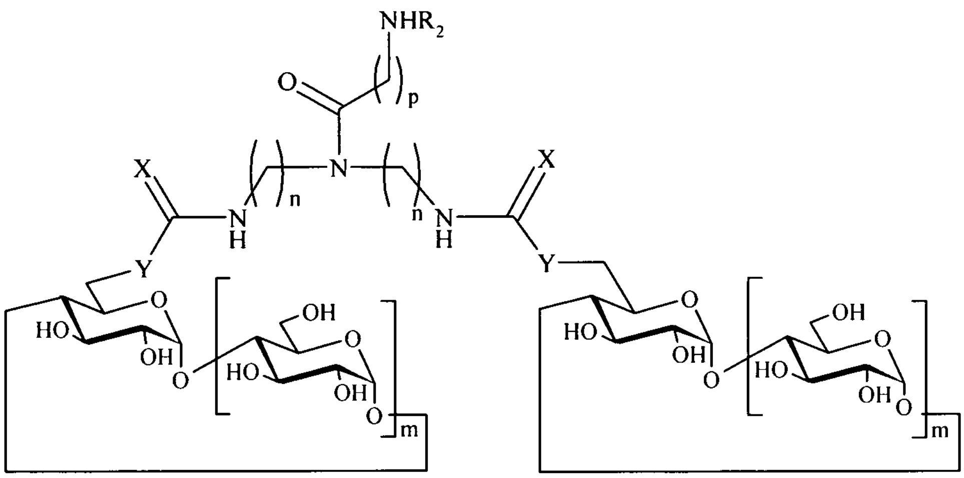

(I-g)

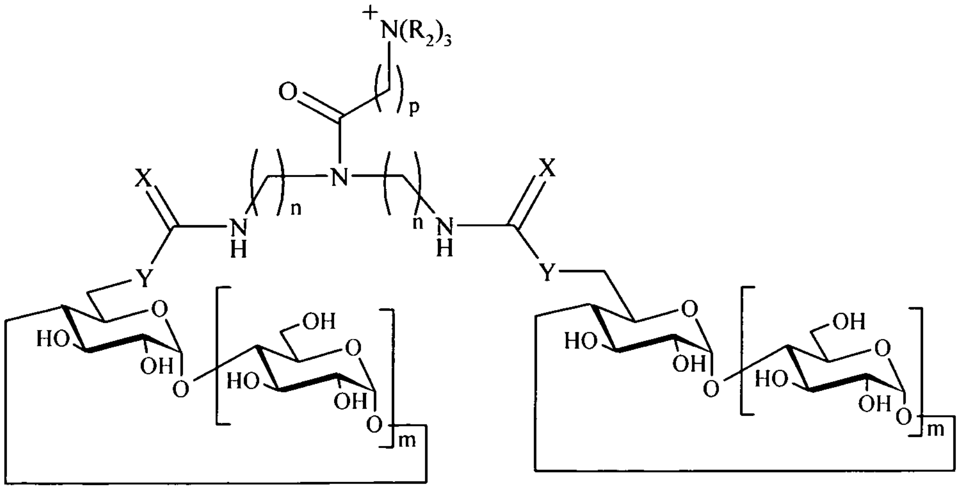

(I-h)

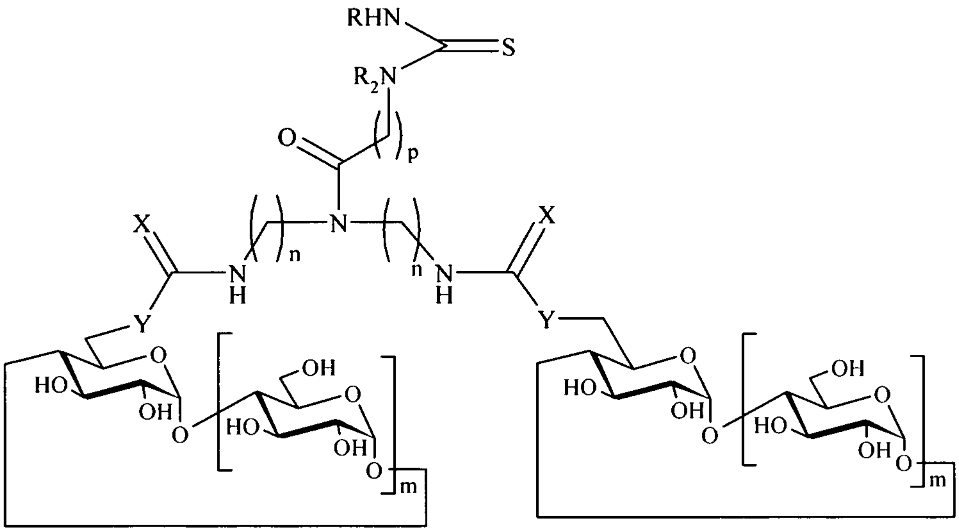

(I-i)

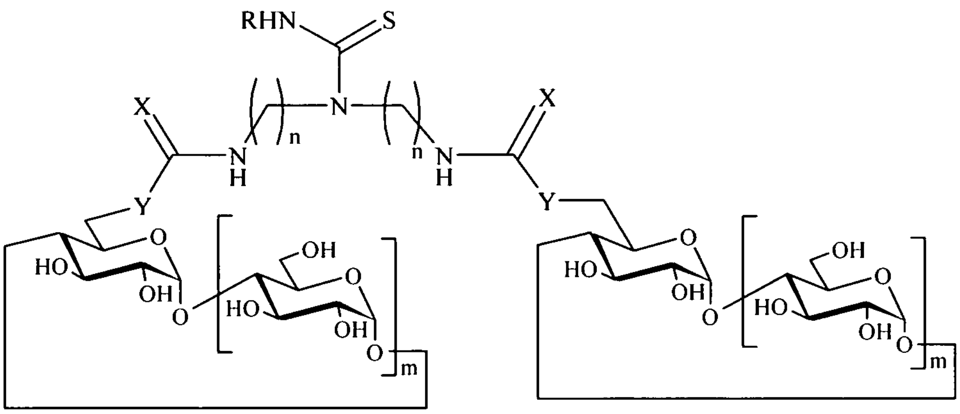

(I-j)

in which n, m, p, X, Y, R and $R_2$ are as defined in claim 1.

**7.** Compound according to any one of claims 1 to 6, **characterized in that** R is chosen from the following groups:

- an alkyl group of 1 to 12 carbon atoms, linear or branched, and preferably being the methyl group;
- an aromatic group such as phenyl, benzyl, naphthyl or derivatives of these groups carrying substituents on the aromatic ring, and preferably being the phenyl group;
- the α-D-mannopyranosyl group, of the following formula (III):

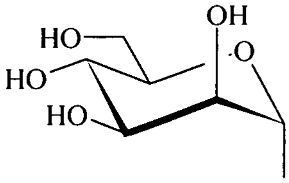

(III)

- the β-lactosyl group, of the following formula (III-a):

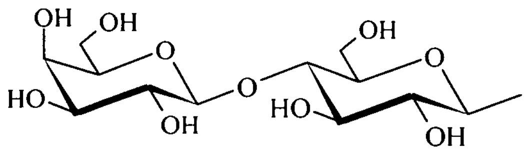

**(III-a)**

- the group derived from Lewis X trisaccharide or from sialyl Lewis X tetrasaccharide, of the following formulae (III-b) and (III-c) respectively:

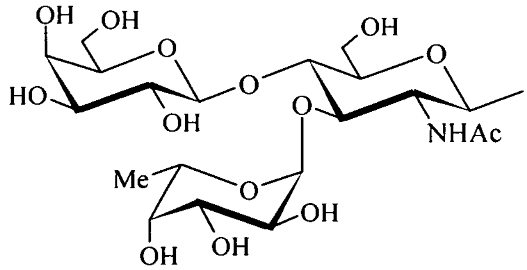

**(III-b)**

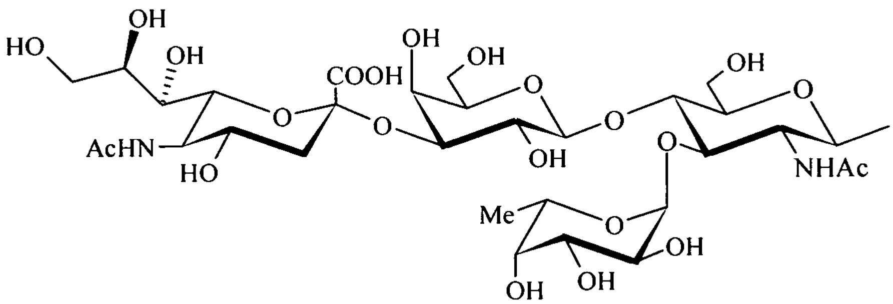

**(III-c)**

- an oligosaccharide derived from heparin, of the following formula (III-d):

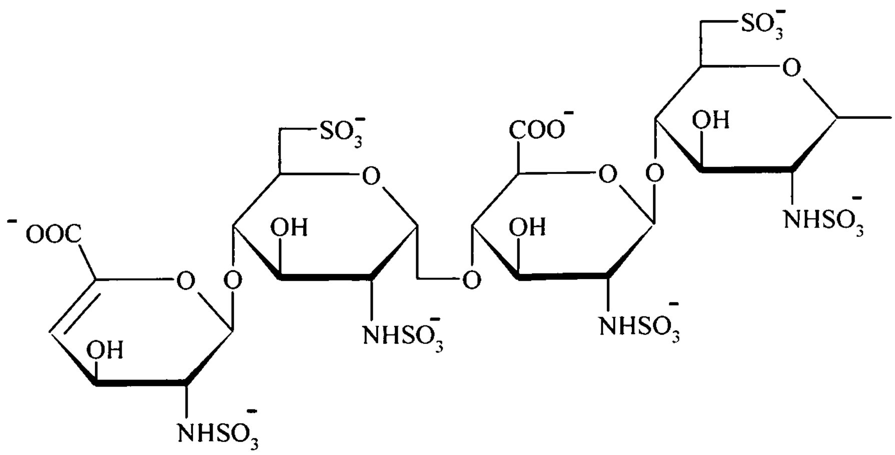

**(III-d)**

8. Compound according to any one of claims 1 to 6, **characterized in that** R comprises a branching element derived from tris(2-hydroxymethyl)methylamine, and represents one of the following groups:

- the tris(α-D-mannopyranosyloxymethyl)methyl group, of the following formula (IV):

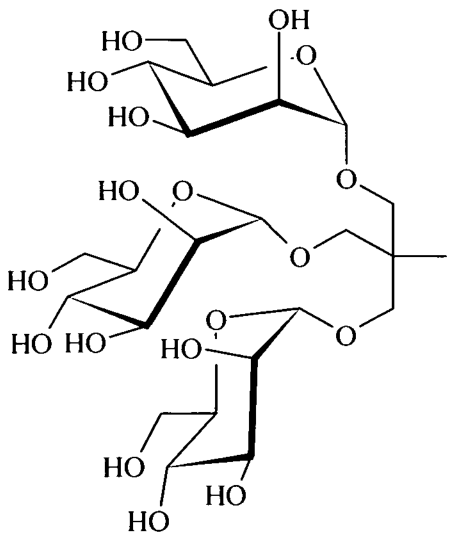

(IV)

- the tris(β-lactosyloxymethyl)methyl group, of the following formula (IV-a):

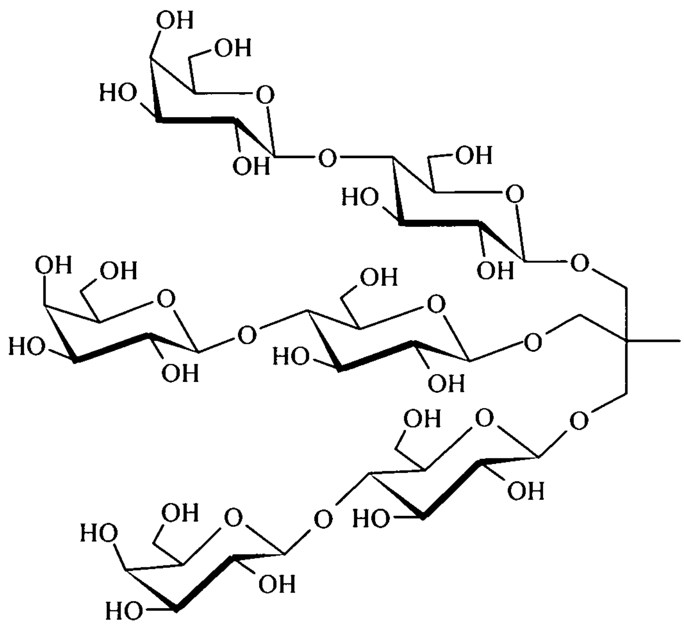

(IV-a)

**9.** Compound according to any one of claims 1 to 6, **characterized in that** R comprises a branching element derived from pentaerythritol, said compound corresponding to one of the following formulae:

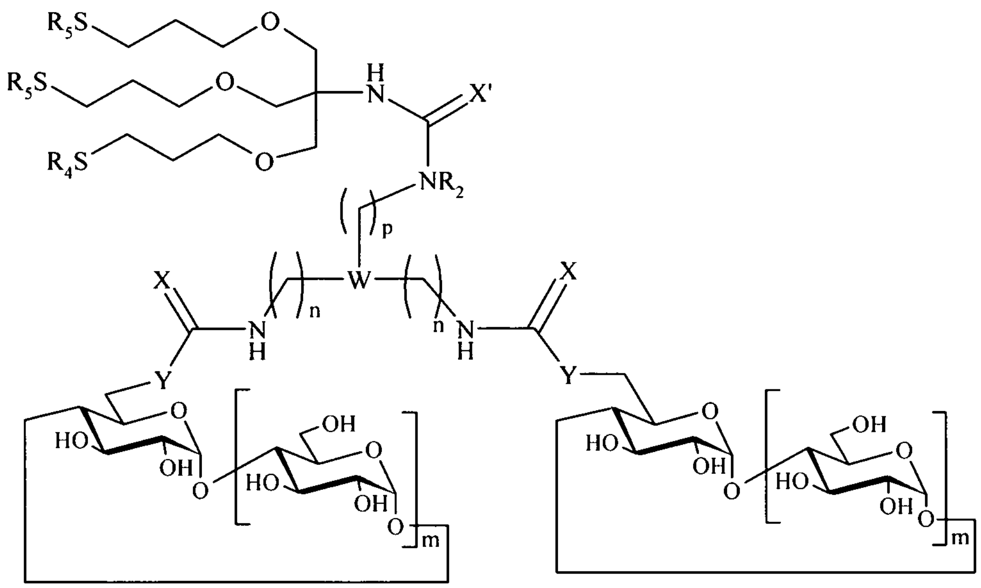

(II-a)

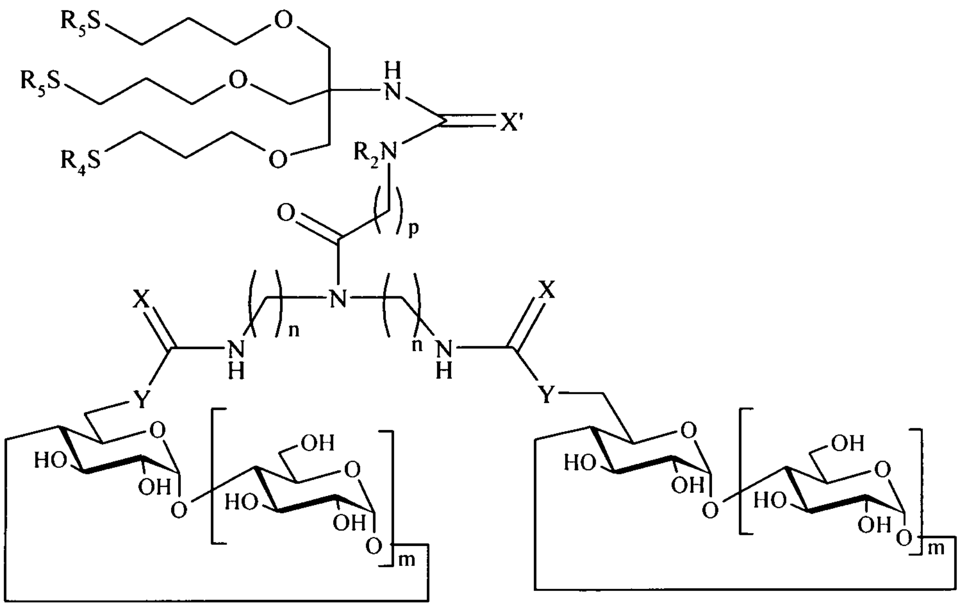

(II-b)

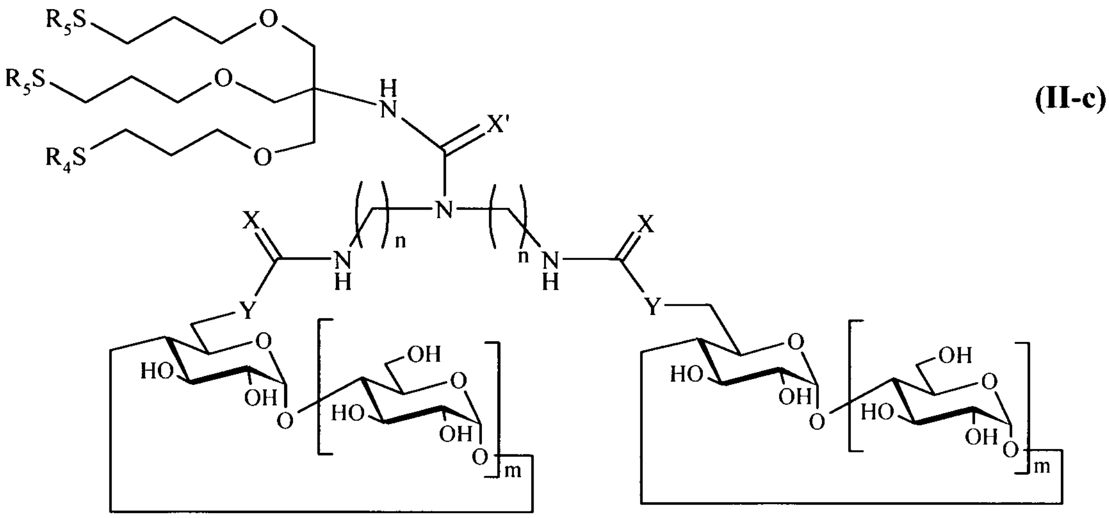

(II-c)

in which m, n, p, X, X', Y are as defined in claim 1, and
$R_5$ and $R_4$ represent glucide derivatives which can be different or identical or also a fluorescent or radioactive probe.

**10.** Compound according to claim 9, **characterized in that** $R_5$ and $R_4$ represent one of the following groups:

- the α-D-mannopyranosyl group, of formula (III) as defined in claim 7, or
- the β-lactosyl group, of formula (III-a) as defined in claim 7, or
- the β-D-glucopyranosyl group, of the following formula (VI):

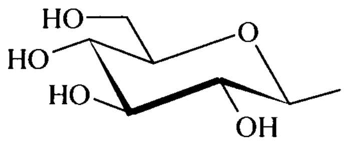

(VI)

$R^5$ and $R^4$ being able to be identical or different.

**11.** Compound according to any one of claims 1 to 6, **characterized in that** R comprises a branching element derived from tris(2-aminoethyl)amine (TREN), said compound corresponding to one of the following formulae:

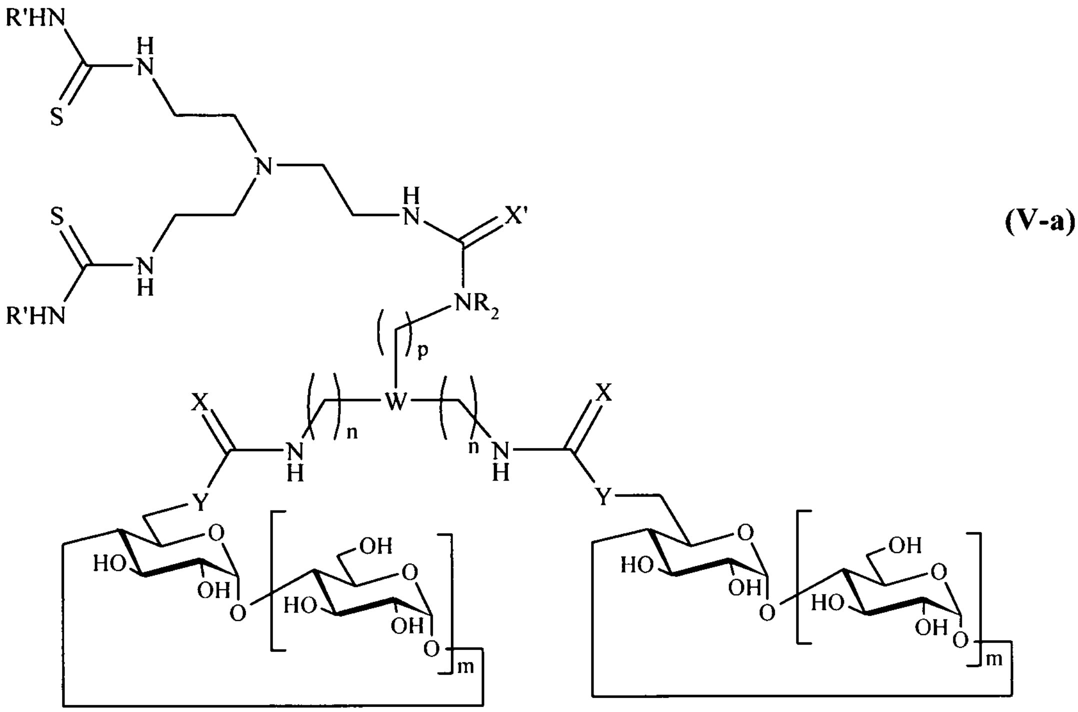

(V-a)

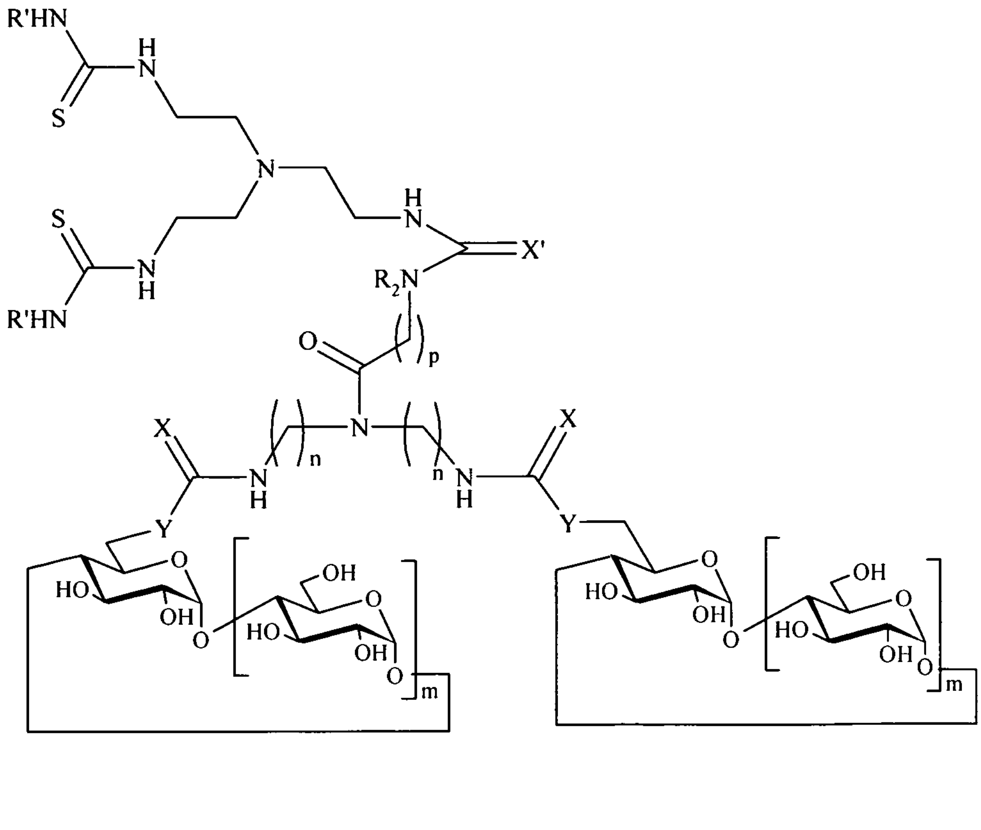

(V-b)

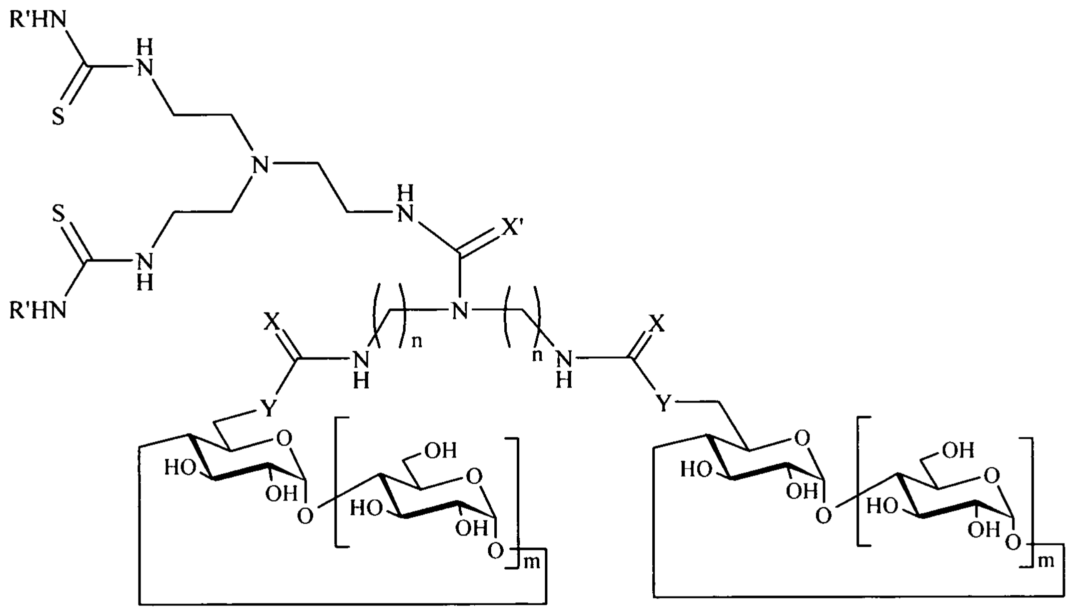

(V-c)

m, n, p, X, X', Y being as defined in claim 1, and
R' having the definition given previously for R.

**12.** Compound according to claim 11, **characterized in that** R' represents

- the α-D-mannopyranosyl group, of formula (III), or
- the β-lactosyl group of formula (III-a), or
- the tris(α-D-mannopyranosyloxymethyl)methyl group, of formula (IV), or
- the tris(β-lactyloxymethyl)methyl group, of formula (IV-a).

**13.** Compound according to one of claims 1 to 12, **characterized in that** m is equal to 6.

**14.** Method for preparing a compound according to claim 1, **characterized in that** it comprises the following stages:

- the reaction of a compound selectively functionalized in primary alcohol position with an amine group, of the following formula (VII):

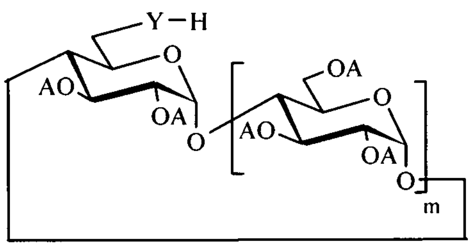

(VII)

m, A and Y being as defined previously in claim 1, and A preferably being a hydrogen atom,
with a dimerization element of diisocyanate or diisothiocyanate type, in particular carrying a protected amine functionality in the form of a carbamate group or carrying a positively charged quaternary ammonium salt functionality, of the following formula (VIII):

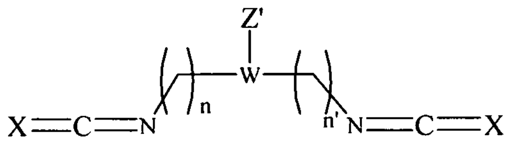

**(VIII)**

* n and n' being as defined previously in claim 1, and preferably being equal,
* W and X being as defined in claim 1,
* Z' representing a group corresponding to one of the following formulae:

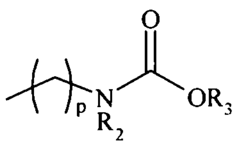 or 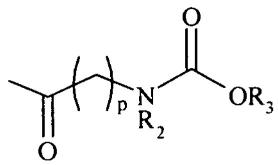 or 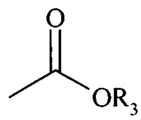 or

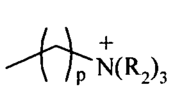 or 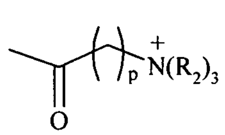 or 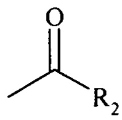

p, $R_2$ and $R_3$ being as defined in claim 1, in order to obtain a compound according to claim 1, corresponding to the following formula (IX):

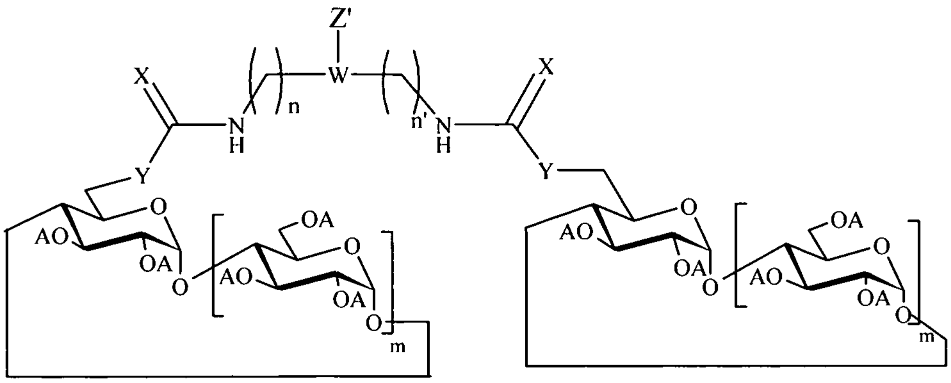

**(IX)**

- and optionally the hydrolysis reaction of the

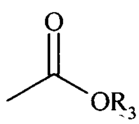

group as defined above, present in the compounds of the abovementioned formula (IX), in which Z' contains such a group, in order to obtain a compound carrying a free amine functionality and corresponding to the following formula (X):

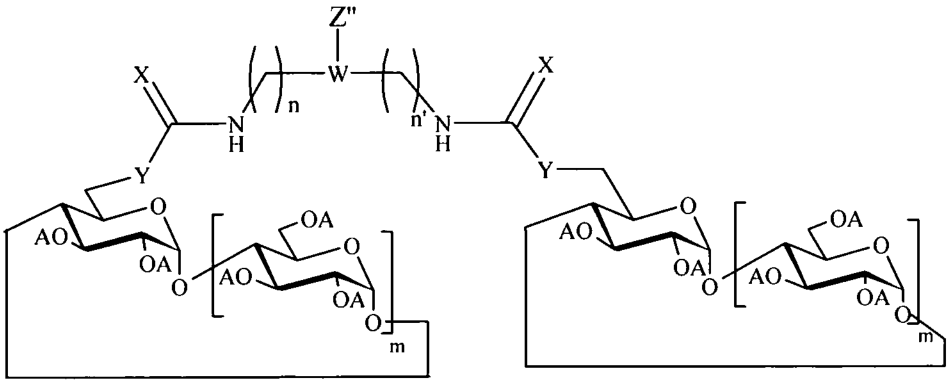

(X)

* n, n,' A, X, Y, W and m being as defined previously, and
* Z" corresponding to the hydrolysate of the Z' group containing a $-COOR_3$ function, and representing a hydrogen atom or corresponding to one of the following formulae:

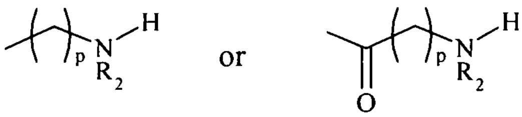

p and $R_2$ being as defined in claim 1,

- and optionally the reaction of a compound of formula (X) as obtained in the preceding stage, with an isocyanate or an isothiocyanate of the following formula (XI):

R-N=C=X'

R and X' being as defined in claim 1,
in order to obtain a compound according to claim 1 corresponding to the following formula (XII):

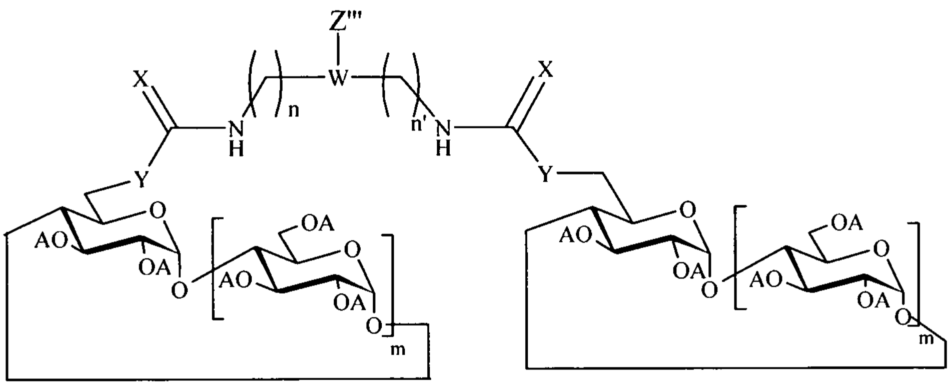

(XII)

* n, n,' A, X, Y, W and m being as defined previously, and
* Z"' corresponding to one of the following formulae:

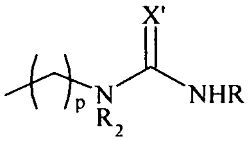 or 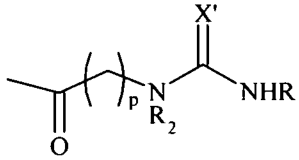 or 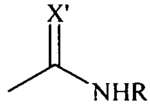

p, $R_2$, X' and R being as defined in claim 1.

**15.** Inclusion complex of a compound according to any one of claims 1 to 13, with a pharmacologically active molecule, the molar ratio between the compound according to one of claims 1 to 13 and the pharmacologically active molecule being approximately 10:1 1 to approximately 1:2.

**16.** Complex according to claim 15, **characterized in that** the pharmacologically active molecule is a ditopic molecule, capable of interacting simultaneously with two cyclodextrin sub-units, such as a molecule having two aromatic rings, such as for example a Taxol derivative, or a sufficiently large size, such as for example a steroid.

**17.** Complex according to any one of claims 15 or 16, **characterized in that** the pharmacologically active molecule is an antineoplastic agent, belonging in particular to the taxol family.

**18.** Pharmaceutical composition comprising a compound according to any one of claims 1 to 13, or an inclusion complex according to one of claims 15 to 17, with a pharmacologically acceptable vehicle.

**19.** Pharmaceutical composition according to claim 18, in the form of aqueous solution.

**20.** Pharmaceutical composition according to any one of claims 18 or 19, **characterized in that** it contains per unit dose approximately 50 mg to approximately 500 mg of one of the compounds according to any one of claims 1 to 13, or **in that** it contains per unit dose approximately 100 mg to approximately 750 mg of one of the complexes according to one of claims 15 to 17.

**Patentansprüche**

**1.** Verbindung, die der folgenden allgemeinen Formel entspricht:

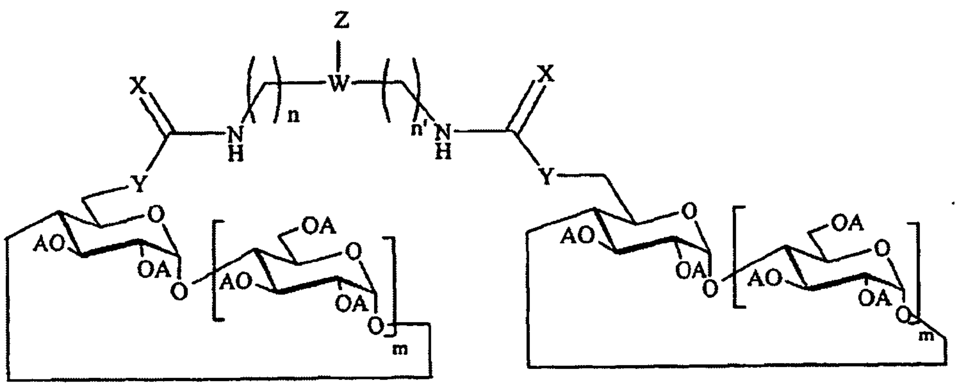 (I)

worin:

- m für eine ganze Zahl gleich 5, 6 oder 7 steht;
- n und n' für eine ganze Zahl im Bereich von 1 bis 5 stehen, wobei n und n' gleich oder verschieden sein können;
- die Gruppen A, gleich oder verschieden, für ein Wasserstoffatom, eine Acyl-, Alkyl-, Hydroxyalkyl- oder Sulfoalkylgruppe mit 1 bis 16 Kohlenstoffatomen stehen,
- X für O oder S steht,

- Y für Folgendes steht:

    * eine Gruppe $-NR_1-$, wobei $R_1$ für ein Wasserstoffatom oder eine Alkylgruppe steht, die 1 bis 6 Kohlenstoffatome umfasst, oder
    * eine Amidgruppe der Formel $-NH-CO(CH_2)_q-NR_1-$, wobei q für eine ganze Zahl im Bereich von 1 bis 5 steht und $R_1$ so ist, wie oben definiert, oder
    * eine Cysteaminylgruppe der Formel $-S-(CH_2)_r-NR_1-$, wobei r für eine ganze Zahl im Bereich von 2 bis 5 steht und $R_1$ so ist, wie oben definiert,

- W für CH oder N steht;
- Z für Folgendes steht:

    * ein Wasserstoffatom oder
    * einen Carbamatsubstituenten der Formel

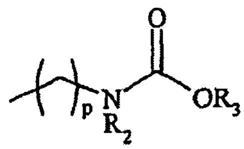

oder
* einen Aminsubstituenten der Formel

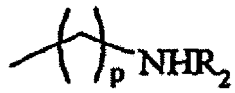

oder
* eine quartäre Ammoniumgruppe der Formel

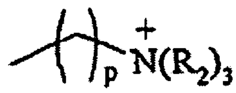

oder
* einen Harnstoff- oder Thioharnstoffsubstituenten der Formel

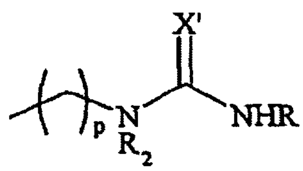

oder
* eine Gruppe der Formel

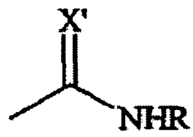

oder

* eine Gruppe der Form $C(=O)OR_3$, eine Gruppe der Form $C(=O)R_2$ oder eine Gruppe, die die Amin-, quartären Ammonium-, Harnstoff- oder Thioharnstofffunktionalitäten tragen, mit den jeweiligen Formeln

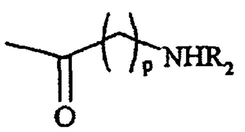

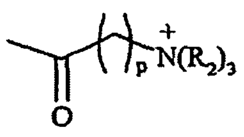

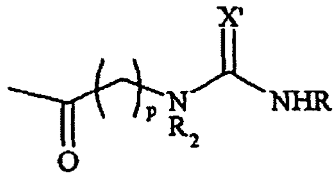

wobei p für eine ganze Zahl im Bereich von 0 bis 5 steht, wenn W für CH steht, und von 2 bis 5, wenn W für N steht, X' für O oder S steht,

$R_2$ für ein Wasserstoffatom oder eine Alkylgruppe steht, die 1 bis 6 Kohlenstoffatome umfasst, und insbesondere eine Methyl-, Ethyl-, Propyl- oder Butylgruppe ist,

$R_3$ für einen Substituenten steht, der die Hydrolyse der Carbamatgruppe ermöglicht, um die Aminfunktion frei zu setzen, wie etwa die *tert*-Butyl-, 9-Fluorenylmethyl-, Benzyl-, Allyl- oder 2,2,2-Trichlorethylgruppen, und:

R für ein Wasserstoffatom, eine Alkylgruppe, linear oder verzweigt, mit 1 bis 12 Kohlenstoffatomen, oder eine aromatische Gruppe, wie etwa die Phenyl-, Benzyl-oder Naphtylgruppe steht, oder Derivate dieser Gruppen, die Substituenten auf dem aromatischen Ring tragen, wie etwa die Methyl-, Ethyl-, Chlor-, Brom-, Iod-, Nitro-, Hydroxyl-, Methoxyl- oder Acetamidosubstiuenten, oder

R für ein biologisches Erkennungselement, wie etwa ein Aminosäurederivat, ein Peptid, ein Monosaccharid, ein Oligosaccharid, ein Multiplikationselement mit mehreren Verzweigungen, wobei die Verzweigungen Kohlenhydratgruppen umfassen, die gleich oder verschieden sein können, oder auch eine Sonde zur Visualisierung oder zum Fluoreszenz- oder Radioaktivitätsnachweis steht.

2. Verbindung nach Anspruch 1, **dadurch gekennzeichnet, dass** n und n' gleich sind.

3. Verbindung nach Anspruch 1 oder 2, **dadurch gekennzeichnet, dass** alle Gruppen A für ein Wasserstoffatom stehen.

4. Verbindung nach Anspruch 3, **dadurch gekennzeichnet, dass** Y entweder für eine Gruppe $NR_1$ oder eine Gruppe $-NH-CO-(CH_2)_q-NR_1-$ oder eine Gruppe $-S(CH_2)_r NR_1-$ steht und jeweils einer der folgenden Formeln entspricht:

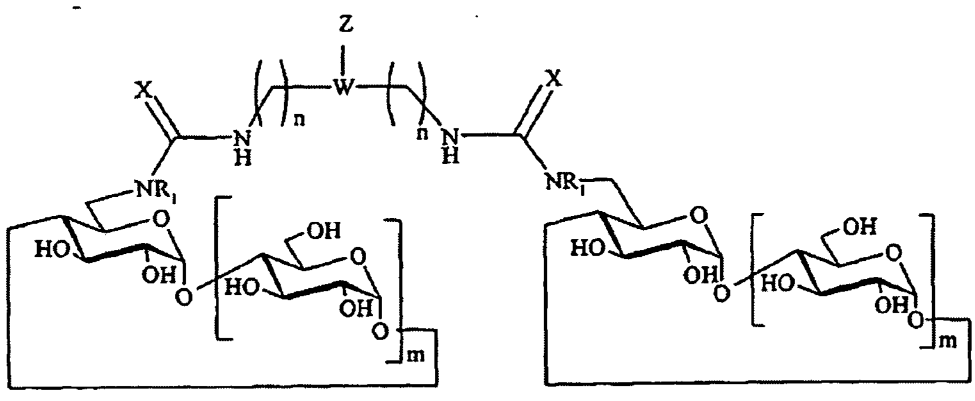

**(I-a)**

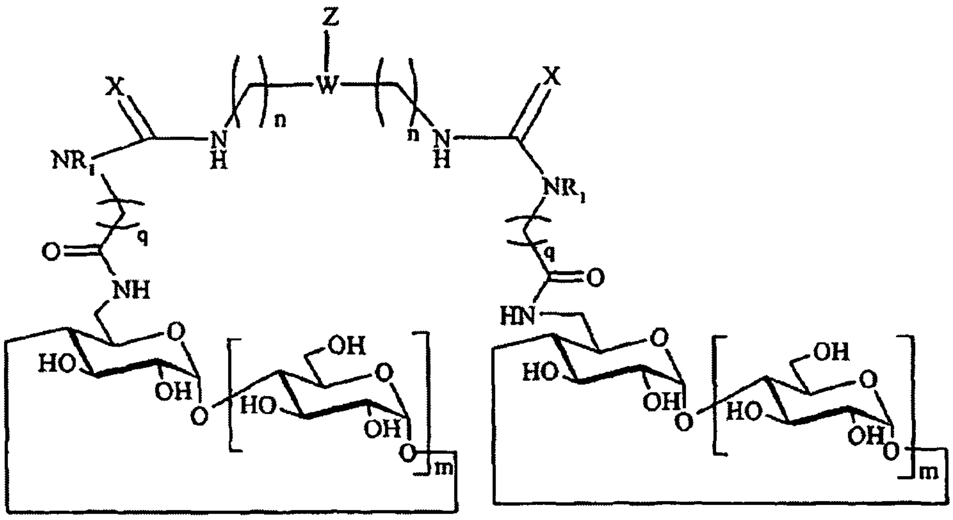

(I-b)

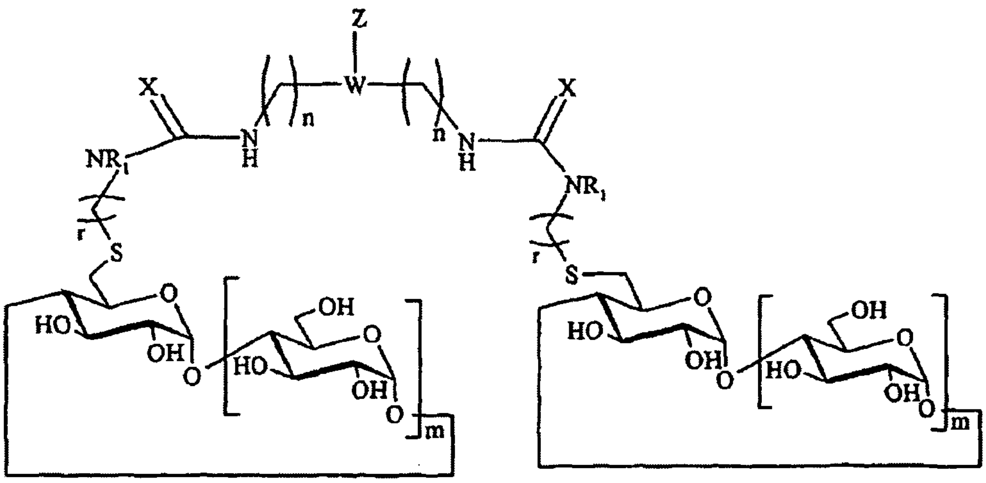

(I-c)

worin n, m, q, r, X, W, Z und $R_1$ so sind, wie in Anspruch 1 definiert.

5. Verbindung nach Anspruch 3 oder 4, **dadurch gekennzeichnet, dass** Z entweder für eine Gruppe - $(CH_2)_p$-$NHR_2$ oder eine Gruppe -$(CH_2)_p$-$N(R_2)_3$ oder eine Gruppe mit der Formel

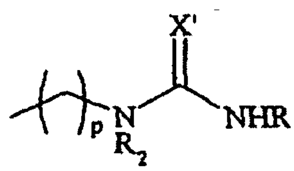

steht, worin X' für ein Schwefelatom steht und jeweils einer der folgenden Formeln entspricht:

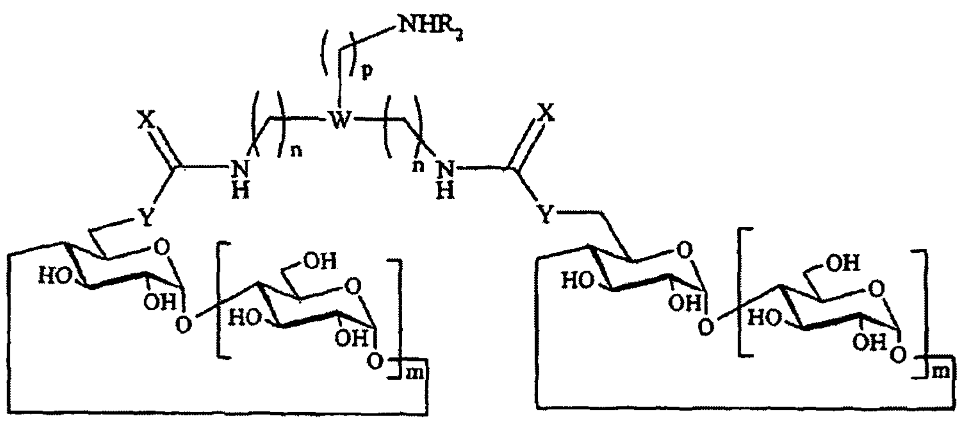

**(I-d)**

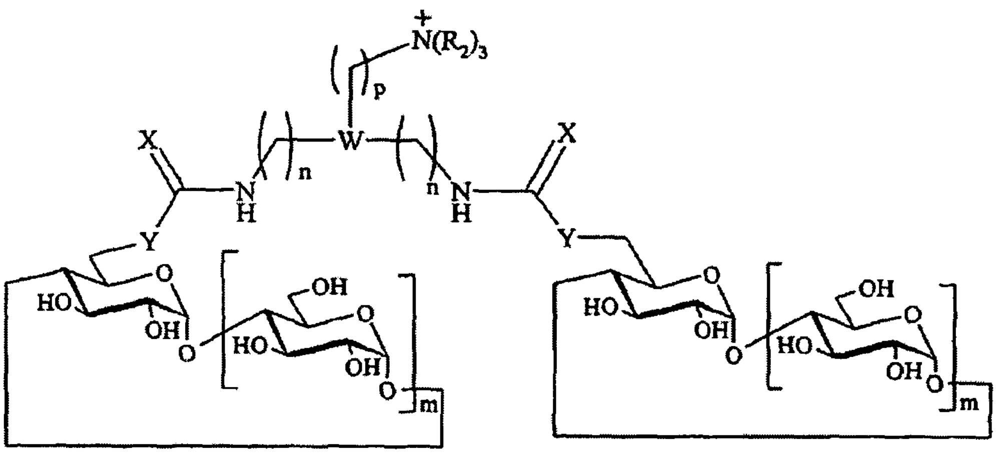

**(I-e)**

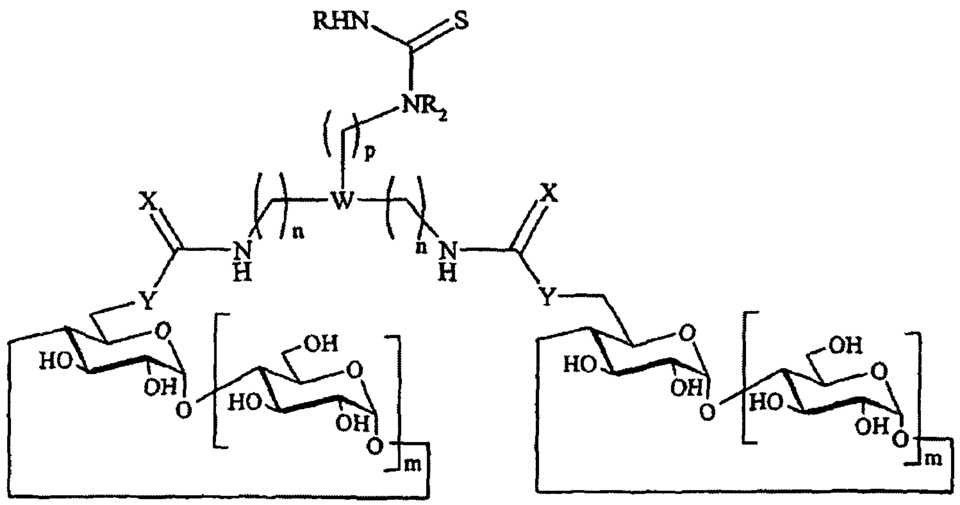

**(I-f)**

worin n, m, p, X, W, Y, R und $R_2$ so sind, wie in Anspruch 1 definiert.

**6.** Verbindung nach einem der Ansprüche 3 bis 5, **dadurch gekennzeichnet, dass** W für ein Stickstoffatom steht und dadurch, dass Z entweder für eine Gruppe der Formel $-CO-(CH_2)_p-NHR_2$ oder eine Gruppe der Formel $-CO-(CH_2)_p-N(R_2)_3$ oder eine Gruppe der Formel

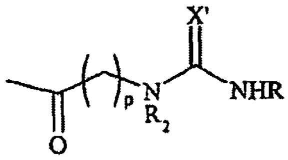

,

worin X' für ein Schwefelatom steht, oder für eine Gruppe der Formel

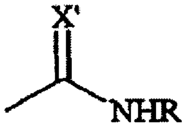

steht, worin X' für ein Schwefelatom steht und jeweils einer der folgenden Formeln entspricht:

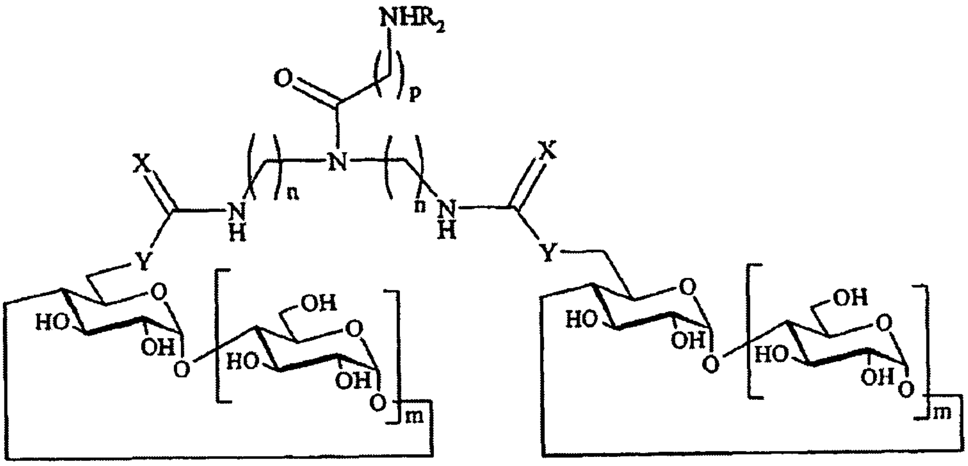

(I-g)

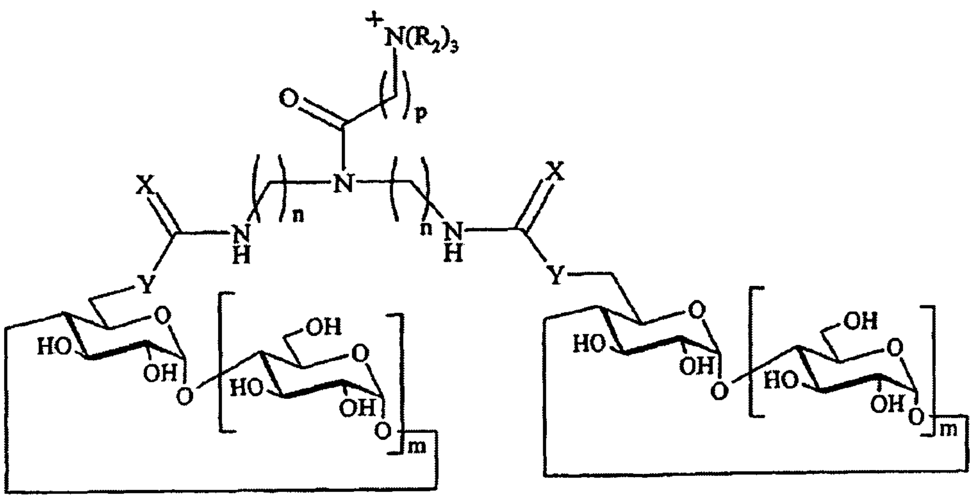

(I-h)

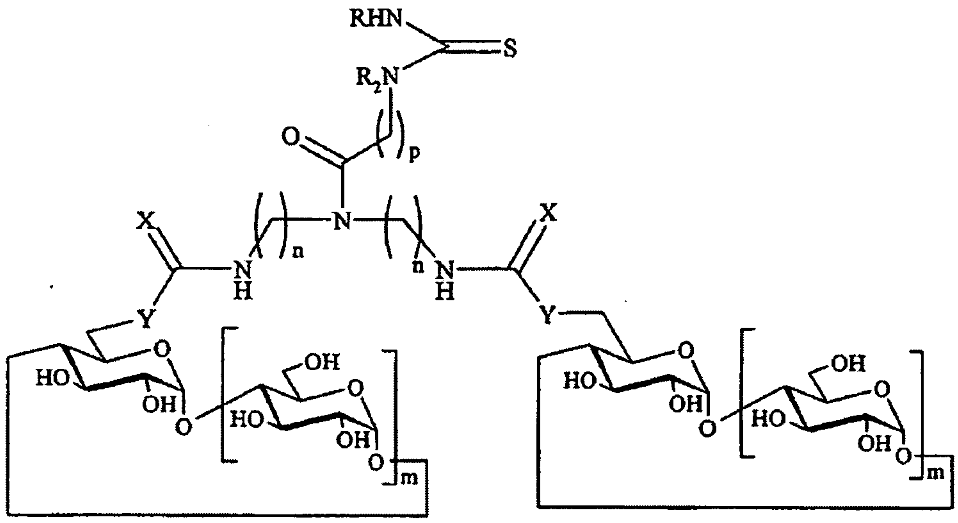

(I-i)

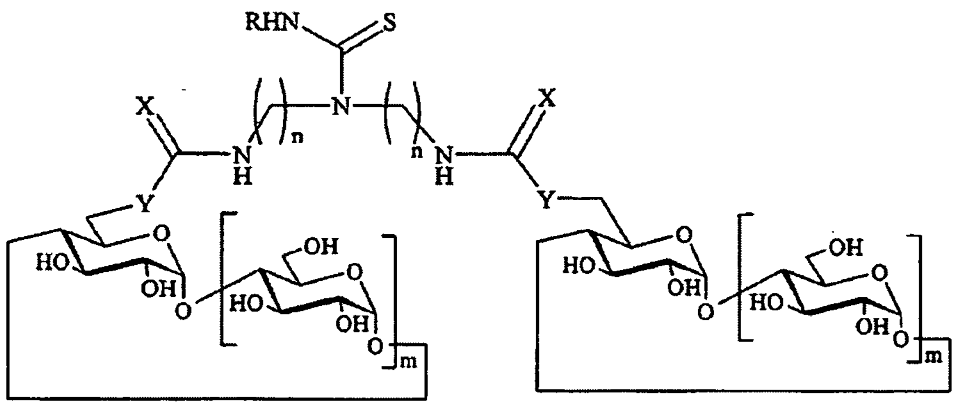

(I-j)

worin n, m, p, X, Y, R und $R_2$ so sind, wie in Anspruch 1 definiert.

7. Verbindung nach einem der Ansprüche 1 bis 6, **dadurch gekennzeichnet, dass** R aus den folgenden Gruppen ausgewählt ist:

- einer Alkylgruppe mit 1 bis 12 Kohlenstoffatomen, linear oder verzweigt, und die bevorzugt die Methylgruppe ist;
- einer aromatischen Gruppe, wie etwa der Phenyl-, Benzyl-, Naphtylgruppe oder Derivaten dieser Gruppen, die Substituenten auf dem aromatischen Ring tragen und die bevorzugt die Phenylgruppe ist;
- der α-D-Mannopyranosylgruppe mit der folgenden Formel (III):

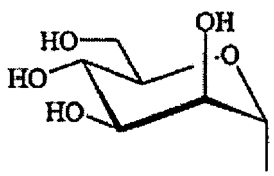

(III)

- der β-Lactosylgruppe mit der folgenden Formel (III-a):

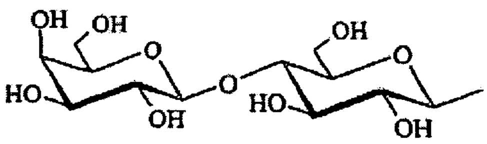

(III-a)

- der Gruppe, die von dem Trisaccharid Lewis-X oder dem Tetrasaccharid Sialyl-Lewis-X abgeleitet ist, mit der folgenden Formel (III-b) bzw. (III-c):

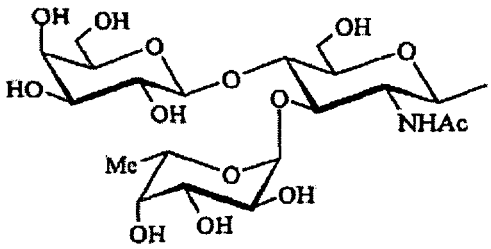

(III-b)

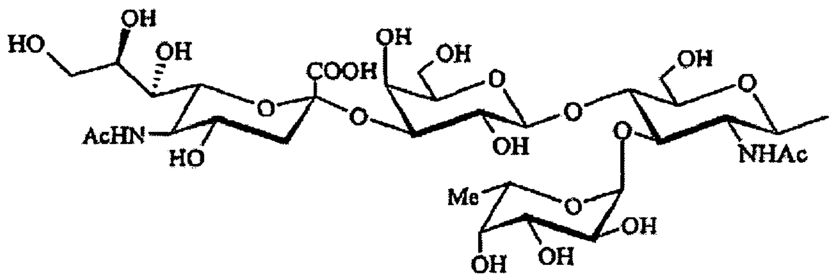

(III-c)

- einem Oligosaccharid, das von Heparin abgeleitet ist, mit der folgenden Formel (III-d):

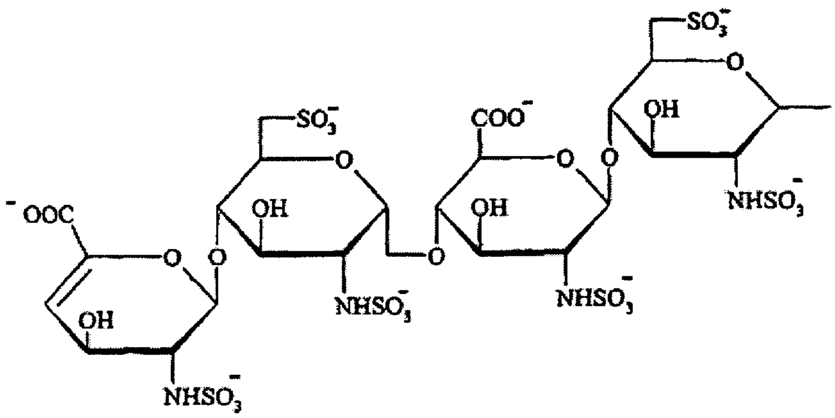

(III-d)

**8.** Verbindung nach einem der Ansprüche 1 bis 6, **dadurch gekennzeichnet, dass** R ein Verzweigungselement umfasst, das von Tris(2-hydroxymethyl)methylamin abgeleitet ist, und für eine der folgenden Gruppen steht:

- der Tris-α-D-mannopyranosyloxymethyl)methylgruppe mit der folgenden Formel (IV):

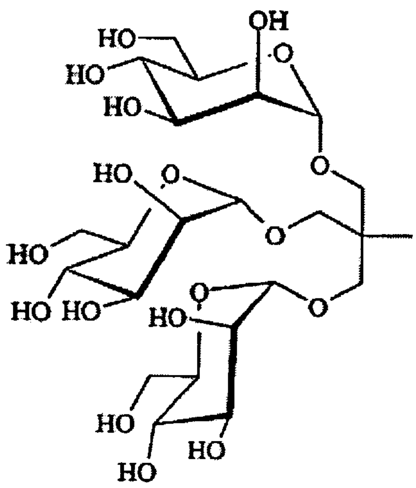

**(IV)**

- der Tris(β-lactosyloxymethyl)methylgruppe mit der folgenden Formel (IV-a):

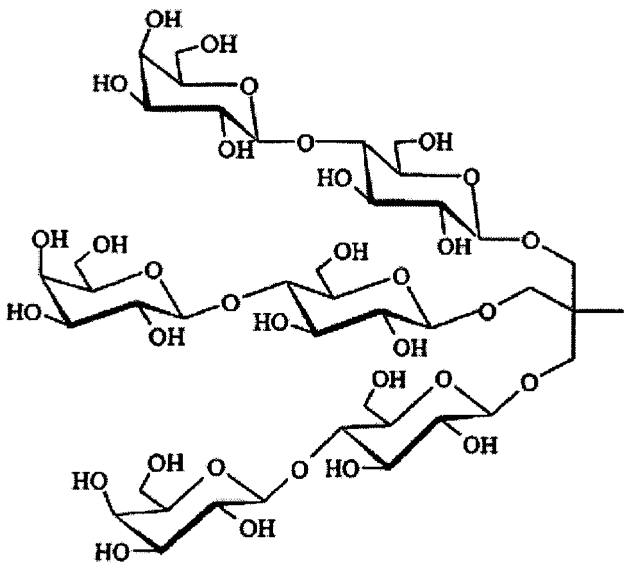

**(IV-a)**

**9.** Verbindung nach einem der Ansprüche 1 bis 6, **dadurch gekennzeichnet, dass** R ein Verzweigungselement umfasst, das von Pentaerythritol abgeleitet ist, wobei die Verbindung einer der folgenden Formeln entspricht:

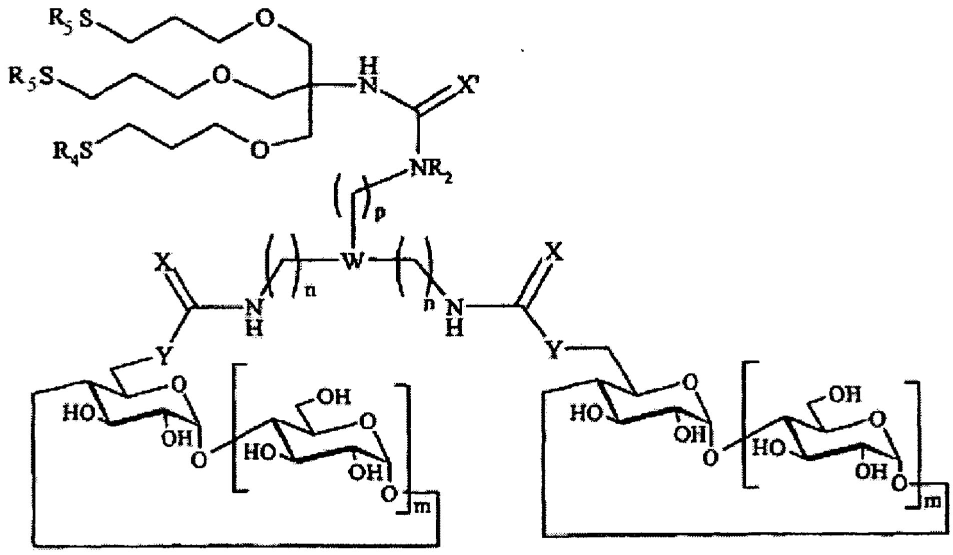

(II-a)

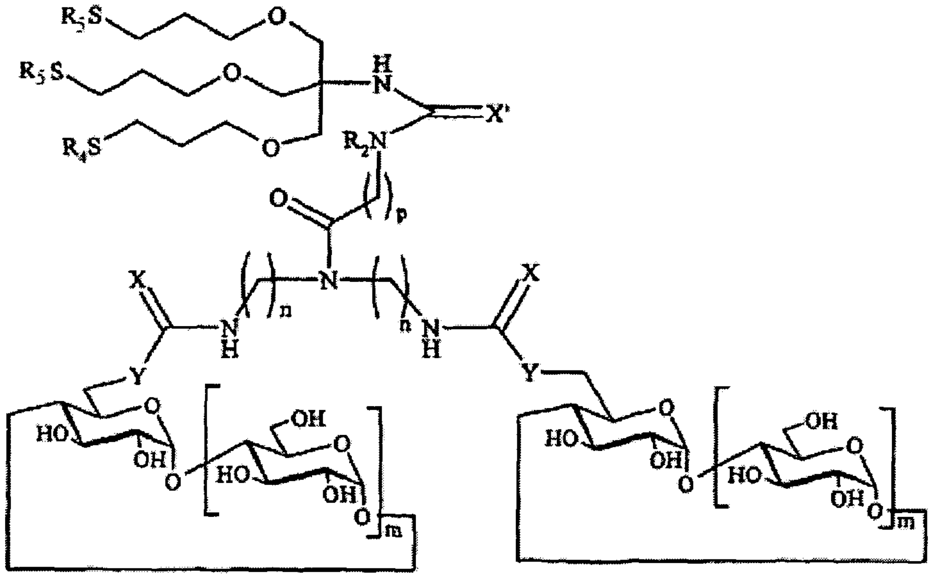

(II-b)

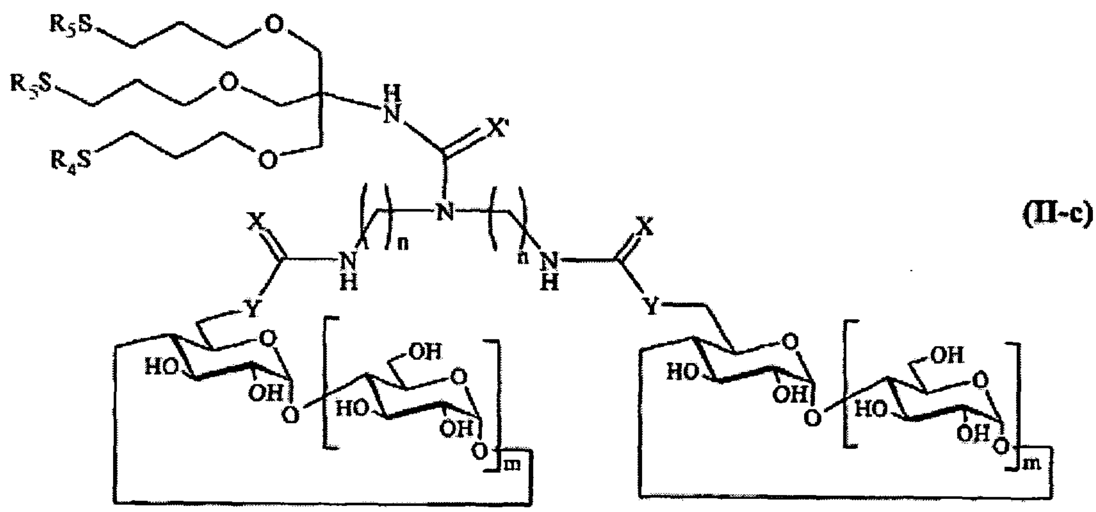

(II-c)

worin m, n, p, X, X', Y so sind, wie in Anspruch 1 definiert, und $R_5$ und $R_4$ für Kohlenhydratderivate, die verschieden oder gleich sein können, oder auch eine fluoreszierende oder radioaktive Sonde stehen.

**10.** Verbindung nach Anspruch 9, **dadurch gekennzeichnet, dass** $R_5$ und $R_4$ für eine der folgenden Gruppen stehen:

- die α-D-Mannopyranosylgruppe der Formel (III), wie in Anspruch 7 definiert, oder
- die β-Lactosylgruppe der Formel (III-a), wie in Anspruch 7 definiert, oder
- die β-D-Glucopyranosylgruppe mit der folgenden Formel (VI):

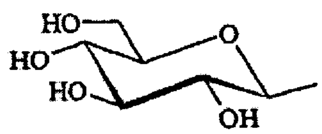

(VI)

wobei $R_5$ und $R_4$ gleich oder verschieden sein können.

**11.** Verbindung nach einem der Ansprüche 1 bis 6, **dadurch gekennzeichnet, dass** R ein Verzweigungselement umfasst, das von Tris(2-aminoethyl)amin (TREN) abgeleitet ist, wobei die Verbindung einer der folgenden Formeln entspricht:

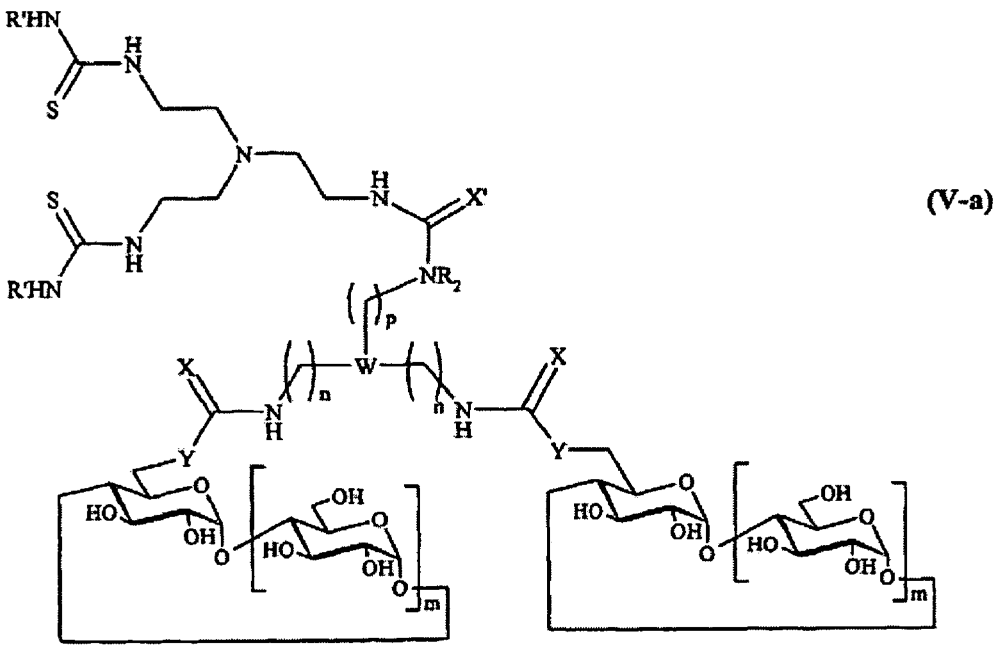

(V-a)

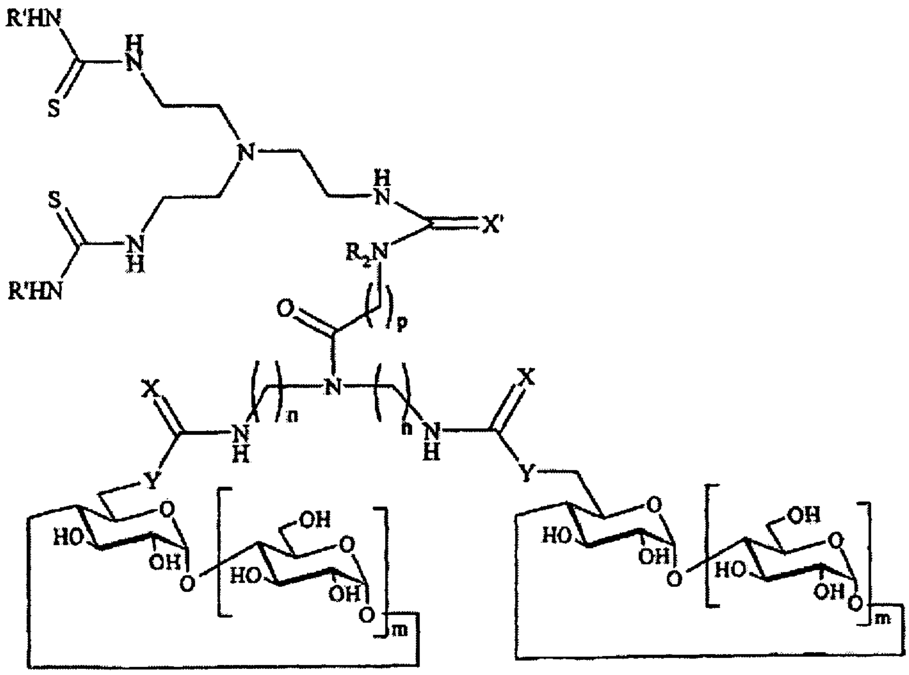

(V-b)

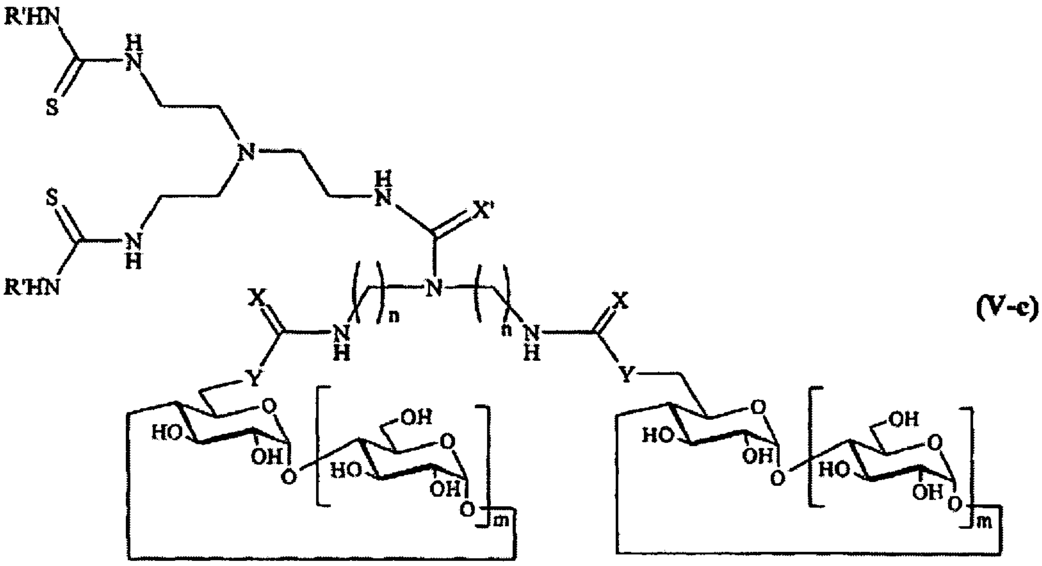

(V-c)

wobei m, n, p, X, X', Y so sind, wie in Anspruch 1 definiert, und R' die Definition aufweist, die zuvor für R angegeben wurde.

**12.** Verbindung nach Anspruch 11, **dadurch gekennzeichnet, dass** R' für

- die α-D-Mannopyranosylgruppe der Formel (III) oder
- die β-Lactosylgruppe der Formel (III-a) oder
- die Tris-α-D-mannopyranosyloxymethyl)methylgruppe der Formel (IV) oder
- die Tris(β-lactosyloxymethyl)methylgruppe der Formel (IV-a) steht.

**13.** Verbindung nach einem der Ansprüche 1 bis 12, **dadurch gekennzeichnet, dass** m gleich 6 ist.

**14.** Verfahren zur Herstellung einer Verbindung nach Anspruch 1, **dadurch gekennzeichnet, dass** es die folgenden Schritte umfasst:

- das Umsetzen einer Verbindung, die an der Primäralkoholposition selektiv mit einer Amingruppe funktionalisiert ist, mit der folgenden Formel (VII):

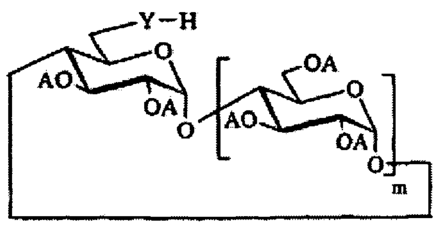

(VII)

wobei m, A und Y so sind, wie zuvor in Anspruch 1 definiert, und A bevorzugt ein Wasserstoffatom ist, mit einem Dimerisierungselement vom Typ Diisocyanat oder Diisothiocyanat, das insbesondere eine geschützte Aminfunktionalität in Form einer Carbamatgruppe trägt oder eine positiv geladene quartäre Ammoniumsalzfunktionalität trägt, mit der folgenden Formel (VIII):

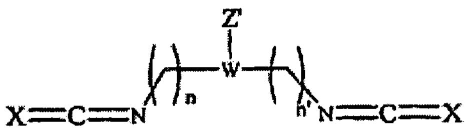

(VIII)

* wobei n und n' so sind, wie zuvor in Anspruch 1 definiert und bevorzugt gleich sind,
* W und X so sind, wie in Anspruch 1 definiert,
* Z' für eine Gruppe steht, die einer der folgenden Formeln entspricht:

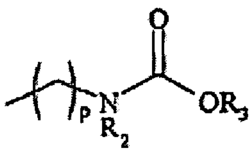 oder 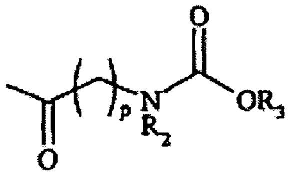 oder 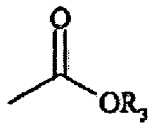

oder

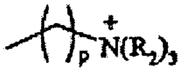 oder 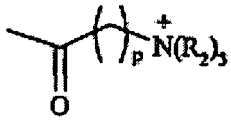 oder 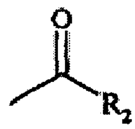

wobei p, $R_2$ und $R_3$ so sind, wie in Anspruch 1 definiert,
um eine Verbindung nach Anspruch 1 zu erhalten, die der folgenden Formel (IX) entspricht:

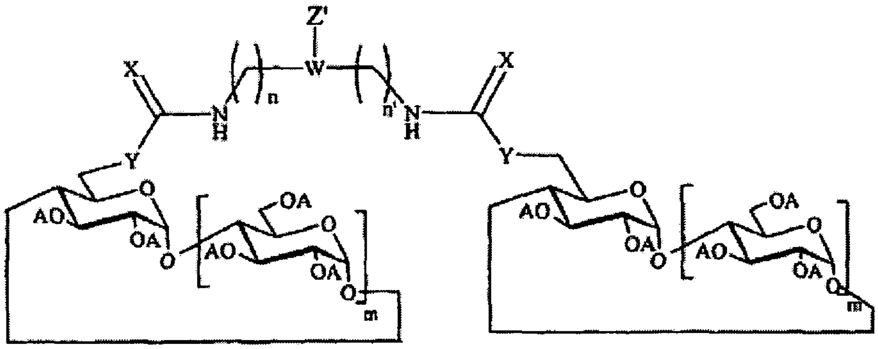

(IX)

und gegebenenfalls das Umsetzen durch Hydrolyse der Gruppe

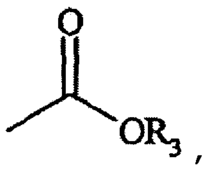

wie oben definiert, die in den Verbindungen der oben genannten Formel (IX) vorhanden ist, worin Z' eine

solche Gruppe enthält, um eine Verbindung zu erhalten, die eine freie Aminfunktionalität trägt und der folgenden Formel (X) entspricht:

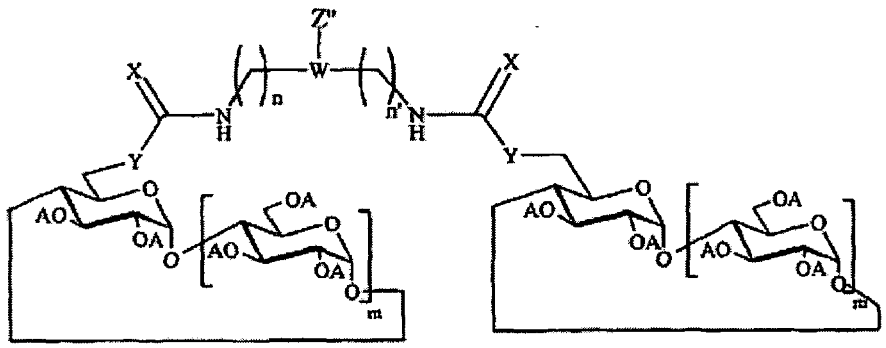

(X)

* wobei n, n' , A, X, Y, W und m so sind, wie zuvor definiert, und
* Z" mit dem Hydrolysat der Gruppe Z' übereinstimmt, das eine Funktion -$COOR_3$ enthält und für ein Wasserstoffatom steht oder einer der folgenden Formeln entspricht:

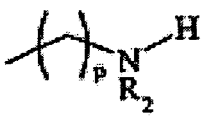

oder

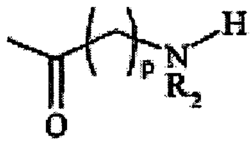

wobei p und $R_2$ so sind, wie in Anspruch 1 definiert,

- und gegebenenfalls das Umsetzen einer Verbindung der Formel (X), wie sie im vorhergehenden Schritt erhalten wurde, mit einem Isocyanat oder einem Isothiocyanat der folgenden Formel (XI):

R-N=C=X'

wobei R und X' so sind, wie in Anspruch 1 definiert,
um eine Verbindung nach Anspruch 1 zu erhalten, die der folgenden Formel (XII) entspricht:

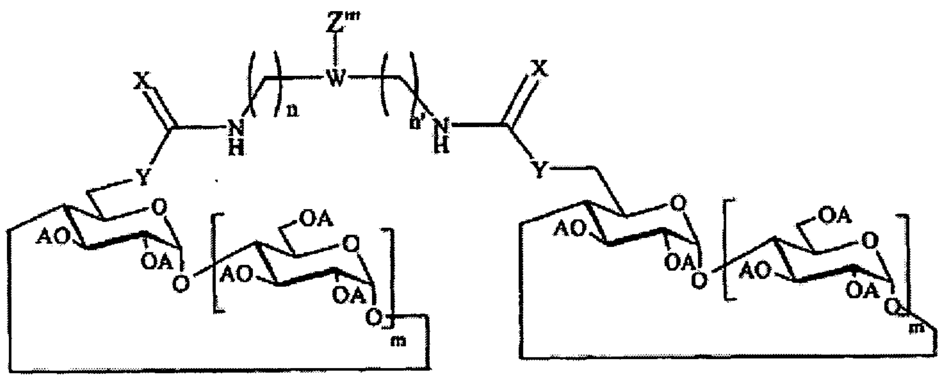

(XII)

* wobei n, n', A, X, Y, W und m so sind, wie zuvor definiert, und
* Z''' einer der folgenden Formeln entspricht:

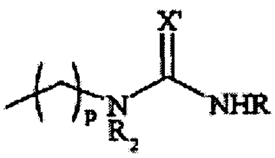 oder 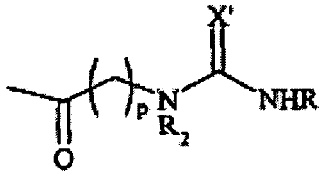 oder 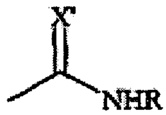

wobei p, $R_2$, X' und R so sind, wie in Anspruch 1 definiert.

**15.** Einschlusskomplex einer Verbindung nach einem der Ansprüche 1 bis 13 mit einem pharmakologisch aktiven Molekül, wobei das Molverhältnis zwischen der Verbindung nach einem der Ansprüche 1 bis 13 und dem pharmakologisch aktiven Molekül etwa 10:1 bis etwa 1:2 beträgt.

**16.** Komplex nach Anspruch 15, **dadurch gekennzeichnet, dass** das pharmakologisch aktive Molekül ein ditopes Molekül ist, das in der Lage ist, gleichzeitig mit zwei Cyclodextrin-Untereinheiten zu interagieren, wie etwa ein Molekül, das zwei aromatische Ringe, wie zum Beispiel ein Taxolderivat, oder eine ausreichend große Größe aufweist, wie zum Beispiel ein Steroid.

**17.** Komplex nach einem der Ansprüche 15 oder 16, **dadurch gekennzeichnet, dass** das pharmakologisch aktive Molekül ein antitumorales Mittel ist, das insbesondere zur Taxolfamilie gehört.

**18.** Pharmazeutische Zusammensetzung, die eine Verbindung nach einem der Ansprüche 1 bis 13 oder einen Einschlusskomplex nach einem der Ansprüche 15 bis 17 mit einem pharmakologisch verträglichen Vehikel umfasst.

**19.** Pharmazeutische Zusammensetzung nach Anspruch 18 in Form einer wässrigen Lösung.

**20.** Pharmazeutische Zusammensetzung nach einem der Ansprüche 18 oder 19, **dadurch gekennzeichnet, dass** sie als Dosiseinheit etwa 50 mg bis etwa 500 mg einer der Verbindungen nach einem der Ansprüche 1 bis 13 enthält, oder dadurch, dass sie als Dosiseinheit etwa 100 mg bis etwa 750 mg einer der Komplexe nach einem der Ansprüche 15 bis 17 enthält.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9519994 A
- WO 9521870 A
- WO 9733919 A
- EP 0403366 A
- EP 0605753 A
- WO 9002141 A
- WO 9961483 A


### Littérature non-brevet citée dans la description


- **P. Potier.** *Chem. Soc. Rev.,* 1992, vol. 21, 113-119
- **V. Lainé et al.** *J. Chem. Soc., Perkin Trans.,* 1995, vol. 2, 1479-1487
- **I. Baussanne et al.** *Chem. Commun,* 2000, 1489-1490
- **J. M. Benito et al.** *Chem. Eur. J.,* 2003
- **I. Baussanne et al.** *ChemBioChem,* 2001, 777-783
- **L. Jicsinszky.** Comprehensive Supramolecular Chemistry. Pergamon, 1996, vol. 3, 57-198
- *Chem. Commun,* 2000, 1489-1490
- **Jicsinszinsky et al.** *Carbohydr. Polym.,* vol. 2041 (45), 139-145
- **R. Richter ; H. Ulrich.** The Chemistry of Cyanates and Their Thio Derivatives. Wiley, 1977, vol. 2, 619-818
- **Y. Ichikawa.** *Synlett,* 2000, 1253-1256
- **J. M. García Fernández ; C. Ortiz Mellet.** *Adv. Carbohydr. Chem. Biochem.,* 1999, vol. 55, 35-135
- **P. R. Ashton et al.** *J. Org. Chem,* 1998, vol. 63, 3429-3437
- **D. A. Fulton ; J. F. Stoddart.** *Org. Lett.,* 2000, vol. 2, 1113-1116
- **X.-B. Meng et al.** *Carbohydr. Res.,* 2002, vol. 337, 977-981
- **J. Defaye ; J. Gelas.** Studies in Natural Products Chemistry. Elsevier, 1991, vol. 8, 315-357
- **H. Driguez.** *Top. Curr. Chem.,* 1997, vol. 187, 85-116
- *Synthesis,* 2002, 2195-2202
- **J. J. Lundquist ; E. J. Toon.** *Chem. Rev.,* 2002, vol. 102, 555-578
- *ChemBioChem,* 2001